# EUROPEAN PATENT APPLICATION

(11) **EP 3 970 752 A1**
(43) Date of publication of application: **23.03.2022**
(21) Application number: 20196705.6
(22) Date of filing: 17.09.2020
(51) Int. Cl.: A61K 47/61, A61K 47/68, A61P 35/00, C07K 16/32

(54) **MOLECULES WITH SOLUBILITY TAG AND RELATED METHODS**

(71) Applicant: Merck Patent GmbH, 64293 Darmstadt (DE)
(72) Inventor: The designation of the inventor has not yet been filed

(57) **Abstract**

The present disclosure relates to molecules comprising a targeting moiety, at least one payload wherein said at least one payload is a therapeutic agent or a detectable lable, a linker/linkers covalently linking said payload/payloads and said targeting moiety, and at least one solubility tag, wherein said solubility tag comprises at least 3 and up to 12 monosaccharide units.

SEQUENCE LISTING

<110> Merck Patent GmbH

<120> Molecules With Solubility Tag And Related Methods

<130> P20-059

<160> 2

<170> BiSSAP 1.3.6

<210> 1
<211> 220
<212> PRT
<213> Artificial Sequence

<220>
<223> Light chain of Trastuzumab-T

<400> 1
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Asn Thr Ala
            20                  25                  30
Val Ala Trp Tyr Gln Gln Lys Pro Gly Lys Ala Pro Lys Leu Leu Ile
        35                  40                  45
Tyr Ser Ala Ser Phe Leu Tyr Ser Gly Val Pro Ser Arg Phe Ser Gly
    50                  55                  60
Ser Arg Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Thr Thr Pro Pro
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Gly Leu Leu Gln Gly
    210                 215                 220

<210> 2
<211> 451
<212> PRT
<213> Artificial Sequence

<220>
<223> Heavy chain of Trastuzumab-T

<400> 2
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30
Tyr Ile His Trp Val Arg Gln Ala Pro Gly Lys Gly Leu Glu Trp Val
        35                  40                  45
Ala Arg Ile Tyr Pro Thr Asn Gly Tyr Thr Arg Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ser Arg Trp Gly Gly Asp Gly Phe Tyr Ala Met Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Ala Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

## Description

### FIELD OF THE INVENTION

The present disclosure relates to molecules with solubility tag and to methods for increasing the solubility of a molecule. Moreover, the present disclosure relates to antibody-drug conjugates with solubility tag, methods and compounds for preparing such antibody-drug conjugates, methods for increasing the solubility of antibody-drug conjugates, antibody-drug conjugates prepared by such methods, as well as the use of such antibody-drug conjugates in medical treatment.

### BACKGROUND OF THE INVENTION

Highly specialized chemical molecules are essential components in many important applications of modern-day life, for example in clinical diagnosis, in medical treatment or as research tools. Often, however, the options which molecules can be used for these applications are severely limited because insufficient solubility prevents the use of molecules that otherwise could be perfectly suited for a specific purpose. Thus, very frequently and in many fields of technology a need arises to provide molecules with increased solubility or to increase the solubility of specific molecules.

A typical example for this issue is antibody-drug conjugates (ADCs) in medical diagnosis and treatment. In recent years, ADCs have emerged as a new class of biotherapeutics that allow to combine the specificity of an antibody with the potency of a small molecule therapeutic.

Structurally, ADCs are cell targeting conjugates typically comprising three covalently linked main components: (i) an antibody component, (ii) a linker, and (iii) a medical drug ("payload"). Upon administration to a patient, the antibody component guides the ADC, via specific binding of the antibody component to its target antigen, to the target cells or the desired site within the body. Typically, the ADC is then internalized into the cell, e.g. by receptor-mediated endocytosis. Subsequently, the medical drug is released, for example by protease- or pH-dependent linker cleavage or by antibody degradation. The released medical drug will then fulfill its therapeutic function inside of the cell.

While non-targeted drugs typically reach their site of action by whole-body distribution and passive diffusion, ADCs are not distributed evenly across the whole body. Rather, due to the interaction of the antibody component with its target antigen, an ADC is concentrated mainly at its site of action. Consequently, ADCs require smaller dosages while still allowing the drug to reach therapeutically effective levels inside the target cells, thus improving the therapeutic window. The targeting by formation of an ADC is therefore a powerful method to enhance specificity and decrease systemic toxicity of a medical drug, and to allow for the therapeutic use of medical drugs that are less suitable or even unsuitable as systemic drugs.

Suitability of ADC for clinical use depends on multiple factors, such as the selection of an appropriate target antigen and antibody component, linker design and stability, and the use of a drug payload with appropriate characteristics and potency.

A further critical factor is the overall solubility of the ADC. Common drug payloads, such as camptothecin and duocarmycin, are often hydrophobic and significantly reduce the solubility of the ADC molecule. Therefore, newly developed ADCs often have solubility issues and are prone to aggregation and particle formation. This, in turn, means that only ADCs with low drug-to-antibody ratio (DAR) can be synthesized, while for larger or more hydrophobic payloads synthesis of an ADC is not possible at all. Even in cases where synthesis is possible, reduced solubility and predisposition to aggregation still complicates analytical characterization of the ADC, hinders efficient manufacturing with high reproducibility, and reduces long term stability. In clinical applications, the low solubility of ADCs often leads to reduced efficacy, significant side effects and a small therapeutic window, which may be inacceptable for reasons of patient safety.

In view of these issues, lack of solubility is a significant risk in the development of new ADCs that drives costs and often leads to project termination.

To some degree the solubility of an ADC can be improved by selection of more hydrophilic linkers or payloads. However, such compromises limit the options for ADC design and do not allow to modulate and fine-tune the drug-to-antibody ratio, which would be required for ADC optimization. Thus, this approach often leads to ADCs with inferior therapeutic characteristics.

Alternatively, the incorporation of PEG chains of different length in the linker is a common scheme to address solubility issues in the ADC landscape. (see e.g. Simmons et al., Toxicology and Applied Pharmacology (2020), vol. 392, p. 114932; Tiberghien, Organic Process Research & Development (2018), vol. 22(9), p. 1241-1256). However, administration of ADCs with PEG chains often leads to the development of anti-PEG antibodies. This obstructs the positive effect of the PEG chain on solubility and often leads to an immune response against the ADC (Thi et al., Polymers (2020), vol. 12(2), p. 298; Hong, Journal of Pharmacological and Toxicological Methods (2020), vol. 102, p. 106678).

Accordingly, there is a need in the art for improved ways to increase the solubility of ADCs. Moreover, there is a need in the art for improved ways to reduce/prevent aggregation of ADCs. Moreover, there is a need in the art for improved ways to increase the drug-to-antibody ratio (DAR) of ADCs. Moreover, there is a need in the art for improved ADCs, in particular for improved ADCs that have a longer serum half-life, improved pharmacokinetics, higher efficacy, less side effects, reduced toxicity, reduced immunogenicity, a larger therapeutic window and/or improved patient safety. Moreover, there is a need in the art for improved ways to achieve optimized clinical characteristics of an ADC (e.g. pharmacokinetics, efficacy, side effects, therapeutic window, patient safety). Moreover, there is a need in the art for approaches that allow to form ADCs from payloads that otherwise are too hydrophobic for ADC generation. Moreover, there is a need in the art for approaches that allow to form ADCs with multiple hydrophobic payloads that otherwise cannot be prepared due to issues of solubility and/or aggregation. Moreover, there is a need in the art for ways to address the above-described needs by a "standardized" approach that can be widely used for different ADCs. Moreover, there is a need in the art for ways to address the above-described needs by a modular approach that can be flexibly adapted to different ADCs and the hydrophobicity of specific payloads, and that allows to modulate and fine-tune the drug-to-antibody ratio. Moreover, there is a need in the art for ways to address the above-described needs by an approach that allows for fast synthetic access.

Similar needs as sketched out above also exist for other types of molecules.

The present disclosure overcomes the above-described problems and addresses the above-described needs.

### SUMMARY OF THE INVENTION

The present disclosure addresses the needs described above in the section "Background of the Invention" by the different aspects and embodiments described below.

The present invention is, in part, based on the surprising observation that covalent attachment of an oligosaccharide-based solubility tag as described in the present disclosure to a molecule, such as an antibody-drug conjugate, results in various advantageous effects on the molecule. For example, as shown for ADCs, advantageous effects can include (but are not limited to) an increase in solubility, reduced aggregation, availability of molecules that otherwise cannot be obtained due to solubility issues, accessibility of higher drug-to-antibody ratios and improved clinical characteristics. Moreover, it was found that this approach is not limited to a specific structure, but can be more widely used, and can be applied as a modular approach, allowing for example for flexible adaption to a specific ADC.

In one aspect, the present disclosure relates to a molecule comprising
- a targeting moiety and
- at least one solubility tag.

In another aspect, the present disclosure relates to a molecule comprising
- a targeting moiety,
- at least one functional moiety, and
- at least one solubility tag.

In another aspect, the present disclosure relates to a molecule comprising
- a targeting moiety,
- at least one functional moiety,
- a linker/linkers covalently linking said functional moiety/moieties and said targeting moiety, and
- at least one solubility tag.

In some embodiments, said at least one functional moiety is a payload that is a therapeutic agent or a detectable label. In some embodiments, said solubility tag comprises at least 3 and up to 12 monosaccharide units.

In some embodiments, said solubility tag is/said solubility tags are linked by a covalent bond to said at least one payload and/or to said linker(s).

In some embodiments, said monosaccharide units are individually selected from the group consisting of aldoses, ketoses and chemically modified forms of said aldoses or ketoses. In some embodiments, said monosaccharide units are individually selected from the group consisting of tetroses, pentoses, hexoses, and chemically modified forms of tetroses, pentoses and hexoses, wherein said tetroses are individually selected from the group consisting of erythrose and threose, said pentoses are individually selected from the group consisting of ribose, arabinose, xylose and lyxose, and said hexoses are individually selected from the group consisting of allose, altrose, glucose, mannose, gulose, idose, galactose and talose.

In some embodiments, the solubility tag comprises or consists of a chito-oligosaccharide.

In some embodiments, said chito-oligosaccharide is a chito-oligosaccharide with 3 to 7 monosaccharide units selected from Table 1 below.

In some embodiments, said solubility tag comprises or is a chemical group with a structural formula selected from the group consisting of structural formulas (I) to (IV) defined below.

In some embodiments, said targeting moiety is selected from the group consisting of a protein, a peptide, a peptide mimetic, a nucleic acid, an oligonucleotide and a small molecule.

In some embodiments, said antibody is an antibody against an antigen present on the surface of a target cell or an antigen-binding fragment of such an antibody.

In some embodiments, said targeting moiety specifically binds to a tumor antigen.

In some embodiments, said therapeutic agent is a cytotoxic agent, anti-inflammatory agent, immunostimulatory agent or immunosuppressive agent.

In some embodiments, said linker/each of said linkers has a molecular weight of up to 1,500 Da.

In another aspect, the present disclosure relates to a molecule consisting of
- a targeting moiety and
- at least one solubility tag.

In another aspect, the present disclosure relates to a molecule consisting of
- a targeting moiety,
- at least one functional moiety, and
- at least one solubility tag.

In another aspect, the present disclosure relates to a molecule consisting of
- a targeting moiety,
- at least one functional moiety,
- a linker/linkers covalently linking said functional moiety/moieties and said targeting moiety, and
- at least one solubility tag.

In another aspect, the present disclosure relates to a method for increasing the solubility of a molecule, said molecule comprising a targeting moiety, wherein said method comprises covalently linking at least one solubility tag to said molecule.

In another aspect, the present disclosure relates to a method for increasing the solubility of a molecule, said molecule comprising
- a targeting moiety and
- at least one functional moiety,
wherein said method comprises covalently linking at least one solubility tag to said molecule.

In another aspect, the present disclosure relates to a method for increasing the solubility of a molecule, said molecule comprising
- a targeting moiety,
- at least one functional moiety, and
- a linker/linkers covalently linking said functional moiety/moieties and said targeting moiety wherein said method comprises covalently linking at least one solubility tag to said molecule.

In another aspect, the present disclosure relates to a method for increasing the solubility of a molecule, said molecule consisting of a targeting moiety, wherein said method comprises covalently linking at least one solubility tag to said molecule.

In another aspect, the present disclosure relates to a method for increasing the solubility of a molecule, said molecule consisting of
- a targeting moiety and
- at least one functional moiety,
   wherein said method comprises covalently linking at least one solubility tag to said molecule.

In another aspect, the present disclosure relates to a method for increasing the solubility of a molecule, said molecule consisting of
- a targeting moiety,
- at least one functional moiety, and
- a linker/linkers covalently linking said functional moiety/moieties and said targeting moiety, wherein said method comprises covalently linking at least one solubility tag to said molecule.

In another aspect, the present disclosure relates to a method for increasing the solubility of a chemical compound, said chemical compound comprising a targeting moiety, wherein said method comprises the preparation of a molecule in which said chemical compound is covalently linked to at least one solubility tag.

In another aspect, the present disclosure relates to a method for increasing the solubility of a chemical compound, said chemical compound comprising
- a targeting moiety and
- at least one functional moiety,
   wherein said method comprises the preparation of a molecule in which said chemical compound is covalently linked to at least one solubility tag.

In another aspect, the present disclosure relates to a method for increasing the solubility of a chemical compound, said chemical compound comprising
- a targeting moiety,
- at least one functional moiety, and
- a linker/linkers covalently linking said functional moiety/moieties and said targeting moiety, wherein said method comprises the preparation of a molecule in which said chemical compound is covalently linked to at least one solubility tag.

In some embodiments, said at least one functional moiety is a payload that is a therapeutic agent or a detectable label. In some embodiments, said solubility tag comprises at least 3 and up to 12 monosaccharide units.

In another aspect, the present disclosure relates to a method for increasing the solubility of a chemical compound, said chemical compound consisting of a targeting moiety, wherein said method comprises the preparation of a molecule in which said chemical compound is covalently linked to at least one solubility tag.

In another aspect, the present disclosure relates to a method for increasing the solubility of a chemical compound, said chemical compound consisting of
- a targeting moiety and
- at least one functional moiety,
   wherein said method comprises the preparation of a molecule in which said chemical compound is covalently linked to at least one solubility tag.

In another aspect, the present disclosure relates to a method for increasing the solubility of a chemical compound, said chemical compound consisting of
- a targeting moiety,
- at least one functional moiety, and
- a linker/linkers covalently linking said functional moiety/moieties and said targeting moiety, wherein said method comprises the preparation of a molecule in which said chemical compound is covalently linked to at least one solubility tag.

In another aspect, the present disclosure relates to the use of a solubility tag for enhancing the solubility of a molecule, said molecule comprising a targeting moiety.

In another aspect, the present disclosure relates to the use of a solubility tag for enhancing the solubility of a molecule, said molecule comprising
- a targeting moiety and
- at least one functional moiety.

In another aspect, the present disclosure relates to the use of a solubility tag for enhancing the solubility of a molecule, said molecule comprising
- a targeting moiety,
- at least one functional moiety, and
- a linker/linkers covalently linking said functional moiety/moieties and said targeting moiety.

In another aspect, the present disclosure relates to the use of a solubility tag for enhancing the solubility of a molecule, said molecule consisting of a targeting moiety.

In another aspect, the present disclosure relates to the use of a solubility tag for enhancing the solubility of a molecule, said molecule consisting of
- a targeting moiety and
- at least one functional moiety.

In another aspect, the present disclosure relates to the use of a solubility tag for enhancing the solubility of a molecule, said molecule consisting of
- a targeting moiety,
- at least one functional moiety, and
- a linker/linkers covalently linking said functional moiety/moieties and said targeting moiety.

In one aspect, the present disclosure relates to an antibody-drug conjugate comprising
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

In another aspect, the present disclosure relates to an antibody-drug conjugate consisting of
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

In another aspect, the present disclosure relates to a method for increasing the solubility of an antibody-drug conjugate, said antibody-drug conjugate comprising
(i) an antibody component,
(ii) at least one payload wherein said at least one payload is a therapeutic agent or a detectable label, and
(iii) a linker/linkers covalently linking said payload/payloads and said antibody component, wherein said method comprises covalently linking at least one solubility tag to said antibody-drug conjugate.

In another aspect, the present disclosure relates to a method for increasing the solubility of an antibody-drug conjugate, said antibody-drug conjugate consisting of
(i) an antibody component,
(ii) at least one payload wherein said at least one payload is a therapeutic agent or a detectable label, and
(iii) a linker/linkers covalently linking said payload/payloads and said antibody component, wherein said method comprises covalently linking at least one solubility tag to said antibody-drug conjugate.

In another aspect, the present disclosure relates to a method for increasing the solubility of an antibody-drug conjugate, said antibody-drug conjugate comprising
(i) an antibody component,
(ii) at least one payload wherein said at least one payload is a therapeutic agent or a detectable label, and
(iii) a linker/linkers covalently linking said payload/payloads and said antibody component, wherein said method comprises the preparation of said antibody-drug conjugate in a form in which said antibody-drug conjugate is covalently linked to at least one solubility tag.

In another aspect, the present disclosure relates to a method for increasing the solubility of an antibody-drug conjugate, said antibody-drug conjugate consisting of
(i) an antibody component,
(ii) at least one payload wherein said at least one payload is a therapeutic agent or a detectable label, and
(iii) a linker/linkers covalently linking said payload/payloads and said antibody component, wherein said method comprises the preparation of said antibody-drug conjugate in a form in which said antibody-drug conjugate is covalently linked to at least one solubility tag.

In another aspect, the present disclosure relates to a method for increasing the solubility of a chemical compound comprising
(i) an antibody component,
(ii) at least one payload wherein said at least one payload is a therapeutic agent or a detectable label, and
(iii) a linker/linkers covalently linking said payload/payloads and said antibody component, wherein said method comprises the preparation of a molecule in which said chemical compound is covalently linked to at least one solubility tag,
   wherein said molecule is an antibody-drug conjugate.

In another aspect, the present disclosure relates to a method for increasing the solubility of a chemical compound consisting of
(i) an antibody component,
(ii) at least one payload wherein said at least one payload is a therapeutic agent or a detectable label, and
(iii) a linker/linkers covalently linking said payload/payloads and said antibody component, wherein said method comprises the preparation of a molecule in which said chemical compound is covalently linked to at least one solubility tag,
   wherein said molecule is an antibody-drug conjugate.

In another aspect, the present disclosure relates to a method for increasing the solubility of a molecule comprising
(i) an antibody component,
(ii) at least one payload wherein said at least one payload is a therapeutic agent or a detectable label, and
(iii) a linker/linkers covalently linking said payload/payloads and said antibody component, wherein said method comprises the preparation of said molecule in a form in which said molecule is covalently linked to at least one solubility tag, thus resulting in an antibody-drug conjugate comprising
   (i) an antibody component,
   (ii) at least one payload wherein said at least one payload is a therapeutic agent or a detectable label,
   (iii) a linker/linkers covalently linking said payload/payloads and said antibody component, and
   (iv) at least one solubility tag.

In another aspect, the present disclosure relates to a method for increasing the solubility of a molecule consisting of
(i) an antibody component,
(ii) at least one payload wherein said at least one payload is a therapeutic agent or a detectable label, and
(iii) a linker/linkers covalently linking said payload/payloads and said antibody component, wherein said method comprises the preparation of said molecule in a form in which said molecule is covalently linked to at least one solubility tag, thus resulting in an antibody-drug conjugate consisting of
   (i) an antibody component,
   (ii) at least one payload wherein said at least one payload is a therapeutic agent or a detectable label,
   (iii) a linker/linkers covalently linking said payload/payloads and said antibody component, and
   (iv) at least one solubility tag.

In another aspect, the present disclosure relates to the use of a solubility tag for enhancing the solubility of an antibody-drug conjugate.

In some embodiments, said use involves the step of covalently linking said solubility tag to said antibody-drug conjugate.

In some embodiments of said use, said antibody-drug conjugate comprises
(i) an antibody component,
(ii) at least one payload wherein said at least one payload is a therapeutic agent or a detectable label, and
(iii) a linker/linkers covalently linking said payload/payloads and said antibody component.

In some embodiments of said use, said antibody-drug conjugate consists of
( i) an antibody component,
( ii) at least one payload wherein said at least one payload is a therapeutic agent or a detectable label, and
( iii) a linker/linkers covalently linking said payload/payloads and said antibody component.

In another aspect, the present disclosure relates to a method for preparing an antibody-drug conjugate as defined in the present disclosure, said method comprising the step of
- carrying out a reaction resulting in the formation of a covalent bond between (a) a molecule comprising an antibody component as defined in the present disclosure, a payload as defined in the present disclosure and a linker as defined in the present disclosure and (b) a solubility tag as defined in the present disclosure, or
- carrying out a reaction resulting in the formation of a covalent bond between (a) an antibody component as defined in the present disclosure and (b) a molecule comprising a payload as defined in the present disclosure, a linker as defined in the present disclosure and a solubility tag as defined in the present disclosure, or
- carrying out a reaction resulting in the formation of a covalent bond between (a) a molecule comprising an antibody component as defined in the present disclosure, a linker as defined in the present disclosure and a solubility tag as defined in the present disclosure and (b) a payload as defined in the present disclosure, or
- carrying out a reaction resulting in the formation of a covalent bond between (a) a molecule comprising an antibody component as defined in the present disclosure and a linker as defined in the present disclosure and (b) a molecule comprising a solubility tag as defined in the present disclosure and a payload as defined in the present disclosure,
   to yield an antibody-drug conjugate with a covalently linked solubility tag.

In another aspect, the present disclosure relates to a compound for use in the preparation of an antibody-drug conjugate according to the present disclosure, wherein said compound comprises a solubility tag as defined in the present disclosure linked to an activator group.

In another aspect, the present disclosure relates to an antibody-drug conjugate that has been prepared by a method according to the present disclosure.

In another aspect, the present disclosure relates to a pharmaceutical composition comprising the antibody-drug conjugate of the present disclosure or an antibody-drug conjugate prepared by the method according to the present disclosure.

In some embodiments, said pharmaceutical composition comprises a pharmaceutically acceptable carrier, diluent and/or excipient.

In another aspect, the present disclosure relates to an antibody-drug conjugate according to the present disclosure or a pharmaceutical composition according to the present disclosure for use as a medicament or for use in the treatment of a disease as defined below.

In another aspect, the present disclosure relates to a method for treating a disease in a patient in need thereof, comprising the step of administering to said patient a therapeutically effective amount of the antibody-drug conjugate of the present disclosure or the pharmaceutical composition of the present disclosure.

In another aspect, the present disclosure relates to the use of the antibody-drug conjugate of the present disclosure or of the pharmaceutical composition of the present disclosure for the manufacture of a medicament, preferably for the manufacture of a medicament for the treatment of a disease or disorder as defined below.

### BRIEF DESCRIPTION OF THE FIGURES

In the following, reference is made to the figures. All methods referred to in the figure descriptions below were carried out as described in detail in the examples.

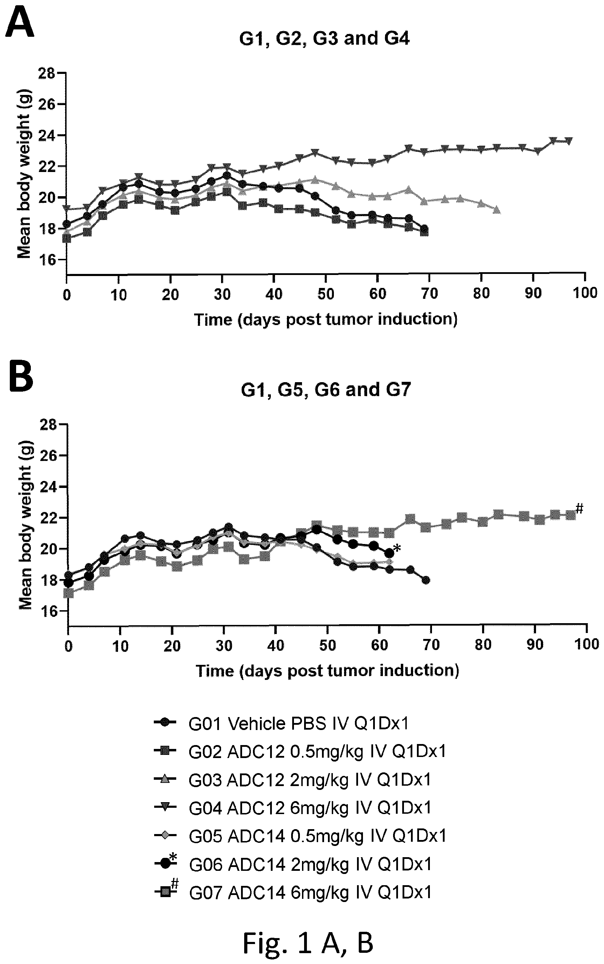

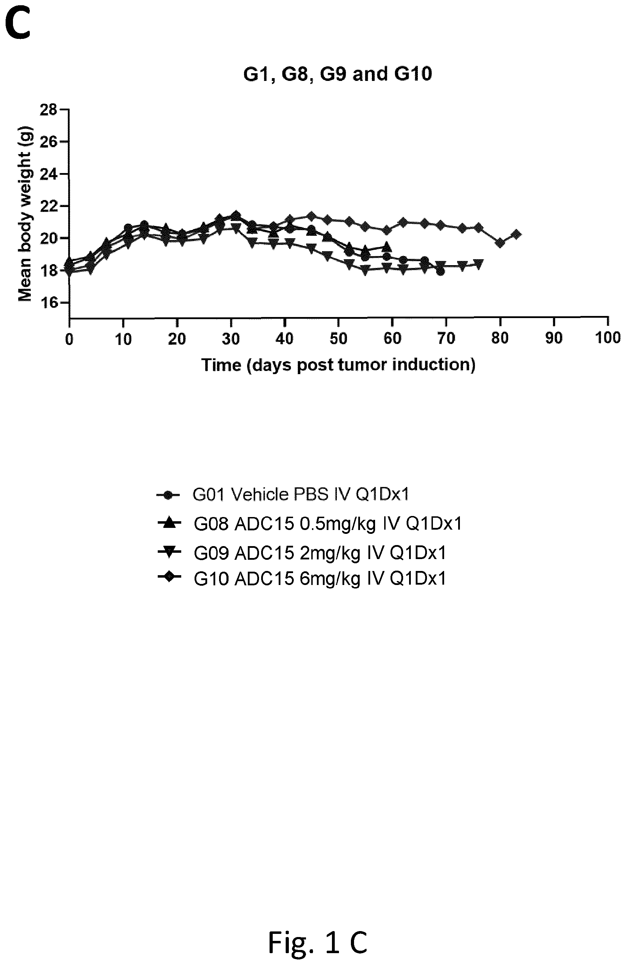

**Figure 1** shows mean body weight curves of BALB/c Nude mice bearing subcutaneous SK-OV-3 tumors obtained in Example 8. Each point represents the mean of the recorded body weight per group. Animals were randomized and treated on D31. D97 was the last day of the study. Values are shown for all time points where at least 80% of the animals of the respective group were still present. "Q1Dx1" indicates that treatment occurred in one single dose (which was administered on D31).

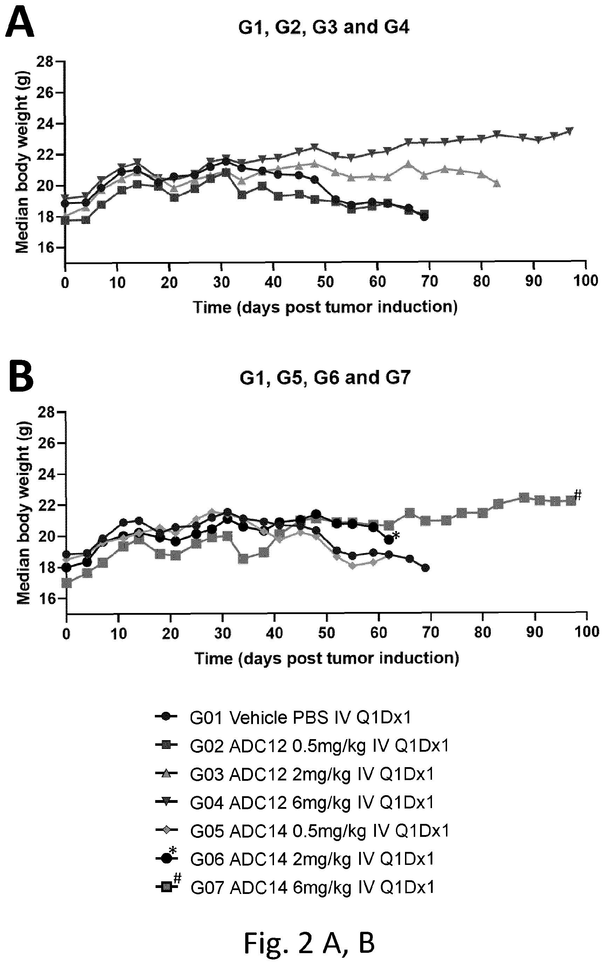

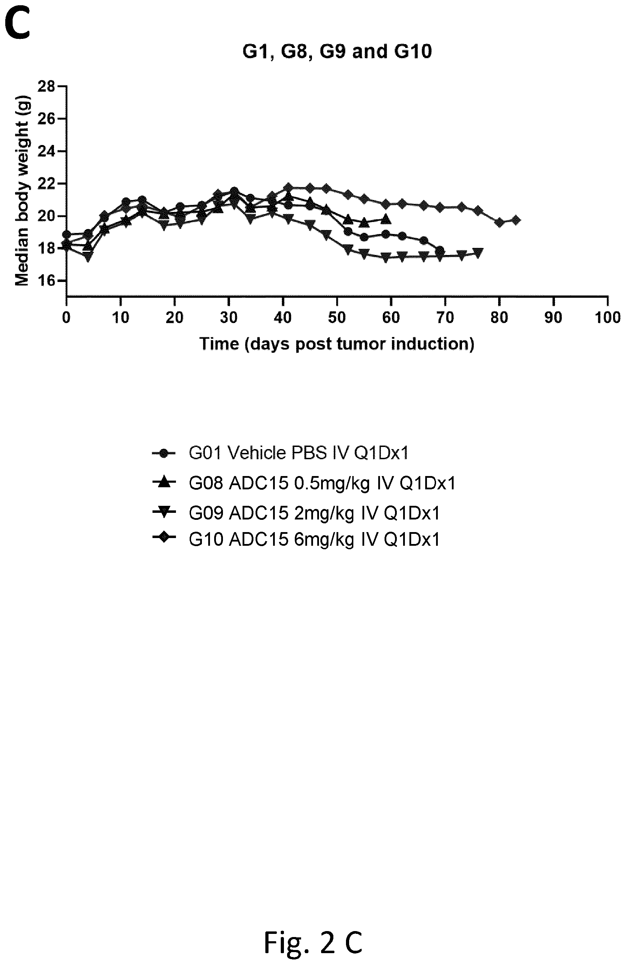

**Figure 2** shows median body weight curves of BALB/c Nude mice bearing subcutaneous SK-OV-3 tumors obtained in Example 8. Each point represents the median of the recorded body weight per group. Values are shown for all time points where at least 80% of the animals of the respective group were still present.
**Figure 3** shows mean tumor volume curves of BALB/c Nude mice bearing subcutaneous SK-OV-3 tumors obtained in Example 8. Each point represents the mean of the recorded tumor volume per group. Values are shown for all time points where at least 80% of the animals of the respective group were still present.
**Figure 4** shows median tumor volume curves of BALB/c Nude mice bearing subcutaneous SK-OV-3 tumors obtained in Example 8. Values are shown for all time points where at least 80% of the animals of the respective group were still present.
**Figure 5** shows a summary of the tumor volume of BALB/c Nude mice bearing subcutaneous SK-OV-3 tumors on D59 in Example 8.
**Figure 6** shows tumor growth inhibition (T/C%) in treated BALB/c Nude mice bearing subcutaneous SK-OV-3 tumors in Example 8. Group 1 was used as the control. No further calculations were possible after D83 since there were less than four mice remaining in the control group.

### DETAILED DESCRIPTION OF CERTAIN EMBODIMENTS

Although the present disclosure is described in detail above and below, it is to be understood that this disclosure is not limited to the particular methodologies, protocols and reagents described by the present disclosure, as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

In the following, certain elements of the present disclosure will be described in more detail, including the description of specific embodiments. However, the variously described examples and preferred embodiments should not be construed to limit the present disclosure to only the explicitly described embodiments. This description should be understood to support and encompass embodiments which combine the explicitly described embodiments with any number of the disclosed and/or preferred elements and in any manner. Furthermore, any permutations and combinations of all described elements in this application should be considered disclosed by the description of the present application except for where this leads to logical contradictions or the context indicates otherwise.

Unless defined otherwise herein, scientific and technical terms used in connection with the present disclosure shall have the meanings that are commonly understood by those of ordinary skill in the art. Generally, nomenclatures and techniques referred to in the present disclosure, e.g. nomenclatures and techniques of organic chemistry, chemical synthesis, biology, medicinal and pharmaceutical chemistry, medicine, pharmacology or toxicology, are those well-known and commonly used in the art. The methods and techniques of the present disclosure are generally performed according to conventional methods well-known in the art and as described in the references cited and discussed throughout the present disclosure unless otherwise indicated.

According to one aspect, the present disclosure relates to a molecule comprising
- a targeting moiety and
- at least one solubility tag.

In another aspect, the present disclosure relates to a molecule comprising
- a targeting moiety,
- at least one functional moiety, and
- at least one solubility tag.

In another aspect, the present disclosure relates to a molecule comprising
- a targeting moiety,
- at least one functional moiety,
- a linker/linkers covalently linking said functional moiety/moieties and said targeting moiety, and
- at least one solubility tag.

In another aspect, the present disclosure relates to a molecule consisting of
- a targeting moiety and
- at least one solubility tag.

In another aspect, the present disclosure relates to a molecule consisting of
- a targeting moiety,
- at least one functional moiety, and
- at least one solubility tag.

In another aspect, the present disclosure relates to a molecule consisting of
- a targeting moiety,
- at least one functional moiety,
- a linker/linkers covalently linking said functional moiety/moieties and said targeting moiety, and
- at least one solubility tag.

In another aspect, the present disclosure relates to a method for increasing the solubility of a molecule, said molecule comprising a targeting moiety, wherein said method comprises covalently linking at least one solubility tag to said molecule.

In another aspect, the present disclosure relates to a method for increasing the solubility of a molecule, said molecule comprising
- a targeting moiety and
- at least one functional moiety,
   wherein said method comprises covalently linking at least one solubility tag to said molecule.

In another aspect, the present disclosure relates to a method for increasing the solubility of a molecule, said molecule comprising
- a targeting moiety,
- at least one functional moiety, and
- a linker/linkers covalently linking said functional moiety/moieties and said targeting moiety,
   wherein said method comprises covalently linking at least one solubility tag to said molecule.

In another aspect, the present disclosure relates to a method for increasing the solubility of a molecule, said molecule consisting of a targeting moiety, wherein said method comprises covalently linking at least one solubility tag to said molecule.

In another aspect, the present disclosure relates to a method for increasing the solubility of a molecule, said molecule consisting of
- a targeting moiety and
- at least one functional moiety,
   wherein said method comprises covalently linking at least one solubility tag to said molecule.

In another aspect, the present disclosure relates to a method for increasing the solubility of a molecule, said molecule consisting of
- a targeting moiety,
- at least one functional moiety, and
- a linker/linkers covalently linking said functional moiety/moieties and said targeting moiety, wherein said method comprises covalently linking at least one solubility tag to said molecule.

In another aspect, the present disclosure relates to a method for increasing the solubility of a chemical compound, said chemical compound comprising a targeting moiety, wherein said method comprises the preparation of a molecule in which said chemical compound is covalently linked to at least one solubility tag.

As a skilled person understands, this "molecule in which said chemical compound is covalently linked to at least one solubility tag" is the molecule referred to in the first six aspects mentioned in the section "Antibody-drug conjugates" and can be characterized by all the other features disclosed in this application with regard to any of these aspects.

In another aspect, the present disclosure relates to a method for increasing the solubility of a chemical compound, said chemical compound comprising
- a targeting moiety and
- at least one functional moiety,
   wherein said method comprises the preparation of a molecule in which said chemical compound is covalently linked to at least one solubility tag.

In another aspect, the present disclosure relates to a method for increasing the solubility of a chemical compound, said chemical compound comprising
- a targeting moiety,
- at least one functional moiety, and
- a linker/linkers covalently linking said functional moiety/moieties and said targeting moiety, wherein said method comprises the preparation of a molecule in which said chemical compound is covalently linked to at least one solubility tag.

In another aspect, the present disclosure relates to a method for increasing the solubility of a chemical compound, said chemical compound consisting of a targeting moiety, wherein said method comprises the preparation of a molecule in which said chemical compound is covalently linked to at least one solubility tag.

In another aspect, the present disclosure relates to a method for increasing the solubility of a chemical compound, said chemical compound consisting of
- a targeting moiety and
- at least one functional moiety,
   wherein said method comprises the preparation of a molecule in which said chemical compound is covalently linked to at least one solubility tag.

In another aspect, the present disclosure relates to a method for increasing the solubility of a chemical compound, said chemical compound consisting of
- a targeting moiety,
- at least one functional moiety, and
- a linker/linkers covalently linking said functional moiety/moieties and said targeting moiety, wherein said method comprises the preparation of a molecule in which said chemical compound is covalently linked to at least one solubility tag.

In some embodiments of any of the methods described above, all components of said chemical compound are covalently linked.

In another aspect, the present disclosure relates to the use of a solubility tag for enhancing the solubility of a molecule, said molecule comprising a targeting moiety.

In another aspect, the present disclosure relates to the use of a solubility tag for enhancing the solubility of a molecule, said molecule comprising
- a targeting moiety and
- at least one functional moiety.

In another aspect, the present disclosure relates to the use of a solubility tag for enhancing the solubility of a molecule, said molecule comprising
- a targeting moiety,
- at least one functional moiety, and
- a linker/linkers covalently linking said functional moiety/moieties and said targeting moiety.

In another aspect, the present disclosure relates to the use of a solubility tag for enhancing the solubility of a molecule, said molecule consisting of a targeting moiety.

In another aspect, the present disclosure relates to the use of a solubility tag for enhancing the solubility of a molecule, said molecule consisting of
- a targeting moiety and
- at least one functional moiety.

In another aspect, the present disclosure relates to the use of a solubility tag for enhancing the solubility of a molecule, said molecule consisting of
- a targeting moiety,
- at least one functional moiety, and
- a linker/linkers covalently linking said functional moiety/moieties and said targeting moiety.

In some embodiments, the use involves the step of covalently linking at least one solubility tag to said molecule.

The following embodiments relate to any of the molecules, methods or uses defined above.

In some embodiments, all components of said molecule are covalently linked.

In some embodiments, said targeting moiety is a molecular group that specifically binds to a target molecule or fragment thereof.

In some embodiments, said target molecule is a biomolecule.

In some embodiments, said target molecule is a receptor at the surface of a cell.

In some embodiments, said target molecule is an antigen that is present on the surface of a target cell.

In some embodiments, said targeting moiety is capable of specifically binding to an antigen that is present on the surface of a target cell.

In some embodiments, said targeting moiety comprises a protein, a peptide, a peptide mimetic, a nucleic acid, an oligonucleotide or a small molecule.

In some embodiments, said targeting moiety is selected from the group consisting of a protein, a peptide, a peptide mimetic, a nucleic acid, an oligonucleotide and a small molecule.

In some embodiments, said targeting moiety comprises a protein.

In some embodiments, said targeting moiety is a protein.

In some embodiments, said targeting moiety comprises or is a protein which is a protein ligand that specifically binds to a receptor at the surface of a cell.

In some embodiments, said targeting moiety comprises or is a protein which is an antibody or an antigen-binding fragment thereof.

In some embodiments, said targeting moiety comprises or is a protein which is an antibody component.

In some embodiments, said targeting moiety comprises or is a protein which comprises at least 30 amino acids.

In some embodiments, said targeting moiety comprises or is a peptide which consists of 2 to 30 amino acids.

In some embodiments, said targeting moiety comprises a peptide.

In some embodiments, said targeting moiety is a peptide.

In some embodiments, said targeting moiety comprises a peptide mimetic.

In some embodiments, said targeting moiety is a peptide mimetic.

In some embodiments, said targeting moiety comprises or is a nucleic acid which is a DNA or an RNA.

In some embodiments, said targeting moiety comprises a nucleic acid.

In some embodiments, said targeting moiety is a nucleic acid.

In some embodiments, said targeting moiety comprises an oligonucleotide.

In some embodiments, said targeting moiety is an oligonucleotide.

In some embodiments, said targeting moiety comprises or is a small molecule with a molecular weight < 1000 Da.

In some embodiments, said targeting moiety comprises a small molecule.

In some embodiments, said targeting moiety is a small molecule.

In some embodiments, said targeting moiety is not a sugar.

In some embodiments, said targeting moiety does not comprise a sugar.

In some embodiments, said targeting moiety has a molecular weight of at least 100 Da.

In some embodiments, said targeting moiety has a molecular weight of at least 500 Da.

In some embodiments, said targeting moiety has a molecular weight of at least 1 000 Da.

In some embodiments, said targeting moiety has a molecular weight of at least 2 000 Da.

In some embodiments, said targeting moiety has a molecular weight of at least 10 kDa.

In some embodiments, said targeting moiety has a molecular weight of at least 50 kDa.

In some embodiments, said targeting moiety has a molecular weight of at least 100 kDa.

In some embodiments, said targeting moiety has a molecular weight of up to 1 000 Da.

In some embodiments, said targeting moiety has a molecular weight of up to 2 000 Da.

In some embodiments, said targeting moiety has a molecular weight of up to 10 kDa.

In some embodiments, said targeting moiety has a molecular weight of up to 50 kDa.

In some embodiments, said targeting moiety has a molecular weight of up to 200 kDa.

In some embodiments, said targeting moiety has a molecular weight of up to 1 MDa.

In some embodiments, said targeting moiety has a molecular weight of up to 5 MDa.

In some embodiments, said targeting moiety has a molecular weight of up to 10 MDa.

In some embodiments, said at least one functional moiety is a chemical entity which is capable of fulfilling a biological, chemical, therapeutic and/or diagnostic function in the human body.

As the skilled person understands, the term "chemical entity" includes any type of chemical group or molecule of any substance class and is only limited by the recited requirement that it must be capable of fulfilling (in the context of the molecule according to the present disclosure) a biological, chemical, therapeutic and/or diagnostic function in the human body.

In some embodiments, said at least one functional moiety is a therapeutic agent or a detectable label.

In some embodiments, said at least one functional moiety is a therapeutic agent.

In some embodiments, said at least one functional moiety is a detectable label.

In some embodiments, said at least one functional moiety is a payload that is a therapeutic agent or a detectable label.

In some embodiments, said payload is a therapeutic agent.

In some embodiments, said payload is a detectable label.

In some embodiments, said at least one functional moiety comprises a protein, a peptide, a peptide mimetic, a nucleic acid, an oligonucleotide or a small molecule.

In some embodiments, said at least one functional moiety is a protein, a peptide, a peptide mimetic, a nucleic acid, an oligonucleotide or a small molecule.

In some embodiments, said at least one functional moiety comprises a protein.

In some embodiments, said at least one functional moiety is a protein.

In some embodiments, said at least one functional moiety comprises or is a protein which comprises at least 30 amino acids.

In some embodiments, said at least one functional moiety comprises or is a peptide which consists of 2 to 30 amino acids.

In some embodiments, said at least one functional moiety comprises a peptide.

In some embodiments, said at least one functional moiety is a peptide.

In some embodiments, said at least one functional moiety comprises a peptide mimetic.

In some embodiments, said at least one functional moiety is a peptide mimetic.

In some embodiments, said at least one functional moiety comprises or is a nucleic acid which is a DNA or an RNA.

In some embodiments, said at least one functional moiety comprises a nucleic acid.

In some embodiments, said at least one functional moiety is a nucleic acid.

In some embodiments, said at least one functional moiety comprises an oligonucleotide.

In some embodiments, said at least one functional moiety is an oligonucleotide.

In some embodiments, said at least one functional moiety comprises a small molecule.

In some embodiments, said at least one functional moiety is a small molecule.

In some embodiments, said at least one functional moiety comprises or is a small molecule with a molecular weight < 1000 Da.

In some embodiments, said at least one functional moiety comprises a small molecule.

In some embodiments, said at least one functional moiety is a small molecule.

In some embodiments, said at least one functional moiety is not a sugar.

In some embodiments, said at least one functional moiety does not comprise a sugar.

In some embodiments, said at least one functional moiety has a molecular weight of at least 100 Da.

In some embodiments, said at least one functional moiety has a molecular weight of at least 500 Da.

In some embodiments, said at least one functional moiety has a molecular weight of at least 1 000 Da.

In some embodiments, said at least one functional moiety has a molecular weight of at least 2 000 Da.

In some embodiments, said at least one functional moiety has a molecular weight of at least 10 kDa.

In some embodiments, said at least one functional moiety has a molecular weight of at least 50 kDa.

In some embodiments, said at least one functional moiety has a molecular weight of at least 100 kDa.

In some embodiments, said at least one functional moiety has a molecular weight of up to 1 000 Da.

In some embodiments, said at least one functional moiety has a molecular weight of up to 2 000 Da.

In some embodiments, said at least one functional moiety has a molecular weight of up to 10 kDa.

In some embodiments, said at least one functional moiety has a molecular weight of up to 50 kDa.

In some embodiments, said at least one functional moiety has a molecular weight of up to 200 kDa.

In some embodiments, said at least one functional moiety has a molecular weight of up to 1 MDa.

In some embodiments, said at least one functional moiety has a molecular weight of up to 5 MDa.

In some embodiments, a comparative molecule with an identical structure as said molecule, but lacking said solubility tag(s), has an isoelectric point (pI) of 5-9.

In some embodiments, a comparative molecule with an identical structure as said molecule, but lacking said solubility tag(s), has a solubility (indicated in g of compound per ml of PBS; solubility measured at 25 °C in PBS (Phosphate-buffered saline: 137 mM NaCl, 2.7 mM KCl, 10 mM Na2HPO4, 1.8 mM KH2PO4, pH 7.4)) that falls within a range of ± 50%, preferably ± 30%, relative to the solubility under the same conditions of a compound consisting of the antibody Trastuzumab with two copies of auristatin covalently linked to its Fc region.

In some embodiments, the number of functional moieties per molecule is in the range of from 1 to 15.

In some embodiments, the number of functional moieties per molecule is in the range of from 1 to 10.

In some embodiments, the number of functional moieties per molecule is in the range of from 1 to 8.

In some embodiments, the number of functional moieties per molecule is in the range of from 1 to 4.

In some embodiments, the molecule comprises one, but not more than one functional moieties.

In some embodiments, the number of functional moieties per molecule is in the range of from 2 to 8.

In some embodiments, the number of functional moieties per molecule is in the range of from 4 to 8.

In some embodiments, the number of targeting moieties per molecule is in the range of from 1 to 15.

In some embodiments, the number of targeting moieties per molecule is in the range of from 1 to 10.

In some embodiments, the number of targeting moieties per molecule is in the range of from 1 to 8.

In some embodiments, the number of targeting moieties per molecule is in the range of from 1 to 4.

In some embodiments, the molecule comprises one, but not more than one targeting moieties.

In some embodiments, the number of targeting moieties per molecule is in the range of from 2 to 8.

In some embodiments, the number of targeting moieties per molecule is in the range of from 4 to 8.

In some embodiments, said molecule is an antibody-drug conjugate comprising
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

In some embodiments, said molecule is an antibody-drug conjugate that consists of
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

### Antibody-drug conjugates

In another aspect, the present disclosure relates to an antibody-drug conjugate comprising
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

As used herein, an "antibody-drug conjugate" (abbreviated "ADC") is a molecule comprising an antibody (the "antibody component" of the ADC, see below) that is conjugated via a linker to a payload ("drug"). In the ADC of the present disclosure, the payload is a therapeutic agent or a detectable label. The different components of the antibody-drug conjugate are covalently linked.

By binding to its antigen, the antibody component of the ADC serves as targeting component that can direct the ADC to its target site. For example, if the antigen of the antibody component is a tumor antigen, the ADC will e.g. be directed to tumor cells expressing this tumor antigen at their cell surface. Upon recruitment of the ADC to the target site, the payload can mediate a therapeutic action (e.g. killing of a cancer cell, local reduction of an inflammation, local stimulation or suppression of the immune system) or, if the payload is a detectable label, the target site can be identified by detection of the detectable label.

Non-targeted drugs typically reach their site of action by whole-body distribution and passive diffusion. In contrast, ADCs are targeted compounds that are not distributed evenly across the whole body. Due to the interaction of the antibody component with its target antigen, an ADC is concentrated preferentially at its site target site. Therefore, ADCs with a therapeutic agent as payload require lower dosages to be therapeutically effective, thus improving the therapeutic window.

In many cases, upon binding to its target cell an ADC will be internalized into the cell, e.g. by receptor-mediated endocytosis. If the linker is a cleavable linker, the linker may be cleaved after cellular degradation (e.g. by enzymatic or chemical cleavage). Alternatively, the antibody may be degraded inside of the cell. In either case, the payload is released into the cellular interior. If the payload is a medical drug, it can then fulfill its therapeutic function inside of the cell. If the payload is a detectable label it may be detected inside of the cell.

Antibody-drug conjugates, their structure, preparation and use are described in detail e.g. in Antibody-Drug Conjugates: Fundamentals, Drug Development, and Clinical Outcomes to Target Cancer, 1st edition (2016), editors Olivier and Hurvitz, publisher John Wiley & Sons, Inc. (U.S.); Toader, Topics in Medicinal Chemistry (2018), vol. 28 (Cancer II), p. 289-332; Chau, Lancet (2019), vol. 394 (10200), p. 793-804; Nimoy, Pharmaceuticals (2018), vol. 11 (2), p. 32/1-32/22; Fei et al., Journal of Biomedical Nanotechnology (2018), vol. 14(3), p. 405-429; Gorka et al., Accounts of Chemical Research (2018), vol. 51(12), p. 3226-3235; Tiberghien et al., Journal of Organic Chemistry (2019), vol. 84(8), p. 4830-4836; Rohrer, in: Process Scale Purification of Antibodies, 2nd edition (2017), editor: Gottschalk, John Wiley & Sons, Inc., p. 595-614; Vaklavas and Forero, Methods in Molecular Biology (2012), vol. 899, p. 489-497.

As a skilled person will understand, in practice ADCs are often populations of molecules that slightly vary with regard to their characteristics. For example, a population of ADC molecules may for the most part include ADC molecules with 4 payloads per ADC molecule, but may also contain a small fraction of ADC molecules with 3 payloads and a small fraction of ADC molecules with 5 payloads per ADC molecule. In such a case where there is slight variation in an ADC population with regard to a characteristic, the numbers indicated below will typically relate to the rounded average number over the population.

For the purposes of the present disclosure, a high homogeneity between the ADCs within the population of interest is usually desirable. A higher homogeneity can typically be achieved by additional steps of purification/separation, e.g. by HIC (hydrophobic interaction chromatography), SEC (size exclusion chromatography) and HPLC/reversed phase HPLC. The homogeneity of an ADC population can be determined e.g. by HIC, HPLC/reversed phase HPLC, SDS-PAGE analysis and MS (mass spectrometry) analysis. For an analysis differentiating between the antibody heavy and light chain, SEC under reducing conditions or SDS-PAGE followed by MS analysis can be carried out.

### Antibody component

The term "antibody component", as used herein, refers to an immunoglobulin molecule that is used or can be used as part of an antibody-drug conjugate. The term "antibody component" can encompass intact antibodies and antigen-binding fragments of intact antibodies (i.e. fragments of an intact antibody that are still capable of binding the same antigen to which the corresponding intact antibody binds). In some embodiments, the term also includes molecules in which an intact antibody or antigen-binding fragment of an intact antibody is covalently linked to one or more further intact antibodies and/or one or more further antigen-binding fragments of antibodies and/or another molecular structure. Thus, the antibody component is an immunoglobulin molecule that recognizes and specifically binds to a target (the antigen, see below), through at least one antigen-binding site within the variable region of the immunoglobulin molecule.

As used herein, an "intact" antibody refers to an antibody that includes the complete, full-length sequence of an antibody of the respective antibody class. Thus, an intact antibody includes the antigen-binding region(s) (i.e. the complete VL and VH domains), as well as complete light and heavy chain constant domains, as appropriate for the antibody class, wherein the antibody domains remain associated through at least one non-covalent interaction. The constant domains may be native sequence constant domains (e.g., human native sequence constant domains) or amino acid sequence variants thereof.

As used herein, a "fragment" of an antibody is a portion of an intact antibody. An "antigen-binding fragment" of an (intact) antibody is a portion of said antibody that binds the same antigen as the intact antibody. Typically, this means that the fragment comprises the same antigen-binding region as the intact antibody. Examples of antibody fragments include, but are not limited to Fab, Fab', F(ab')2, and Fv fragments and single chain Fv (scFv) antibodies. In some embodiments, the term "fragment" of an antibody also encompasses bi- or multivalent antibody constructs generated by joining two or more of the aforementioned antibody fragments together.

As used herein, "antigen" refers to a substance that can specifically bind to the variable region of an antibody. An antigen may e.g. be a protein, polypeptide, peptide, carbohydrate, polynucleotide, lipid, or a combination of the foregoing.

A "variable region" of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of the antibody. There are at least two techniques for determining CDRs: (1) an approach based on cross-species sequence variability (Sequences of Proteins of Immunological Interest, 5th ed. (1991), editors Kabat et al., National Institutes of Health (Bethesda, USA)); and (2) an approach based on crystallographic studies of antigen-antibody complexes (Al-Lazikani et al., J. Molec. Biol. (1997), vol. 273, p. 927-948)). In addition, combinations of these two approaches are sometimes used in the art to determine CDRs.

The terms "epitope" or "antigenic determinant" are used interchangeably herein and refer to the portion of an antigen that is recognized and specifically bound by a particular antibody. When the antigen is a polypeptide, epitopes can be formed both from contiguous amino acids and noncontiguous amino acids juxtaposed by tertiary folding of a protein. Epitopes formed from contiguous amino acids are typically retained upon protein denaturing, whereas epitopes formed by tertiary folding are typically lost upon protein denaturing. An epitope typically includes at least 3, and more usually, at least 5 or 8-10 amino acids in a unique spatial conformation.

Each ADC molecule according to the present disclosure comprises one antibody component (but may comprise more than one payload and more than one linker).

With respect to the type and source of the antibody, the antibody component is not particularly limited, as long as it contains at least one antigen-binding site and shows binding to its target antigen. Standard techniques of antibody design and preparation are known to a skilled person (see e.g. Antibodies: A Laboratory Manual, 2nd edition (2014), editor Greenfield, Cold Spring Harbor Laboratory Press (U.S.); Antibody Engineering - Methods and Protocols, 2nd edition (2010), editors Nevoltris and Chames, publisher Springer (Germany); Handbook of Therapeutic Antibodies (2014), editors Dübel and Reichert, publisher Wiley-VCH Verlag GmbH & Co. KGaA (Germany); Harper, Methods in Molecular Biology (2013), vol. 1045, p. 41-49).

The following embodiments relate to any of the molecules, antibody-drug conjugates, methods or uses defined above.

In some embodiments, said antibody component is an intact antibody or an antigen-binding fragment thereof.

In some embodiments, said antibody component is an intact antibody.

In some embodiments, said antibody component is an antigen-binding fragment of an intact antibody.

In some embodiments, the antibody component (resp. said antibody that is included as targeting moiety in the "molecule" defined above) is a monoclonal antibody or a polyclonal antibody. Preferably, the antibody component (resp. said antibody that is included as targeting moiety in the "molecule" defined above) is a monoclonal antibody.

A "monoclonal" antibody", as used herein, means an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody arising from the growth of a single cell clone and is generally characterized by heavy chains of one and only one class and subclass, and light chains of only one type. Monoclonal antibodies are directed against a single antigen. In addition, in contrast with preparations of polyclonal antibodies which typically include various antibodies directed against various epitopes, each monoclonal antibody is directed against a single epitope of the antigen. Monoclonal antibodies are typically produced by a single clone of B lymphocytes ("B cells"). Monoclonal antibodies may be obtained using a variety of techniques known to those skilled in the art, including standard hybridoma technology (see e.g. Kohler and Milstein, Eur. J. Immunol. (1976), vol. 5, p. 511-519; Antibodies: A Laboratory Manual, 2nd edition (2014), editor Greenfield, Cold Spring Harbor Laboratory Press (USA); Immunobiology, 5th ed. (2001), editors Janeway et al., Garland Publishing (USA)) and e.g. expression from a eukaryotic host cell transfected with a DNA molecule coding for the homogeneous antibody or from a prokaryotic host cell transfected with a DNA molecule coding for the homogeneous antibody.

As used herein, "polyclonal" antibody refers to a heterogeneous population of antibodies, typically obtained by purification from the sera of immunized animals by standard techniques known to a skilled person (see e.g. Antibodies: A Laboratory Manual, 2nd edition (2014), editor Greenfield, Cold Spring Harbor Laboratory Press (USA)).

In some embodiments, the antibody component (resp. said antibody that is included as targeting moiety in the "molecule" defined above) is a monospecific antibody or a bispecific antibody.

A "monospecific antibody", as used herein, is an antibody that is capable of binding only to one antigen.

The term "bispecific antibody", as used in the present disclosure, refers to an antibody that is capable of specifically binding to two different epitopes at the same time. The epitopes can be from the same antigen or from two different antigens. Preferably, the epitopes are from two different antigens. Typically, a bispecific antibody has two antigen-binding sites, wherein e.g. each of the two pairs of heavy chain and light chain (HC/LC) is specifically binding to a different antigen, i.e. the first heavy and the first light chain are specifically binding together to a first antigen, and, the second heavy and the second light chain are specifically binding together to a second antigen. Methods for making bispecific antibodies are known in the art. For example, bispecific antibodies can be produced recombinantly using the co-expression of two immunoglobulin heavy chain/light chain pairs (see e.g. Milstein et al., Nature (1983), vol. 305, p. 537-539). Alternatively, bispecific antibodies can be prepared using chemical linkage (see e.g. Brennan et al., Science (1985), vol. 229, p. 81). A bispecific antibody can also for example be prepared by the SEED technology (an approach for generation of bispecific antibodies in which structurally related sequences within the conserved CH3 domains of human IgA and IgG are exchanged to form two asymmetric but complementary domains, see WO 2016/087650).

In some embodiments, said antibody component (resp. said antibody that is included as targeting moiety in the "molecule" defined above) is a bispecific antibody or an antigen-binding fragment thereof that is capable of binding both antigens for which said bispecific antibody is specific. Thus, said antigen-binding fragment of said bispecific antibody binds to the same two antigens as said bispecific antibody.

In some embodiments, said antibody that is included as targeting moiety in the "molecule" defined above is a bispecific antibody.

The antibody component of the present disclosure (resp. said antibody that is included as targeting moiety in the "molecule" defined above) may be monovalent, bivalent or multivalent. A "monovalent" antibody/antibody component has one antigen-binding site. A "bivalent" antibody/antibody component has two antigen-binding sites. These two antigen-binding sites may bind the same or different antigens. A "multivalent" antibody/antibody component has more than two antigen-binding sites. These more than two antigen-binding sites may bind the same or different antigens.

In some embodiments, said antibody component (resp. said antibody that is included as targeting moiety in the "molecule" defined above) is an antibody selected from the group consisting of a chimeric antibody, a humanized antibody and a human antibody.

As used in this disclosure, a "chimeric" antibody is an antibody in which a portion of the heavy and/or light chain is identical with or homologous to corresponding sequences in antibodies derived from a particular species or belonging to a particular antibody class or subclass, while the remainder of the chain(s) is/are identical with or homologous to corresponding sequences in antibodies derived from another species or belonging to another antibody class or subclass, as well as fragments of such antibodies, so long as they exhibit the desired biological activity (U.S. Patent 4,816,567; Morrison et al., Proc. Natl. Acad. Sci USA (1984), vol. 81, p. 6851-6855). As used herein, "humanized antibody" is used a subset of "chimeric antibodies."

A "humanized antibody", as used herein, is a "humanized" form of non-human (e.g., murine) antibody. A "humanized antibody", is a chimeric antibody that contains minimal sequence derived from non-human immunoglobulin. In one embodiment, a humanized antibody is a human immunoglobulin (recipient antibody) in which residues from an HVR (hereinafter defined) of the recipient are replaced by residues from an HVR of a non-human species (donor antibody) such as mouse, rat, rabbit or non-human primate having the desired specificity, affinity, and/or capacity. In some instances, framework (FR) residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, etc. The number of these amino acid substitutions in the FR are typically no more than 6 in the H chain, and in the L chain, no more than 3. The humanized antibody optionally will also comprise at least a portion of an immunoglobulin constant region (Fc), typically that of a human immunoglobulin. For further details, see e.g. Jones et al., Nature (1986), vol. 321, p. 522-525; Riechmann et al., Nature (1988), vol. 332, p. 323-329; and Presta, Curr. Op. Struct. Biol. (1992), vol. 2, p. 593-596. See also, for example, Vaswani and Hamilton, Ann. Allergy, Asthma & Immunol. (1998), vol. 1, p. 105-115; Harris, Biochem. Soc. Transactions (1995), vol. 23, p. 1035-1038; Hurle and Gross, Curr. Op. Biotech. (1994), vol. 5, p. 428-433; U.S. Patent 6,982,321; U.S. Patent 7,087,409. A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries (Hoogenboom and Winter, J. Mol. Biol. (1991), vol. 227, p. 381; Marks et al., J. Mol. Biol. (1991), vol. 222, p. 581). Also available for the preparation of human monoclonal antibodies are methods described in Cole et al., in: Monoclonal Antibodies and Cancer Therapy (1985), editors Reisfeld and Sell, publisher Alan R. Liss Inc. (New York), p. 77-96; Boerner et al., J. Immunol. (1991), vol. 147(1), p. 86-95; van Dijk and van de Winkel, Curr. Opin. Pharmacol. (2001), vol. 5, p. 368-374. Human antibodies can be prepared by administering the antigen to a transgenic animal that has been modified to produce such antibodies in response to antigenic challenge, but whose endogenous loci have been disabled, e.g., immunized xenomice (see e.g. U.S. Patent 6,075,181 and U.S. Patent 6,150,584 regarding XENOMOUSE^{™} technology). See also, for example, Li et al., Proc. Natl. Acad. Sci. USA (2006), vol. 103, p. 3557-3562 regarding human antibodies generated via a human B-cell hybridoma technology.

The antibody component according to the present disclosure can be of any class (e.g. IgA, IgD, IgE, IgG, and IgM, preferably IgG), or subclass (e.g., IgG1, IgG2, IgG3, IgG4, IgA1 and IgA2, preferably IgG1). The different classes of immunoglobulins have different and well-known subunit structures and three-dimensional configurations (Immunobiology, 5th ed. (2001), editors Janeway et al., Garland Publishing (USA)).

In some embodiments, said antibody component (resp. said antibody that is included as targeting moiety in the "molecule" defined above) is an antibody selected from the group consisting of an IgG1 antibody, an IgG2 antibody, an IgG3 antibody, IgG4 antibody, an IgA antibody, an IgM antibody, and hybrids thereof.

An antibody consisting of a "hybrid" of two antibodies of different class/subclasses refers to an antibody that contains sequences from these two antibodies of different class/subclass. For example, a bispecific antibody prepared by the SEED technology (WO 2016/087650) typically contains sequences from both IgG and IgA and thus would be considered a "hybrid" of an IgG antibody and an IgA antibody.

In some embodiments, said antigen-binding fragment is selected from the group consisting of a Fab, a Fab', a (Fab')2, a Fv, a scFv, a diabody and a VHH

"Fab" fragments are obtained by papain digestion of an antibody, which produces two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site.

"F(ab')2" fragments are obtained by pepsin treatment of an antibody, which yields a single large F(ab')2 fragment which roughly corresponds to two disulfide linked Fab fragments having different antigen-binding activity and is still capable of cross-linking antigen.

"Fab' " fragments differ from Fab fragments by having a few additional residues at the carboxy terminus of the CH1 domain including one or more cysteines from the antibody hinge region. Fab'-SH is the designation for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and - binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

"Single-chain Fv", also abbreviated as "scFv", are antibody fragments that comprise the VH and VL antibody domains connected into a single polypeptide chain. Preferably, the scFv polypeptide further comprises a polypeptide linker between the VH and VL domains which enables the scFv to form the desired structure for antigen binding. For a review of the scFv, see Pluckthun, in: The Pharmacology of Monoclonal Antibodies, vol. 113 (1994), editors Rosenburg and Moore, Springer-Verlag (New York), p. 269-315.

The term "diabody" refers to a small antibody fragment prepared by constructing scFv fragments (see preceding paragraph) with short linkers (about 5-10) residues) between the VH and VL domains such that inter-chain but not intra-chain pairing of the V domains is achieved, thereby resulting in a bivalent fragment, i.e., a fragment having two antigen-binding sites. Bispecific diabodies are heterodimers of two "crossover" scFv fragments in which the VH and VL domains of the two antibodies are present on different polypeptide chains. Diabodies are described in greater detail in, for example, EP 0404097; WO 93/11161; Hollinger et al., Proc. Natl. Acad. Sci. USA (1993), vol. 90, p. 6444-6448.

As used herein, the terms "VHH" and "nanobody" have the same meaning. They refer to single-domain antibodies which are antibody fragments consisting of a single monomeric variable region of a heavy chain of an antibody. Like a whole antibody, a VHH is able to bind selectively to a specific antigen. With a molecular weight of only 12-15 kDa, VHHs are much smaller than common antibodies (150-160 kDa). The first single-domain antibodies were engineered from heavy-chain antibodies found in camelids. (Gibbs and Wayt, Nanobodies, Scientific American Magazine (2005)). Generally, the antibodies with a natural deficiency of the light chain and the heavy chain constant region 1 (CHI) are first obtained, the variable regions of the heavy chain of the antibody are therefore cloned to construct a single domain antibody (VHH) consisting of only one heavy chain variable region.

In some embodiments, said antigen-binding fragment is selected from the group consisting of a Fab, a Fab', a (Fab')2 and a Fv.

In some embodiments, said antigen-binding fragment is selected from the group consisting of a scFv, a diabody and a VHH

In some embodiments, said antigen-binding fragment is an antigen-binding fragment of a monoclonal antibody or a polyclonal antibody.

In some embodiments, said antigen-binding fragment is an antigen-binding fragment of a monoclonal antibody.

In some embodiments, said antigen-binding fragment is an antigen-binding fragment of a monospecific antibody or a bispecific antibody.

In some embodiments, said antigen-binding fragment is an antigen-binding fragment of a bispecific antibody that is capable of binding both antigens for which said bispecific antibody is specific.

In some embodiments, said antigen-binding fragment is an antigen-binding fragment of an antibody selected from the group consisting of a chimeric antibody, a humanized antibody and a human antibody.

In some embodiments, said antigen-binding fragment is an antigen-binding fragment of an antibody selected from the group consisting of an IgG1 antibody, an IgG2 antibody, an IgG3 antibody, IgG4 antibody, an IgA antibody, an IgM antibody, and hybrids thereof.

In some embodiments, said targeting moiety/said antibody component (resp. said antibody that is included as targeting moiety in the "molecule" defined above) is capable of specifically binding to an antigen that is present on the surface of a target cell.

As used herein, an "antigen that is present on the surface of a target cell" is an antigen that is present on the surface of the target cell in such a manner that it is accessible from the extracellular environment (i.e. an antibody can bind to it from the extracellular environment). For example, CD8 is a transmembrane protein of cytotoxic T cells, and its extracellular domain is accessible for antibodies directed against the extracellular domain of CD8 from the extracellular environment. Thus, in the sense of the present disclosure, CD8 is an antigen that is present on the surface of cytotoxic T cells. In an embodiment, said "antigen that is present on the surface of a target cell" is a protein that is present on the surface of a target cell.

An antibody/antibody component "binds" an antigen of interest is an antibody/antibody component that is capable of binding that antigen with sufficient affinity such that the antibody/antibody component is useful in targeting to a cell expressing the antigen.

If the present disclosure refers to a first molecule/molecular group (e.g. an antibody/antibody component) "specifically binding"/that "specifically binds" to a second molecule/molecular group (e.g. an antigen of interest), this means that the first molecule/molecular group (in this example the antibody) binds to said second molecule/molecular group (in this example the antigen of interest) with an affinity that is at least ten-fold greater than its affinity for other molecules/molecular groups, in particular other molecule/molecular group in the human body (in this example at least ten-fold greater than its affinity for binding to non-specific antigens (e.g., BSA, casein) other than said antigen of interest (or closely related antigens)). In a preferred embodiment, a first molecule/molecular group (e.g. an antibody/antibody component) that "specifically binds" to a second molecule/molecular group (e.g. an antigen of interest) binds to said antigen with an affinity that is at least 100-fold greater than its affinity for other molecules/molecular groups, in particular other molecule/molecular group in the human body (in this example at least 100-fold greater than its affinity for binding to non-specific antigens other than said antigen of interest (or closely related antigens)). Typically said binding will be determined under physiological conditions. A first molecule/molecular group that "specifically binds" to a second molecule/molecular group may bind to that second molecule/molecular group with an affinity of at least about 1×10⁷ M⁻¹. An antibody/antibody component that "specifically binds" to an antigen of interest may bind to that antigen with an affinity of at least about 1×10⁷ M⁻¹.

In some embodiments, said antibody component (resp. said antibody that is included as targeting moiety in the "molecule" defined above) is an antibody against an antigen that is present on the surface of a target cell or an antigen-binding fragment of such an antibody.

An antibody/antibody component "against" a certain antigen is an antibody/antibody component with an antigen-binding site that binds to said antigen. If an antibody binds to an antigen can e.g. be determined by testing in an immunofluorescence experiment with cultured cells whether the antibody binds to cells that express the antigen at their cell surface.

In some embodiments, said antigen that is present on the surface of said target cell is more abundant on the surface of said target cell than on the surface of other cell types.

The abundance of a surface antigen on a cell type can be determined by standard methods known to a skilled person, e.g. flow cytometry (e.g. by exposing cell of said cell type to the antibody of interest, subsequently staining with a fluorescently labelled secondary antibody directed against the antibody of interest, and detection of fluorescent label by flow cytometry).

In some embodiments, said antigen that is present on the surface of said target cell is "present on the surface of said target cell, but substantially not on the surface of other cell types".

As used herein, an antigen that is "present on the surface of said target cell, but substantially not on the surface of other cell types" is sufficiently abundant at the surface of the target cell to allow for recruitment of an ADC with an antibody component against said antigen under physiological conditions. In contrast, abundance of said antigen at the surface of other cell types is so low that recruitment of said ADC under physiological conditions is barely above background binding.

In some embodiments, said antigen that is present on the surface of said target cell is present on the surface of said target cell, but not on the surface of other cell types.

As used herein, an antigen that is "present on the surface of said target cell, but not on the surface of other cell types" is sufficiently abundant at the surface of the target cell to allow for recruitment of an ADC with an antibody component against said antigen under physiological conditions. In contrast, abundance of said antigen at the surface of other cell types is so low that recruitment of said ADC under physiological conditions is not above background binding.

In some embodiments, said binding of said antibody component (resp. said antibody that is included as targeting moiety in the "molecule" defined above) to said antigen that is present on the surface of said target cell allows to recruit the antibody-drug conjugate specifically to said target cell.

The term "allows to recruit the antibody-drug conjugate specifically to said target cell" means that the ADC is recruited to said target cell under physiological conditions with an efficiency that is at least 10 times higher, preferably at least 100 times higher, than the recruitment to other cell types (i.e. to other cell types to which said ADC may be exposed in the body during administration of said ADC).

In some embodiments, said antigen that is present on the surface of said target cell is selected from the group consisting of a tumor antigen and an immune cell antigen.

In some embodiments, said antigen that is present on the surface of said target cell is a tumor antigen.

In some embodiments, said targeting moiety/said antibody component (resp. said antibody that is included as targeting moiety in the "molecule" defined above) is capable of specifically binding to an antigen selected from the group consisting of a tumor antigen and an immune cell antigen.

In some embodiments, said targeting moiety/said antibody component (resp. said antibody that is included as targeting moiety in the "molecule" defined above) is capable of specifically binding to a tumor antigen.

The term "tumor", as used herein, refers to an abnormal cell mass formed by neoplastic cell growth. A tumor can be benign or malignant. Preferably, in the present disclosure the term "tumor" refers to a malignant tumor. The tumor can be, but is not limited to, a tumor present in myeloma, hematological cancers such as leukemias and lymphomas (such as B cell lymphoma, T cell lymphoma, Hodgkin's lymphoma, non-Hodgkin's lymphoma), hematopoietic neoplasms, thymoma, head and neck cancer, sarcoma, lung cancer, liver cancer, genitourinary cancers (such as ovarian cancer, vaginal cancer, cervical cancer, uterine cancer, bladder cancer, testicular cancer, prostate cancer or penile cancer), adenocarcinoma, breast cancer, pancreatic cancer, lung cancer, renal cancer, liver cancer, primary or metastatic melanoma, squamous cell carcinoma, basal cell carcinoma, neurological tumors including brain tumors such as astrocystomas and glioblastomas, angiosarcoma, hemangiosarcoma, head and neck cancer, thyroid carcinoma, soft tissue sarcoma, bone cancer such as bone sarcoma, vascular cancer, gastrointestinal cancer (such as gastric, stomach or colon cancer) (see Rosenberg, Ann. Rev. Med. (1996), vol. 47, p. 481-491).

As used herein, the term "cancer" refers to a malignant neoplasm. Cancer can include a hematological cancer or a solid tumor. For example, the cancer can be a leukemia (e.g., acute myeloid leukemia (AML), acute monocytic leukemia, promyelocytic leukemia, eosinophilic leukaemia, acute lymphoblastic leukemia (ALL) such as acute B lymphoblastic leukemia (BALL), chronic myelogenous leukemia (CML), chronic lymphocytic leukemia (CLL)) or lymphoma (e.g., non-Hodgkin lymphoma), myelodysplastic syndrome (MDS)" melanoma, lung cancer (e.g., non-small cell lung cancer; NSCLC), ovarian cancer, endometrial cancer, peritoneal cancer, pancreatic cancer, breast cancer, prostate cancer, squamous cell carcinoma of the head and neck, or cervical cancer. Preferably, in the present disclosure the term "cancer" refers to a solid malignant tumor.

As used herein, a "tumor antigen" is, in its broadest sense, an antigen that allows recruitment of an ADC to the site of a tumor, such that a therapeutic action or diagnostic (e.g. labelling of the tumor site) can be achieved. The tumor antigen may either be an antigen that is present on the surface of the tumor cells or an antigen associated with the tumor microenvironment.

Sources for information on cell surface expression and methods to identify and verify tumor antigens are known to a skilled person and described in the literature (see e.g. Bornstein, AAPS J. (2015), vol. 17(3), p. 525-534; Bander, Methods Mol Biol (2013), vol. 1045, p. 29-40; Antibody-Drug Conjugates: Fundamentals, Drug Development, and Clinical Outcomes to Target Cancer", 1st edition (2016), editors Olivier and Hurvitz, publisher John Wiley & Sons, Inc. (U.S.); Vigneron et al., Cancer Immun. (2013), vol. 13, p. 15; Hong et al., BMC Syst Biol. (2018), vol. 12 (Suppl 2), p. 17; de Souza et al., Cancer Immun. (2012), vol. 12, p. 15; Immune Epitope Database and Analysis Resource (https://www.iedb.org); Cancer Cell Line Encyclopedia (https://portals.broadinstitute.org/ccle); OASIS Database (http://oasis-genomics.org/)).

In preferred embodiments, said tumor antigen is an antigen that is present on the surface of a tumor cell. In these embodiments, the term "tumor antigen" indicates an antigen that is present at the cell surface of a tumor cell and allows for distinction of the tumor cell over other cell types. A tumor antigen may be part of a molecule (e.g. a protein) that is expressed by a tumor cell and accessible from the extracellular environment. A tumor antigen may differ (i.e. qualitatively differ) from its counterpart in corresponding non-tumor cells (e.g., where the molecule is a protein by one or more amino acid residues). Alternatively, the tumor antigen may be identical to its counterpart in corresponding non-tumor cells, but present on the surface of the tumor cells at a higher level than on the surface of corresponding non-tumor cells. For example, the tumor antigen may be present only on the surface of the tumor cells, but not on the surface of non-tumor cells, or the tumor antigen may be present on the surface of tumor cells at a higher level (e.g. at least 5-fold higher, preferably at least 100-fold higher) than on the surface of non-tumor cells. In an embodiment, the tumor antigen is present on the surface of tumor cells at a level that is at least 1000-fold higher than on the surface of non-tumor cells.

Preferably, the tumor to which said tumor antigen relates is a cancer (i.e. the tumor antigen that is present on the surface of a tumor cell is present on a cancer cell).

In some embodiments, said tumor antigen is selected from the group consisting of CD1 Ia, CD4, CD19, CD20, CD21, CD22, CD23, CD25, CD52, CD30, CD33, CD37, CD40L, CD52, CD56, CD70, CD72, CD74, CD79a, CD79b, CD138, CD163, Her2, Her3, EGFR, Mucl8, ,integrin, PSMA, CEA, BLys, ROR1, NaPi2b, NaPi3b, CEACAM5, Muc1, integrin avb6, Met, Trop2, BCMA, disialoganglioside GD2, B-PR1B, E16, STEAP1, 0772P, Sema 5b, ETBR, MSG783, STEAP2, Trp4, CRIPTO, FcRH1, FcRH2, NCA, IL20R-alpha, Brevican, EphB2R, ASLG659, PSCA, GEDA, BAFF-R, CXCR5, HLA-DOB, P2X5, LY64, IRTA2, TENB2, PSMA, FOLH1, STR5, SSTR1, SSTR2, SSTR3, SSTR4, TGAV, ITGB6, CA9, EGFRvlll, IL2RA, AXL, CD3Q, TNFRSF8, TNFRSF17, CTAGs, CTA; CD174/Fucosyltransferase 3 (Lewis Blood Group), CLEC14A, GRP78, HSPA5, ASG-5, ENPP3, PRR4, GCC, GUCY2C, Liv-1, SLC39A8, 5T4, NCMA1, CanAg, FOLR1, GPN B, TIM-1, HAVCR1, Mindin/RG-1, B7-H4, VTCN1, PTK7, SDC1, a claudin (preferably claudin 18.2), RON, MST1 R, EPHA2, MS4A1, TNC (Tenascin C), FAP, DKK-1, CS1/SLAMF7, ENG (Endoglin), ANXA1 (Annexin A1), VCAM-1 (CD106) and folate receptor alpha.

In some embodiments, said tumor antigen is selected from the group consisting of xCT, gpNMB, carbonic anhydrase IX (CAIX), cKIT, c-MET, Tumor-associated glycoprotein 72 (TAG-72), TROP-2, TRA-1-60, TRA, TNF-alpha, TM4SF1, TIM-1, TAA, TA-MUC1 (tumor-specific epitope of mucin-1), Sortilin (SORT1), STn, STING, STEAP-1, SSTR2, SSEA-4, SLITRK6, SLC44A4, SLAMF7, SAIL, Receptor tyrosine kinase (RTK), ROR2, ROR1, RNF43, Prolactin Receptor (PRLR), Polymorphic epithelial mucin (PEM), Phosphatidylserine (PS), Phosphatidyl Serine, PTK7, PSMA, PD-L1, P-Cadherin, OX001L, OAcGD2, Nectin-4, NaPi2b, NOTCH3, Mesothelin (MSLN), MUC16, MTX5, MTX3, MT1-MMP, MRC2, MET, MAGE, Ly6E, Lewis Y antigen, LRRC15, LRP-1, LIV-1, LHRH, LGR5, LGALS3BP, LAMP-1, KLK2, KAAG-1, IL4R, IL7R, IL1RAP, IL-4, IL-3, IL-2, IL-13R, IGF-1R, HSP90, HLA-DR, HER-3, HER-2, Globo H, GPR20, GPC3, GPC-1, GD3, GD2, GCC, FSH, FOLR-alpha, FOLR, FLT3, FGFR3, FGFR2, FCRH5, EphA3, EphA2, EpCAM, ETBR, ENPP3, EGFRviii, EGFR, EFNA4, Dysadherin, DR5 (Death receptor 5), DPEP3, DLL3, DLK-1, DCLK1, Cripto, Cathepsin D, CanAg, CXCR5, CSP-1, CLL-1, CLDN6, CLDN18.2, CEACAM6, CEACAM5, CEA, CDH6, CD79b, CD74, CD71, CD70, CD56, CD51, CD48, CD46, CD45, CD44v6, CD40L, CD38, CD37, CD352, CD33, CD317, CD30, CD300f, CD3, CD25, CD248, CD228, CD22, CD205, CD20, CD19, CD184, CD166, CD147, CD142, CD138, CD123, CCR7, CA9, CA6, C4.4a, BCMA, B7-H4, B7, -H3, Axl, ASCT2, AMHRII, ALK, AG-7, ADAM-9, 5T4, 4-1BB.

Insofar as the designations of antigens indicated in the present disclosure are gene designations, these designations refer to the protein(s) encoded by said gene.

In some embodiments, said antibody component (resp. said antibody that is included as targeting moiety in the "molecule" defined above) has a first and a second antigen-binding site. Preferably, said first and said second antigen-binding site are capable of binding to different antigens. In some embodiments, said first antigen-binding site is capable of specifically binding to a tumor antigen and said second antigen-binding site is capable of specifically binding to a tumor antigen.

In some embodiments, said targeting moiety/said antibody component (resp. said antibody that is included as targeting moiety in the "molecule" defined above) is capable of specifically binding to an immune cell antigen.

Preferably, said immune cell antigen is
- an antigen present on the surface of an immune cell,
- an antigen which is a molecule that is secreted by an immune cell, or
- an antigen which is a molecule that interacts with a receptor on an immune cell.

More preferably, said immune cell antigen is an antigen present on the surface of an immune cell.

In some embodiments, said antigen that is present on the surface of said target cell is an immune cell antigen that is present on the surface of an immune cell.

In some embodiments, said immune cell is a B cell, a T cell or a dendritic cell. Preferably, said immune cell is a T cell.

In some embodiments, said immune cell antigen is selected from the group consisting of CD80, CD86, B7H3, TNF-α, TGF-β, TGF-β2, TGF-1, IL-1, IL-4, IL-5, IL-6, IL-12, IL-13, IL-22, IL-23, interferon receptor, PD-1, PD-L1, CTLA4, MSR1 and folate receptor beta.

Binding of said antibody component (resp. said antibody that is included as targeting moiety in the "molecule" defined above) to said immune cell antigen may have an immunostimulatory or immunosuppressive effect.

### Payload

In some embodiments, said "molecule" defined above comprises only one kind of functional moiety.

As described above, the ADC of the present disclosure comprises a payload.

The term "payload", as used herein, refers to a chemical moiety that is conjugated to an antibody component as part of an antibody-drug conjugate. In the antibody-drug conjugate according to the present disclosure, the payload is linked to the antibody component by covalent binding through a linker. As described above, the payload in the ADC of the present disclosure (resp. the functional moiety) is a therapeutic agent or a detectable label. Upon recruitment of the ADC to its target site by binding of the antibody component to its target antigen, the payload can fulfill its function at the target site. For example, if the antibody component is specific for the tumor antigen, the payload may be a cytotoxic agent that kills tumor cells, e.g. a maytansinoid or duocarmycin. Or if the antibody component is specific for an antigen indicating inflammation, the payload may be an anti-inflammatory agent, e.g. a glucocorticoid receptor antagonist like cortisol or prednisolone. Or the payload may be a detectable agent that allows to detect the presence of the target antigen or identify the target site.

The payload can be introduced into the ADC at different stages of preparation. In one approach, a linker-payload construct (i.e. a construct in which the payload is covalently linked to the linker) is synthesized by standard methods of organic chemistry (as shown in the examples) and subsequently this linker-payload payload construct is conjugated to the antibody component. However, the antibody component, linker and payload can also be prepared and conjugated in different order (e.g. the linker is conjugated to the antibody component and subsequently the payload attached to the linker).

Different payloads, their preparation, conjugation and use in antibody-drug conjugates are described e.g. in Nicolaou et al., Accounts of Chemical Research (2019), vol. 52(1), p. 127-139; Maderna et al., Molecular Pharmaceutics (2015), vol. 12(6), p. 1798-1812; Gromek et al., Current Topics in Medicinal Chemistry (2014), vol. 14(24), p. 2822-2834.

The ADC according to the present disclosure may comprise only one type of payload (i.e. one ADC molecule is linked to only one kind of payload, e.g. auristatin E, wherein one or more copies of the payload (in this example auristatin E) may be linked to the ADC molecule) or several types of payloads (i.e. one ADC molecule is linked to two or more kinds of payload, e.g. auristatin E and DM4, wherein one or more copies of each payload (in this example one or more copies of auristatin E and one or more copies of DM4) may be linked to the ADC molecule). Preferably, the antibody-drug conjugate according to the present disclosure comprises only one kind of payload.

The copy number payloads linked to one ADC molecule (i.e. in the first example above the number of auristatin E molecules linked to one ADC molecule, and in the second example above the number of auristatin E molecules plus the number of DM4 molecules linked to one ADC molecule) is reflected in the drug-antibody ratio.

As used herein, the "drug-antibody ratio" of an ADC (abbreviated as "DAR") is the (average) number of payloads per ADC molecule divided by the number of antibody components per ADC molecule. The DAR of an ADC can e.g. be determined by identifying the molecular components of an ADC molecule by mass spectrometry and subsequently dividing the number of payload molecules ("drug" molecules, which includes also detectable labels if the payload is a detectable label) in an ADC molecule to the number of antibody components in the ADC molecule (the ADC according to the present disclosure contains one antibody component per ADC molecule). The DAR values of the embodiments defined below are preferably determined by this approach, i.e. calculated from structural information obtained by mass spectrometry.

ADCs with different DAR can be prepared by linking different numbers of payloads to the ADC molecule. For example, a linker-payload construct including one payload copy per linker can be prepared, and subsequently multiple copies of this linker-payload construct are linked to each antibody component. The number of linker-payload constructs that are linked per antibody component can be influenced by the reaction conditions (e.g. the concentrations of the components, degree of activation of components, duration of conjugation reaction etc.), as known to a skilled person and described in Example 3 below. See also section on Conjugation below.

Typically, the drug-antibody ratio (DAR) of the antibody-drug conjugate according to the present disclosure is in the range of from 1 to 15, preferably in the range of from 1 to 10, more preferably in the range of from 1 to 8, even more preferably in the range of from 1 to 4.

In another embodiment, the drug-antibody ratio (DAR) of the antibody-drug conjugate according to the present disclosure is in the range of from 4 to 8.

In particularly preferred embodiments, the drug-antibody ratio (DAR) of the antibody-drug conjugate according to the present disclosure is in the range of from 2 to 8.

As pointed out above, the payload of the ADC of the present disclosure (resp. said functional moiety) can be a therapeutic agent.

A "therapeutic agent", as used herein, is an agent that exerts an effect that is linked to a therapeutic benefit if administered to a patient (e.g. by killing a tumor cells, reducing an undesired inflammation, stimulating the activity of the immune system against an infection, or suppressing the immune response in case of an autoimmune disease). Therapeutic agents useful in accordance with the present disclosure include, but are not limited to, cytotoxic agents, anti-inflammatory agents, immunostimulatory agents and immunosuppressive agents.

In some embodiments, the therapeutic agent is a cytotoxic agent, anti-inflammatory agent, immunostimulatory agent or immunosuppressive agent.

In some preferred embodiments, the therapeutic agent is a cytotoxic agent.

As used herein, a "cytotoxic agent" is a substance that is toxic to cells (i.e. causes cell death or destruction). A cytotoxic agent according to the present disclosure is typically a small molecule chemical compound, peptide, or nucleic acid molecule. Various cytotoxic agents that can be used in ADCs are known to the skilled person (Nicolaou et al., Accounts of Chemical Research (2019), vol. 52(1), p. 127-139; Maderna et al., Molecular Pharmaceutics (2015), vol. 12(6), p. 1798-1812; Gromek et al., Current Topics in Medicinal Chemistry (2014), vol. 14(24), p. 2822-2834; Garcia-Echeverria, Journal of Medicinal Chemistry (2014), vol. 57(19), p. 7888-7889). Examples of cytotoxic agents include, but are not limited to, auristatins (e.g. auristatin E, MMAE (monomethyl auristatin E), MMAF (monomethyl auristatin F), dolastatin 10, dolastatin 15), maytansinoids (e.g. maytansin, DM1, DM2, DM3, DM4; since maytansinoids are derived from maytansin, they are sometimes referred to herein also as "maytansins"), tubulysin, exatecan, camptothecin, SN38, Dxd, exatecan, duocarmycin, CBI dimer (Cyclopropanebenz[e]indoline dimer, also referred to herein as "CBI"), doxorubicin or diazepines (e.g. pyrrolobenzodiazepine or indolinobenzodiazepine).

In some embodiments, the cytotoxic agent according to the present disclosure is a chemotherapeutic agent or a radioactive isotope. Preferably, the cytotoxic agent is a chemotherapeutic agent.

In some embodiments, the therapeutic agent is an Eg5 inhibitor, a V-ATPase inhibitor, a HSP90 inhibitor, an IAP inhibitor, an mTor inhibitor, a microtubule stabilizer, a microtubule destabilizer, an auristatin, a dolastatin, a MetAP (methionine aminopeptidase), an inhibitor of nuclear export of proteins CRM1, a DPPIV inhibitor, an inhibitor of phosphoryl transfer reactions in mitochondria, a protein synthesis inhibitor, a kinase inhibitor, a CDK2 inhibitor, a CDK9 inhibitor, a proteasome inhibitor, a kinesin inhibitor, an HDAC inhibitor, a DNA damaging agent, a DNA alkylating agent, a DNA intercalator, a DNA minor groove binder, an RNA polymerase inhibitor, a topoisomerase inhibitor and a DHFR inhibitor. Methods for attaching each of these to a linker compatible with the antibodies and method of the present disclosure are known in the art (see e.g. Singh et al., Therapeutic Antibodies: Methods and Protocols (2009), vol. 525, p 445-457).

In preferred embodiments, the chemotherapeutic agent may for example be are a maytansinoid (such as DM1, DM2, DM3, or DM4), an anti-metabolite (e.g., methotrexate, 6-mercaptopurine, 6-thioguanine, cytarabine, 5-fluorouracil decarbazine), an ablating agent (e.g., mechlorethamine, thiotepa chlorambucil, meiphalan, carmustine (BSNU), lomustine (CCNU), cyclophosphamide, busulfan, dibromomannitol, streptozotocin, mitomycin C, cis-dichlorodiamine platinum (II) (DDP) cisplatin, anthracycline (e.g., daunorubicin (formerly daunomycin), doxorubicin), an antibiotic (e.g., dactinomycin (formerly actinomycin), bleomycin, mithramycin, anthramycin (AMC)), and anti-mitotic agents (e.g., vincristine, vinblastine) (see e.g., Seattle Genetics US 2009/0304721), a benzodiazepine compound (e.g. a pyrrolobenzodiazepine or indolinobenzodiazepines), a taxoid, CC-1065, CC-1065 analog, duocarmycin, duocarmycin analog, enediyne (such as calicheamicin), a dolastatin or dolastatin analog (e.g. auristatin), a tomaymycin derivative, a leptomycin derivative, adriamicin, cisplatin, carboplatin, etoposide, melphalan, chlorambucil, calicheamicin, taxanes (see WO 01/038318 and WO 03/097625), DNA-alkylating agents (e.g., CC-1065 or a CC-1065 analog), anthracyclines, tubulysin analogs, cytochalasin B, gramicidin D, ethidium bromide, emetine (including derivatives thereof). Further details about cytotoxic payloads for ADCs can for example be found in Cytotoxic Payloads for Antibody-Drug Conjugates (Drug Discovery, Band 71), 1st edition (2019), editors Thurston and Jackson, Royal Society of Chemistry (U.K.).

Examples for cytotoxic agents that are radioactive isotopes are At²¹¹, I¹³¹, I¹²⁵, Y⁹⁰, Re¹⁸⁶, Re¹⁸⁸, Sm¹⁵³, Bi²¹², P³², P²¹², Zr⁸⁹ and radioactive isotopes of Lu.

Cytotoxic agents may achieve cell killing by different mechanisms and thus divided into different classes according to their mechanism of action (Nicolaou et al., Accounts of Chemical Research (2019), vol. 52(1), p. 127-139). In some embodiments, the cytotoxic agent included in the ADC of the present disclosure is selected from the group consisting of an inhibitor of microtubule formation, an EG5 inhibitor and a DNA damaging agent (e.g. Anderl et al., Methods in Molecular Biology (2013), vol. 1045, p. 51-70).

An "inhibitor of microtubule formation", as used herein, is an inhibitor that acts by inhibiting tubulin polymerization or microtubule assembly, and thus has anti-proliferative/toxic effects on cells. In a preferred embodiment, said inhibitor of microtubule formation is selected from the group consisting of an auristatin (preferably auristatin E, MMAE or MMAF), a maytansinoid (preferably maytansin, DM1, DM2, DM3 or DM4) and tubulysin.

An "EG5 inhibitor", as used herein, is an inhibitor that inhibits the protein EG5, and thus is toxic to cells. EG5 refers to member 11 of the human kinesin family, which is also known as KIF11, HKSP, KNSL1 or TRIP5. EG5 inhibitors are for example those described in Macroni et al., Molecules (2019), vol. 24(21), p. 3948 or Karpov et al., ACS Medicinal Chemistry Letters (2019), vol. 10(12), p. 1674-1679. In a preferred embodiment, said EG5 inhibitor is selected from the group consisting of structures described in ispenisib, filanesib, litronesib and K858 (Chen et al., ACS Chem Biol. (2017), vol. 12(4), p. 1038-1046).

A "DNA damaging agent", as used herein, is an agent that acts to damage cellular DNA, e.g. by inducing double-strand breaks, cross-linking specific sites of DNA or intercalating between DNA base pairs. In a preferred embodiment, said DNA damaging agent is selected from the group consisting of a topoisomerase I inhibitor, a topoisomerase II inhibitor and a DNA alkylating agent. In some embodiments, said cytotoxic agent is a topoisomerase I inhibitor. In some embodiments, said cytotoxic agent is a topoisomerase II inhibitor. In some embodiments, said cytotoxic agent is a DNA alkylating agent. Preferably, said topoisomerase I inhibitor is selected from the group consisting of exatecan, camptothecin, SN38, Dxd and variants thereof, wherein, preferably, said topoisomerase I inhibitor is exatecan, SN38 or Dxd. Preferably, said topoismerase II inhibitor is doxorubicin or a variant thereof, preferably doxorubicin. Preferably, said DNA alkylating agent is selected from the group consisting of duocarmycin, a CBI dimer, a pyrrolobenzodiazepine and variants thereof, wherein, preferably, said DNA alkylating agent is selected from the group consisting of duocarmycin, a CBI dimer and a diazepine (preferably a pyrrolobenzodiazepine or indolinobenzodiazepine).

In some embodiments, the cytotoxic agent is an exatecan, a duocarmycin or a CBI dimer.

In some embodiments, the therapeutic agent is selected from the group consisting of auristatin, MMAE (monomethyl auristatin E), duocarmycin, CBI (Cyclopropanebenz[e]indoline) dimer, maytansin, pyrrolobenzodiazepine and indolinobenzodiazepine. In some embodiments, the therapeutic agent is selected from the group consisting of an auristatin, a duocarmycin, a CBI (Cyclopropanebenz[e]indoline) dimer and a maytansinoid. In some embodiments, the therapeutic agent is selected from the group consisting of MMAE (monomethyl auristatin E), duocarmycin, CBI (Cyclopropanebenz[e]indoline) dimer and maytansinoid DM4.

In some embodiments, the therapeutic agent is selected from the group consisting of a dolastatin, an auristatin, MMAE, MMAF, amberstatin 269, auristatin 101, auristatin f, auristatin w, CEN-106, CM1, DGN462, DGN549, DM1, DM2, DM4, doxorubicin, duocarmycin, exatecan, OX-4235, PNU-159682, rapamycin, SG3199, SG1882, SN-38, tubulysin, amanitin, aminopterin, anthracycline, calicheamicin, camptothecin, fujimycin, hemiasterlin, a maytansinoid, PBD, rapamycin, vinblastine.

In some embodiments, the therapeutic agent is an anti-inflammatory agent.

As used herein, an "anti-inflammatory agent" is a substance that reduces inflammation. This means that said anti-inflammatory agent results in the reduction of an undesired inflammation as compared to the administration of a control molecule that does not include said anti-inflammatory agent. By recruiting the anti-inflammatory agent to specific immune cells as target cells, the anti-inflammatory effects can be focused to the site of inflammation (where these immune cells may be enriched) or to a specific type of immune cell.

Preferably, the anti-inflammatory agent may be a glucocorticoid receptor agonist.

In some embodiments, said anti-inflammatory agent is a steroid, preferably, selected from the group consisting of cortisol, cortisone acetate, beclometasone, prednisone, prednisolone, methylprednisolone, betamethasone, trimcinolone, budesonide, dexamethasone, fluticasone, fluticasone propionate, fluticasone furoate and a mometasone.

In some embodiments, aid anti-inflammatory agent is a non-steroidal anti-inflammatory agent, e.g. a Cox2 inhibitor.

In some embodiments, the therapeutic agent is an immunostimulatory agent.

As used herein, an "immunostimulatory agent" is a substance that enhances the development or maintenance of an immunologic response. The immunostimulatory agent may be an agonist of an immunostimulatory molecule or an antagonists of a molecule inhibiting an immunologic response. In some embodiments, the immunostimulatory agent comprises an agonist of an immunostimulatory molecule, such as an agonist of a costimulatory molecule found on immune cells such (as T cells) or an agonist of a costimulatory molecule found on cells involved in innate immunity (such as NK cells). In some embodiments, the immunostimulatory agent comprises an antagonist of an immunosuppressive molecule, e.g. an antagonist of a cosuppressive molecule found on cells involved in innate immunity (such as NK cells). Preferably, administration of an ADC with an immunostimulatory agent as payload results in an improvement of a desired immune response. In some embodiments, administration of an ADC with an immunostimulatory agent as payload results in an improved anti-tumor response in an animal cancer model, such as a xenograft model, as compared to the administration of a control molecule that does not include said immunostimulatory agent.

In some embodiments, the immunostimulatory agent is or comprises an antagonist of an inhibitor of T cell activation. In some embodiments, the immunostimulatory agent is or comprises an agonists of a stimulant of T cell activation. In some embodiments, the immunostimulatory agent is or comprises an agent that antagonizes or prevents cytokines that inhibit T cell activation, such as IL-6, IL-10, TGFβ, VEGF. In some embodiments, the at least one immunostimulatory agent comprises an antagonist of a chemokine such as CXCR2, CXCR4, CCR2 or CCR4. In some embodiments, the immunostimulatory agent is or comprises an agonist of a cytokine that stimulates T cell activation, such as IL-2, IL-7, IL-12, IL-15, IL-21 and IFNα.

In preferred embodiments, the immunostimulatory agent is selected from the group consisting of a TLR7 agonist, a TLR8 agonist, a TLR7 antagonist, a TLR8 antagonist, a Sting inhibitor, a TGF beta inhibitor, an a2A inhibitor and an a2B inhibitor.

In some embodiments, the therapeutic agent is an immunosuppressive agent.

As used herein, an "immunosuppressive agent" is an agent that inhibits the development or maintenance of an immunologic response. Such inhibition by an immunosuppressive agent can be effected by, for example, elimination of immune cells (e.g., T or B lymphocytes); induction or generation of immune cells that can modulate (e.g., down-regulate) the functional capacity of other cells; induction of an unresponsive state in immune cells (e.g., anergy); or increasing, decreasing or changing the activity or function of immune cells, including, for example, altering the pattern of proteins expressed by these cells (e.g., altered production and/or secretion of certain classes of molecules such as cytokines, chemokines, growth factors, transcription factors, kinases, costimulatory molecules or other cell surface receptors, and the like). In typical embodiments, an immunosuppressive agent has a cytotoxic or cytostatic effect on an immune cell that promotes an immune response. In some embodiments, said immunosuppressive agent results in the reduction of an undesired immune response as compared to the administration of a control molecule that does not include said immunosuppressive agent.

"Immune cell", as used herein, means any cell of hematopoietic lineage involved in regulating an immune response against an antigen (e.g., an autoantigen), such as a T cell (T lymphocyte), a B cell (B lymphocyte) or a dendritic cell. Preferably, an immune cell according to the present disclosure is a T cell or B cell.

In preferred embodiments, the immunosuppressive agent according to the present disclosure is selected from the group consisting of an IMDH (inosine monophosphate dehydrogenase) inhibitor, an mTor (mechanistic target of rapamycin) inhibitor, a SYK (spleen tyrosine kinase) inhibitor, a JAK (janus kinase) inhibitor and a calcineurin inhibitor.

In some embodiments of the antibody-drug conjugate according to the present disclosure, the payload is a detectable label.

As used herein, "detectable label" refers to a molecule capable of detection (i.e. capable of detection using detection methods known in the art, e.g. detection methods based on radiography, fluorescence, chemiluminescence, enzymatic activity or absorbance).

An ADC with a detectable label as payload can be useful for diagnosing a disease, identifying the site of a disease, or for monitoring or prognosing the onset, development, progression and/or severity of a disease or disorder as part of a clinical testing procedure, such as determining the efficacy of a particular therapy. There are even situations where a detectable label and a therapeutic agent can be used in combination (e.g. Rondon and Degoul, Bioconjugate Chemistry (2020), vol. 31(2), p. 159-173).

A detectable label as payload may e.g. be an enzyme (such as horseradish peroxidase, alkaline phosphatase, beta-galactosidase, or acetylcholinesterase), a prosthetic group (such as streptavidin/biotin and avidin/biotin), a fluorescent material (such as Alexa Fluor^{®} 350, Alexa Fluor^{®} 405, Alexa Fluor^{®} 430, Alexa Fluor^{®} 488, Alexa Fluor^{®} 500, Alexa Fluor^{®} 514, Alexa Fluor^{®} 532, Alexa Fluor^{®} 546, Alexa Fluor^{®} 555, Alexa Fluor^{®} 568, Alexa Fluor^{®} 594, Alexa Fluor^{®} 610, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, umbelliferone, fluorescein, fluorescein isothiocyanate, rhodamine, dichlorotriazinylamine fluorescein, dansyl chloride or phycoerythrin), a luminescent material (such as luminol), a bioluminescent material (such as luciferase, luciferin or aequorin), a radioactive material (such as iodine (¹³¹I, ¹²⁵I, ¹²³I or ¹²¹I), carbon (¹⁴C), sulfur (³⁵S), tritium (³H), indium (¹¹⁵In, ¹¹³In, ¹¹²In or ¹¹¹In), technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ⁶⁴Cu, ¹¹³Sn, and ¹¹⁷Sn), a positron-emitting metal (for positron emission tomography), or a non-radioactive paramagnetic metal ion. Alternatively, a detectable label can for example be a fluorophore, a spin label, an infrared probe, an affinity probe, a spectroscopic probe, a radioactive probe, or a quantum dot.

In some embodiments, the detectable label is a radioisotope, fluorophore, chromophore, enzyme, dye, metal ion, ligand (such as biotin, avidin, streptavidin or hapten) or quantum dot.

In some embodiments, the detectable label is a radioisotope, fluorescent compound or enzyme.

In some embodiments, the detectable label is selected from the group consisting of a cyanine dye, a sulfo-cyanine dye, an Alexa Fluor^{®} dye (Molecular Probes/Thermo Fisher Scientific), a DyLight^{®} Fluor dye (Dyomics/Thermo Fisher Scientific), FluoProbes^{®} dyes (Interchim), a Seta^{®} dye (SETA BioMedicals) and an IRIS^{™} dyes (Cyanine Technologies). Preferably, said detectable label is a cyanine dye or sulfo-cyanine dye.

In some embodiments, the detectable label is a cyanine dye selected from the group consisting of Cy2, Cy3, Cy3B, Cy3.5, Cy5, Cy5.5, Cy7.

In some embodiments, the detectable label is a sulfo-cyanine dye selected from the group consisting of sulfo-Cy2, sulfo-Cy3, sulfo-Cy3B, sulfo-Cy3.5, sulfo-Cy5, sulfo-Cy5.5, sulfo-Cy7.

### Linker

As pointed out above, the antibody-drug conjugate according to the present disclosure comprises a linker. The linker is a molecular group that covalently links the payload and the antibody component of the ADC.

A variety of linkers that can be used for the ADC of the present disclosure and related methods are described in WO 2004/010957 entitled "Drug Conjugates and Their Use for Treating Cancer, An Autoimmune Disease or an Infectious Disease".

Typically, there will be one linker per payload (i.e. one linker molecule for each individual occurrence of a payload in an ADC; this means that, if two copies of a payload are present in an ADC, there will be two linkers, wherein the first linker covalently links the first payload to the antibody component, and the second linker covalently links the second payload to the antibody component). However, it is also possible that one linker links more than one payload moiety to the antibody component of the ADC.

Depending on the number of payloads to be linked to the antibody component and on the number of payloads per linker, there may be one or more linkers in an ADC.

Covalent linking of the antibody component and the payload via a linker can for example be achieved by a linker having two reactive functional groups (i.e. a linker that is bivalent in a reactive sense). Bivalent linker reagents which are useful to attach two or more functional or biologically active components are known to the skilled person (see e.g. Hermanson, Bioconjugate Techniques (1996), Academic Press (New York), p 234-242).

Alternatively, a linker-payload construct comprising payload(s) covalently attached to a linker may be prepared by methods of organic synthesis. Subsequently, one or more copies of this linker-payload construct can then be conjugated to the antibody component by methods known to the skilled person (see e.g. Behrens et al., Molecular Pharmaceutics (2015), vol. 12(11), p. 3986-3998; Stefano, Methods in Molecular Biology (2013), vol. 1045, p. 145-171; Dickgiesser et al., in: Methods in Molecular Biology: Enzyme-Mediated Ligation Methods (2019), editors Nuijens and Schmidt, vol. 2012, p. 135-149; Dickgiesser et al., Bioconjugate Chem. (2020), vol. 31(4), p. 1070-1076) and described in Example 3 below.

A linker in the antibody-drug conjugate of the present disclosure is preferably stable extracellularly (i.e. outside of the cell, e.g. in plasma). Thus, before transport or delivery into a cell, the ADC is preferably stable and remains intact, i.e. the antibody remains linked to the payload. An effective linker will: (i) maintain the specific binding properties of the antibody; (ii) allow intracellular delivery of the payload; (iii) remain stable and intact, i.e. not cleaved, until the conjugate has been delivered or transported to its targeted site; and (iv) maintain the therapeutic efficacy of the payload (e.g. the cytotoxic, cell-killing effect of the payload).

Whether a linker is stable in the extracellular environment can be determined, for example, by incubating independently with plasma both (a) the ADC (the "ADC sample") and (b) an equal molar amount of unconjugated antibody or therapeutic agent (the "control sample") for a predetermined time period (e.g. 8 hours) and then comparing the amount of unconjugated antibody or therapeutic agent present in the ADC sample with that present in the control sample, as measured, for example, by high performance liquid chromatography.

A linker that is stable outside of the target cell may be cleaved at some efficacious rate inside the target cell. In some embodiments, the linker that is cleavable under intracellular conditions is cleavable by a cleaving agent that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. Typically, the peptidyl linker is at least two amino acids long or at least three amino acids long. Cleaving agents can include cathepsins B and D and plasmin, all of which are known to hydrolyze dipeptide drug derivatives resulting in the release of active drug inside target cells (see e.g. Dubowchik and Walker, Pharm. Therapeutics (1999), vol. 83, p. 67-123). For example, a peptidyl linker that is cleavable by the thiol-dependent protease cathepsin B, which is highly expressed in cancerous tissue, can be used (e.g. a Phe-Leu or a Gly-Phe-Leu-Gly linker). Other such linkers are described e.g. in U.S. Patent 6,214,345. In specific embodiments, the peptidyl linker cleavable by an intracellular protease is a Val-Cit linker or a Phe-Lys linker (see e.g. U.S. Patent 6,214,345, which describes the synthesis of doxorubicin with the Val-Cit linker). One advantage of using intracellular proteolytic release of a therapeutic agent is that the agent is typically attenuated when conjugated and the serum stabilities of the conjugates are typically high.

In other embodiments, the cleavable linker is pH-sensitive, i.e., sensitive to hydrolysis at certain pH values. Typically, the pH-sensitive linker is hydrolyzable under acidic conditions. For example, an acid-labile linker that is hydrolyzable in the lysosome (e.g. a hydrazone, semicarbazone, thiosemicarbazone, cis-aconitic amide, orthoester, acetal, ketal, or the like) can be used (see e.g. U.S. Patent 5,122,368; U.S. Patent 5,824,805; U.S. Patent 5,622,929; Dubowchik and Walker, Pharm. Therapeutics (1999), vol. 83, p. 67-123; Neville et al., Biol. Chem. (1989), vol. 264, p. 14653-14661). Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as a thioether attached to the therapeutic agent via an acylhydrazone bond, see e.g. U.S. Patent 5,622,929).

In yet other embodiments, the linker is cleavable under reducing conditions (e.g., a disulfide linker). A variety of disulfide linkers are known in the art, including, for example, those that can be formed using SATA (N-succinimidyl-5-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene), SPDB and SMPT (see e.g. Thorpe et al., Cancer Res. (1987), vol. 47, p. 5924-5931; Wawrzynczak et al., in: Immunoconjugates: Antibody Conjugates in Radioimagery and Therapy of Cancer (1987), editor Vogel, Oxford U. Press; U.S. Patent 4,880,935).

In other embodiments, the linker is not cleavable inside the target cell, but the payload is released, for example, by antibody degradation.

In yet other specific embodiments, the linker is a malonate linker (Johnson et al., Anticancer Res. (1995), vol. 15, p. 1387-1393), a maleimidobenzoyl linker (Lau et al., Bioorg-Med-Chem. (1995), vol. 3(10), p. 1299-1304), or a 3'-N-amide analog (Lau et al., Bioorg-Med-Chem. (1995), vol. 3(10), p. 1305-1312).

The linker may be cleavable under intracellular conditions (as described above) or not cleavable under intracellular conditions. In some embodiments, the linker is not cleavable under intracellular conditions. In other embodiments, the linker is cleavable under intracellular conditions. Such a linker is particularly preferred if the payload is a therapeutic agent. Preferably, the linker is cleavable under intracellular conditions, such that cleavage of the linker releases the payload from the antibody component in the intracellular environment.

Whether the linker of an ADC is stable or cleaved may be examined by exposing the ADC to the conditions to be tested and then verifying the integrity of the linker in the treated sample and an untreated control sample by standard analytical techniques such as mass spectroscopy, HPLC, and the separation/analysis technique LC/MS.

In some embodiments, said linker/each of said linkers has a molecular weight of up to 1,500 Da, preferably up to 1,000 Da, more preferably up to 500 Da.

In some embodiments, said linker is/said linkers are stable in the extracellular environment.

The designation that a linker is "stable in the extracellular environment" preferably means that said linker is stable in human serum. A linker is "stable in human serum" if in an assay in which ADC molecules including the linker are exposed to human serum, after an incubation of 48 h at 37 °C at least 50%, preferably at least 75% of the linkers in the ADCs have been neither cleaved nor degraded.

In some embodiments, said linker is/said linkers are stable in the intracellular environment.

Preferably, a linker that is "stable in the intracellular environment" is a linker that has such a structure that if ADC molecules including the linker are taken up by cells (i.e. enter into the intracellular environment of the cells), after an incubation of 24 h at 37 °C at least 50%, preferably at least 75% of the linkers in the ADC molecules have been neither cleaved nor degraded.

In some embodiments, said linker is/said linkers are cleaved upon exposure to the intracellular environment.

Preferably, a linker that is "cleaved upon exposure to the intracellular environment" is a linker that has such a structure that if ADC molecules including the linker are taken up by cells (i.e. enter into the intracellular environment of the cells), the linkers in the ADC molecules are cleaved efficiently (preferably at least 90% of the linkers are cleaved within 24 h, more preferably within 12 h). As the skilled person understands, this allows for release of the payload into the target cells.

In some embodiments, said linker is/said linkers are stable in the extracellular environment, but cleaved upon exposure to the intracellular environment.

Preferably, a linker that is "stable in the extracellular environment, but cleaved upon exposure to the intracellular environment" is preferably a linker that is stable in human serum, but has such a structure that if ADC molecules including the linker are taken up by cells (i.e. enter into the intracellular environment of the cells), the linkers in the ADC molecules are cleaved efficiently (preferably at least 90% of the linkers are cleaved within 24 h, more preferably within 12 h). As the skilled person understands, this allows for release of the payload into the target cells.

In some embodiments, said linker is/said linkers are cleavable by enzymatic or chemical cleavage.

As used herein, a linker that is "cleavable by enzymatic cleavage" is a linker that is cleaved in the presence of a certain enzyme, but stable in the absence of this enzyme. For the purposes of the ADC of the present disclosure, this enzyme will typically be an enzyme that the ADC is not exposed to in the extracellular environment, but exposed to upon uptake of the ADC into the target cell, resulting in a linker that is extracellularly stable, but cleaved upon entry into the target cell.

As used herein, a linker that is "cleavable by chemical cleavage" is a linker that is cleaved by a non-enzymatic reaction that results in the breakage of a covalent chemical bond. Examples are linkers that are pH-sensitive or cleavable under reducing conditions (see above).

In some embodiments, said linker is/said linkers are cleavable by enzymatic cleavage.

In some embodiments, said enzymatic cleavage is cleavage by exposure to a glycosidase, protease or esterase.

A glycosidase is an enzyme of E.C. (Enzyme classification) 3.2.1 that catalyzes the hydrolysis of glycosidic bonds in complex sugars. A protease is an enzyme of E.C. 3.4 that catalzyes the cleavage of peptide bonds. An esterase is an enzyme of E.C. 3.1 that catalyzes the cleavage of ester bonds.

Preferably, said glycosidase is a glucuronidase. A glucuronidase is an enzyme of E.C. 3.2.1.31 that catalzyes the cleavage of β-Glucuronides.

Preferably, said protease is a cathepsin (most preferably cathepsin B). Cathepsins are a group of proteases within E.C. 3.4 that catalyze the proteolytic cleavage of peptide bonds. For the purpose of the present disclosure, the use of a lysosomal, endoproteolytic cathepsin is particularly advantageous, since these become activated at low pH (as in lysosomes) and cleave within a peptide sequence. Cathepsin B is a cathepsin classified as E.C. 3.4.22.1.

In some embodiments, said enzymatic cleavage is by exposure to a tumor-specific enzyme, preferably a tumor-specific protease or esterase. A "tumor-specific" enzyme is an enzyme that is present in a certain tumor (i.e. there is enzymatic activity of said enzyme in the tumor), whereas the enzyme is substantially absent from other cells and tissues (i.e. outside of said tumor substantially no, preferably no, enzymatic activity of said enzyme).

In some embodiments, said linker includes/said linkers include a protease cleavage site, preferably a cathepsin B cleavage site.

In some embodiments, said linker includes/said linkers include a glucuronide (which is a molecular group that can be cleaved by glucuronidase).

In some embodiments, said linker/said linkers are is cleavable by chemical cleavage.

In some embodiments, said linker that is cleavable by chemical cleavage is a pH-sensitive linker/said linkers that are cleavable by chemical cleavage are pH-sensitive linkers. Preferably, said linker includes/said linkers include a hydrazone.

In some embodiments, said linker that is cleavable by chemical cleavage is cleavable under reducing conditions/wherein said linkers that are cleavable by chemical cleavage are cleavable under reducing conditions. Preferably, said linker includes/said linkers include a disulfide linkage.

In some embodiments, said linker comprises/said linkers comprise a cathepsin B cleavage site, a glucuronide or a disulfide linkage.

### Solubility tag

The antibody-drug conjugate according to the present disclosure (resp. the "molecule" as defined above) comprises a solubility tag.

As a skilled person understands, a "solubility tag" is a molecular group linked to a molecule of interest that has the purpose of increasing the solubility of the molecule of interest in aqueous environment, compared to the same molecule of interest without the solubility tag. Thus, it is intended that the molecule with the solubility tag linked to it has a higher solubility in aqueous environment than the same molecule without the solubility tag linked to it.

The solubility tag of the present disclosure is based on an oligosaccharide. As shown in the examples of the present disclosure, inclusion of such a solubility tag in an ADC results in various advantageous effects.

The ADC according to the present disclosure may comprise one or more than one solubility tag per ADC molecule. Typically, the solubility tag(s) will be covalently attached to the antibody-drug conjugate.

Typically, in the solubility tag will be linked to the ADC of the present disclosure by a covalent bond between the solubility tag and the linker. However, the solubility tag can also be linked to the ADC by a covalent bond between the solubility tag and a component of the ADC other than the linker.

If the present disclosure states that a certain molecule or moiety A is attached/linked "by a covalent bond" to another molecule or moiety B, this indicates that the molecule/moiety A is directly linked to said molecule/moiety B by a chemical bond, without further atoms or molecular groups between molecule/moiety A and molecule/moiety B.

In some embodiments, said solubility tag is/said solubility tags are linked by a covalent bond to the targeting moiety, the functional moiety/moieties or the linker(s).

In some embodiments, said solubility tag is/said solubility tags are linked by a covalent bond to the targeting moiety.

In some embodiments, said solubility tag is/said solubility tags are linked by a covalent bond to the functional moiety/moieties.

In some embodiments, said solubility tag is/said solubility tags are linked by a covalent bond to the linker(s).

In some embodiments, said solubility tag is/said solubility tags are linked by a covalent bond to the antibody component, the at least one payload or the linker(s). In some embodiments, said solubility tag is/said solubility tags are linked by a covalent bond to said antibody component. In some embodiments, said solubility tag is/said solubility tags are linked by a covalent bond to said at least one payload. In some embodiments, said solubility tag is/said solubility tags are linked by a covalent bond to said linker(s).

In some embodiments, said solubility tag is/said solubility tags are linked by a covalent bond
- to the antibody component, but not to the at least one payload or the linker(s), or
- to the at least one payload, but not to the antibody component or the linker(s), or
- to the linker(s), but not to the antibody component or the at least one payload.

In some embodiments, said solubility tag is/said solubility tags are linked by a covalent bond only to the antibody component (but not to the at least one payload or the linker(s)). In some embodiments, said solubility tag is/said solubility tags are linked by a covalent bond only to the at least one payload (but not to the antibody component or the linker(s)). In some embodiments, said solubility tag is/said solubility tags are linked by a covalent bond only to the linker(s) (but not to the antibody component or the at least one payload).

In some embodiments, said solubility tag is/said solubility tags are linked by a covalent bond to at least one of
(i) the antibody component,
(ii) the at least one payload,
(iii) the linker/linkers covalently linking said payload/payloads and said antibody component
of the ADC according to the present disclosure.

In some embodiments, the antibody component (i), the payload(s) (ii) and the linker(s) (iii) are covalently linked.

In some embodiments, the antibody component (i), the payload(s) (ii), the linker(s) (iii) and the solubility tag(s) (iv) are covalently linked.

The antibody-drug conjugate according to the present disclosure can comprise one or more solubility tags.

In some embodiments, said molecule comprises only one solubility tag. In some embodiments, said molecule comprises at least one, preferably at least 2, more preferably at least 3, more preferably at least 4 solubility tags. In some embodiments, said molecule comprises up to 10, preferably up to 8, more preferably up to 6, more preferably up to 4, more preferably up to 2 solubility tags, more preferably only one solubility tag. In some embodiments, said molecule comprises at least 1 and up to 4 solubility tags. In some embodiments, said molecule comprises at least 3 and up to 10 solubility tags.

In some embodiments, at least one solubility tag is covalently linked to said molecule. In other embodiments, at least 2, preferably at least 3, more preferably at least 4 solubility tags are covalently linked to said molecule. In some embodiments, up to 10, preferably up to 6, more preferably up to 4, more preferably up to 2 solubility tags are covalently linked to said molecule, more preferably only 1 solubility tag is covalently linked to said molecule. In some embodiments, at least 1 and up to 4 solubility tags are covalently linked to said molecule. In some embodiments, at least 3 and up to 10 solubility tags are covalently linked to said molecule. As a skilled person will understand, the number of solubility tags covalently linked to the molecule is an average number (which is determined over a population of said molecules). Preferably, said population is a homogeneous population.

In some embodiments, said antibody-drug conjugate comprises at least one solubility tag. In some embodiments, said antibody-drug conjugate comprises at least 2, preferably at least 3, more preferably at least 4 solubility tags. In some embodiments, said antibody-drug conjugate comprises up to 10, preferably up to 8, more preferably up to 6, more preferably up to 4, more preferably up to 2 solubility tags. In some embodiments, said antibody-drug conjugate comprises at least 1 and up to 4 solubility tags. In some embodiments, said antibody-drug conjugate comprises at least 3 and up to 10 solubility tags. In some embodiments, said antibody-drug conjugate comprises only one solubility tag.

In some embodiments, at least one solubility tag is covalently linked to said antibody-drug conjugate. In other embodiments, at least 2, preferably at least 3, more preferably at least 4 solubility tags are covalently linked to said antibody-drug conjugate. In some embodiments, only one, preferably up to 2, more preferably up to 4, more preferably up to 6, more preferably up to 8, more preferably up to 10 solubility tags are covalently linked to said antibody-drug conjugate. In some embodiments, at least 1 and up to 4 solubility tags are covalently linked to said antibody-drug conjugate. In some embodiments, at least 3 and up to 10 solubility tags are covalently linked to said antibody-drug conjugate. As a skilled person will understand, the number of solubility tags covalently linked to the antibody-drug conjugate is an average number (which is determined over a population of molecules of the ADC). Preferably, said population is a homogeneous population.

In some embodiments, not more than three solubility tags are covalently linked per linker. In preferred embodiments, not more than two solubility tags are covalently linked per linker. In more preferred embodiments, not more than one solubility tag is covalently linked per linker.

In some embodiments, not more than three solubility tags are covalently linked per payload. In preferred embodiments, not more than two solubility tags are covalently linked per payload. In more preferred embodiments, not more than one solubility tag is covalently linked per payload.

In some embodiments, not more than three solubility tags are covalently linked per antibody component. In preferred embodiments, not more than two solubility tags are covalently linked per antibody component. In more preferred embodiments, not more than one solubility tag is covalently linked per antibody component.

In preferred embodiments, only one kind of solubility tag is covalently attached to the antibody-drug conjugate. This means that all solubility tags covalently attached to the antibody-drug conjugate are identical (they are of the same kind with regard to their molecular structure).

In some embodiments, more than one kind of solubility tag is covalently attached to said antibody-drug conjugate. This means that there are at least two different types of solubility tags with different structure covalently attached to the antibody-drug conjugate (i.e. the solubility tags covalently attached to the ADC are of more than one kind with regard to their molecular structure).

In some embodiments, up to two kinds of solubility tags are covalently attached to said antibody-drug conjugate.

In preferred embodiments, said antibody-drug conjugate comprises only one kind of solubility tag. This means that all solubility tags comprised by the antibody-drug conjugate are identical (they are of the same kind with regard to their molecular structure).

In some embodiments, said antibody-drug conjugate comprises more than one kind of solubility tag. This means that the antibody-drug conjugate comprises at least two different types of solubility tags with different structure (i.e. the solubility tags covalently attached to the ADC are of more than one kind with regard to their molecular structure).

In some embodiments, said antibody-drug conjugate comprises up to two different kinds of solubility tags.

Typically, the solubility tag of the ADC of present disclosure will comprise/consist of monosaccharide units that are linked by covalent bonds.

In some embodiments, said solubility tag comprises/said solubility tags comprise monosaccharide units. In some embodiments, said solubility tag consists of/said solubility tags consist of monosaccharide units.

As used herein, a "monosaccharide" is a sugar that is not decomposable into simpler sugars by hydrolysis, is classed as either an aldose or ketose, and contains one or more hydroxyl groups

(-OH) per molecule. Examples of monosaccharides include glucose (dextrose), fructose (levulose), and galactose. Monosaccharides are the building blocks of disaccharides (such as sucrose and lactose), oligosaccharides, and polysaccharides (such as cellulose and starch). The present disclosure uses the term "monosaccharide" or "monosaccharide unit" to refer to a single monosaccharide residue in an oligosaccharide. Within the context of an oligosaccharide, an monosaccharide unit is a monosaccharide that is linked to another monosaccharide via covalent bond formed by a hydroxyl group of said monosaccharide (e.g. a glycosidic bond).

As used herein, "oligosaccharide" refers to a compound containing two or more monosaccharide units. Preferably, the term "oligosaccharide" refers to a compound containing 2-12 monosaccharide units connected by glycosidic bonds. In accordance with accepted nomenclature, oligosaccharides are depicted herein with a non-reducing end on the left and a reducing end on the right.

Monosaccharides and oligosaccharides can be chemically synthesized by standard methods of carbohydrate chemistry (see e.g. Preparative Carbohydrate Chemistry (1997), editor Hanessian, publisher Marcel Dekker, Inc. (New York); Carbohydrate Chemistry: Proven Synthetic Methods (2015), editors Roy and Vidal, CRC Press; Carbohydrate Chemistry: State of the Art and Challenges for Drug Development (2016), editor Cipolla, Imperial College Press (London); CRC Handbook of Oligosaccharides (1990), Vol. I-III, (published 2019), editors: Liptak et al., CHR Press, Inc.; Liaqat and Eltem, Carbohydrate Polymers (2018), vol. 184, p. 243-259).

Alternatively, oligosaccharides can be prepared by biotechnological methods (see e.g. Meyer et al., Biotechnological Production of Oligosaccharides - Applications in the Food Industry, Food Production and Industry (2015), Ayman Hafiz Amer Eissa, IntechOpen, DOI: 10.5772/60934; Liaqat and Eltem, Carbohydrate Polymers (2018), vol. 184, p. 243-259; Samain et al., Carbohydrate Research (1997), vol. 302, p. 35-42; Samain et al., Biotechnol. (1999), vol. 72, p. 33-47).

To further increase the purity of oligosaccharides prepared by the above-described approaches, the oligosaccharide can be purified by standard methods of organic chemistry, including e.g. precipitation, re-crystallization, ultrafiltration, nanofiltration, gel permeation chromatography, ion exchange chromatograpy, capillary electrophoresis, HPLC purification, UPLC purification, or membrane and carrier approaches as described e.g. in Pinelo et al., Separation and Purification Technology (2009), vol. 70(1), p. 1-11.

Monosaccharides and oligosaccharides can be characterized by standard methods known to a person of skill in the art (see e.g. Carbohydrate Chemistry (1988), editor El Khadem, Academic Press (San Diego)). This includes, for example, LC-MS/ESI MS methods, 1D and 2D NMR, gel permeation chromatography or ion mobility-mass spectrometry (Seeberger et al., Nature (2015), vol. 526(7572), p. 241-244).

In some embodiments, said solubility tag comprises/said solubility tags comprise an oligosaccharide consisting of monosaccharide units. In some embodiments, said solubility tag consists of/said solubility tags consist of an oligosaccharide consisting of monosaccharide units.

In some embodiments, the solubility tag comprises/each solubility tag comprises up to 25, preferably up to 20, more preferably up to 15, more preferably up to 12, more preferably up to 10, more preferably up to 9, more preferably up to 8, more preferably up to 7, more preferably up to 6, more preferably up to 5 monosaccharide units.

In some embodiments, the solubility tag comprises/each solubility tag comprises at least 2, preferably at least 3, more preferably at least 4, more preferably at least 5 monosaccharide units.

In some embodiments, the solubility tag comprises/each solubility tag comprises 5 monosaccharide units.

In some embodiments, the solubility tag/each solubility tag consists of 3 to 8, preferably 4 to 8, more preferably 4 to 7, more preferably 4 to 6, more preferably 4 or 5 monosaccharide units.

In some embodiments, the solubility tag/each solubility tag consists of 3 to 8, preferably 4 to 8, more preferably 4 to 7 monosaccharide units.

In a particularly preferred embodiment, the solubility tag/each solubility tag consists of 4 to 6 monosaccharide units.

In some embodiments, the solubility tag/each solubility tag consists of 4 or 5 monosaccharide units. In some embodiments, the solubility tag/each solubility tag consists 5 or 6 monosaccharide units. In some embodiments, the solubility tag/each solubility tag consists of 5 monosaccharide units.

In some embodiments, the monosaccharide units of which said solubility tag/each solubility tag consists are linked by covalent bonds, forming an oligosaccharide.

The solubility tag of the antibody-drug conjugate according to the present disclosure may comprise or consist of a chito-oligosaccharide.

As used herein, the term "chito-oligosaccharide" (abbreviated herein as "CO") refers to oligosaccharides obtained upon hydrolysis of (not deacetylated, partially deacetylated or fully deacetylated) chitin with diluted aqueous mineral acid. This results in a mixture of various chito-oligosaccharides that can be further separated into different chito-oligosaccharide species e.g. by ultrafiltration, gel permeation chromatography, cation exchange chromatography and capillary electrophoresis. The preparation of chito-oligosaccharides by this approach is e.g. described in Schmitz et al., Marine Drugs (2019), vol. 17(8), p. 452.

Chito-oligosaccharides may alternatively be obtained by chemical synthesis (Bohe and Crich, in: Comprehensive Organic Synthesis, 2nd edition (2014), vol. 6, editors Knochel and Molander, Elsevier Ltd.; Solid Support Oligosaccharide Synthesis and Combinatorial Carbohydrate Libraries (2001), editor Seeberger, John Wiley & Sons, Inc.).

As a further alternative, chito-oligosaccharides may be obtained by a biotechnological approach (see e.g. Samain et al., Carbohydrate Research (1997), vol. 302, p. 35-42; Samain et al., Biotechnol. (1999), vol. 72, p. 33-47).

Chito-oligosaccharides may then be further purified and characterized as described above for oligosaccharides in general.

Typically, chito-oligosaccharides are composed of D-glucosamine (GlcN) and/or N-acetyl-D-glucosamine (GlcNAc), resulting in the general formula (GlcNAc)ₘ(GlcN)ₙ., wherein m and n are integer numbers. The monosaccharide units in chito-oligosacharides are typically linked by β-(1,4)-glycosidic linkages.

As used herein, "N-acetyl-D-glucosamine", also referred to as "N-acetylglucosamine" and abbreviated as "GlcNAc", is 2-acetylamino-2-deoxy-D-glucose (also termed 2-acetamido-2-deoxy-D-glucose). Glucose is abbreviated herein as "Glc".

As used herein, D-glucosamine, also referred to as "glucosamine" and abbreviated as "GlcN", is 2-amino-2-deoxy-D-glucose.

Examples of chito-oligosaccharides are summarized in Table 1 below. The CAS^{®} Registry Number to the exact definition of the compound is provided.

**Table 1: Chito-oligosaccharides**

| **Number of mono-saccharide units** | **Chito-oligosaccharide** | **CAS^{®} Registry Number** |
|---|---|---|
| 2 | (GlcN)₂ / Chitobiose | 577-76-4 1160435-72-2 (α-Anomer) 77224-08-9 (β-Anomer) |
| 2 | GlcN-GlcNAc | 245733-17-9 (β-Anomer) |
| 2 | GlcNAc-GlcN | 934746-16-4 (β-Anomer) 1242095-05-1 (α-Anomer) |
| 2 | (GlcNAc)₂ / Di-*N-*acetylchitobiose | 35061-50-8 35991-83-4 (β-Anomer) 34147-27-8 (α-Anomer) |
| 3 | (GlcN)₃ / Chitotriose | 41708-93-4 147780-25-4 (α-Anomer) 133442-53-2 (β-Anomer) |
| 3 | (GlcN)₂-GlcNAc | 138430-54-3 |
| 3 | (GlcNAc)₂-GlcN | 66592-57-2 |
| 3 | GlcN-(GlcNAc)₂ | 138430-53-2 |
| 3 | (GlcNAc)₃ / Tri-*N-*acetylchitotriose | 38864-21-0 13319-32-9 (β-Anomer) 147695-57-6 (α-Anomer) |
| 4 | (GlcN)₄ / Chitotetraose | 5567-52-2 133359-41-8 (β-Anomer) |
| 4 | (GlcN)₃-GlcNAc | 879688-12-7 |
| 4 | (GlcNAc)₂-(GlcN)₂ | 1283726-32-8 |
| 4 | GlcNAc-GlcN-GlcNAc-GlcN | 66748-38-7 |
| 4 | GlcNAc-(GlcN)₂-GlcNAc | 1283726-36-2 |
| 4 | GlcN-(GlcNAc)₂-GlcN | 1283726-38-4 |
| 4 | GlcN-GlcNAc-GlcN-GlcNAc | 1203584-86-4 |
| 4 | (GlcN)₂-(GlcNAc)₂ | 1283726-39-5 |
| 4 | (GlcNAc)₃-GlcN | 66649-50-1 |
| 4 | (GlcNAc)₂-GlcN-GlcNAc | 909107-65-9 |
| 4 | GlcNAc-GlcN-(GlcNAc)₂ | 66649-51-2 |
| 4 | GlcN-(GlcNAc)₃ | 163777-01-3 |
| 4 | (GlcNAc)₄ / Tetra-*N-*acetylchitotetraose | 2706-65-2 53290-51-0 (β-Anomer) 59817-31-1 (α-Anomer) |
| 5 | (GlcN)₅ / Chitopentaose | 41708-94-5 13531-87-8 (β-Anomer) |
| 5 | (GlcN)₃-(GlcNAc)₂ | 207619-75-8 |
| 5 | (GlcNAc)₂-(GlcN)₂-GlcNAc | 1283726-47-5 |
| 5 | GlcNAc-GlcN-GlcNAc-GlcN-GlcNAc | 1283726-46-4 |
| 5 | GlcNAc-(GlcN)₂-(GlcNAc)₂ | 1283726-45-3 |
| 5 | GlcN-(GlcNAc)₂-GlcN-GlcNAc | 1283726-44-2 |
| 5 | GlcN-GlcNAc-GlcN-(GlcNAc)₂ | 890705-26-7 |
| 5 | GlcN-(GlcNAc)₄ | 163777-02-4 |
| 5 | (GlcNAc)₅ / Penta-*N-*acetylchitopentaose | 36467-68-2 175775-91-4 (α-Anomer) 81520-72-1 (β-Anomer) |
| 6 | (GlcN)₆ / Chitohexaose | 41708-95-6 6734-92-5 (β-Anomer) |
| 6 | (GlcN)₂-GlcNAc-(GlcN)₃ | 207619-72-5 |
| 6 | GlcNAc-(GlcN)₄-GlcNAc | 207619-74-7 |
| 6 | GlcN-GlcNAc-GlcN-GlcNAc-(GlcN)₂ | 207619-73-6 |
| 6 | GlcNAc-(GlcN)₂-GlcNAc-GlcN-GlcNAc | 1283726-50-0 |
| 6 | GlcNAc-(GlcN)₃-(GlcNAc)₂ | 1283726-49-7 |
| 6 | GlcN-GlcNAc-GlcN-(GlcNAc)₃ | 1283726-55-5 |
| 6 | GlcNAc-(GlcN)₂-(GlcNAc)₃ | 1283726-53-3 |
| 6 | (GlcNAc)₆ / Hexa-*N-*acetylchitohexaose | 38854-46-5 13319-33-0 (β-Anomer) 174388-75-1 (α-Anomer) |
| 7 | (GlcN)₇ / Chitoheptaose | 68232-35-9 |
| 7 | (GlcNAc)₇ / Hepta-*N-*acetylchitoheptaose | 79127-58-5 |

In some embodiments, the solubility tag/each solubility tag comprises a chito-oligosaccharide.

In preferred embodiments, the solubility tag/each solubility tag is a chito-oligosaccharide (i.e. the solubility tag/each solubility tag consists of a chito-oligosaccharide).

In some embodiments, said chito-oligosaccharide is selected from the chito-oligosaccharides shown in Table 1. Preferably, said chito-oligosaccharide is a chito-oligosaccharide with 3 to 7 monosaccharide units shown in Table 1. More preferably, said chito-oligosaccharide is a chito-oligosaccharide with 4 to 6 monosaccharide units shown in Table 1. More preferably, said chito-oligosaccharide is a chito-oligosaccharide with 4 or 5 monosaccharide units shown in Table 1.

In some embodiments, the monosaccharide units of the solubility tag according to the present disclosure (i.e. the monosaccharide units that are comprised in the solubility tag according to the present disclosure resp. of which the solubility tag according to the present disclosure consists) are independently selected from the group consisting of aldoses, ketoses and chemically modified forms of said aldoses or ketoses.

As used herein, the term "aldose" refers to a monosaccharide that is a polyhydroxy aldehyde with the generic chemical formula Cₙ(H₂O)ₙ (wherein n is an integer number) and a single aldehyde group (-CH=O) per molecule (during ring formation, the aldehyde group reacts with a hydroxyl group to form a hemiacetal). Non-limiting examples of aldoses include aldohexose (all six-carbon, aldehyde-containing sugars, including e.g. glucose, mannose and galactose), aldopentose (all five-carbon aldehyde containing sugars, including e.g. xylose and arabinose) and aldotetrose (all four-carbon, aldehyde containing sugars, including e.g. erythrose).

As used herein, the term "ketose" refers to a monosaccharide that is a polyhydroxy ketone with the generic chemical formula Cₙ(H₂O)ₙ (wherein n is an integer number) and a single ketone group (=O) per molecule (during ring formation, the ketone group reacts with a hydroxyl group to form a hemiketal). Non-limiting examples of ketoses include ketohexose (all six-carbon, ketone-containing sugars, including e.g. fructose), ketopentose (all five-carbon ketone containing sugars, including e.g. xylulose and ribulose) and ketotetrose (all four-carbon, ketose containing sugars, including e.g. erythrulose.

In the present disclosure, aldoses and ketoses as defined above are considered as "unmodified" monosaccharides.

A "chemically modified form" of a monosaccharide designates a monosaccharide that is not an unmodified monosaccharide as defined above and that differs from an unmodified monosaccharide as defined above by addition, removal or substitution of at least one atom or molecular group.

CO-V, the oligosaccharide used in the examples (see structural formula (I)), is lightly modified compared to an unmodified oligosaccharide: GlcN vs. Glc (i.e. replacement of a hydroxyl group in glucose with an amino group) at one occasion and GlcNAc vs. Glc (i.e. replacement of a hydroxyl group in glucose with an N-acetylamino group) at four occasions. As the skilled person is aware, stronger chemical modification compared to the solubility tags in the examples may result in a more pronounced change of the characteristics of the solubility tag compared to the solubility tag in the examples. Thus, while the present disclosure embraces solubility tags with further chemical modification compared to the examples, it is understood that a degree of chemical modification that results in less deviation from the solubility tags in the examples is preferable over a more excessive degree of chemical modification.

In some embodiments, said monosaccharide units are independently selected from the group consisting of aldoses and chemically modified forms of said aldoses.

In some embodiments, said monosaccharide units are individually selected from the group consisting of tetroses, pentoses, hexoses, heptoses, octoses and chemically modified forms of tetroses, pentoses, hexoses, heptoses and octoses. Preferably, said monosaccharide units are individually selected from the group consisting of tetroses, pentoses, hexoses, and chemically modified forms of tetroses, pentoses and hexoses. More preferably, said monosaccharide units are individually selected from the group consisting of pentoses, hexoses and chemically modified forms of pentoses and hexoses. More preferably, said monosaccharide units are selected from the group consisting of hexoses and chemically modified forms of hexoses.

The term "individually selected" indicates that for each individual monosaccharide unit, an independent selection from the listed options can be made. For example, if the monosaccharide units of an oligosaccharide consisting of three monosaccharide units are "individually selected" from the group consisting of pentoses (P) and hexoses (H), the first, second and third monosaccharide unit of said oligosaccharide may each be independently selected from the group consisting of pentoses (P) and hexoses (H). Thus, in this example the combinations PPP, PPH, PHP, PHH, HPP, HPH, HHP and HHH are encompassed.

As the skilled person is aware, tetroses, pentoses, hexoses, heptoses and octoses have the chemical formula Cₙ(H₂O)ₙ, wherein for a tetrose n = 4, for a pentose n = 5, for a hexose n = 6, for a heptose n = 7, and for an octose n = 8. Tetroses, pentoses, hexoses, heptoses and octoses can exist as aldoses or as ketoses.

In some embodiments, said monosaccharide units are individually selected from the group consisting of tetroses, pentoses, hexoses, heptoses and octoses. Preferably, said monosaccharide units are individually selected from the group consisting of tetroses, pentoses and hexoses. More preferably, said monosaccharide units are individually selected from the group consisting of pentoses and hexoses. More preferably, said monosaccharide units are hexoses.

In some embodiments, said monosaccharide units of said solubility tag are not modified by chemical modification.

In some embodiments, said tetroses, pentoses, hexoses, heptoses and octoses have the chemical formula Cₙ(H₂O)ₙ. wherein for tetroses n = 4, for pentoses n = 5, for hexoses n = 6, for heptoses n = 7, and for octoses n = 8 n.

In some embodiments, said aldoses have the chemical formula Cₙ(H₂O)ₙ, wherein preferably n is an integer number and 4 ≤ n ≤ 12, more preferably n is an integer number and 4 ≤ n ≤ 8, more preferably n is an integer number and 5 ≤ n ≤ 7, more preferably n is 5 or 6, more preferably n is 6.

In some embodiments, said ketoses have the chemical formula Cₙ(H₂O)ₙ, wherein preferably n is an integer number and 4 ≤ n ≤ 12, more preferably n is an integer number and 4 ≤ n ≤ 8, more preferably n is an integer number and 5 ≤ n ≤ 7, more preferably n is 5 or 6, more preferably n is 6.

Preferably, said tetroses are individually selected from the group consisting of erythrose and threose.

Preferably, said pentoses are individually selected from the group consisting of ribose, arabinose, xylose and lyxose.

Preferably, said hexoses are individually selected from the group consisting of allose, altrose, glucose, mannose, gulose, idose, galactose and talose.

In some embodiments, said monosaccharide units are D-sugars. The term "D-sugars", as used herein, refers to the Fischer projection known to a skilled person in the field.

In some embodiments, at least one monosaccharide unit of said solubility tag is modified by at least one chemical modification.

In some embodiments, all monosaccharide units of said solubility tag are modified by at least one chemical modification.

In some embodiments, no monosaccharide unit of said solubility tag is modified by more than three, preferably by more than two, more preferably by more than one chemical modifications.

In some embodiments, the average number of chemical modifications per monosaccharide unit of said solubility tag is ≤ 3. Preferably, the average number of chemical modifications per monosaccharide unit of said solubility tag is ≤ 2. More preferably, the average number of chemical modifications per monosaccharide unit of said solubility tag is ≤ 1.5. More preferably, the average number of chemical modifications per monosaccharide unit of said solubility tag is ≤ 1.

As the skilled person understands, the average number of chemical modifications per monosaccharide unit of said solubility tag is calculated by dividing the overall number of chemical modifications on all monosaccharide units of said solubility tag by the number of monosaccharide units in said solubility tag.

In some embodiments, said chemically modified forms of said monosaccharide units are forms of said monosaccharide units with at least one chemical modification.

In some embodiments, said chemically modified forms of said monosaccharide units are forms of said monosaccharide units with up to three chemical modifications, preferably up to two chemical modifications, more preferably one chemical modification.

In some embodiments, said chemical modification(s) are individually selected from the following:
- replacement of a hydroxyl group by a substituent selected from the group consisting of hydrogen, alkyl, acyl, acyloxy, alkenyl, alkynyl, O-alkyl, S-alkyl, carboxyalkyl, halogen, amino, N-acylamino, azido, sulfate, selenyl and azido;
- replacement of a hydroxyl group by a sulfur-containing moiety selected from the group consisting of a sulfoxide, a sulfone, a sulfuric acid, a sulfuric ester, a thiosulfate, a thioester, a thioether and a sulfoximine;
- replacement of a hydroxyl group by a phosphor-containing moiety selected from the group consisting of a phosphate, a phosphonate, a phosphines, a phosphoric acid and a phosphoester;
- replacement of a hydroxyl group by a silyl group or covalent linkage of a silyl group to said hydroxyl group by formation of a silyl ether;
- replacement of a hydroxyl group by a branched polyalcohol;
- replacement of a hydroxyl group by an amino acid or a peptide of up to 3 amino acids, or covalent linkage of an amino acid or a peptide of up to 3 amino acids to said monosaccharide unit;
- acetal formation with a hydroxyl group of said monosaccharide unit;
- covalent linkage of a PEG (polyethylene glycol) group to said monosaccharide unit;
- covalent linkage to an aromatic or heteroaromatic substituent;
- endocyclic double-bond formation within the sugar ring of said monosaccharide unit.

Chemical modifications of carbohydrates are known to a skilled person and e.g. described in The Organic Chemistry of Sugars, editors Levy and Fügedi (2005), CRC Press/Taylor & Francis (USA); Tamburrini et al., Medicinal Research Reviews (2020), vol. 40(2), p. 495-531; Koviach-Cote and Pirinelli, Chemical Reviews (2018), vol. 118(17), p. 7986-8004; Saloranta and Leino, Synlett (2015), vol. 26(4), p. 421-425; Corsaro et al., in: Microwaves in Organic Synthesis, 2nd edition (2006), publisher Wiley-VCH Verlag, p. 579-614; Lichtenthaler, in: Methods and Reagents for Green Chemistry (2007), editors Tundo et al., publisher John Wiley & Sons, p. 23-63.

In some embodiments, said chemical modification(s) are individually selected from the following: replacement of a hydroxyl group by a substituent selected from the group consisting of hydrogen, alkyl, acyl, acyloxy, alkenyl, alkynyl, O-alkyl, S-alkyl, carboxyalkyl, halogen, amino, N-acylamino, azido, sulfate, selenyl and azido.

In some embodiments, said alkyl is substituted or unsubstituted C₁-C₅ alkyl, said acyl is substituted or unsubstituted C₁-C₅ acyl, said acyloxy is substituted or unsubstituted C₁-C₅ acyloxy, said alkenyl is substituted or unsubstituted C₁-C₅ alkenyl, said alkynyl is substituted or unsubstituted C₁-C₅ alkynyl, said O-alkyl is substituted or unsubstituted C₁-C₅ O-alkyl, said S-alkyl is substituted or unsubstituted C₁-C₅ S-alkyl, said carboxyalkyl is substituted or unsubstituted C₁-C₅ carboxyalkyl, and said N-acylamino is substituted or unsubstituted C₁-C₅ N-acylamino. Preferably, said alkyl is unsubstituted C₁-C₅ alkyl, said acyl is unsubstituted C₁-C₅ acyl, said acyloxy is unsubstituted C₁-C₅ acyloxy, said alkenyl is unsubstituted C₁-C₅ alkenyl, said alkynyl is unsubstituted C₁-C₅ alkynyl, said O-alkyl is unsubstituted C₁-C₅ O-alkyl, said S-alkyl is unsubstituted C₁-C₅ S-alkyl, said carboxyalkyl is unsubstituted C₁-C₅ carboxyalkyl, and said N-acylamino is unsubstituted C₁-C₅ N-acylamino.

In some preferred embodiments, said alkyl is substituted or unsubstituted C₁-C₂ alkyl, said acyl is substituted or unsubstituted C₁-C₂ acyl, said acyloxy is substituted or unsubstituted C₁-C₂ acyloxy, said alkenyl is substituted or unsubstituted C₁-C₂ alkenyl, said alkynyl is substituted or unsubstituted C₁-C₂ alkynyl, said O-alkyl is substituted or unsubstituted C₁-C₂ O-alkyl, said S-alkyl is substituted or unsubstituted C₁-C₂ S-alkyl, said carboxyalkyl is substituted or unsubstituted C₁-C₂ carboxyalkyl, and said N-acylamino is substituted or unsubstituted C₁-C₂ N-acylamino. Preferably, said alkyl is unsubstituted C₁-C₂ alkyl, said acyl is unsubstituted C₁-C₂ acyl, said acyloxy is unsubstituted C₁-C₂ acyloxy, said alkenyl is unsubstituted C₁-C₂ alkenyl, said alkynyl is unsubstituted C₁-C₂ alkynyl, said O-alkyl is unsubstituted C₁-C₂ O-alkyl, said S-alkyl is unsubstituted C₁-C₂ S-alkyl, said carboxyalkyl is unsubstituted C₁-C₂ carboxyalkyl, and said N-acylamino is unsubstituted C₁-C₂ N-acylamino.

In some embodiments, said alkyl, acyl, acyloxy, alkenyl, alkynyl, O-alkyl, S-alkyl, carboxyalkyl, or N-acylamino is linear or branched. Preferably, said alkyl, acyl, acyloxy, alkenyl, alkynyl, O-alkyl, S-alkyl, carboxyalkyl, or N-acylamino is linear.

In some embodiments, said substituted alkyl, acyl, acyloxy, alkenyl, alkynyl, O-alkyl, S-alkyl, carboxyalkyl, or N-acylamino is substituted with a group selected from halogen, CN, OH, amino, methyl, ethyl, methoxy, ethoxy.

In some embodiments, said substituted alkyl, acyl, acyloxy, alkenyl, alkynyl, O-alkyl, S-alkyl, carboxyalkyl, or N-acylamino is substituted with an atom or group having a molecular weight of ≤ 100 Da, preferably a molecular weight of ≤ 80 Da, more preferably a molecular weight of ≤ 50 Da.

In some embodiments, said chemical modification(s) are individually selected from the following: replacement of a hydroxyl group by a sulfur-containing moiety selected from the group consisting of a sulfoxide, a sulfone, a sulfuric acid, a sulfuric ester, a thiosulfate, a thioester, a thioether and a sulfoximine.

In some preferred embodiments, said sulfur-containing moiety has a molecular weight of up to 100 Da, more preferably up to 50 Da.

In some embodiments, said chemical modification(s) are individually selected from the following: replacement of a hydroxyl group by a phosphor-containing moiety selected from the group consisting of a phosphate, a phosphonate, a phosphines, a phosphoric acid and a phosphoester.

In some preferred embodiments, said phosphor-containing moiety has a molecular weight of up to 100 Da, more preferably up to 50 Da.

In some embodiments, said chemical modification(s) are individually selected from the following: replacement of a hydroxyl group by a silyl group or covalent linkage of a silyl group to said hydroxyl group by formation of a silyl ether.

In some preferred embodiments, said silyl group has a molecular weight of up to 150 Da, more preferably up to 100 Da.

In some embodiments, said chemical modification(s) are individually selected from the following: replacement of a hydroxyl group by an amino acid or a peptide of up to 3 amino acids or covalent linkage of an amino acid or a peptide of up to 3 amino acids to said monosaccharide unit;

In some embodiments, said chemical modification(s) are individually selected from the following: acetal formation with a hydroxyl group of said monosaccharide unit.

In some preferred embodiments, each substituent of the acetal that is not a covalent linkage to the monosaccharide unit has a molecular weight of up to 100 Da, more preferably up to 50 Da.

In some embodiments, said chemical modification(s) are individually selected from the following: replacement of a hydroxyl group by a branched polyalcohol.

In some preferred embodiments, said branched polyalcohol is a branched polyalcohol with up to 8 hydroxyl groups, more preferably up to 5 hydroxyl groups.

In some embodiments, said chemical modification(s) are individually selected from the following: covalent linkage of a PEG (polyethylene glycol) group to said monosaccharide unit.

In some preferred embodiments, said PEG group has a molecular weight of up to 1000 Da, more preferably up to 500 Da, more preferably up to 120 Da.

In some embodiments, said chemical modification(s) are individually selected from the following: covalent linkage to an aromatic or heteroaromatic substituent.

In some preferred embodiments, said aromatic or heteroaromatic substituent has a molecular weight of up to 200 Da, more preferably up to 100 Da.

In some embodiments, said chemical modification(s) are individually selected from the following: endocyclic double-bond formation within the sugar ring of said monosaccharide unit.

In some embodiments, said chemical modification of said monosaccharides is the replacement of a hydroxyl group of said monosaccharide by a substituent selected from the group consisting of hydrogen, amino, N-acetylamino, methyl, methoxy and sulfate.

In some embodiments, said chemical modification of said monosaccharides is the replacement of a hydroxyl group of said monosaccharide by a substituent selected from the group consisting of hydrogen, amino, N-acetylamino, methyl and methoxy.

In some embodiments, said chemical modification of said monosaccharides is the replacement of a hydroxyl group of said monosaccharide by a substituent selected from the group consisting of hydrogen, amino and N-acetylamino.

In some embodiments, said chemically modified forms of said monosaccharide units are forms of said monosaccharide units with one, two or three chemical modifications, preferably with one or two chemical modifications, more preferably with one chemical modification.

In some embodiments, said chemically modified forms of said monosaccharide units are forms of said monosaccharide units in which one, two or three hydroxyl groups have independently been replaced. In some preferred embodiments, said chemically modified forms of said monosaccharide units are forms of said monosaccharide units in which one or two hydroxyl groups have independently been replaced. In some more preferred embodiments, said chemically modified forms of said monosaccharide units are forms of said monosaccharide units in which one hydroxyl group has independently been replaced.

In some embodiments, said chemically modified forms of said monosaccharide units are forms of said monosaccharide units in which one or two hydroxyl groups have independently been replaced by a hydrogen, amino group, N-acetylamino group, methyl group, methoxy group or sulfate group.

In some embodiments, said chemically modified forms of said monosaccharide units are forms of said monosaccharide units in which one or two hydroxyl groups have independently been replaced by a hydrogen, amino group, N-acetylamino group, methyl group or methoxy group.

In some embodiments, said chemically modified forms of said monosaccharide units are forms of said monosaccharide units in which one or two hydroxyl groups have independently been replaced by a hydrogen, amino group or N-acetylamino group.

In some embodiments, the substituent to the carbon backbone of the monosaccharide unit that results from the chemical modification has a molecular weight of not more than 800 Dalton, preferably not more than 400 Dalton, more preferably not more than 200 Dalton, more preferably not more than 100 Dalton.

In some embodiments, said solubility tag comprises/said solubility tags comprise no monosaccharide units other than monosaccharide units selected from the group consisting of glucose, chemically modified forms of glucose, galactose and chemically modified forms of galactose.

In some embodiments, said solubility tag comprises/said solubility tags comprise no monosaccharide units other than monosaccharides selected from glucosamine (GlcN), N-acetyl-glucosamine (GlcNAc), fucose (Fuc) and 6-methyl-fucose. In some preferred embodiments, said solubility tag comprises/said solubility tags comprise only monosaccharides selected from glucosamine (GlcN) and N-acetyl-glucosamine (GlcNAc) as monosaccharide units. In some more preferred embodiments, said solubility tag comprises/said solubility tags comprise only N-acetyl-glucosamine (GlcNAc) as monosaccharide units.

As used herein, "fucose" (abbreviated "Fuc") is 6-deoxy-L-galactose.

In some embodiments, said solubility tag consists/said solubility tags consist only of covalently linked monosaccharide units selected from the group consisting of glucosamine (GlcN), N-acetyl-glucosamine (GlcNAc) and 6-methyl-fucose. In some preferred embodiments, said solubility tag consists/said solubility tags consist only of covalently linked monosaccharide units selected from the group consisting of glucosamine (GlcN) and N-acetyl-glucosamine (GlcNAc). In some more preferred embodiments, said solubility tag consists/said solubility tags consist only of covalently linked N-acetyl-glucosamine (GlcNAc) units.

In some embodiments, said solubility tag comprises/said solubility tags comprise at least three N-acetyl-glucosamine (GlcNAc) units, preferably at least four N-acetyl-glucosamine (GlcNAc) units.

In some embodiments, said solubility tag comprises/said solubility tags comprise one glucosamine (GlcN) and four N-acetyl-glucosamine (GlcNAc) units. In some preferred embodiments, said solubility tag consists/said solubility tags consist of one glucosamine (GlcN) and four N-acetyl-glucosamine (GlcNAc) units.

In some embodiments, the monosaccharide units of said solubility tag are linked in a linear fashion without branches.

In some embodiments, the monosaccharide units of said solubility tag are linked in a branched fashion.

In some embodiments, said monosaccharide units are linked covalently by linkages individually selected from the group consisting of a glycosidic linkage, an ether bond, an ester bond and exchange of the hydroxy group at the anomeric center of a monosaccharide unit against a bond to another monosaccharide. In some embodiments, said monosaccharide units are linked covalently by glycosidic linkages. As the skilled person is aware, this means that the monosaccharide units are linked by covalent bonds formed between the hemiacetal or hemiketal group of a monosaccharide unit and a hydroxyl group of another monosaccharide unit.

In some embodiments, the monosaccharide units of the solubility tag are in ring form. As the skilled person is aware, monosaccharides can exist either in cyclic form ("ring form") or in open chain form. It is understood that in the case of a terminal monosaccharide of an oligosaccharide, the ring form may be in equilibrium with an open chain form. According to the present disclosure, such a terminal monosaccharide would still be considered as being in ring form.

In some embodiments, said solubility tag is/said solubility tags are linked to said antibody-drug conjugate (resp. said molecule) via a covalent bond between a GlcN monosaccharide unit of said solubility tag and said linker.

In some embodiments, said solubility tag is/said solubility tags are linked to said antibody-drug conjugate (resp. said molecule) via a beta-alanine group that covalently links a GlcN monosaccharide unit of said solubility tag to said linker.

In some embodiments, said solubility tag is/said solubility tags are linked to said antibody-drug conjugate (resp. said molecule) by formation of a covalent bond between a GlcN monosaccharide unit of said solubility tag and said linker.

In some embodiments, said solubility tag is/said solubility tags are linked to said antibody-drug conjugate (resp. said molecule) via a covalent bond between said linker and an amino group linked to carbon 2 of a monosaccharide unit of said solubility tag. The number in "carbon 2" refers to standard numbering of carbon atoms in carbon backbones of monosaccharides (i.e. the carbon atoms are numbered from 1 to x along the backbone, starting from the end that is closest to the C=O group (in the oligosaccharide the C=O group may form an acetal or hemiacetal).

Preferably, said amino group is linked to the carbon C-2 of the terminal monosaccharide unit at the non-reducing end of said oligosaccharide (i.e. of the oligosaccharide comprised in said solubility tag/of which said solubility tag consists).

The number of solubility tags per ADC molecule can be conveniently controlled if the solubility tag is attached to a linker or linker-payload construct by chemical reaction prior to conjugation to the antibody component. To increase the number of solubility tags in the final ADC, either more solubility tags can be included per linker/linker-payload construct or the number of solubility-tagged linkers/linker-payload constructs in the ADC can be increased.

As the skilled person is aware, attachment of more solubility tags per linker/linker-payload construct is facilitated by the use of appropriate reactive groups e.g. in the linker motif. With an appropriate combination of two reactive groups, one on the linker/linker-payload construct and the other one on the tag (e.g. carboxylic acids for amide bond formation with the amino group of a glucosamine unit in the oligosaccharide tag, or combinations like azide/alkyne or halogen/thiol. The tags can be added at an early or late stage in the synthesis of the linker resp. linker-payload construct.

Alternatively or in addition, the number of linkers/linker-payload constructs per antibody component can be controlled by changes in the conjugation method. While a site-specific conjugation e.g. with transglutaminase limits the number of linkers/linker-payload constructs per antibody component to the number of recognition sequences (there are typically two transglutaminase recognition sequences in the sequence of a full-length antibody, but this can be adapted by altered by mutating the antibody sequence), the use of unspecific lysine conjugation or of the more specific interchain cysteine allows the modulation of the ratio. These approaches also allow to control the number of solubility tags per antibody component if the solubility tags are coupled directly (i.e. not via the linker or payload) to the antibody component.

In some embodiments, the solubility tag comprises/each solubility tag comprises the oligosaccharide

GlcN-GlcNAc-GlcNAc-GlcNAc-GlcNAc.

In some embodiments, the solubility tag consists of/each solubility tag consists of the oligosaccharide

GlcN-GlcNAc-GlcNAc-GlcNAc-GlcNAc.

Preferably, said oligosaccharide GlcN-GlcNAc-GlcNAc-GlcNAc-GlcNAc is linked to another component of the antibody-drug conjugate by a covalent bond to the GlcN monosaccharide unit of said oligosaccharide.

In some embodiments, said solubility tag is or comprises (preferably is) *O*-(2-desoxy-2-amino-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D-*glucopyranosyl)-(1→4)-*O*-(2-acetamido-2-desoxy-*D*-glucopyranose.

In some embodiments, said solubility tag is or comprises (preferably is) *O*-(2-Desoxy-2-amino-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D-*glucopyranosyl)-(1→4)-*O*-{(6-desoxy-2-*O*-methyl-*α*-*L*-galactopyranosyl)-(1→6)-*O*}-(2-acetamido-2-desoxy-D-glucopyranose).

In some embodiments, said solubility tag is or comprises (preferably is) *O*-(2-Desoxy-2-amino-**-***β-*D-glucopyranosyl)-(1→4)-*O*-(2-acetamido-2-desoxy-*β*-*D*-glucopyranosyl)-(1→4)-*O*-(2-acetamido-2-desoxy-*β*-*D*-glucopyranosyl)-(1→4)-*O*-(2-acetamido-2-desoxy-*D-*glucopyranose).

In some embodiments, said solubility tag is or comprises (preferably is) the sodium salt of *O-*(2-Desoxy-2-amino-*β*-*D*-glucopyranosyl)-(1→4)-*O*-(2-acetamido-2-desoxy-*β*-*D-*glucopyranosyl)-(1→4)-*O*-(2-acetamido-2-desoxy-*β*-*D*-glucopyranosyl)-(1→4)-*O*-(2-acetamido-2-desoxy-6-*O*-sulfo-*D*-glucopyranose).

In some embodiments, said solubility tag is or comprises (preferably is) *O*-(2-Desoxy-2-amino-*β*-*D*-glucopyranosyl)-(1→4)-*O*-(2-acetamido-2-desoxy-*D*-glucopyranose).

In some embodiments, said solubility tag is or comprises (preferably is) N-[(3R,4R,6R)-5-[(2S,3R,4R,6R)-3-acetamido-5-[(2S,3R,4R,6R)-3-acetamido-5-[(2S,3R,4R,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide.

In some embodiments, said solubility tag is or comprises (preferably is) N-[(3R,4R,5S,6R)-5-[(2S,3R,4R,5S,6R)-3-acetamido-5-[(2S,3R,4R,5S,6R)-3-acetamido-5-[(2S,3R,4R,5S,6R)-3-acetamido-5-[(2S,R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-6-[[(2R,3S,4R,5S,6S)-4,5-dihydroxy-3-methoxy-6-methyl-tetrahydropyran-2-yl]oxymethyl]-2,4-dihydroxy-tetrahydropyran-3-yl]acetamide.

In some embodiments, said solubility tag is or comprises (preferably is) N-[(3R,4R,5S,6R)-5-[(2S,3R,4R,5S,6R)-3-acetamido-5-[(2S,3R,4R,5S,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide.

In some embodiments, said solubility tag is or comprises (preferably is) ((2R,3S,4R,5R)-5-acetamido-3-(((2S,3R,4R,5S,6R)-3-acetamido-5-(((2S,3R,4R,5S,6R)-3-acetamido-5-(((2S,3R,4R,5S,6R)-3-amino-4,5-dihydroxy-6-(hydroxymethyl)tetrahydro-2H-pyran-2-yl)oxy)-4-hydroxy-6-(hydroxymethyl)tetrahydro-2H-pyran-2-yl)oxy)-4-hydroxy-6-(hydroxymethyl)tetrahydro-2H-pyran-2-yl)oxy)-4,6-dihydroxytetrahydro-2H-pyran-2-yl)methyl sulfate Natrium (I).

In some embodiments, said solubility tag is or comprises (preferably is) N-[(3R,4R,5S,6R)-5-[(2S,3R,4R,5S,6R)-3-amino-4,5-dihydroxy-6-(hydroxymethyl)tetrahydropyran-2-yl]oxy-2,4-dihydroxy-6-(hydroxymethyl)tetrahydropyran-3-yl]acetamide.

In some embodiments, said solubility tag comprises a chemical group with the structural formula (I):

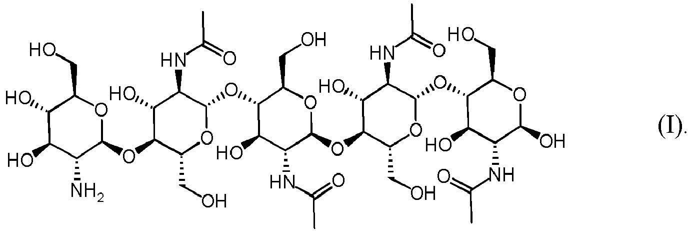

In some embodiments, said solubility tag comprises a chemical group with the structural formula (II):

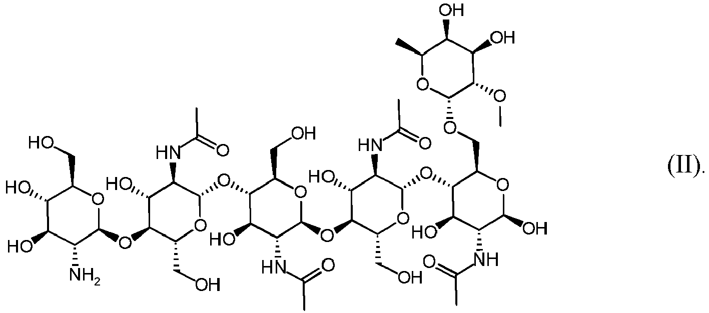

In some embodiments, said solubility tag comprises a chemical group with the structural formula (III):

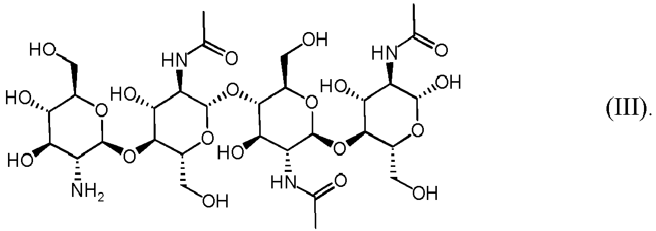

In some embodiments, said solubility tag comprises a chemical group with the structural formula (IV):

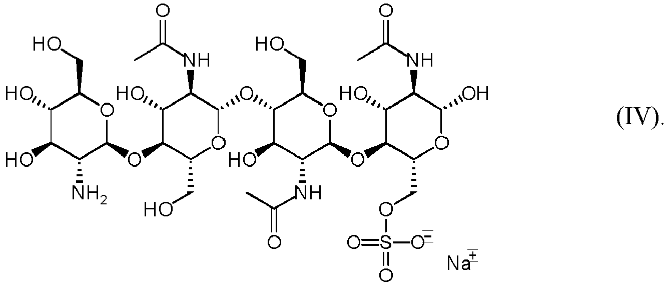

In some embodiments, said solubility tag is a chemical group with the structural formula (I):

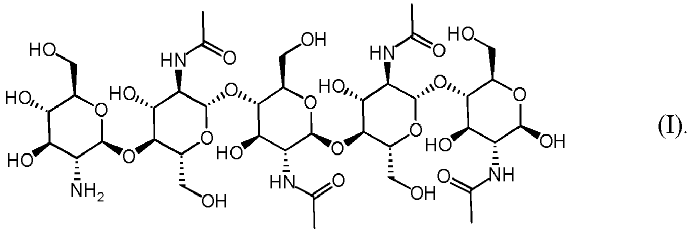

In some embodiments, said solubility tag is a chemical group with the structural formula (II):

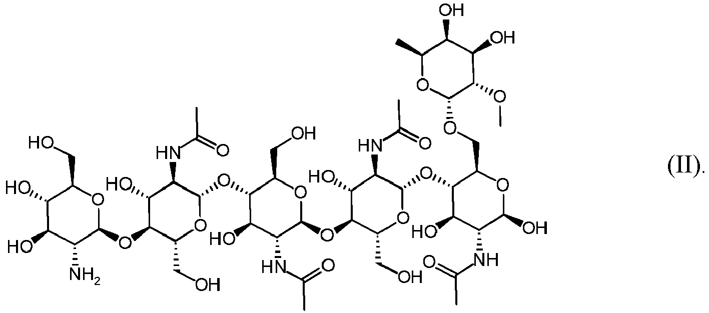

In some embodiments, said solubility tag is a chemical group with the structural formula (III):

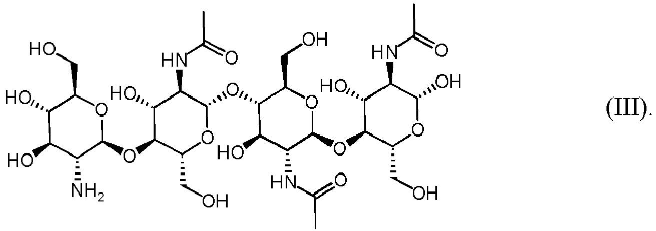

In some embodiments, said solubility tag is a chemical group with the structural formula (IV):

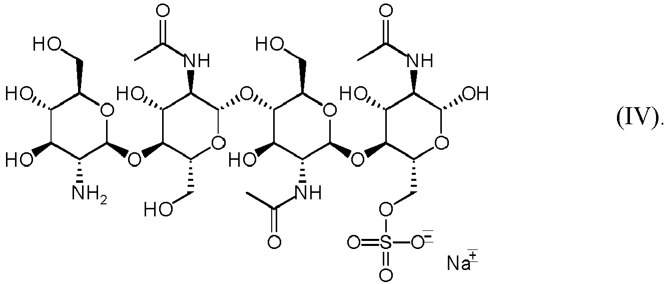

As the skilled person understands, the chemical groups with the structural formula (I) to (IV) can be linked to said molecule/antibody-drug conjugate resp. a component thereof e.g. by one of the linkages described above or in the examples (such as a covalent bond between said molecule/antibody-drug conjugate/component thereof and an amino group linked to carbon 2 of a monosaccharide unit within said chemical group). It is understood that if the chemical group with structural formula (I) to (IV) is covalently attached to said molecule/antibody-drug conjugate resp. a component thereof, e.g. a hydrogen atom in the structural formula (I) to (IV) may be replaced by said molecule/antibody-drug conjugate (i.e. a covalent bond that in the structural formula (I) to (IV) is shown as being linked to a hydrogen atom instead forms the covalent linkage by which said chemical group is linked to said molecule/antibody-drug conjugate/component thereof).

As the skilled person understands, the natural antibody glycosylation is not a solubility tag according to the present disclosure.

In some embodiments, said solubility tag is not an N-linked glycan. In some embodiments, said solubility tag is not an N-linked glycan, does not comprise an N-linked glycan and is not a molecular group within an N-linked glycan.

As used herein, the term "N-linked glycan" is a monosaccharide, oligosaccharide or polysaccharide that is attached by a covalent bond to the nitrogen in the side chain of asparagine within a polypeptide as part of a glycoprotein.

As used herein, a molecular group "within an N-linked glycan" is a molecular group that is part of the chemical structure of said N-linked glycan.

In some embodiments, said solubility tag is not an N-linked glycan or O-linked glycan. In some embodiments, said solubility tag is not an N-linked glycan or O-linked glycan, does not comprise an N-linked glycan or O-linked glycan, and is not a molecular group within an N-linked glycan or O-linked glycan.

As used herein, an "O-linked glycan" is a monosaccharide (typically N-acetylgalactosamine, galactose, or xylose) that is attached by a covalent bond to the oxygen in the side chain of serine or threonine within a polypeptide as part of a glycoprotein.

In some embodiments, said antibody component is either not glycosylated or only carries a glycosylation at position Asn 297 (EU numbering) of the IgG1 heavy chain. Preferably, said glycosylation at Asn 297 is the natural antibody glycosylation.

The term "EU numbering " will be understood to mean the numbering of an antibody heavy chain is according to the EU index as taught in Sequences of Proteins of Immunological Interest, 5th ed. (1991), editors Kabat et al., National Institutes of Health (Bethesda, USA). The EU index is based on the residue numbering of the human IgG1 EU antibody (Edelman et al., Proc Natl Acad Sci USA (1969), vol. 63, p. 78-85).

In some embodiments, said antibody component (resp. said antibody or antigen-binding fragment of item [38]) does not carry any monosaccharides or carries no monosaccharides besides the monosaccharides in the antibody glycosylation. In some embodiments, said antibody component (resp. said antibody or antigen-binding fragment of item [38]) does not carry any antibody glycosylation.

In some embodiments, said antibody component (resp. said antibody or antigen-binding fragment of item [38]) does not comprise any chito-oligosaccharide. In some embodiments, said antibody component does not comprise any monosaccharide units.

In some embodiments, said antibody-drug conjugate (resp. said molecule) comprises no chito-oligosaccharide beyond the chito-oligosaccharide(s) of the solubility tag(s). In some embodiments, said antibody-drug conjugate comprises no other monosaccharide units beyond the monosaccharide units of the solubility tag(s) and optionally monosaccharide units that are part of the glycosylation of the antibody component (resp. of the antibody or antigen-binding fragment of item [38]). In some embodiments, said antibody-drug conjugate (resp. said molecule) comprises no other monosaccharide units beyond the monosaccharide units of the solubility tag(s).

In some embodiments, said molecule with solubility tag(s) has a higher solubility than a molecule of the same structure, but without said solubility tag(s).

In some embodiments, said ADC with solubility tag(s) has a higher solubility than an ADC of the same structure, but without said solubility tag(s).

The solubility of an ADC can be assessed by measuring the formation of aggregates under different ADC concentrations in appropriate buffers during formulation development (Kalonia et al., J. Phys. Chem. B (2016), vol. 120, p. 7062-7075; Duerr and Friess, European Journal of Pharmaceutics and Biopharmaceutics (2019), vol. 139, p. 168-176), preferably by the method of Duerr and Friess. This approach allows to compare the solubility of an ADC with and without a certain solubility tag. A good first appraisal if a tag increases the solubility of an ADC can also be obtained by HIC (hydrophobic interaction chromatography) experiments, as described in the experimental section below.

In some embodiments, a corresponding molecule without said payload (i.e. a molecule composed of only the antibody component, the linker and the solubility tag of said antibody-drug conjugate) is non-toxic to mice at a dose of 6 mg per kg of body weight of said mice administered by intravenous administration.

In some embodiments, said antibody-drug conjugate is such that a corresponding molecule without said payload (i.e. a molecule composed of only the antibody component, the linker and the solubility tag of said antibody-drug conjugate) does not lead to any signs of liver toxicity in an animal study with mice.

Preferably, said animal study with mice involves the administration of said molecule without said payload to adult female BALB/c Nude mice in a single intravenous administration at a dose of 6 mg of ADC per kg of body weight of said mice, preparation of formalin-fixed liver tissue stained with hematoxylin & eosin and histopathological analysis under the light microscope, wherein the absence of visible lesions under the light microscope indicates the absence of liver toxicity.

Further details how this experiment can be carried out can be found in Example 8 below.

In another aspect, the present disclosure relates to an antibody-drug conjugate consisting of
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

Preferably, said antibody-drug conjugate, said antibody component, said at least one payload, said therapeutic agent, said detectable label, said linker/linkers and said at least one solubility tag are (insofar as this does not lead to logical contradictions) as defined in any of the embodiments above, or as defined by a combination of any of the embodiments described above.

### Methods for Solubility Enhancement

As described above and in the examples section below, covalent linkage of a solubility tag according to the present disclosure allows to increase the solubility of an antibody-drug conjugate. Thus, in further aspects, the present disclosure provides methods to increase the solubility of an antibody-drug conjugate.

In another aspect, the present disclosure relates to a method for increasing the solubility of an antibody-drug conjugate, said antibody-drug conjugate comprising
(i) an antibody component,
(ii) at least one payload wherein said at least one payload is a therapeutic agent or a detectable label, and
(iii) a linker/linkers covalently linking said payload/payloads and said antibody component, wherein said method comprises covalently linking at least one solubility tag to said antibody-drug conjugate.

In another aspect, the present disclosure relates to a method for increasing the solubility of an antibody-drug conjugate, said antibody-drug conjugate consisting of
(i) an antibody component,
(ii) at least one payload wherein said at least one payload is a therapeutic agent or a detectable label, and
(iii) a linker/linkers covalently linking said payload/payloads and said antibody component,
   wherein said method comprises covalently linking at least one solubility tag to said antibody-drug conjugate.

Preferably, said antibody-drug conjugate, said antibody component, said at least one payload, said therapeutic agent, said detectable label, said linker/linkers and said at least one solubility tag are (insofar as this does not lead to logical contradictions) as defined in any of the embodiments above, or as defined by a combination of any of the embodiments described above.

General methods for the preparation of ADCs by different approaches can be found for example in Matsuda et al., Organic Process Research & Development (2019), vol. 23(12), p. 2647-2654; Walker et al., Bioconjugate Chemistry (2019), vol. 30(9), p. 2452-2457; Barfield and Rabuka, Methods in Molecular Biology (2018), vol. 1728 (Noncanonical Amino Acids), p. 3-16.

The antibody component, payload and linker can be prepared and covalently linked by standard biotechnological methods and methods synthetic organic chemistry, as described above in the section "Antibody-Drug Conjugates" and shown in detail in the examples section.

As described above in the section "Antibody-Drug Conjugates", an oligosaccharide-based solubility tag according to the present application can be chemically synthesized by standard methods of carbohydrate chemistry (see e.g. Preparative Carbohydrate Chemistry (1997), editor Hanessian, publisher Marcel Dekker, Inc. (New York); Carbohydrate Chemistry: Proven Synthetic Methods (2015), editors Roy and Vidal, CRC Press; Carbohydrate Chemistry: State of the Art and Challenges for Drug Development (2016), editor Cipolla, Imperial College Press (London); CRC Handbook of Oligosaccharides (1990), Vol. I-III, (published 2019), editors: Liptak et al., CHR Press, Inc.; Liaqat and Eltem, Carbohydrate Polymers (2018), vol. 184, p. 243-259).

Alternatively, an oligosaccharide tag can be prepared by biotechnological methods (see e.g. Meyer et al., Biotechnological Production of Oligosaccharides - Applications in the Food Industry, Food Production and Industry (2015), Ayman Hafiz Amer Eissa, IntechOpen, DOI: 10.5772/60934; Liaqat and Eltem, Carbohydrate Polymers (2018), vol. 184, p. 243-259; Samain et al., Carbohydrate Research (1997), vol. 302, p. 35-42; Samain et al., Biotechnol. (1999), vol. 72, p. 33-47).

Further purification can be carried out by standard methods of organic chemistry, including e.g. re-crystallization or precipitation, nanofiltration, ultrafiltration, gel permeation chromatography, ion exchange chromatography, capillary electrophoresis, HPLC purification, UPLC purification, or membrane and carrier approaches as described e.g. in Pinelo et al., Separation and Purification Technology (2009), vol. 70(1), p. 1-11.

The intermediate mono- and oligosaccharides and the final solubility tag before attachment can be characterized by standard methods known to a person of skill in the art e.g. by LC-MS/ESI MS methods, 1D and 2D NMR, GPC (gel permeation chromatography) or ion mobility-mass spectrometry (see e.g. Carbohydrate Chemistry (1988), editor El Khadem, Academic Press (San Diego); Seeberger et al., Nature (2015), vol. 526(7572), p. 241-244).

By use of orthogonal conjugation methods for the linkage of the different components (such as combination of transglutaminase and cysteine conjugation), a high level of control can be achieved. Attaching the solubility tag to the linker has the advantage that it is usually straightforward to include an appropriate reactive group in the linker during synthesis. On the other hand, specific attachment of the solubility tag to the antibody component can typically be achieved by enzymatic conjugation approaches.

While the reaction environment during enzymatic addition of the solubility tag is typically not harmful to the protein components of the ADC, the conditions for attachment by chemical reaction must be appropriately chosen to avoid damage to the protein components. Suitable conditions can be inferred from the conditions for chemical linker conjugation to the antibody component. Thus, the tag can for example be added via a cycloaddition reaction such as Click Chemistry or Diels Alder type modifications (Rossin et al., Bioconjugate Chemistry (2016), vol. 27(7), p. 1697-1706) or using aldehydes (Barfield and Rabuka, Methods in Molecular Biology (2018), vol. 1728, p. 3-16).

Optionally, the covalent attachment of the solubility group to the antibody-drug conjugate may be carried out with an intermediate compound as described below. To obtain this intermediate compound, the solubility tag is activated by introduction of an activator group by standard methods of organic synthesis. The activator group is a reactive functional group and may for example be a maleimide, halogen-acetamide, alkyl halogen, Michael acceptor (such as a vinylpyridine) or a group suitable for cycloaddition (e.g. a ketone, hydrazone, semicarbazone, carboxylic acid, alkene or alkyne suitable for cycloaddition). Subsequently, the solubility tag is covalently linked to the antibody-drug conjugate by a reaction of said activator group with an appropriate molecular group within the antibody-drug conjugate. As the skilled person will understand, covalent linkage of the solubility tag to the antibody-drug conjugate may also be achieved with an activator group on the antibody-drug conjugate rather than on the solubility tag.

Successful covalent linkage of the solubility tag to the antibody-drug conjugate can then be confirmed by standard methods known to a skilled person, e.g. by LC-MS.

If the present disclosure indicates that a modification or action is for "increasing the solubility of an antibody-drug conjugate", this refers to a situation where the solubility of the antibody-drug conjugate after carrying out said method is higher than before carrying out said method.

To verify whether an increase in the solubility of the antibody-drug conjugate has indeed been achieved, the solubility of the antibody-drug conjugate before and after covalent linkage of the solubility tag(s) can be assessed e.g. by measuring the formation of aggregates under different ADC concentrations in appropriate buffers during formulation development (Kalonia et al., J. Phys. Chem. B (2016), vol. 120, p. 7062-7075; Duerr and Friess, European Journal of Pharmaceutics and Biopharmaceutics (2019), vol. 139, p. 168-176), preferably by the method of Duerr and Friess. This approach allows to compare the solubility of an ADC with and without a certain solubility tag.

Alternatively, HIC (hydrophobic interaction chromatography) experiments may be carried out, as described in Example 4 below. Comparability between samples can be ensured by the use of internal standards. Shorter retention times in hydrophobic interaction chromatography indicate an increase in solubility.

In another aspect, the present disclosure relates to the use of a solubility tag for enhancing the solubility of an antibody-drug conjugate.

In some embodiments, said use involves the step of covalently linking said solubility tag to said antibody-drug conjugate.

In some embodiments of said use, said antibody-drug conjugate comprises
(i) an antibody component,
(ii) at least one payload wherein said at least one payload is a therapeutic agent or a detectable label, and
(iii) a linker/linkers covalently linking said payload/payloads and said antibody component.

In some embodiments of said use, said antibody-drug conjugate consists of
(i) an antibody component,
(ii) at least one payload wherein said at least one payload is a therapeutic agent or a detectable label, and
(iii) a linker/linkers covalently linking said payload/payloads and said antibody component.

Preferably, in such use said antibody-drug conjugate, said antibody component, said at least one payload, said therapeutic agent, said detectable label, said linker/linkers and said at least one solubility tag are (insofar as this does not lead to logical contradictions) as defined in any of the embodiments above, or as defined by a combination of any of the embodiments described above (see above, section "Antibody-Drug Conjugates").

Preparation and covalent linkage of the antibody component, payload and linker, preparation, purification and characterization of the solubility tag, covalent linkage of the solubility tag, confirmation of successful linkage of the solubility tag and verification that the solubility tag has indeed resulted in an enhancement of the solubility of an antibody-drug conjugate can all be carried out as described with regard to the method for increasing the solubility of an antibody-drug conjugate above.

In another aspect, the present disclosure relates to a method for increasing the solubility of an antibody-drug conjugate, said antibody-drug conjugate comprising
(i) an antibody component,
(ii) at least one payload wherein said at least one payload is a therapeutic agent or a detectable label, and
(iii) a linker/linkers covalently linking said payload/payloads and said antibody component,
   wherein said method comprises the preparation of said antibody-drug conjugate in a form in which said antibody-drug conjugate is covalently linked to at least one solubility tag.

In another aspect, the present disclosure relates to a method for increasing the solubility of an antibody-drug conjugate, said antibody-drug conjugate consisting of
(i) an antibody component,
(ii) at least one payload wherein said at least one payload is a therapeutic agent or a detectable label, and
(iii) a linker/linkers covalently linking said payload/payloads and said antibody component,
   wherein said method comprises the preparation of said antibody-drug conjugate in a form in which said antibody-drug conjugate is covalently linked to at least one solubility tag.

In another aspect, the present disclosure relates to a method for increasing the solubility of a chemical compound comprising
(i) an antibody component,
(ii) at least one payload wherein said at least one payload is a therapeutic agent or a detectable label, and
(iii) a linker/linkers covalently linking said payload/payloads and said antibody component,
   wherein said method comprises the preparation of a molecule in which said chemical compound is covalently linked to at least one solubility tag,
   wherein said molecule is an antibody-drug conjugate.

In another aspect, the present disclosure relates to a method for increasing the solubility of a chemical compound consisting of
(i) an antibody component,
(ii) at least one payload wherein said at least one payload is a therapeutic agent or a detectable label, and
(iii) a linker/linkers covalently linking said payload/payloads and said antibody component, wherein said method comprises the preparation of a molecule in which said chemical compound is covalently linked to at least one solubility tag,
   wherein said molecule is an antibody-drug conjugate.

In another aspect, the present disclosure relates to a method for increasing the solubility of a molecule comprising
(i) an antibody component,
(ii) at least one payload wherein said at least one payload is a therapeutic agent or a detectable label, and
(iii) a linker/linkers covalently linking said payload/payloads and said antibody component,
wherein said method comprises the preparation of said molecule in a form in which said molecule is covalently linked to at least one solubility tag, thus resulting in an antibody-drug conjugate comprising
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

In another aspect, the present disclosure relates to a method for increasing the solubility of a molecule consisting of
(i) an antibody component,
(ii) at least one payload wherein said at least one payload is a therapeutic agent or a detectable label, and
(iii) a linker/linkers covalently linking said payload/payloads and said antibody component,
wherein said method comprises the preparation of said molecule in a form in which said molecule is covalently linked to at least one solubility tag, thus resulting in an antibody-drug conjugate consisting of
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.

Preferably, in such methods said antibody-drug conjugate, said antibody component, said at least one payload, said therapeutic agent, said detectable label, said linker/linkers and said at least one solubility tag are (insofar as this does not lead to logical contradictions) as defined in any of the embodiments above, or as defined by a combination of any of the embodiments described above (see above, section "Antibody-Drug Conjugates").

The preparation of the antibody component, payload and linker and preparation, purification and characterization of the solubility tag can be carried out as described above for the method for increasing the solubility of an antibody-drug conjugate.

Preparing the antibody-drug conjugate/molecule "in a form in which said antibody-drug conjugate/molecule is covalently linked to at least one solubility tag" means generating a compound in which the antibody-drug conjugate/molecule is covalently linked said at least one solubility tag. This may be achieved either by preparing and characterizing the individual components (i.e. the antibody component, payload, linker and solubility tag) as described above for the method for increasing the solubility of an antibody-drug conjugate, and then linking them covalently. The order in which the individual components are linked is not limited. Thus, it is e.g. also possible to prepare by methods of synthetic organic chemistry a linker-payload construct covalently linked to a solubility tag and conjugate this construct in a final step to the antibody component by standard methods of conjugation as described in the literature (see e.g. Behrens et al., Molecular Pharmaceutics (2015), vol. 12(11), p. 3986-3998; Stefano, Methods in Molecular Biology (2013), vol. 1045, p. 145-171; Dickgiesser et al., in: Methods in Molecular Biology: Enzyme-Mediated Ligation Methods (2019), editors Nuijens and Schmidt, vol. 2012, p. 135-149; Dickgiesser et al., Bioconjugate Chem. (2020), vol. 31(4), p. 1070-1076) and in Example 3 below.

Confirmation that the desired compound was obtained and verification that inclusion of the solubility tag indeed results in an increased solubility can then be carried out as described above.

### Methods and Tools for Preparing an ADC According to the Present Disclosure

An antibody-drug conjugate with an oligosaccharide-based solubility tag according to the present disclosure can be prepared as described above in the sections "Antibody-Drug Conjugates" and "Methods for Solubility Enhancement". Further methods and tools for preparing an antibody-drug conjugate according to the present disclosure are disclosed below.

In another aspect, the present disclosure relates to a method for preparing an antibody-drug conjugate as defined in the present disclosure, said method comprising the step of
- carrying out a reaction resulting in the formation of a covalent bond between (a) a molecule comprising an antibody component as defined in the present disclosure, a payload as defined in the present disclosure and a linker as defined in the present disclosure and (b) a solubility tag as defined in the present disclosure, or
- carrying out a reaction resulting in the formation of a covalent bond between (a) an antibody component as defined in the present disclosure and (b) a molecule comprising a payload as defined in the present disclosure, a linker as defined in the present disclosure and a solubility tag as defined in the present disclosure, or
- carrying out a reaction resulting in the formation of a covalent bond between (a) a molecule comprising an antibody component as defined in the present disclosure, a linker as defined in the present disclosure and a solubility tag as defined in the present disclosure and (b) a payload as defined in the present disclosure, or
- carrying out a reaction resulting in the formation of a covalent bond between (a) a molecule comprising an antibody component as defined in the present disclosure and a linker as defined in the present disclosure and (b) a molecule comprising a solubility tag as defined in the present disclosure and a payload as defined in the present disclosure,
   to yield an antibody-drug conjugate with a covalently linked solubility tag.

Preferably, said payload is a therapeutic agent or a detectable label.

Preferably, in such method said antibody-drug conjugate, said antibody component, said payload, said therapeutic agent, said detectable label, said linker and said solubility tag are (insofar as this does not lead to logical contradictions) as defined in any of the embodiments above, or as defined by a combination of any of the embodiments described above (see above, section "Antibody-Drug Conjugates").

Preparation, purification and characterization of the antibody component, payload, linker and solubility tag can be carried out as described.

Alternatively, intermediates of the ADC synthesis (e.g. a construct comprising a linker, payload and solubility tag) may be built not by covalently linking previously prepared components, but by gradually synthesizing the complete construct in one molecule (as shown e.g. in Example 1 below). The synthetic sequence and its flexibility is driven be the desired structure and various approaches have been described (e.g. Quiles et al., Journal of Medicinal Chemistry (2010), vol. 53(2), p. 586-594; Feuillatre et al., ACS Omega (2020), vol. 5(3), p. 1557-1565; Sonzini, Bioconjugate Chemistry (2020), vol. 31(1), p. 123-129; Watkinson, BioProcess International (2017), vol. 15(10), p. 22-33).

For covalent linkage of the individual components, any standard methods of synthetic organic chemistry can be used (see above). Confirmation of successful preparation of the ADC including a solubility tag can be carried out as described above and in the examples.

Covalent linkage of the solubility tag to another component of the antibody-drug conjugate may be achieved by "activating" the solubility tag (i.e. by forming an intermediate with a reactive chemical group) and subsequently carrying out a reaction in which that activated intermediate is covalently linked to the other component of the antibody drug conjugate.

Thus, in another aspect, the present disclosure relates to a compound for use in the preparation of an antibody-drug conjugate according to the present disclosure, wherein said compound comprises a solubility tag as defined in the present disclosure linked to an activator group.

In some embodiments, said compound consists of a solubility tag as defined in the present disclosure linked to an activator group.

Preferably, said antibody-drug conjugate and said solubility tag are (insofar as this does not lead to logical contradictions) as defined in any of the embodiments above, or as defined by a combination of any of the embodiments described above (see above, section "Antibody-Drug Conjugates").

As used herein, an "activator group" is a reactive chemical group useful for covalently linking the solubility tag to another compound or an antibody-drug conjugate, e.g. an antibody component, linker or payload as defined in the present disclosure. As the skilled person will understand, the reactive groups must be selected based on compatibility and selectivity as described above for conjugation reactions.

In some embodiments, said activator group is selected from the group consisting of a maleimide, a halogen-acetamide, an alkyl halogen, a Michael acceptor (wherein said Michael acceptor is preferably a vinyl-pyridine) and a group suitable for cycloaddition (wherein said group suitable for cycloaddition is preferably a ketone, hydrazone, semicarbazone, carboxylic acid, alkene or alkyne suitable for cycloaddition).

In another aspect, the present disclosure relates to an antibody-drug conjugate that has been prepared by a method according to the present disclosure. Said method according to the present disclosure may be any of the methods for increasing the solubility of an antibody-drug conjugate/of a molecule according to the present disclosure, the use of a solubility tag for enhancing the solubility of an antibody-drug conjugate according to the present disclosure, or the method for preparing an antibody-drug conjugate according to the present disclosure.

Preferably, said antibody-drug conjugate is (insofar as this does not lead to logical contradictions) as defined in any of the embodiments above, or as defined by a combination of any of the embodiments described above (see above, section "Antibody-Drug Conjugates").

### Pharmaceutical Composition and Medical Uses

In another aspect, the present disclosure relates to a pharmaceutical composition comprising the antibody-drug conjugate of the present disclosure or an antibody-drug conjugate prepared by a method according to the present disclosure.

Preferably, said antibody-drug conjugate and said method are as defined in any of the embodiments above, or as defined by a combination of any of the embodiments described above (see above, section "Antibody-Drug Conjugates").

Methods for preparing pharmaceutical compositions are known to a skilled person in the art (Remington: The Science and Practice of Pharmacy, 22nd ed. (2012), Pharmaceutical Press). In some embodiments, said pharmaceutical composition comprises a pharmaceutically acceptable carrier, diluent and/or excipient.

The term "pharmaceutically acceptable" designates that said carrier, diluent or excipient is a non-toxic, inert material that is compatible with the other ingredients of the pharmaceutical composition and not harmful to the patient that the pharmaceutical composition is administered to, such that it can be used in a pharmaceutical product. Substances suitable as carriers, diluents or excipients in pharmaceutical compositions are known to a skilled person in the art (Remington: The Science and Practice of Pharmacy, 22nd ed. (2012), Pharmaceutical Press). The pharmaceutical composition may further include e.g. additional adjuvants, antioxidants, buffering agents, bulking agents, colorants, emulsifiers, fillers, flavoring agents, preservatives, stabilizers, suspending agents and/or other customary pharmaceutical auxiliaries.

In some embodiments, said pharmaceutical composition further includes at least one additional adjuvant, antioxidant, buffering agent, bulking agent, colorant, emulsifier, filler, flavoring agent, preservative, stabilizer, suspending agent and/or other customary pharmaceutical auxiliary.

In another aspect, the present disclosure relates to an antibody-drug conjugate according to the present disclosure or a pharmaceutical composition according to the present disclosure for use as a medicament.

In another aspect, the present disclosure relates to an antibody-drug conjugate according to the present disclosure or a pharmaceutical composition according to the present disclosure for use in the treatment of cancer. In another aspect, the present disclosure relates to an antibody-drug conjugate according to the present disclosure or a pharmaceutical composition according to the present disclosure for use in the treatment of a malignant tumor. In another aspect, the present disclosure relates to an antibody-drug conjugate according to the present disclosure or a pharmaceutical composition according to the present disclosure for use in the treatment of an inflammatory disease.

In some embodiments, said antibody-drug conjugate and said pharmaceutical composition are for use in the treatment of a human.

Preferably, said antibody-drug conjugate and said pharmaceutical composition are as defined in any of the embodiments above, or as defined by a combination of any of the embodiments described above (see in particular the section "Antibody-Drug Conjugates" above).

The production of medicaments containing the antibody-drug conjugate of the present disclosure according or a pharmaceutical composition according to the present disclosure can be performed according to well-known pharmaceutical methods. Further details on techniques for formulation and administration may be found e.g. in Remington: The Science and Practice of Pharmacy, 22nd ed. (2012), Pharmaceutical Press.

As used herein, "treatment" of a disease and "treating" a disease refers to the process of providing a subject with a pharmaceutical treatment, e.g., the administration of a drug, such that said disease is alleviated, reduced, minimized, halted or even healed, and/or such that the chances of a relapse into the disease are reduced or a relapse into the disease is even prevented.

The use of antibody-drug conjugates in the treatment of diseases is known to a skilled person in the art (see e.g. Coats et al., Clinical Cancer Research (2019), vol. 25(18), p. 5441-5448; Rudra, Bioconjugate Chemistry (2020), vol. 31(3), p. 462-473). Thus, the skilled person is aware that the components of the antibody-drug conjugate, in particular the antibody component and the payload, must be selected appropriately in order to allow for successful treatment. For example, for treatment of a specific cancer, the antibody component of the ADC must be selected such that binding of the antibody component to its target antigen directs the ADC to said cancer (e.g. by using an antibody component against a tumor antigen that is specifically found on the surface of the cancer cells). Similarly, the payload should be selected such that the desired treatment effect is achieved. For example, for the treatment of a cancer, a cytotoxic drug may be selected as payload.

In another aspect, the present disclosure relates to a method for treating a disease in a patient in need thereof, comprising the step of administering to said patient a therapeutically effective amount of the antibody-drug conjugate of the present disclosure or the pharmaceutical composition of the present disclosure.

Preferably, said antibody-drug conjugate and said pharmaceutical composition are as defined in any of the embodiments above, or as defined by a combination of any of the embodiments described above (see in particular the section "Antibody-Drug Conjugates" above).

By "therapeutically effective amount" is meant the amount of an agent required to ameliorate the symptoms of a disease. The effective amount of active agent(s) (e.g., an antibody drug conjugate (ADC)) used for therapeutic treatment of a disease according to the present disclosure varies depending upon the manner of administration, the age, body weight, and general health of the subject. Ultimately, the attending physician or veterinarian will decide the appropriate amount and dosage regimen. Such amount is referred to as a "therapeutically effective" amount.

The term "patient", as used herein, refers to a mammal (such as a human, rat, mouse, monkey, pig, goat, cow, horse, dog or cat). Preferably, the patient is a human.

In some embodiments, said disease is cancer. In some embodiments, said disease is a malignant tumor. In some embodiments, said disease is an inflammatory disease

In another aspect, the present disclosure relates to the use of the antibody-drug conjugate of the present disclosure or of the pharmaceutical composition of the present disclosure for the manufacture of a medicament.

In another aspect, the present disclosure relates to the use of the antibody-drug conjugate of the present disclosure or of the pharmaceutical composition of the present disclosure for the manufacture of a medicament for the treatment of cancer. In another aspect, the present disclosure relates to the use of the antibody-drug conjugate of the present disclosure or of the pharmaceutical composition of the present disclosure for the manufacture of a medicament for the treatment of a malignant tumor. In another aspect, the present disclosure relates to the use of the antibody-drug conjugate of the present disclosure or of the pharmaceutical composition of the present disclosure for the manufacture of a medicament for the treatment of an inflammatory disease.

Preferably, said antibody-drug conjugate and said pharmaceutical composition are as defined in any of the embodiments above, or as defined by a combination of any of the embodiments described above (see in particular the section "Antibody-Drug Conjugates" above).

In some embodiments, said medicament is prepared for administration to a human.

In some embodiments of the different medical uses disclosed above, said inflammatory disease is an autoimmune disease.

In some embodiments of the different medical uses disclosed above, said inflammatory disease is selected from the group consisting of inflammatory bowel disease (IBD), systemic lupus erythematosus (SLE), multiple sclerosis, rheumatoid arthritis, Sjogren's syndrome and Hidradenitis suppurativa (HS).

In some embodiments of the different medical uses disclosed above, said cancer, malignant tumor or inflammatory disease is a human disease.

Also disclosed with regard to the above-described subject matter is the following:
[1] A molecule comprising
- a targeting moiety and
- at least one solubility tag.
[2] A molecule comprising
- a targeting moiety,
- at least one functional moiety, and
- at least one solubility tag.
[3] A molecule comprising
- a targeting moiety,
- at least one functional moiety,
- a linker/linkers covalently linking said functional moiety/moieties and said targeting moiety, and
- at least one solubility tag.
[4] A molecule consisting of
- a targeting moiety and
- at least one solubility tag.
[5] A molecule consisting of
- a targeting moiety,
- at least one functional moiety, and
- at least one solubility tag.
[6] A molecule consisting of
- a targeting moiety,
- at least one functional moiety,
- a linker/linkers covalently linking said functional moiety/moieties and said targeting moiety, and
- at least one solubility tag.
[7] A method for increasing the solubility of a molecule, said molecule comprising a targeting moiety, wherein said method comprises covalently linking at least one solubility tag to said molecule.
[8] A method for increasing the solubility of a molecule, said molecule comprising
- a targeting moiety and
- at least one functional moiety,
   wherein said method comprises covalently linking at least one solubility tag to said molecule.
[9] A method for increasing the solubility of a molecule, said molecule comprising
- a targeting moiety,
- at least one functional moiety, and
- a linker/linkers covalently linking said functional moiety/moieties and said targeting moiety,
   wherein said method comprises covalently linking at least one solubility tag to said molecule.
[10] A method for increasing the solubility of a molecule, said molecule consisting of a targeting moiety, wherein said method comprises covalently linking at least one solubility tag to said molecule.
[11] A method for increasing the solubility of a molecule, said molecule consisting of
- a targeting moiety and
- at least one functional moiety,
   wherein said method comprises covalently linking at least one solubility tag to said molecule.
[12] A method for increasing the solubility of a molecule, said molecule consisting of
- a targeting moiety,
- at least one functional moiety, and
- a linker/linkers covalently linking said functional moiety/moieties and said targeting moiety,
   wherein said method comprises covalently linking at least one solubility tag to said molecule.
[13] A method for increasing the solubility of a chemical compound, said chemical compound comprising a targeting moiety, wherein said method comprises the preparation of a molecule in which said chemical compound is covalently linked to at least one solubility tag.
[14] A method for increasing the solubility of a chemical compound, said chemical compound comprising
- a targeting moiety and
- at least one functional moiety,
   wherein said method comprises the preparation of a molecule in which said chemical compound is covalently linked to at least one solubility tag.
[15] A method for increasing the solubility of a chemical compound, said chemical compound comprising
- a targeting moiety,
- at least one functional moiety, and
- a linker/linkers covalently linking said functional moiety/moieties and said targeting moiety,
   wherein said method comprises the preparation of a molecule in which said chemical compound is covalently linked to at least one solubility tag.
[16] A method for increasing the solubility of a chemical compound, said chemical compound consisting of a targeting moiety, wherein said method comprises the preparation of a molecule in which said chemical compound is covalently linked to at least one solubility tag.
[17] A method for increasing the solubility of a chemical compound, said chemical compound consisting of
- a targeting moiety and
- at least one functional moiety,
   wherein said method comprises the preparation of a molecule in which said chemical compound is covalently linked to at least one solubility tag.
[18] A method for increasing the solubility of a chemical compound, said chemical compound consisting of
- a targeting moiety,
- at least one functional moiety, and
- a linker/linkers covalently linking said functional moiety/moieties and said targeting moiety,
   wherein said method comprises the preparation of a molecule in which said chemical compound is covalently linked to at least one solubility tag.
[19] The method according to any one of items [13] to [18], wherein all components of said chemical compound are covalently linked.
[20] Use of a solubility tag for enhancing the solubility of a molecule, said molecule comprising a targeting moiety.
[21] Use of a solubility tag for enhancing the solubility of a molecule, said molecule comprising
- a targeting moiety and
- at least one functional moiety.
[22] Use of a solubility tag for enhancing the solubility of a molecule, said molecule comprising
- a targeting moiety,
- at least one functional moiety, and
- a linker/linkers covalently linking said functional moiety/moieties and said targeting moiety.
[23] Use of a solubility tag for enhancing the solubility of a molecule, said molecule consisting of a targeting moiety.
[24] Use of a solubility tag for enhancing the solubility of a molecule, said molecule consisting of
- a targeting moiety and
- at least one functional moiety.
[25] Use of a solubility tag for enhancing the solubility of a molecule, said molecule consisting of
- a targeting moiety,
- at least one functional moiety, and
- a linker/linkers covalently linking said functional moiety/moieties and said targeting moiety.
[26] The use according to any one of items [20] to [25], wherein said use involves the step of covalently linking at least one solubility tag to said molecule.
[27] The molecule according to any one of items [1] to [6], the method according to any one of items [7] to [19], or the use according to any one of items [20] to [26], wherein all components of said molecule are covalently linked.
[28] The molecule according to any one of items [1] to [6] or [27], the method according to any one of items [7] to [19] or [27], or the use according to any one of items [20] to [27], wherein said targeting moiety is a molecular group that specifically binds to a target molecule or fragment thereof.
[29] The molecule or the method or the use according to item [28], wherein said target molecule is a biomolecule.
[30] The molecule or the method or the use according to any one of items [28] to [29], wherein said target molecule is a receptor at the surface of a cell.
[31] The molecule or the method or the use according to any one of items [28] to [30], wherein said target molecule is an antigen that is present on the surface of a target cell.
[32] The molecule according to any one of items [1] to [6] or [27] to [31], the method according to any one of items [7] to [19] or [27] to [31], or the use according to any one of items [20] to [31], wherein said targeting moiety is capable of specifically binding to an antigen that is present on the surface of a target cell.
[33] The molecule according to any one of items [1] to [6] or [27] to [32], the method according to any one of items [7] to [19] or [27] to [32], or the use according to any one of items [20] to [32], wherein said targeting moiety comprises a protein, a peptide, a peptide mimetic, a nucleic acid, an oligonucleotide or a small molecule.
[34] The molecule according to any one of items [1] to [6] or [27] to [33], the method according to any one of items [7] to [19] or [27] to [33], or the use according to any one of items [20] to [33], wherein said targeting moiety is selected from the group consisting of a protein, a peptide, a peptide mimetic, a nucleic acid, an oligonucleotide and a small molecule.
[35] The molecule according to any one of items [1] to [6] or [27] to [34], the method according to any one of items [7] to [19] or [27] to [34], or the use according to any one of items [20] to [34], wherein said targeting moiety comprises a protein.
[36] The molecule according to any one of items [1] to [6] or [27] to [35], the method according to any one of items [7] to [19] or [27] to [35], or the use according to any one of items [20] to [35], wherein said targeting moiety is a protein.
[37] The molecule according to any one of items [1] to [6] or [27] to [36], the method according to any one of items [7] to [19] or [27] to [36], or the use according to any one of items [20] to [36], wherein said targeting moiety comprises or is a protein which is a protein ligand that specifically binds to a receptor at the surface of a cell.
[38] The molecule according to any one of items [1] to [6] or [27] to [37], the method according to any one of items [7] to [19] or [27] to [37], or the use according to any one of items [20] to [37], wherein said targeting moiety comprises or is a protein which is an antibody or an antigen-binding fragment thereof.
[39] The molecule according to any one of items [1] to [6] or [27] to [38], the method according to any one of items [7] to [19] or [27] to [38], or the use according to any one of items [20] to [38], wherein said targeting moiety comprises or is a protein which is an antibody component.
[40] The molecule according to any one of items [1] to [6] or [27] to [39], the method according to any one of items [7] to [19] or [27] to [39], or the use according to any one of items [20] to [39], wherein said targeting moiety comprises or is a protein which comprises at least 30 amino acids.
[41] The molecule according to any one of items [1] to [6] or [27] to [40], the method according to any one of items [7] to [19] or [27] to [40], or the use according to any one of items [20] to [40], wherein said targeting moiety comprises or is a peptide which consists of 2 to 30 amino acids.
[42] The molecule according to any one of items [1] to [6] or [27] to [41], the method according to any one of items [7] to [19] or [27] to [41], or the use according to any one of items [20] to [41], wherein said targeting moiety comprises a peptide.
[43] The molecule according to any one of items [1] to [6] or [27] to [34] or [41] to [42], the method according to any one of items [7] to [19] or [27] to [34] or [41] to [42], or the use according to any one of items [20] to [34] or [41] to [42], wherein said targeting moiety is a peptide.
[44] The molecule according to any one of items [1] to [6] or [27] to [35] or [37] to [42], the method according to any one of items [7] to [19] or [27] to [35] or [37] to [42], or the use according to any one of items [20] to [35] or [37] to [42], wherein said targeting moiety comprises a peptide mimetic.
[45] The molecule according to any one of items [1] to [6] or [27] to [34] or [44], the method according to any one of items [7] to [19] or [27] to [34] or [44], or the use according to any one of items [20] to [34] or [44], wherein said targeting moiety is a peptide mimetic.
[46] The molecule according to any one of items [1] to [6] or [27] to [35] or [37] to [42] or [44], the method according to any one of items [7] to [19] or [27] to [35] or [37] to [42] or [44], or the use according to any one of items [20] to [35] or [37] to [42] or [44], wherein said targeting moiety comprises or is a nucleic acid which is a DNA or an RNA.
[47] The molecule according to any one of items [1] to [6] or [27] to [35] or [37] to [42] or [44] or [46], the method according to any one of items [7] to [19] or [27] to [35] or [37] to [42] or [44] or [46], or the use according to any one of items [20] to [35] or [37] to [42] or [44] or [46], wherein said targeting moiety comprises a nucleic acid.
[48] The molecule according to any one of items [1] to [6] or [27] to [34] or [46] to [47], the method according to any one of items [7] to [19] or [27] to [34] or [46] to [47], or the use according to any one of items [20] to [34] or [46] to [47], wherein said targeting moiety is a nucleic acid.
[49] The molecule according to any one of items [1] to [6] or [27] to [35] or [37] to [42] or [44] or [46] to [48], the method according to any one of items [7] to [19] or [27] to [35] or [37] to [42] or [44] or [46] to [48], or the use according to any one of items [20] to [35] or [37] to [42] or [44] or [46] to [48], wherein said targeting moiety comprises an oligonucleotide.
[50] The molecule according to any one of items [1] to [6] or [27] to [34] or [46] to [49], the method according to any one of items [7] to [19] or [27] to [34] or [46] to [49], or the use according to any one of items [20] to [34] or [46] to [49], wherein said targeting moiety is an oligonucleotide.
[51] The molecule according to any one of items [1] to [6] or [27] to [35] or [37] to [42] or [44] or [46] to [47] or [49], the method according to any one of items [7] to [19] or [27] to [35] or [37] to [42] or [44] or [46] to [47] or [49], or the use according to any one of items [20] to [35] or [37] to [42] or [44] or [46] to [47] or [49], wherein said targeting moiety comprises or is a small molecule with a molecular weight < 1000 Da.
[52] The molecule according to any one of items [1] to [6] or [27]to [35] or [37] to [42] or [44] or [46] to [47] or [49] or [51], the method according to any one of items [7] to [19] or [27] to [35] or [37] to [42] or [44] or [46] to [47] or [49] or [51], or the use according to any one of items [20] to [35] or [37] to [42] or [44] or [46] to [47] or [49] or [51], wherein said targeting moiety comprises a small molecule.
[53] The molecule according to any one of items [1] to [6] or [27] to [34] or [51] to [52], the method according to any one of items [7] to [19] or [27] to [34] or [51] to [52], or the use according to any one of items [20] to [34] or [51] to [52], wherein said targeting moiety is a small molecule.
[54] The molecule according to any one of items [1] to [6] or [27] to [53], the method according to any one of items [7] to [19] or [27] to [53], or the use according to any one of items [20] to [53], wherein said targeting moiety is not a sugar.
[55] The molecule according to any one of items [1] to [6] or [27] to [54], the method according to any one of items [7] to [19] or [27] to [54], or the use according to any one of items [20] to [54], wherein said targeting moiety does not comprise a sugar.
[56] The molecule according to any one of items [1] to [6] or [27] to [55], the method according to any one of items [7] to [19] or [27] to [55], or the use according to any one of items [20] to [55], wherein said targeting moiety has a molecular weight of at least 100 Da.
[57] The molecule according to any one of items [1] to [6] or [27] to [56], the method according to any one of items [7] to [19] or [27] to [56], or the use according to any one of items [20] to [56], wherein said targeting moiety has a molecular weight of at least 500 Da.
[58] The molecule according to any one of items [1] to [6] or [27] to [50] to [57], the method according to any one of items [7] to [19] or [27] to [50] to [57], or the use according to any one of items [20] to [50] to [57], wherein said targeting moiety has a molecular weight of at least 1 000 Da.
[59] The molecule according to any one of items [1] to [6] or [27] to [52] or [54] to [58], the method according to any one of items [7] to [19] or [27] to [52] or [54] to [58], or the use according to any one of items [20] to [52] or [54] to [58], wherein said targeting moiety has a molecular weight of at least 2 000 Da.
[60] The molecule according to any one of items [1] to [6] or [27] to [42] or [44] to [49] or [52] or [54] to [59], the method according to any one of items [7] to [19] or [27] to [42] or [44] to [49] or [52] or [54] to [59], or the use according to any one of items [20] to [42] or [44] to [49] or [52] or [54] to [59], wherein said targeting moiety has a molecular weight of at least 10 kDa.
[61] The molecule according to any one of items [1] to [6] or [27] to [42] or [44] to [49] or [52] or [54] to [60], the method according to any one of items [7] to [19] or [27] to [42] or [44] to [49] or [52] or [54] to [60], or the use according to any one of items [20] to [42] or [44] to [49] or [52] or [54] to [60], wherein said targeting moiety has a molecular weight of at least 50 kDa.
[62] The molecule according to any one of items [1] to [6] or [27] to [42] or [44] to [49] or [52] or [54] to [61], the method according to any one of items [7] to [19] or [27] to [42] or [44] to [49] or [52] or [54] to [61], or the use according to any one of items [20] to [42] or [44] to [49] or [52] or [54] to [61], wherein said targeting moiety has a molecular weight of at least 100 kDa.
[63] The molecule according to any one of items [1] to [6] or [27] to [62], the method according to any one of items [7] to [19] or [27] to [62], or the use according to any one of items [20] to [62], wherein said targeting moiety has a molecular weight of up to 10 MDa.
[64] The molecule according to any one of items [1] to [6] or [27] to [63], the method according to any one of items [7] to [19] or [27] to [63], or the use according to any one of items [20] to [63], wherein said targeting moiety has a molecular weight of up to 5 MDa.
[65] The molecule according to any one of items [1] to [6] or [27] to [64], the method according to any one of items [7] to [19] or [27] to [64], or the use according to any one of items [20] to [64], wherein said targeting moiety has a molecular weight of up to 1 MDa.
[66] The molecule according to any one of items [1] to [6] or [27] to [65], the method according to any one of items [7] to [19] or [27] to [65], or the use according to any one of items [20] to [65], wherein said targeting moiety has a molecular weight of up to 200 kDa.
[67] The molecule according to any one of items [1] to [6] or [27] to [61] or [63] to [66], the method according to any one of items [7] to [19] or [27] to [61] or [63] to [66], or the use according to any one of items [20] to [61] or [63] to [66], wherein said targeting moiety has a molecular weight of up to 50 kDa.
[68] The molecule according to any one of items [1] to [6] or [27] to [60] or [63] to [67], the method according to any one of items [7] to [19] or [27] to [60] or [63] to [67], or the use according to any one of items [20] to [60] or [63] to [67], wherein said targeting moiety has a molecular weight of up to 10 kDa.
[69] The molecule according to any one of items [1] to [6] or [27] to [59] or [63] to [68], the method according to any one of items [7] to [19] or [27] to [59] or [63] to [68], or the use according to any one of items [20] to [59] or [63] to [68], wherein said targeting moiety has a molecular weight of up to 2 000 Da.
[70] The molecule according to any one of items [1] to [6] or [27] to [58] or [63] to [69], the method according to any one of items [7] to [19] or [27] to [58] or [63] to [69], or the use according to any one of items [20] to [58] or [63] to [69], wherein said targeting moiety has a molecular weight of up to 1 000 Da.
[71] The molecule according to any one of items [2] to [3] or [5] to [6] or [27] to [70], the method according to any one of items [8] to [9] or [11] to [12] or [14] to [15] or [17] to [19] or [27] to [70], or the use according to any one of items [21] to [22] or [24] to [70], wherein said at least one functional moiety is a chemical entity which is capable of fulfilling a biological, chemical, therapeutic and/or diagnostic function in the human body.
[72] The molecule according to any one of items [2] to [3] or [5] to [6] or [27] to [71], the method according to any one of items [8] to [9] or [11] to [12] or [14] to [15] or [17] to [19] or [27] to [71], or the use according to any one of items [21] to [22] or [24] to [71], wherein said at least one functional moiety is a therapeutic agent or a detectable label.
[73] The molecule according to any one of items [2] to [3] or [5] to [6] or [27] to [72], the method according to any one of items [8] to [9] or [11] to [12] or [14] to [15] or [17] to [19] or [27] to [72], or the use according to any one of items [21] to [22] or [24] to [72], wherein said at least one functional moiety is a therapeutic agent.
[74] The molecule according to any one of items [2] to [3] or [5] to [6] or [27] to [72], the method according to any one of items [8] to [9] or [11] to [12] or [14] to [15] or [17] to [19] or [27] to [72], or the use according to any one of items [21] to [22] or [24] to [72], wherein said at least one functional moiety is a detectable label.
[75] The molecule according to any one of items [2] to [3] or [5] to [6] or [27] to [74], the method according to any one of items [8] to [9] or [11] to [12] or [14] to [15] or [17] to [19] or [27] to [74], or the use according to any one of items [21] to [22] or [24] to [74], wherein said at least one functional moiety is a payload that is a therapeutic agent or a detectable label.
[76] The molecule or the method or the use according to item [75], wherein said payload is a therapeutic agent.
[77] The molecule or the method or the use according to item [75], wherein said payload is a detectable label.
[78] The molecule according to any one of items [2] to [3] or [5] to [6] or [27] to [77], the method according to any one of items [8] to [9] or [11] to [12] or [14] to [15] or [17] to [19] or [27] to [77], or the use according to any one of items [21] to [22] or [24] to [77], wherein said at least one functional moiety comprises a protein, a peptide, a peptide mimetic, a nucleic acid, an oligonucleotide or a small molecule.
[79] The molecule according to any one of items [2] to [3] or [5] to [6] or [27] to [78], the method according to any one of items [8] to [9] or [11] to [12] or [14] to [15] or [17] to [19] or [27] to [78], or the use according to any one of items [21] to [22] or [24] to [78], wherein said at least one functional moiety is a protein, a peptide, a peptide mimetic, a nucleic acid, an oligonucleotide or a small molecule.
[80] The molecule according to any one of items [2] to [3] or [5] to [6] or [27] to [79], the method according to any one of items [8] to [9] or [11] to [12] or [14] to [15] or [17] to [19] or [27] to [79], or the use according to any one of items [21] to [22] or [24] to [79], wherein said at least one functional moiety comprises a protein.
[81] The molecule according to any one of items [2] to [3] or [5] to [6] or [27] to [80], the method according to any one of items [8] to [9] or [11] to [12] or [14] to [15] or [17] to [19] or [27] to [80], or the use according to any one of items [21] to [22] or [24] to [80], wherein said at least one functional moiety is a protein.
[82] The molecule according to any one of items [2] to [3] or [5] to [6] or [27] to [81], the method according to any one of items [8] to [9] or [11] to [12] or [14] to [15] or [17] to [19] or [27] to [81], or the use according to any one of items [21] to [22] or [24] to [81], wherein said at least one functional moiety comprises or is a protein which comprises at least 30 amino acids.
[83] The molecule according to any one of items [2] to [3] or [5] to [6] or [27] to [82], the method according to any one of items [8] to [9] or [11] to [12] or [14] to [15] or [17] to [19] or [27] to [82], or the use according to any one of items [21] to [22] or [24] to [82], wherein said at least one functional moiety comprises or is a peptide which consists of 2 to 30 amino acids.
[84] The molecule according to any one of items [2] to [3] or [5] to [6] or [27] to [83], the method according to any one of items [8] to [9] or [11] to [12] or [14] to [15] or [17] to [19] or [27] to [83], or the use according to any one of items [21] to [22] or [24] to [83], wherein said at least one functional moiety comprises a peptide.
[85] The molecule according to any one of items [2] to [3] or [5] to [6] or [27] to [79] or [83] to [84], the method according to any one of items [8] to [9] or [11] to [12] or [14] to [15] or [17] to [19] or [27] to [79] or [83] to [84], or the use according to any one of items [21] to [22] or [24] to [79] or [83] to [84], wherein said at least one functional moiety is a peptide.
[86] The molecule according to any one of items [2] to [3] or [5] to [6] or [27] to [79], the method according to any one of items [8] to [9] or [11] to [12] or [14] to [15] or [17] to [19] or [27] to [79], or the use according to any one of items [21] to [22] or [24] to [79], wherein said at least one functional moiety comprises a peptide mimetic.
[87] The molecule according to any one of items [2] to [3] or [5] to [6] or [27] to [79] or [86], the method according to any one of items [8] to [9] or [11] to [12] or [14] to [15] or [17] to [19] or [27] to [79] or [86], or the use according to any one of items [21] to [22] or [24] to [79] or [86], wherein said at least one functional moiety is a peptide mimetic.
[88] The molecule according to any one of items [2] to [3] or [5] to [6] or [27] to [80] or [82] to [84] or [86], the method according to any one of items [8] to [9] or [11] to [12] or [14] to [15] or [17] to [19] or [27] to [80] or [82] to [84] or [86], or the use according to any one of items [21] to [22] or [24] to [80] or [82] to [84] or [86], wherein said at least one functional moiety comprises or is a nucleic acid which is a DNA or an RNA.
[89] The molecule according to any one of items [2] to [3] or [5] to [6] or [27] to [80] or [82] to [84] or [86] or [88], the method according to any one of items [8] to [9] or [11] to [12] or [14] to [15] or [17] to [19] or [27] to [80] or [82] to [84] or [86] or [88], or the use according to any one of items [21] to [22] or [24] to [80] or [82] to [84] or [86] or [88], wherein said at least one functional moiety comprises a nucleic acid.
[90] The molecule according to any one of items [2] to [3] or [5] to [6] or [27] to [79] or [88] to [89], the method according to any one of items [8] to [9] or [11] to [12] or [14] to [15] or [17] to [19] or [27] to [79] or [88] to [89], or the use according to any one of items [21] to [22] or [24] to [79] or [88] to [89], wherein said at least one functional moiety is a nucleic acid.
[91] The molecule according to any one of items [2] to [3] or [5] to [6] or [27] to [80] or [82] to [84] or [86] or [88] to [90], the method according to any one of items [8] to [9] or [11] to [12] or [14] to [15] or [17] to [19] or [27] to [80] or [82] to [84] or [86] or [88] to [90], or the use according to any one of items [21] to [22] or [24] to [80] or [82] to [84] or [86] or [88] to [90], wherein said at least one functional moiety comprises an oligonucleotide.
[92] The molecule according to any one of items [2] to [3] or [5] to [6] or [27] to [79] or [88] to [91], the method according to any one of items [8] to [9] or [11] to [12] or [14] to [15] or [17] to [19] or [27] to [79] or [88] to [91], or the use according to any one of items [21] to [22] or [24] to [79] or [88] to [91], wherein said at least one functional moiety is an oligonucleotide.
[93] The molecule according to any one of items [2] to [3] or [5] to [6] or [27] to [80] or [82] to [84] or [86] or [88] to [89] or [91], the method according to any one of items [8] to [9] or [11] to [12] or [14] to [15] or [17] to [19] or [27] to [80] or [82] to [84] or [86] or [88] to [89] or [91], or the use according to any one of items [21] to [22] or [24] to [79], wherein said at least one functional moiety comprises a small molecule.
[94] The molecule according to any one of items [2] to [3] or [5] to [6] or [27] to [79] or [93], the method according to any one of items [8] to [9] or [11] to [12] or [14] to [15] or [17] to [19] or [27] to [79] or [93], or the use according to any one of items [21] to [22] or [24] to [79] or [93], wherein said at least one functional moiety is a small molecule.
[95] The molecule according to any one of items [2] to [3] or [5] to [6] or [27] to [80] or [82] to [84] or [86] or [88] to [89] or [91] or [93] to [94], the method according to any one of items [8] to [9] or [11] to [12] or [14] to [15] or [17] to [19] or [27] to [80] or [82] to [84] or [86] or [88] to [89] or [91] or [93] to [94], or the use according to any one of items [21] to [22] or [24] to [80] or [82] to [84] or [86] or [88] to [89] or [91] or [93] to [94], wherein said at least one functional moiety comprises or is a small molecule with a molecular weight < 1000 Da.
[96] The molecule according to any one of items [2] to [3] or [5] to [6] or [27] to [80] or [82] to [84] or [86] or [88] to [89] or [91] or [93] to [95], the method according to any one of items [8] to [9] or [11] to [12] or [14] to [15] or [17] to [19] or [27] to [80] or [82] to [84] or [86] or [88] to [89] or [91] or [93] to [95], or the use according to any one of items [21] to [22] or [24] to [80] or [82] to [84] or [86] or [88] to [89] or [91] or [93] to [95], wherein said at least one functional moiety comprises a small molecule.
[97] The molecule according to any one of items [2] to [3] or [5] to [6] or [27] to [79] or [93] to [96], the method according to any one of items [8] to [9] or [11] to [12] or [14] to [15] or [17] to [19] or [27] to [79] or [93] to [96], or the use according to any one of items [21] to [22] or [24] to [79] or [93] to [96], wherein said at least one functional moiety is a small molecule.
[98] The molecule according to any one of items [2] to [3] or [5] to [6] or [27] to [97], the method according to any one of items [8] to [9] or [11] to [12] or [14] to [15] or [17] to [19] or [27] to [97], or the use according to any one of items [21] to [22] or [24] to [97], wherein said at least one functional moiety is not a sugar.
[99] The molecule according to any one of items [2] to [3] or [5] to [6] or [27] to [98], the method according to any one of items [8] to [9] or [11] to [12] or [14] to [15] or [17] to [19] or [27] to [98], or the use according to any one of items [21] to [22] or [24] to [98], wherein said at least one functional moiety does not comprise a sugar.
[100] The molecule according to any one of items [2] to [3] or [5] to [6] or [27] to [99], the method according to any one of items [8] to [9] or [11] to [12] or [14] to [15] or [17] to [19] or [27] to [99], or the use according to any one of items [21] to [22] or [24] to [99], wherein said at least one functional moiety has a molecular weight of at least 100 Da.
[101] The molecule according to any one of items [2] to [3] or [5] to [6] or [27] to [100], the method according to any one of items [8] to [9] or [11] to [12] or [14] to [15] or [17] to [19] or [27] to [100], or the use according to any one of items [21] to [22] or [24] to [100], wherein said at least one functional moiety has a molecular weight of at least 500 Da.
[102] The molecule according to any one of items [2] to [3] or [5] to [6] or [27] to [101], the method according to any one of items [8] to [9] or [11] to [12] or [14] to [15] or [17] to [19] or [27] to [101], or the use according to any one of items [21] to [22] or [24] to [101], wherein said at least one functional moiety has a molecular weight of at least 1 000 Da.
[103] The molecule according to any one of items [2] to [3] or [5] to [6] or [27] to [102], the method according to any one of items [8] to [9] or [11] to [12] or [14] to [15] or [17] to [19] or [27] to [102], or the use according to any one of items [21] to [22] or [24] to [102], wherein said at least one functional moiety has a molecular weight of at least 2 000 Da.
[104] The molecule according to any one of items [2] to [3] or [5] to [6] or [27] to [84] or [86] to [91] or [93] or [95] to [103], the method according to any one of items [8] to [9] or [11] to [12] or [14] to [15] or [17] to [19] or [27] to [84] or [86] to [91] or [93] or [95] to [103], or the use according to any one of items [21] to [22] or [24] to [84] or [86] to [91] or [93] or [95] to [103], wherein said at least one functional moiety has a molecular weight of at least 10 kDa.
[105] The molecule according to any one of items [2] to [3] or [5] to [6] or [27] to [84] or [86] to [91] or [93] or [95] to [104], the method according to any one of items [8] to [9] or [11] to [12] or [14] to [15] or [17] to [19] or [27] to [84] or [86] to [91] or [93] or [95] to [104], or the use according to any one of items [21] to [22] or [24] to [84] or [86] to [91] or [93] or [95] to [104], wherein said at least one functional moiety has a molecular weight of at least 50 kDa.
[106] The molecule according to any one of items [2] to [3] or [5] to [6] or [27] to [84] or [86] to [91] or [93] or [95] to [105], the method according to any one of items [8] to [9] or [11] to [12] or [14] to [15] or [17] to [19] or [27] to [84] or [86] to [91] or [93] or [95] to [105], or the use according to any one of items [21] to [22] or [24] to [84] or [86] to [91] or [93] or [95] to [105], wherein said at least one functional moiety has a molecular weight of at least 100 kDa.
[107] The molecule according to any one of items [2] to [3] or [5] to [6] or [27] to [102], the method according to any one of items [8] to [9] or [11] to [12] or [14] to [15] or [17] to [19] or [27] to [102], or the use according to any one of items [21] to [22] or [24] to [102], wherein said at least one functional moiety has a molecular weight of up to 1 000 Da.
[108] The molecule according to any one of items [2] to [3] or [5] to [6] or [27] to [103], the method according to any one of items [8] to [9] or [11] to [12] or [14] to [15] or [17] to [19] or [27] to [103], or the use according to any one of items [21] to [22] or [24] to [103], wherein said at least one functional moiety has a molecular weight of up to 2 000 Da.
[109] The molecule according to any one of items [2] to [3] or [5] to [6] or [27] to [104], the method according to any one of items [8] to [9] or [11] to [12] or [14] to [15] or [17] to [19] or [27] to [104], or the use according to any one of items [21] to [22] or [24] to [104], wherein said at least one functional moiety has a molecular weight of up to 10 kDa.
[110] The molecule according to any one of items [2] to [3] or [5] to [6] or [27] to [105], the method according to any one of items [8] to [9] or [11] to [12] or [14] to [15] or [17] to [19] or [27] to [105], or the use according to any one of items [21] to [22] or [24] to [105], wherein said at least one functional moiety has a molecular weight of up to 50 kDa.
[111] The molecule according to any one of items [2] to [3] or [5] to [6] or [27] to [106], the method according to any one of items [8] to [9] or [11] to [12] or [14] to [15] or [17] to [19] or [27] to [106], or the use according to any one of items [21] to [22] or [24] to [106], wherein said at least one functional moiety has a molecular weight of up to 200 kDa.
[112] The molecule according to any one of items [2] to [3] or [5] to [6] or [27] to [106], the method according to any one of items [8] to [9] or [11] to [12] or [14] to [15] or [17] to [19] or [27] to [106], or the use according to any one of items [21] to [22] or [24] to [106], wherein said at least one functional moiety has a molecular weight of up to 1 MDa.
[113] The molecule according to any one of items [2] to [3] or [5] to [6] or [27] to [106], the method according to any one of items [8] to [9] or [11] to [12] or [14] to [15] or [17] to [19] or [27] to [106], or the use according to any one of items [21] to [22] or [24] to [106], wherein said at least one functional moiety has a molecular weight of up to 5 MDa.
[114] The molecule according to any one of items [1] to [6] or [27] to [113], the method according to any one of items [7] to [19] or [27] to [113], or the use according to any one of items [20] to [113], wherein a comparative molecule with an identical structure as said molecule, but lacking said solubility tag(s), has an isoelectric point (pI) of 5-9.
[115] The molecule according to any one of items [1] to [6] or [27] to [114], the method according to any one of items [7] to [19] or [27] to [114], or the use according to any one of items [20] to [114], wherein a comparative molecule with an identical structure as said molecule, but lacking said solubility tag(s), has a solubility (indicated in g of compound per ml of PBS; solubility measured at 25 °C in PBS (Phosphate-buffered saline: 137 mM NaCl, 2.7 mM KCl, 10 mM Na₂HPO₄, 1.8 mM Na₂HPO₄, pH 7.4)) that falls within a range of ± 50%, preferably ± 30%, relative to the solubility under the same conditions of a compound consisting of the antibody Trastuzumab with two copies of auristatin covalently linked to its Fc region.
[116] The molecule according to any one of items [1] to [6] or [27] to [115], the method according to any one of items [7] to [19] or [27] to [115], or the use according to any one of items [20] to [115], wherein the number of functional moieties per molecule is in the range of from 1 to 15.
[117] The molecule according to any one of items [1] to [6] or [27] to [116], the method according to any one of items [7] to [19] or [27] to [116], or the use according to any one of items [20] to [116], wherein the number of functional moieties per molecule is in the range of from 1 to 10.
[118] The molecule according to any one of items [1] to [6] or [27] to [117], the method according to any one of items [7] to [19] or [27] to [117], or the use according to any one of items [20] to [117], wherein the number of functional moieties per molecule is in the range of from 1 to 8.
[119] The molecule according to any one of items [1] to [6] or [27] to [118], the method according to any one of items [7] to [19] or [27] to [118], or the use according to any one of items [20] to [118], wherein the number of functional moieties per molecule is in the range of from 1 to 4.
[120] The molecule according to any one of items [1] to [6] or [27] to [119], the method according to any one of items [7] to [19] or [27] to [119], or the use according to any one of items [20] to [119], wherein the molecule comprises one, but not more than one functional moieties.
[121] The molecule according to any one of items [1] to [6] or [27] to [119], the method according to any one of items [7] to [19] or [27] to [119], or the use according to any one of items [20] to [119], wherein the number of functional moieties per molecule is in the range of from 2 to 8.
[122] The molecule according to any one of items [1] to [6] or [27] to [119], the method according to any one of items [7] to [19] or [27] to [119], or the use according to any one of items [20] to [119], wherein the number of functional moieties per molecule is in the range of from 4 to 8.
[123] The molecule according to any one of items [1] to [6] or [27] to [122], the method according to any one of items [7] to [19] or [27] to [122], or the use according to any one of items [20] to [122], wherein the number of targeting moieties per molecule is in the range of from 1 to 15.
[124] The molecule according to any one of items [1] to [6] or [27] to [123], the method according to any one of items [7] to [19] or [27] to [123], or the use according to any one of items [20] to [123], wherein the number of targeting moieties per molecule is in the range of from 1 to 10.
[125] The molecule according to any one of items [1] to [6] or [27] to [124], the method according to any one of items [7] to [19] or [27] to [124], or the use according to any one of items [20] to [124], wherein the number of targeting moieties per molecule is in the range of from 1 to 8.
[126] The molecule according to any one of items [1] to [6] or [27] to [125], the method according to any one of items [7] to [19] or [27] to [125], or the use according to any one of items [20] to [125], wherein the number of targeting moieties per molecule is in the range of from 1 to 4.
[127] The molecule according to any one of items [1] to [6] or [27] to [126], the method according to any one of items [7] to [19] or [27] to [126], or the use according to any one of items [20] to [126], wherein the molecule comprises one, but not more than one targeting moieties.
[128] The molecule according to any one of items [1] to [6] or [27] to [126], the method according to any one of items [7] to [19] or [27] to [126], or the use according to any one of items [20] to [126], wherein the number of targeting moieties per molecule is in the range of from 2 to 8.
[129] The molecule according to any one of items [1] to [6] or [27] to [126], the method according to any one of items [7] to [19] or [27] to [126], or the use according to any one of items [20] to [126], wherein the number of targeting moieties per molecule is in the range of from 4 to 8.
[130] The molecule according to any one of items [1] to [6] or [27] to [129], the method according to any one of items [7] to [19] or [27] to [129], or the use according to any one of items [20] to [129], wherein said molecule is an antibody-drug conjugate comprising
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.
[131] The molecule according to any one of items [1] to [6] or [27] to [130], the method according to any one of items [7] to [19] or [27] to [130], or the use according to any one of items [20] to [130], wherein said molecule is an antibody-drug conjugate that consists of
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.
[132] An antibody-drug conjugate comprising
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.
[133] An antibody-drug conjugate consisting of
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.
[134] A method for increasing the solubility of an antibody-drug conjugate, said antibody-drug conjugate comprising
(i) an antibody component,
(ii) at least one payload wherein said at least one payload is a therapeutic agent or a detectable label, and
(iii) a linker/linkers covalently linking said payload/payloads and said antibody component,
   wherein said method comprises covalently linking at least one solubility tag to said antibody-drug conjugate.
[135] A method for increasing the solubility of an antibody-drug conjugate, said antibody-drug conjugate consisting of
(i) an antibody component,
(ii) at least one payload wherein said at least one payload is a therapeutic agent or a detectable label, and
(iii) a linker/linkers covalently linking said payload/payloads and said antibody component,
   wherein said method comprises covalently linking at least one solubility tag to said antibody-drug conjugate.
[136] A method for increasing the solubility of an antibody-drug conjugate, said antibody-drug conjugate comprising
(i) an antibody component,
(ii) at least one payload wherein said at least one payload is a therapeutic agent or a detectable label, and
(iii) a linker/linkers covalently linking said payload/payloads and said antibody component,
   wherein said method comprises the preparation of said antibody-drug conjugate in a form in which said antibody-drug conjugate is covalently linked to at least one solubility tag.
[137] A method for increasing the solubility of an antibody-drug conjugate, said antibody-drug conjugate consisting of
(i) an antibody component,
(ii) at least one payload wherein said at least one payload is a therapeutic agent or a detectable label, and
(iii) a linker/linkers covalently linking said payload/payloads and said antibody component,
   wherein said method comprises the preparation of said antibody-drug conjugate in a form in which said antibody-drug conjugate is covalently linked to at least one solubility tag.
[138] A method for increasing the solubility of a chemical compound comprising
(i) an antibody component,
(ii) at least one payload wherein said at least one payload is a therapeutic agent or a detectable label, and
(iii) a linker/linkers covalently linking said payload/payloads and said antibody component,
   wherein said method comprises the preparation of a molecule in which said chemical compound is covalently linked to at least one solubility tag,
   wherein said molecule is an antibody-drug conjugate.
[139] A method for increasing the solubility of a chemical compound consisting of
(i) an antibody component,
(ii) at least one payload wherein said at least one payload is a therapeutic agent or a detectable label, and
(iii) a linker/linkers covalently linking said payload/payloads and said antibody component,
   wherein said method comprises the preparation of a molecule in which said chemical compound is covalently linked to at least one solubility tag,
   wherein said molecule is an antibody-drug conjugate.
[140] A method for increasing the solubility of a molecule comprising
(i) an antibody component,
(ii) at least one payload wherein said at least one payload is a therapeutic agent or a detectable label, and
(iii) a linker/linkers covalently linking said payload/payloads and said antibody component,
wherein said method comprises the preparation of said molecule in a form in which said molecule is covalently linked to at least one solubility tag, thus resulting in an antibody-drug conjugate comprising
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.
[141] A method for increasing the solubility of a molecule consisting of
(i) an antibody component,
(ii) at least one payload wherein said at least one payload is a therapeutic agent or a detectable label, and
(iii) a linker/linkers covalently linking said payload/payloads and said antibody component,
wherein said method comprises the preparation of said molecule in a form in which said molecule is covalently linked to at least one solubility tag, thus resulting in an antibody-drug conjugate consisting of
(i) an antibody component,
(ii) at least one payload wherein said at least one payload is a therapeutic agent or a detectable label,
(iii) a linker/linkers covalently linking said payload/payloads and said antibody component, and
(iv) at least one solubility tag.
[142] Use of a solubility tag for enhancing the solubility of an antibody-drug conjugate.
[143] The use according to item [142], wherein said use involves the step of covalently linking said solubility tag to said antibody-drug conjugate.
[144] The use according to any one of items [142] to [143], wherein said antibody-drug conjugate comprises
(i) an antibody component,
(ii) at least one payload wherein said at least one payload is a therapeutic agent or a detectable label, and
(iii) a linker/linkers covalently linking said payload/payloads and said antibody component.
[145] The use according to any one of items [142] to [144], wherein said antibody-drug conjugate consists of
(i) an antibody component,
(ii) at least one payload wherein said at least one payload is a therapeutic agent or a detectable label, and
(iii) a linker/linkers covalently linking said payload/payloads and said antibody component.
[146] The molecule according to any one of items [39] to [40] or [54] to [131] or the antibody-drug conjugate according to any one of items [132] to [133] or the method according to any one of items [39] to [40] or [54] to [131] or [134] to [141] or the use according to any one of items [39] to [40] or [54] to [131] or [144] to [145], wherein said antibody component is an intact antibody or an antigen-binding fragment thereof.
[147] The molecule according to any one of items [39] to [40] or [54] to [131] or [146] or the antibody-drug conjugate according to any one of items [132] to [133] or [146] or the method according to any one of items [39] to [40] or [54] to [131] or [134] to [141] or [146] or the use according to any one of items [39] to [40] or [54] to [131] or [144] to [146], wherein said antibody component is an intact antibody.
[148] The molecule according to any one of items [39] to [40] or [54] to [131] or [146] or the antibody-drug conjugate according to any one of items [132] to [133] or [146] or the method according to any one of items [39] to [40] or [54] to [131] or [134] to [141] or [146] or the use according to any one of items [39] to [40] or [54] to [131] or [144] to [146], wherein said antibody component is an antigen-binding fragment of an intact antibody.
[149] The molecule according to any one of items [38] to [40] or [54] to [131] or [146] to [147] or the antibody-drug conjugate according to any one of items [132] to [133] or [146] to [147] or the method according to any one of items [38] to [40] or [54] to [131] or [134] to [141] or [146] to [147] or the use according to any one of items [38] to [40] or [54] to [131] or [144] to [147], wherein said antibody component (resp. said antibody of item [38]) is a monoclonal antibody or a polyclonal antibody.
[150] The molecule according to any one of items [38] to [40] or [54] to [131] or [146] to [147] or [149] or the antibody-drug conjugate according to any one of items [132] to [133] or [146] to [147] or [149] or the method according to any one of items [38] to [40] or [54] to [131] or [134] to [141] or [146] to [147] or [149] or the use according to any one of items [38] to [40] or [54] to [131] or [144] to [147] or [149], wherein said antibody component (resp. said antibody of item [38]) is a monoclonal antibody.
[151] The molecule according to any one of items [38] to [40] or [54] to [131] or [146] to [147] or [149] to [150] or the antibody-drug conjugate according to any one of items [132] to [133] or [146] to [147] or [149] to [150] or the method according to any one of items [38] to [40] or [54] to [131] or [134] to [141] or [146] to [147] or [149] to [150] or the use according to any one of items [38] to [40] or [54] to [131] or [144] to [147] or [149] to [150], wherein said antibody component (resp. said antibody of item [38]) is a monospecific antibody or a bispecific antibody.
[152] The molecule according to any one of items [38] to [40] or [54] to [131] or [146] to [151] or the antibody-drug conjugate according to any one of items [132] to [133] or [146] to [151] or the method according to any one of items [38] to [40] or [54] to [131] or [134] to [141] or [146] to [151] or the use according to any one of items [38] to [40] or [54] to [131] or [144] to [151], wherein said antibody component (resp. said antibody of item [38]) is a bispecific antibody or an antigen-binding fragment thereof that is capable of binding both antigens for which said bispecific antibody is specific.
[153] The molecule according to any one of items [38] to [40] or [54] to [131] or [146] to [147] or [149] to [152] or the method according to any one of items [38] to [40] or [54] to [131] or [134] to [141] or [146] to [147] or [149] to [152] or the use according to any one of items [38] to [40] or [54] to [131] or [144] to [147] or [149] to [152], wherein said antibody of item [38] is a bispecific antibody.
[154] The molecule according to any one of items [38] to [40] or [54] to [131] or [146] to [147] or [149] to [153] or the antibody-drug conjugate according to any one of items [132] to [133] or [146] to [147] or [149] to [153] or the method according to any one of items [38] to [40] or [54] to [131] or [134] to [141] or [146] to [147] or [149] to [153] or the use according to any one of items [38] to [40] or [54] to [131] or [144] to [147] or [149] to [153], wherein said antibody component (resp. said antibody of item [38]) is an antibody selected from the group consisting of a chimeric antibody, a humanized antibody and a human antibody.
[155] The molecule according to any one of items [38] to [40] or [54] to [131] or [146] to [147] or [149] to [154] or the antibody-drug conjugate according to any one of items [132] to [133] or [146] to [147] or [149] to [154] or the method according to any one of items [38] to [40] or [54] to [131] or [134] to [141] or [146] to [147] or [149] to [154] or the use according to any one of items [38] to [40] or [54] to [131] or [144] to [147] or [149] to [154], wherein said antibody component (resp. said antibody of item [38]) is an antibody selected from the group consisting of an IgG1 antibody, an IgG2 antibody, an IgG3 antibody, IgG4 antibody, an IgA antibody, an IgM antibody, and hybrids thereof.
[156] The molecule according to any one of items [38] to [40] or [54] to [131] or [146] or [148] or [152] the antibody-drug conjugate according to any one of items [132] to [133] or [146] or [148] or [152] or the method according to any one of items [38] to [40] or [54] to [131] or [134] to [141] or [146] or [148] or [152] or the use according to any one of items [38] to [40] or [54] to [131] or [144] to [146] or [148] or [152], wherein said antigen-binding fragment is selected from the group consisting of a Fab, a Fab', a (Fab')2, a Fv, a scFv, a diabody and a VHH
[157] The molecule according to any one of items [38] to [40] or [54] to [131] or [146] or [148] or [152] or [156] or the antibody-drug conjugate according to any one of items [132] to [133] or [146] or [148] or [152] or [156] or the method according to any one of items [38] to [40] or [54] to [131] or [134] to [141] or [146] or [148] or [152] or [156] or the use according to any one of items [38] to [40] or [54] to [131] or [144] to [146] or [148] or [152] or [156], wherein said antigen-binding fragment is selected from the group consisting of a Fab, a Fab', a (Fab')2 and a Fv.
[158] The molecule according to any one of items [38] to [40] or [54] to [131] or [146] or [148] or [152] or [156] to [157] or the antibody-drug conjugate according to any one of items [132] to [133] or [146] or [148] or [152] or [156] to [157] or the method according to any one of items [38] to [40] or [54] to [131] or [134] to [141] or [146] or [148] or [152] or [156] to [157] or the use according to any one of items [38] to [40] or [54] to [131] or [144] to [146] or [148] or [152] or [156] to [157], wherein said antigen-binding fragment is selected from the group consisting of a scFv, a diabody and a VHH
[159] The molecule according to any one of items [38] to [40] or [54] to [131] or [146] or [148] or [152] or [156] to [158] or the antibody-drug conjugate according to any one of items [132] to [133] or [146] or [148] or [152] or [156] to [158] or the method according to any one of items [38] to [40] or [54] to [131] or [134] to [141] or [146] or [148] or [152] or [156] to [158] or the use according to any one of items [38] to [40] or [54] to [131] or [144] to [146] or [148] or [152] or [156] to [158], wherein said antigen-binding fragment is an antigen-binding fragment of a monoclonal antibody or a polyclonal antibody.
[160] The molecule according to any one of items [38] to [40] or [54] to [131] or [146] or [148] or [152] or [156] to [159] or the antibody-drug conjugate according to any one of items [132] to [133] or [146] or [148] or [152] or [156] to [159] or the method according to any one of items [38] to [40] or [54] to [131] or [134] to [141] or [146] or [148] or [152] or [156] to [159] or the use according to any one of items [38] to [40] or [54] to [131] or [144] to [146] or [148] or [152] or [156] to [159], wherein said antigen-binding fragment is an antigen-binding fragment of a monoclonal antibody.
[161] The molecule according to any one of items [38] to [40] or [54] to [131] or [146] or [148] or [152] or [156] to [160] or the antibody-drug conjugate according to any one of items [132] to [133] or [146] or [148] or [152] or [156] to [160] or the method according to any one of items [38] to [40] or [54] to [131] or [134] to [141] or [146] or [148] or [152] or [156] to [160] or the use according to any one of items [38] to [40] or [54] to [131] or [144] to [146] or [148] or [152] or [156] to [160], wherein said antigen-binding fragment is an antigen-binding fragment of a monospecific antibody or a bispecific antibody.
[162] The molecule according to any one of items [38] to [40] or [54] to [131] or [146] or [148] or [152] or [156] to [161] or the method according to any one of items [38] to [40] or [54] to [131] or [134] to [141] or [146] or [148] or [152] or [156] to [161] or the use according to any one of items [38] to [40] or [54] to [131] or [144] to [146] or [148] or [152] or [156] to [161], wherein said antigen-binding fragment of item [38] is an antigen-binding fragment of a bispecific antibody that is capable of binding both antigens for which said bispecific antibody is specific.
[163] The molecule according to any one of items [38] to [40] or [54] to [131] or [146] or [148] or [152] or [156] to [162] or the antibody-drug conjugate according to any one of items [132] to [133] or [146] or [148] or [152] or [156] to [162] or the method according to any one of items [38] to [40] or [54] to [131] or [134] to [141] or [146] or [148] or [152] or [156] to [162] or the use according to any one of items [38] to [40] or [54] to [131] or [144] to [146] or [148] or [152] or [156] to [162], wherein said antigen-binding fragment is an antigen-binding fragment of an antibody selected from the group consisting of a chimeric antibody, a humanized antibody and a human antibody.
[164] The molecule according to any one of items [38] to [40] or [54] to [131] or [146] or [148] or [152] or [156] to [163] or the antibody-drug conjugate according to any one of items [132] to [133] or [146] or [148] or [152] or [156] to [163] or the method according to any one of items [38] to [40] or [54] to [131] or [134] to [141] or [146] or [148] or [152] or [156] to [163] or the use according to any one of items [38] to [40] or [54] to [131] or [144] to [146] or [148] or [152] or [156] to [163], wherein said antigen-binding fragment is an antigen-binding fragment of an antibody selected from the group consisting of an IgG1 antibody, an IgG2 antibody, an IgG3 antibody, IgG4 antibody, an IgA antibody, an IgM antibody, and hybrids thereof.
[165] The molecule according to any one of items [1] to [6] or [27] to [131] or [146] to [164] or the antibody-drug conjugate according to any one of items [132] to [133] or [146] to [164] or the method according to any one of items [7] to [19] or [27] to [131] or [134] to [141] or [146] to [164] or the use according to any one of items [20] to [131] or [144] to [164], wherein said targeting moiety/said antibody component (resp. said antibody of item [38]) is capable of specifically binding to an antigen that is present on the surface of a target cell.
[166] The molecule according to any one of items [38] to [40] or [54] to [131] or [146] to [165] or the antibody-drug conjugate according to any one of items [132] to [133] or [146] to [165] or the method according to any one of items [38] to [40] or [54] to [131] or [134] to [141] or [146] to [165] or the use according to any one of items [38] to [40] or [54] to [131] or [144] to [165], wherein said antibody component (resp. said antibody of item [38]) is an antibody against an antigen that is present on the surface of a target cell or an antigen-binding fragment of such an antibody.
[167] The molecule or the antibody-drug conjugate or the method or the use according to any one of items [165] to [166], wherein said antigen that is present on the surface of said target cell is more abundant on the surface of said target cell than on the surface of other cell types.
[168] The molecule or the antibody-drug conjugate or the method or the use according to any one of items [165] to [167], wherein said antigen that is present on the surface of said target cell is present on the surface of said target cell, but substantially not on the surface of other cell types.
[169] The molecule or the antibody-drug conjugate or the method or the use according to any one of items [165] to [168], wherein said antigen that is present on the surface of said target cell is present on the surface of said target cell, but not on the surface of other cell types.
[170] The molecule or the antibody-drug conjugate or the method or the use according to any one of items [165] to [169], wherein said binding of said antibody component (resp. said antibody of item [38]) to said antigen that is present on the surface of said target cell allows to recruit the antibody-drug conjugate specifically to said target cell.
[171] The molecule or the antibody-drug conjugate or the method or the use according to any one of items [165] to [170], wherein said antigen that is present on the surface of said target cell is selected from the group consisting of a tumor antigen and an immune cell antigen.
[172] The molecule or the antibody-drug conjugate or the method or the use according to any one of items [165] to [171], wherein said antigen that is present on the surface of said target cell is a tumor antigen.
[173] The molecule according to any one of items [1] to [6] or [27] to [131] or [146] to [172] or the antibody-drug conjugate according to any one of items [132] to [133] or [146] to [172] or the method according to any one of items [7] to [19] or [27] to [131] or [134] to [141] or [146] to [172] or the use according to any one of items [20] to [131] or [144] to [172], wherein said targeting moiety/said antibody component (resp. said antibody of item [38]) is capable of specifically binding to an antigen selected from the group consisting of a tumor antigen and an immune cell antigen.
[174] The molecule according to any one of items [1] to [6] or [27] to [131] or [146] to [173] or the antibody-drug conjugate according to any one of items [132] to [133] or [146] to [173] or the method according to any one of items [7] to [19] or [27] to [131] or [134] to [141] or [146] to [173] or the use according to any one of items [20] to [131] or [144] to [173], wherein said targeting moiety/said antibody component (resp. said antibody of item [38]) is capable of specifically binding to a tumor antigen.
[175] The molecule or the antibody-drug conjugate or the method or the use according to any one of items [171] to [174], wherein said tumor antigen is an antigen that is present on the surface of a tumor cell.
[176] The molecule or the antibody-drug conjugate or the method or the use according to any one of items [171] to [175], wherein said tumor antigen is selected from the group consisting of CD1 Ia, CD4, CD19, CD20, CD21, CD22, CD23, CD25, CD52, CD30, CD33, CD37, CD40L, CD52, CD56, CD70, CD72, CD74, CD79a, CD79b, CD138, CD163, Her2, Her3, EGFR, Mucl8, ,integrin, PSMA, CEA, BLys, ROR1, NaPi2b, NaPi3b, CEACAM5, Mucl, integrin avb6, Met, Trop2, BCMA, disialoganglioside GD2, B-PR1B, E16, STEAP1, 0772P, Sema 5b, ETBR, MSG783, STEAP2, Trp4, CRIPTO, FcRH1, FcRH2, NCA, IL20R-alpha, Brevican, EphB2R, ASLG659, PSCA, GEDA, BAFF-R, CXCR5, HLA-DOB, P2X5, LY64, IRTA2, TENB2, PSMA, FOLH1, STR5, SSTR1, SSTR2, SSTR3, SSTR4, TGAV, ITGB6, CA9, EGFRvlll, IL2RA, AXL, CD3Q, TNFRSF8, TNFRSF17, CTAGs, CTA; CD174/Fucosyltransferase 3 (Lewis Blood Group), CLEC14A, GRP78, HSPA5, ASG-5, ENPP3, PRR4, GCC, GUCY2C, Liv-1, SLC39A8, 5T4, NCMA1, CanAg, FOLR1, GPN B, TIM-1, HAVCR1, Mindin/RG-1, B7-H4, VTCN1, PTK7, SDC1, a claudin (preferably claudin 18.2), RON, MST1 R, EPHA2, MS4A1, TNC (Tenascin C), FAP, DKK-1, CS1/SLAMF7, ENG (Endoglin), ANXA1 (Annexin A1), VCAM-1 (CD106) and folate receptor alpha.
[177] The molecule or the antibody-drug conjugate or the method or the use according to any one of items [171] to [176], wherein said tumor antigen is selected from the group consisting of xCT, gpNMB, carbonic anhydrase IX (CAIX), cKIT, c-MET, Tumor-associated glycoprotein 72 (TAG-72), TROP-2, TRA-1-60, TRA, TNF- alpha, TM4SF1, TIM-1, TAA, TA-MUC1 (tumor-specific epitope of mucin-1), Sortilin (SORT1), STn, STING, STEAP-1, SSTR2, SSEA-4, SLITRK6, SLC44A4, SLAMF7, SAIL, Receptor tyrosine kinase (RTK), ROR2, ROR1, RNF43, Prolactin Receptor (PRLR), Polymorphic epithelial mucin (PEM), Phosphatidylserine (PS), Phosphatidyl Serine, PTK7, PSMA, PD-L1, P-Cadherin, OX001L, OAcGD2, Nectin-4, NaPi2b, NOTCH3, Mesothelin (MSLN), MUC16, MTX5, MTX3, MT1-MMP, MRC2, MET, MAGE, Ly6E, Lewis Y antigen, LRRC15, LRP-1, LIV-1, LHRH, LGR5, LGALS3BP, LAMP-1, KLK2, KAAG-1, IL4R, IL7R, IL1RAP, IL-4, IL-3, IL-2, IL-13R, IGF-1R, HSP90, HLA-DR, HER-3, HER-2, Globo H, GPR20, GPC3, GPC-1, GD3, GD2, GCC, FSH, FOLR-alpha, FOLR, FLT3, FGFR3, FGFR2, FCRH5, EphA3, EphA2, EpCAM, ETBR, ENPP3, EGFRviii, EGFR, EFNA4 , Dysadherin, DR5 (Death receptor 5), DPEP3, DLL3, DLK-1, DCLK1, Cripto, Cathepsin D, CanAg, CXCR5, CSP-1, CLL-1, CLDN6, CLDN18.2, CEACAM6, CEACAM5, CEA, CDH6, CD79b, CD74, CD71, CD70, CD56, CD51, CD48, CD46, CD45, CD44v6, CD40L, CD38, CD37, CD352, CD33, CD317, CD30, CD300f, CD3, CD25, CD248, CD228, CD22, CD205, CD20, CD19, CD184, CD166, CD147, CD142, CD138, CD123, CCR7, CA9, CA6, C4.4a, BCMA, B7-H4, B7, -H3, Axl, ASCT2, AMHRII, ALK, AG-7, ADAM-9, 5T4, 4-1BB.
[178] The molecule according to any one of items [38] to [40] or [54] to [131] or [146] to [177] or the antibody-drug conjugate according to any one of items [132] to [133] or [146] to [177] or the method according to any one of items [38] to [40] or [54] to [131] or [134] to [141] or [146] to [177] or the use according to any one of items [38] to [40] or [54] to [131] or [144] to [177], wherein said antibody component (resp. said antibody of item [38]) has a first and a second antigen-binding site.
[179] The molecule or the antibody-drug conjugate or the method or the use according to item [178], wherein said first antigen-binding site is capable of specifically binding to a tumor antigen and said second antigen-binding site is capable of specifically binding to a tumor antigen.
[180] The molecule or the antibody-drug conjugate or the method or the use according to any one of items [178] to [179], wherein said first and said second antigen-binding site are capable of binding to different antigens.
[181] The molecule or the antibody-drug conjugate or the method or the use according to any one of items [165] to [171], wherein said antigen that is present on the surface of said target cell is an immune cell antigen.
[182] The molecule according to any one of items [1] to [6] or [27] to [131] or [146] to [178] or [180] to [181] or the antibody-drug conjugate according to any one of items [132] to [133] or [146] to [178] or [180] to [181] or the method according to any one of items [7] to [19] or [27] to [131] or [134] to [141] or [146] to [178] or [180] to [181] or the use according to any one of items [20] to [131] or [144] to [178] or [180] to [181], wherein said targeting moiety/said antibody component (resp. said antibody of item [38]) is capable of specifically binding to an immune cell antigen.
[183] The molecule or the antibody-drug conjugate or the method or the use according to any one of items [171] or [173] or [178] or [180] to [182], wherein said immune cell antigen is
- an antigen present on the surface of an immune cell,
- an antigen which is a molecule that is secreted by an immune cell, or
- an antigen which is a molecule that interacts with a receptor on an immune cell.
[184] The molecule or the antibody-drug conjugate or the method or the use according to any one of items [171] or [173] or [178] or [180] to [183], wherein said immune cell antigen is selected from the group consisting of CD80, CD86, B7H3, TNF-α, TGF-β, TGF-β2, TGF-1, IL-1, IL-4, IL-5, IL-6, IL-12, IL-13, IL-22, IL-23, interferon receptor, PD-1, PD-L1, CTLA4, MSR1 and folate receptor beta.
[185] The molecule or the antibody-drug conjugate or the method or the use according to any one of items [183] to [184], wherein said immune cell is a B cell, a T cell or a dendritic cell.
[186] The molecule or the antibody-drug conjugate or the method or the use according to any one of items [183] to [185], wherein said immune cell is a T cell.
[187] The molecule or the antibody-drug conjugate or the method or the use according to any one of items [171] or [173] or [178] or [180] to [186], wherein binding of said antibody component (resp. said antibody of item [38]) to said immune cell antigen has an immunostimulatory or immunosuppressive effect.
[188] The molecule according to any one of items [2] to [3] or [5] to [6] or [27] to [131] or [146] to [187] or the method according to any one of items [8] to [9] or [11] to [12] or [14] to [15] or [17] to [19] or [27] to [131] or [146] to [187] or the use according to any one of items [21] to [22] or [24] to [131] or [146] to [187], wherein said molecule comprises only one kind of functional moiety.
[189] The molecule according to any one of items [130] to [131] or [146] to [188] or the antibody-drug conjugate according to any one of items [132] to [133] or [146] to [188] or the method according to any one of items [134] to [141] or [146] to [188] or the use according to any one of items [144] to [188], wherein said antibody-drug conjugate comprises only one kind of payload.
[190] The molecule according to any one of items [130] to [131] or [146] to [189] or the antibody-drug conjugate according to any one of items [132] to [133] or [146] to [189] or the method according to any one of items [134] to [141] or [146] to [189] or the use according to any one of items [142] to [189], wherein the drug-antibody ratio (DAR) of the antibody-drug conjugate is in the range of from 1 to 15.
[191] The molecule according to any one of items [130] to [131] or [146] to [190] or the antibody-drug conjugate according to any one of items [132] to [133] or [146] to [190] or the method according to any one of items [134] to [141] or [146] to [190] or the use according to any one of items [142] to [190], wherein the drug-antibody ratio (DAR) of the antibody-drug conjugate is in the range of from 1 to 10.
[192] The molecule according to any one of items [130] to [131] or [146] to [191] or the antibody-drug conjugate according to any one of items [132] to [133] or [146] to [191] or the method according to any one of items [134] to [141] or [146] to [191] or the use according to any one of items [142] to [191], wherein the drug-antibody ratio (DAR) of the antibody-drug conjugate is in the range of from 1 to 8.
[193] The molecule according to any one of items [130] to [131] or [146] to [192] or the antibody-drug conjugate according to any one of items [132] to [133] or [146] to [192] or the method according to any one of items [134] to [141] or [146] to [192] or the use according to any one of items [142] to [192], wherein the drug-antibody ratio (DAR) of the antibody-drug conjugate is in the range of from 1 to 4.
[194] The molecule according to any one of items [130] to [131] or [146] to [193] or the antibody-drug conjugate according to any one of items [132] to [133] or [146] to [193] or the method according to any one of items [134] to [141] or [146] to [193] or the use according to any one of items [142] to [193], wherein the drug-antibody ratio (DAR) of the antibody-drug conjugate is in the range of from 2 to 8.
[195] The molecule according to any one of items [130] to [131] or [146] to [194] or the antibody-drug conjugate according to any one of items [132] to [133] or [146] to [194] or the method according to any one of items [134] to [141] or [146] to [194] or the use according to any one of items [142] to [194], wherein the drug-antibody ratio (DAR) of the antibody-drug conjugate is in the range of from 4 to 8.
[196] The molecule according to any one of items [2] to [3] or [5] to [6] or [27] to [73] or [75] to [76] or [78] to [131] or [146] to [195] or the antibody-drug conjugate according to any one of items [132] to [133] or [146] to [195] or the method according to any one of items [8] to [9] or [11] to [12] or [14] to [15] or [17] to [19] or [27] to [73] or [75] to [76] or [78] to [131] or [134] to [141] or [146] to [195] or the use according to any one of items [21] to [22] or [24] to [73] or [75] to [76] or [78] to [131] or [144] to [195], wherein said payload (resp. said functional moiety) is a therapeutic agent.
[197] The molecule or the antibody-drug conjugate or the method or the use according to item [196], wherein said therapeutic agent is a cytotoxic agent, anti-inflammatory agent, immunostimulatory agent or immunosuppressive agent.
[198] The molecule or the antibody-drug conjugate or the method or the use according to any one of items [196] to [197], wherein said therapeutic agent is a cytotoxic agent.
[199] The molecule or the antibody-drug conjugate or the method or the use according to any one of items [197] to [198], wherein the cytotoxic agent is selected from the group consisting of an inhibitor of microtubule formation, an EG5 inhibitor and a DNA damaging agent.
[200] The molecule or the antibody-drug conjugate or the method or the use according to item [199], wherein said inhibitor of microtubule formation is selected from the group consisting of an auristatin, a maytansinoid and tubulysin.
[201] The molecule or the antibody-drug conjugate or the method or the use according to item [200], wherein said auristatin is auristatin E, MMAE or MMAF.
[202] The molecule or the antibody-drug conjugate or the method or the use according to any one of items [200] to [201], wherein said auristatin is auristatin E or MMAE.
[203] The molecule or the antibody-drug conjugate or the method or the use according to item [200], wherein said maytansinoid is selected from the group consisting of maytansin, DM1, DM2, DM3 and DM4.
[204] The molecule or the antibody-drug conjugate or the method or the use according to any one of items [200] or [203], wherein said maytansinoid is selected from the group consisting of maytansin, DM2 and DM4.
[205] The molecule or the antibody-drug conjugate or the method or the use according to item [199], wherein said EG5 inhibitor is selected from the group consisting of ispenisib, filanesib, litronesib and K858.
[206] The molecule or the antibody-drug conjugate or the method or the use according to item [199], wherein said DNA damaging agent is selected from the group consisting of a topoisomerase I inhibitor, a topoisomerase II inhibitor and a DNA alkylating agent.
[207] The molecule or the antibody-drug conjugate or the method or the use according to any one of items [200] or [206], wherein the cytotoxic agent is a topoismerase I inhibitor.
[208] The molecule or the antibody-drug conjugate or the method or the use according to any one of items [206] or [207], wherein said topoisomerase I inhibitor is selected from the group consisting of exatecan, camptothecin, SN38, Dxd and variants thereof, wherein, preferably, said topoisomerase I inhibitor is exatecan, SN38 or Dxd.
[209] The molecule or the antibody-drug conjugate or the method or the use according to any one of items [200] or [206], wherein the cytotoxic agent is a topoismerase II inhibitor.
[210] The molecule or the antibody-drug conjugate or the method or the use according to any one of items [206] or [209], wherein said topoismerase II inhibitor is doxorubicin or a variant thereof, preferably doxorubicin.
[211] The molecule or the antibody-drug conjugate or the method or the use according to any one of items [200] or [206], wherein the cytotoxic agent is a DNA alkylating agent.
[212] The molecule or the antibody-drug conjugate or the method or the use according to any one of items [206] or [211], wherein said DNA alkylating agent is selected from the group consisting of duocarmycin, a CBI dimer, a pyrrolobenzodiazepine and variants thereof, wherein, preferably, said DNA alkylating agent is selected from the group consisting of duocarmycin, a CBI dimer and a diazepine (preferably a pyrrolobenzodiazepine or indolinobenzodiazepine).
[213] The molecule or the antibody-drug conjugate or the method or the use according to any one of items [196] to [198], wherein the therapeutic agent is selected from the group consisting of auristatin, MMAE (monomethyl auristatin E), duocarmycin, CBI (Cyclopropanebenz[e]indoline) dimer, maytansin, pyrrolobenzodiazepine and indolinobenzodiazepine.
[214] The molecule or the antibody-drug conjugate or the method or the use according to any one of items [196] to [198], wherein the cytotoxic agent is an exatecan, a duocarmycin or a CBI dimer.
[215] The molecule or the antibody-drug conjugate or the method or the use according to any one of items [196] to [198], wherein the therapeutic agent is selected from the group consisting of an auristatin, a duocarmycin, a CBI (Cyclopropanebenz[e]indoline) dimer and a maytansinoid.
[216] The molecule or the antibody-drug conjugate or the method or the use according to any one of items [196] to [198], wherein the therapeutic agent is selected from the group consisting of MMAE (monomethyl auristatin E), duocarmycin, CBI (Cyclopropanebenz[e]indoline) dimer and maytansinoid DM4.
[217] The molecule or the antibody-drug conjugate or the method or the use according to any one of items [196] to [198], wherein the therapeutic agent is selected from the group consisting of a dolastatin, an auristatin, MMAE, MMAF, amberstatin 269, auristatin 101, auristatin f, auristatin w, CEN-106, CM1, DGN462, DGN549, DM1, DM2, DM4, doxorubicin, duocarmycin, exatecan, OX-4235, PNU-159682, rapamycin, SG3199, SG1882, SN-38, tubulysin, amanitin, aminopterin, anthracycline, calicheamicin, camptothecin, fujimycin, hemiasterlin, a maytansinoid, PBD, rapamycin, vinblastine.
[218] The molecule or the antibody-drug conjugate or the method or the use according to any one of items [196] to [197], wherein the therapeutic agent is an anti-inflammatory agent.
[219] The molecule or the antibody-drug conjugate or the method or the use according to any one of items [197] or [218], wherein the anti-inflammatory agent is a glucocorticoid receptor agonist.
[220] The molecule or the antibody-drug conjugate or the method or the use according to any one of items [197] or [218] to [219], wherein said anti-inflammatory agent is a steroid.
[221] The molecule or the antibody-drug conjugate or the method or the use according to any one of items [197] or [218] to [220], wherein the anti-inflammatory agent is selected from the group consisting of cortisol, cortisone acetate, beclometasone, prednisone, prednisolone, methylprednisolone, betamethasone, trimcinolone, budesonide, dexamethasone, fluticasone, fluticasone propionate, fluticasone furoate and a mometasone.
[222] The molecule or the antibody-drug conjugate or the method or the use according to any one of items [197] or [218] to [219], wherein said anti-inflammatory agent is a non-steroidal anti-inflammatory agent.
[223] The molecule or the antibody-drug conjugate or the method or the use according to item [222], wherein said non-steroidal anti-inflammatory agent is a Cox2 inhibitor.
[224] The molecule or the antibody-drug conjugate or the method or the use according to any one of items [196] to [197], wherein the therapeutic agent is an immunostimulatory agent.
[225] The molecule or the antibody-drug conjugate or the method or the use according to any one of items [197] or [224], wherein said immunostimulatory agent is selected from the group consisting of a TLR7 agonist, a TLR8 agonist, a TLR7 antagonist, a TLR8 antagonist, a Sting inhibitor, a TGF beta inhibitor, an a2A inhibitor and an a2B inhibitor.
[226] The molecule or the antibody-drug conjugate or the method or the use according to any one of items [196] to [197], wherein the therapeutic agent is an immunosuppressive agent.
[227] The molecule or the antibody-drug conjugate or the method or the use according to any one of items [197] or [226], wherein said immunosuppressive agent is selected from the group consisting of an IMDH (inosine monophosphate dehydrogenase) inhibitor, an mTor (mechanistic target of rapamycin) inhibitor, a SYK (spleen tyrosine kinase) inhibitor, a JAK (janus kinase) inhibitor and a calcineurin inhibitor.
[228] The molecule according to any one of items [2] to [3] or [5] to [6] or [27] to [72] or [74] to [75] or [77] to [131] or [146] to [195] or the antibody-drug conjugate according to any one of items [132] to [133] or [146] to [195] or the method according to any one of items [8] to [9] or [11] to [12] or [14] to [15] or [17] to [19] or [27] to [72] or [74] to [75] or [77] to [131] or [134] to [141] or [146] to [195] or the use according to any one of items [21] to [22] or [24] to [72] or [74] to [75] or [77] to [131] or [144] to [195], wherein said payload is a detectable label.
[229] The molecule or the antibody-drug conjugate or the method or the use according to item [228], wherein the detectable label is a radioisotope, fluorescent compound or enzyme.
[230] The molecule or the antibody-drug conjugate or the method or the use according to any one of items [228] to [229], wherein the detectable label is selected from the group consisting of a cyanine dye, a sulfo-cyanine dye, an Alexa Fluor^{®} dye (Molecular Probes/Thermo Fisher Scientific), a DyLight^{®} Fluor dye (Dyomics/Thermo Fisher Scientific), FluoProbes^{®} dyes (Interchim), a Seta^{®} dye (SETA BioMedicals) and an IRIS^{™} dyes (Cyanine Technologies).
[231] The molecule or the antibody-drug conjugate or the method or the use according to any one of items [228] to [230], wherein the detectable label is a cyanine dye selected from the group consisting of Cy2, Cy3, Cy3B, Cy3.5, Cy5, Cy5.5, Cy7.
[232] The molecule or the antibody-drug conjugate or the method or the use according to any one of items [228] to [231], wherein the detectable label is a sulfo-cyanine dye selected from the group consisting of sulfo-Cy2, sulfo-Cy3, sulfo-Cy3B, sulfo-Cy3.5, sulfo-Cy5, sulfo-Cy5.5, sulfo-Cy7.
[233] The molecule according to any one of items [3] or [6] or [27] to [131] or [146] to [232] or the antibody-drug conjugate according to any one of items [132] to [133] or [146] to [232] or the method according to any one of items [9] or [12] or [15] or [18] to [19] or [27] to [131] or [134] to [141] or [146] to [232] or the use according to any one of items [22] or [25] or [27] to [131] or [144] to [232], wherein said linker/each of said linkers has a molecular weight of up to 1,500 Da.
[234] The molecule according to any one of items [3] or [6] or [27] to [131] or [146] to [233] or the antibody-drug conjugate according to any one of items [132] to [133] or [146] to [233] or the method according to any one of items [9] or [12] or [15] or [18] to [19] or [27] to [131] or [134] to [141] or [146] to [233] or the use according to any one of items [22] or [25] or [27] to [131] or [144] to [233], wherein said linker/each of said linkers has a molecular weight of up to 1,000 Da.
[235] The molecule according to any one of items [3] or [6] or [27] to [131] or [146] to [234] or the antibody-drug conjugate according to any one of items [132] to [133] or [146] to [234] or the method according to any one of items [9] or [12] or [15] or [18] to [19] or [27] to [131] or [134] to [141] or [146] to [234] or the use according to any one of items [22] or [25] or [27] to [131] or [144] to [234], wherein said linker/each of said linkers has a molecular weight of up to 500 Da.
[236] The molecule according to any one of items [3] or [6] or [27] to [131] or [146] to [235] or the antibody-drug conjugate according to any one of items [132] to [133] or [146] to [235] or the method according to any one of items [9] or [12] or [15] or [18] to [19] or [27] to [131] or [134] to [141] or [146] to [235] or the use according to any one of items [22] or [25] or [27] to [131] or [144] to [235], wherein said linker is/said linkers are stable in the extracellular environment.
[237] The molecule according to any one of items [3] or [6] or [27] to [131] or [146] to [236] or the antibody-drug conjugate according to any one of items [132] to [133] or [146] to [236] or the method according to any one of items [9] or [12] or [15] or [18] to [19] or [27] to [131] or [134] to [141] or [146] to [236] or the use according to any one of items [22] or [25] or [27] to [131] or [144] to [236], wherein said linker is/said linkers are stable in the intracellular environment.
[238] The molecule according to any one of items [3] or [6] or [27] to [131] or [146] to [236] or the antibody-drug conjugate according to any one of items [132] to [133] or [146] to [236] or the method according to any one of items [9] or [12] or [15] or [18] to [19] or [27] to [131] or [134] to [141] or [146] to [236] or the use according to any one of items [22] or [25] or [27] to [131] or [144] to [236], wherein said linker is/said linkers are cleaved upon exposure to the intracellular environment.
[239] The molecule according to any one of items [3] or [6] or [27] to [131] or [146] to [238] or the antibody-drug conjugate according to any one of items [132] to [133] or [146] to [238] or the method according to any one of items [9] or [12] or [15] or [18] to [19] or [27] to [131] or [134] to [141] or [146] to [238] or the use according to any one of items [22] or [25] or [27] to [131] or [144] to [238], wherein said linker is/said linkers are cleavable by enzymatic or chemical cleavage.
[240] The molecule according to any one of items [3] or [6] or [27] to [131] or [146] to [239] or the antibody-drug conjugate according to any one of items [132] to [133] or [146] to [239] or the method according to any one of items [9] or [12] or [15] or [18] to [19] or [27] to [131] or [134] to [141] or [146] to [239] or the use according to any one of items [22] or [25] or [27] to [131] or [144] to [239], wherein said linker is/said linkers are cleavable by enzymatic cleavage.
[241] The molecule according to any one of items [3] or [6] or [27] to [131] or [146] to [239] or the antibody-drug conjugate according to any one of items [132] to [133] or [146] to [239] or the method according to any one of items [9] or [12] or [15] or [18] to [19] or [27] to [131] or [134] to [141] or [146] to [239] or the use according to any one of items [22] or [25] or [27] to [131] or [144] to [239], wherein said linker is/said linkers are cleavable by chemical cleavage.
[242] The molecule or the antibody-drug conjugate or the method or the use according to item [239] or [240], wherein said enzymatic cleavage is cleavage by exposure to a glycosidase, protease or esterase.
[243] The molecule or the antibody-drug conjugate or the method or the use according to any one of items [239] to [242], wherein said enzymatic cleavage is by exposure to a tumor-specific enzyme, preferably a tumor-specific protease or esterase.
[244] The molecule or the antibody-drug conjugate or the method or the use according to any one of items [242] to [243], wherein said protease is cathepsin.
[245] The molecule or the antibody-drug conjugate or the method or the use according to item [242], wherein said glycosidase is a glucuronidase.
[246] The molecule according to any one of items [3] or [6] or [27] to [131] or [146] to [245] or the antibody-drug conjugate according to any one of items [132] to [133] or [146] to [245] or the method according to any one of items [9] or [12] or [15] or [18] to [19] or [27] to [131] or [134] to [141] or [146] to [245] or the use according to any one of items [22] or [25] or [27] to [131] or [144] to [245], wherein said linker includes/said linkers include a protease cleavage site, preferably a cathepsin B cleavage site.
[247] The molecule according to any one of items [3] or [6] or [27] to [131] or [146] to [246] or the antibody-drug conjugate according to any one of items [132] to [133] or [146] to [246] or the method according to any one of items [9] or [12] or [15] or [18] to [19] or [27] to [131] or [134] to [141] or [146] to [246] or the use according to any one of items [22] or [25] or [27] to [131] or [144] to [246], wherein said linker includes/said linkers include a glucuronide.
[248] The molecule according to any one of items [3] or [6] or [27] to [131] or [146] to [239] or [241] or the antibody-drug conjugate according to any one of items [132] to [133] or [146] to [239] or [241] or the method according to any one of items [9] or [12] or [15] or [18] to [19] or [27] to [131] or [134] to [141] or [146] to [239] or [241] or the use according to any one of items [22] or [25] or [27] to [131] or [144] to [239] or [241], wherein said linker that is cleavable by chemical cleavage is a pH-sensitive linker/wherein said linkers that are cleavable by chemical cleavage are pH-sensitive linkers.
[249] The molecule according to any one of items [3] or [6] or [27] to [131] or [146] to [239] or [241] or [248] or the antibody-drug conjugate according to any one of items [132] to [133] or [146] to [239] or [241] or [248] or the method according to any one of items [9] or [12] or [15] or [18] to [19] or [27] to [131] or [134] to [141] or [146] to [239] or [241] or [248] or the use according to any one of items [22] or [25] or [27] to [131] or [144] to [239] or [241] or [248], wherein said linker includes/said linkers include a hydrazone.
[250] The molecule according to any one of items [239] or [241] or the antibody-drug conjugate according to any one of items [239] or [241] or the method according to any one of items [239] or [241] or the use according to any one of items [239] or [241], wherein said linker that is cleavable by chemical cleavage is cleavable under reducing conditions/wherein said linkers that are cleavable by chemical cleavage are cleavable under reducing conditions.
[251] The molecule according to any one of items [3] or [6] or [27] to [131] or [146] to [239] or [241] or [250] or the antibody-drug conjugate according to any one of items [132] to [133] or [146] to [239] or [241] or [250] or the method according to any one of items [9] or [12] or [15] or [18] to [19] or [27] to [131] or [134] to [141] or [146] to [239] or [241] or [250] or the use according to any one of items [22] or [25] or [27] to [131] or [144] to [239] or [241] or [250], wherein said linker includes/said linkers include a disulfide linkage.
[252] The molecule according to any one of items [3] or [6] or [27] to [131] or [146] to [251] or the antibody-drug conjugate according to any one of items [132] to [133] or [146] to [251] or the method according to any one of items [9] or [12] or [15] or [18] to [19] or [27] to [131] or [134] to [141] or [146] to [251] or the use according to any one of items [22] or [25] or [27] to [131] or [144] to [251], wherein said linker comprises/said linkers comprise a cathepsin B cleavage site, a glucuronide or a disulfide linkage.
[253] The molecule according to any one of items [1] to [6] or [27] to [131] or [146] to [252] or the antibody-drug conjugate according to any one of items [132] to [133] or [146] to [252] or the method according to any one of items [7] to [19] or [27] to [131] or [134] to [141] or [146] to [252] or the use according to any one of items [20] to [131] or [142] to [252], wherein said solubility tag is/said solubility tags are linked by a covalent bond to the targeting moiety or the functional moiety/moieties or the linker(s).
[254] The molecule according to any one of items [1] to [6] or [27] to [131] or [146] to [253] or the method according to any one of items [7] to [19] or [27] to [131] or [146] to [253] or the use according to any one of items [20] to [131] or [146] to [253], wherein said solubility tag is/said solubility tags are linked by a covalent bond to the targeting moiety.
[255] The molecule according to any one of items [2] to [3] or [5] to [6] or [27] to [131] or [146] to [254] the antibody-drug conjugate according to any one of items [132] to [133] or [146] to [254] or the method according to any one of items [8] to [9] or [11] to [12] or [14] to [15] or [17] to [19] or [27] to [131] or [146] to [254] or the use according to any one of items [21] to [22] or [24] to [131] or [146] to [254], wherein said solubility tag is/said solubility tags are linked by a covalent bond to the functional moiety/moieties.
[256] The molecule according to any one of items [3] or [6] or [27] to [131] or [146] to [255] or the antibody-drug conjugate according to any one of items [132] to [133] or [146] to [255] or the method according to any one of items [9] or [12] or [15] or [18] to [19] or [27] to [131] or [146] to [255] or the use according to any one of items [22] or [25] to [131] or [146] to [255], wherein said solubility tag is/said solubility tags are linked by a covalent bond to the linker(s).
[257] The molecule according to any one of items [3] or [6] or [27] to [131] or [146] to [256] or the antibody-drug conjugate according to any one of items [132] to [133] or [146] to [256] or the method according to any one of items [9] or [12] or [15] or [18] to [19] or [27] to [131] or [134] to [141] or [146] to [256] or the use according to any one of items [22] or [25] to [131] or [144] to [256], wherein said solubility tag is/said solubility tags are linked by a covalent bond to the antibody component, the at least one payload or the linker(s).
[258] The molecule according to any one of items [39] to [42] or [44] or [46] to [47] or [49] or [51] to [52] or [54] to [131] or [146] to [257] or the antibody-drug conjugate according to any one of items [132] to [133] or [146] to [257] or the method according to any one of items [39] to [42] or [44] or [46] to [47] or [49] or [51] to [52] or [54] to [131] or [134] to [141] or [146] to [257] or the use according to any one of items [39] to [42] or [44] or [46] to [47] or [49] or [51] to [52] or [54] to [131] or [144] to [257], wherein said solubility tag is/said solubility tags are linked by a covalent bond to said antibody component.
[259] The molecule according to any one of items [75] to [131] or [146] to [258] or the antibody-drug conjugate according to any one of items [132] to [133] or [146] to [258] or the method according to any one of items [75] to [131] or [134] to [141] or [146] to [258] or the use according to any one of items [75] to [131] or [144] to [258], wherein said solubility tag is/said solubility tags are linked by a covalent bond to said at least one payload.
[260] The molecule according to any one of items [3] or [6] or [27] to [131] or [146] to [259] or the antibody-drug conjugate according to any one of items [132] to [133] or [146] to [259] or the method according to any one of items [9] or [12] or [15] or [18] to [19] or [27] to [131] or [134] to [141] or [146] to [259] or the use according to any one of items [22] or [25] to [131] or [144] to [259], wherein said solubility tag is/said solubility tags are linked by a covalent bond to said linker(s).
[261] The molecule according to any one of items [3] or [6] or [27] to [131] or [146] to [260] or the antibody-drug conjugate according to any one of items [132] to [133] or [146] to [260] or the method according to any one of items [9] or [12] or [15] or [18] to [19] or [27] to [131] or [134] to [141] or [146] to [260] or the use according to any one of items [22] or [25] to [131] or [144] to [260], wherein said solubility tag is/said solubility tags are linked by a covalent bond
- to the antibody component, but not to the at least one payload or the linker(s), or
- to the at least one payload, but not to the antibody component or the linker(s), or
- to the linker(s), but not to the antibody component or the at least one payload.
[262] The molecule according to any one of items [38] to [42] or [44] or [46] to [47] or [49] or [51] to [52] or [54] to [131] or [146] to [261] or the antibody-drug conjugate according to any one of items [132] to [133] or [146] to [261] or the method according to any one of items [38] to [42] or [44] or [46] to [47] or [49] or [51] to [52] or [54] to [131] or [134] to [141] or [146] to [261] or the use according to any one of items [38] to [42] or [44] or [46] to [47] or [49] or [51] to [52] or [54] to [131] or [144] to [261], wherein said solubility tag is/said solubility tags are linked by a covalent bond only to the antibody component (but not to the at least one payload or the linker(s)).
[263] The molecule according to any one of items [75] to [131] or [146] to [261] or the antibody-drug conjugate according to any one of items [132] to [133] or [146] to [261] or the method according to any one of items [75] to [131] or [134] to [141] or [146] to [261] or the use according to any one of items [75] to [131] or [144] to [261], wherein said solubility tag is/said solubility tags are linked by a covalent bond only to the at least one payload (but not to the antibody component or the linker(s)).
[264] The molecule according to any one of items [3] or [6] or [27] to [131] or [146] to [261] or the antibody-drug conjugate according to any one of items [132] to [133] or [146] to [261] or the method according to any one of items [9] or [12] or [15] or [18] to [19] or [27] to [131] or [134] to [141] or [146] to [261] or the use according to any one of items [22] or [25] to [131] or [144] to [261], wherein said solubility tag is/said solubility tags are linked by a covalent bond only to the linker(s) (but not to the antibody component or the at least one payload).
[265] The molecule according to any one of items [130] to [131] or [146] to [264] or the antibody-drug conjugate according to any one of items [132] to [133] or [146] to [264] or the method according to any one of items [134] to [141] or [146] to [264] or the use according to any one of items [142] to [264], wherein said solubility tag is/said solubility tags are linked by a covalent bond to at least one of (i) to (iii).
[266] The molecule according to any one of items [130] to [131] or [146] to [265] or the antibody-drug conjugate according to any one of items [132] to [133] or [146] to [265] or the method according to any one of items [134] to [141] or [146] to [265] or the use according to any one of items [142] to [265], wherein said solubility tag is/said solubility tags are linked by a covalent bond to at least one of
(i) the antibody component,
(ii) the at least one payload,
(iii) the linker/linkers covalently linking said payload/payloads and said antibody component
   of the antibody-drug conjugate.
[267] The molecule according to any one of items [130] to [131] or [146] to [266] or the antibody-drug conjugate according to any one of items [132] to [133] or [146] to [266] or the method according to any one of items [134] to [141] or [146] to [266] or the use according to any one of items [142] to [266], wherein the antibody component (i), the payload(s) (ii) and the linker(s) (iii) are covalently linked.
[268] The molecule according to any one of items [130] to [131] or [146] to [267] or the antibody-drug conjugate according to any one of items [132] to [133] or [146] to [267] or the method according to any one of items [134] to [141] or [146] to [267] or the use according to any one of items [142] to [267], wherein the antibody component (i), the payload(s) (ii), the linker(s) (iii) and the solubility tag(s) (iv) are covalently linked.
[269] The molecule according to any one of items [1] to [6] or [27] to [131] or [146] to [268] or the method according to any one of items [7] to [19] or [27] to [131] or [134] to [141] or [146] to [268] or the use according to any one of items [20] to [131] or [142] to [268], wherein said molecule comprises at least one solubility tag.
[270] The molecule according to any one of items [1] to [6] or [27] to [131] or [146] to [269] or the method according to any one of items [7] to [19] or [27] to [131] or [134] to [141] or [146] to [269] or the use according to any one of items [20] to [131] or [142] to [269], wherein said molecule comprises at least 2 solubility tags.
[271] The molecule according to any one of items [1] to [6] or [27] to [131] or [146] to [270] or the method according to any one of items [7] to [19] or [27] to [131] or [134] to [141] or [146] to [270] or the use according to any one of items [20] to [131] or [142] to [270], wherein said molecule comprises at least 3 solubility tags.
[272] The molecule according to any one of items [1] to [6] or [27] to [131] or [146] to [271] or the method according to any one of items [7] to [19] or [27] to [131] or [134] to [141] or [146] to [271] or the use according to any one of items [20] to [131] or [142] to [271], wherein said molecule comprises at least 4 solubility tags.
[273] The molecule according to any one of items [1] to [6] or [27] to [131] or [146] to [272] or the method according to any one of items [7] to [19] or [27] to [131] or [134] to [141] or [146] to [272] or the use according to any one of items [20] to [131] or [142] to [272], wherein said molecule comprises up to 10 solubility tags.
[274] The molecule according to any one of items [1] to [6] or [27] to [131] or [146] to [273] or the method according to any one of items [7] to [19] or [27] to [131] or [134] to [141] or [146] to [273] or the use according to any one of items [20] to [131] or [142] to [273], wherein said molecule comprises up to 8 solubility tags.
[275] The molecule according to any one of items [1] to [6] or [27] to [131] or [146] to [274] or the method according to any one of items [7] to [19] or [27] to [131] or [134] to [141] or [146] to [274] or the use according to any one of items [20] to [131] or [142] to [274], wherein said molecule comprises up to 6 solubility tags.
[276] The molecule according to any one of items [1] to [6] or [27] to [131] or [146] to [275] or the method according to any one of items [7] to [19] or [27] to [131] or [134] to [141] or [146] to [275] or the use according to any one of items [20] to [131] or [142] to [275], wherein said molecule comprises up to 4 solubility tags.
[277] The molecule according to any one of items [1] to [6] or [27] to [131] or [146] to [270] or the method according to any one of items [7] to [19] or [27] to [131] or [134] to [141] or [146] to [270] or the use according to any one of items [20] to [131] or [142] to [270], wherein said molecule comprises up to 2 solubility tags.
[278] The molecule according to any one of items [1] to [6] or [27] to [131] or [146] to [269] or the method according to any one of items [7] to [19] or [27] to [131] or [134] to [141] or [146] to [269] or the use according to any one of items [20] to [131] or [142] to [269], wherein said molecule comprises only one solubility tag.
[279] The molecule according to any one of items [1] to [6] or [27] to [131] or [146] to [277] or the method according to any one of items [7] to [19] or [27] to [131] or [134] to [141] or [146] to [277] or the use according to any one of items [20] to [131] or [142] to [277], wherein said molecule comprises at least 1 and up to 4 solubility tags.
[280] The molecule according to any one of items [1] to [6] or [27] to [131] or [146] to [276] or [279] or the method according to any one of items [7] to [19] or [27] to [131] or [134] to [141] or [146] to [276] or [279] or the use according to any one of items [20] to [131] or [142] to [276] or [279], wherein said molecule comprises at least 3 and up to 10 solubility tags.
[281] The molecule according to any one of items [1] to [6] or [27] to [131] or [146] to [280] or the method according to any one of items [7] to [19] or [27] to [131] or [134] to [141] or [146] to [280] or the use according to any one of items [20] to [131] or [142] to [280], wherein at least one solubility tag is covalently linked to said molecule.
[282] The molecule according to any one of items [1] to [6] or [27] to [131] or [146] to [277] or [279] to [281] or the method according to any one of items [7] to [19] or [27] to [131] or [134] to [141] or [146] to [277] or [279] to [281] or the use according to any one of items [20] to [131] or [142] to [277] or [279] to [281], wherein at least 2 solubility tags are covalently linked to said molecule.
[283] The molecule according to any one of items [1] to [6] or [27] to [131] or [146] to [276] or [279] to [282] or the method according to any one of items [7] to [19] or [27] to [131] or [134] to [141] or [146] to [276] or [279] to [282] or the use according to any one of items [20] to [131] or [142] to [276] or [279] to [282], wherein at least 3 solubility tags are covalently linked to said molecule.
[284] The molecule according to any one of items [1] to [6] or [27] to [131] or [146] to [276] or [279] to [283] or the method according to any one of items [7] to [19] or [27] to [131] or [134] to [141] or [146] to [276] or [279] to [283] or the use according to any one of items [20] to [131] or [142] to [276] or [279] to [283], wherein at least 4 solubility tags are covalently linked to said molecule.
[285] The molecule according to any one of items [1] to [6] or [27] to [131] or [146] to [284] or the method according to any one of items [7] to [19] or [27] to [131] or [134] to [141] or [146] to [284] or the use according to any one of items [20] to [131] or [142] to [284], wherein up to 10 solubility tags are covalently linked to said molecule.
[286] The molecule according to any one of items [1] to [6] or [27] to [131] or [146] to [285] or the method according to any one of items [7] to [19] or [27] to [131] or [134] to [141] or [146] to [285] or the use according to any one of items [20] to [131] or [142] to [285], wherein up to 6 solubility tags are covalently linked to said molecule.
[287] The molecule according to any one of items [1] to [6] or [27] to [131] or [146] to [286] or the method according to any one of items [7] to [19] or [27] to [131] or [134] to [141] or [146] to [286] or the use according to any one of items [20] to [131] or [142] to [286], wherein up to 4 solubility tags are covalently linked to said molecule.
[288] The molecule according to any one of items [1] to [6] or [27] to [131] or [146] to [287] or the method according to any one of items [7] to [19] or [27] to [131] or [134] to [141] or [146] to [287] or the use according to any one of items [20] to [131] or [142] to [287], wherein up to 2 solubility tags are covalently linked to said molecule.
[289] The molecule according to any one of items [1] to [6] or [27] to [131] or [146] to [288] or the method according to any one of items [7] to [19] or [27] to [131] or [134] to [141] or [146] to [288] or the use according to any one of items [20] to [131] or [142] to [288], wherein only 1 solubility tag is covalently linked to said molecule.
[290] The molecule according to any one of items [1] to [6] or [27] to [131] or [146] to [289] or the method according to any one of items [7] to [19] or [27] to [131] or [134] to [141] or [146] to [289] or the use according to any one of items [20] to [131] or [142] to [289], wherein at least 1 and up to 4 solubility tags solubility tags are covalently linked to said molecule.
[291] The molecule according to any one of items [1] to [6] or [27] to [131] or [146] to [276] or [279] to [290] or the method according to any one of items [7] to [19] or [27] to [131] or [134] to [141] or [146] to [276] or [279] to [290] or the use according to any one of items [20] to [131] or [142] to [276] or [279] to [290], wherein at least 3 and up to 10 solubility tags solubility tags are covalently linked to said molecule.
[292] The molecule according to any one of items [130] to [131] or [146] to [266] or the antibody-drug conjugate according to any one of items [132] to [133] or [146] to [268] or the method according to any one of items [134] to [141] or [146] to [268] or the use according to any one of items [142] to [268], wherein said antibody-drug conjugate comprises at least one solubility tag.
[293] The molecule according to any one of items [130] to [131] or [146] to [268] or [292] or the antibody-drug conjugate according to any one of items [132] to [133] or [146] to [268] or [292] or the method according to any one of items [134] to [141] or [146] to [268] or [292] or the use according to any one of items [142] to [268] or [292], wherein said antibody-drug conjugate comprises at least 2 solubility tags.
[294] The molecule according to any one of items [130] to [131] or [146] to [268] or [292] to [293] or the antibody-drug conjugate according to any one of items [132] to [133] or [146] to [268] or [292] to [293] or the method according to any one of items [134] to [141] or [146] to [268] or [292] to [293] or the use according to any one of items [142] to [268] or [292] to [293], wherein said antibody-drug conjugate comprises at least 3 solubility tags.
[295] The molecule according to any one of items [130] to [131] or [146] to [268] or [292] to [294] or the antibody-drug conjugate according to any one of items [132] to [133] or [146] to [268] or [292] to [294] or the method according to any one of items [134] to [141] or [146] to [268] or [292] to [294] or the use according to any one of items [142] to [268] or [292] to [294], wherein said antibody-drug conjugate comprises at least 4 solubility tags.
[296] The molecule according to any one of items [130] to [131] or [146] to [268] or [292] to [295] or the antibody-drug conjugate according to any one of items [132] to [133] or [146] to [268] or [292] to [295] or the method according to any one of items [134] to [141] or [146] to [268] or [292] to [295] or the use according to any one of items [142] to [268] or [292] to [295], wherein said antibody-drug conjugate comprises up to 10 solubility tags.
[297] The molecule according to any one of items [130] to [131] or [146] to [268] or [292] to [296] or the antibody-drug conjugate according to any one of items [132] to [133] or [146] to [268] or [292] to [296] or the method according to any one of items [134] to [141] or [146] to [268] or [292] to [296] or the use according to any one of items [142] to [268] or [292] to [296], wherein said antibody-drug conjugate comprises up to 8 solubility tags.
[298] The molecule according to any one of items [130] to [131] or [146] to [268] or [292] to [297] or the antibody-drug conjugate according to any one of items [132] to [133] or [146] to [268] or [292] to [297] or the method according to any one of items [134] to [141] or [146] to [268] or [292] to [297] or the use according to any one of items [142] to [268] or [292] to [297], wherein said antibody-drug conjugate comprises up to 6 solubility tags.
[299] The molecule according to any one of items [130] to [131] or [146] to [268] or [292] to [298] or the antibody-drug conjugate according to any one of items [132] to [133] or [146] to [268] or [292] to [298] or the method according to any one of items [134] to [141] or [146] to [268] or [292] to [298] or the use according to any one of items [142] to [268] or [292] to [298], wherein said antibody-drug conjugate comprises up to 4 solubility tags.
[300] The molecule according to any one of items [130] to [131] or [146] to [268] or [292] to [293] or the antibody-drug conjugate according to any one of items [132] to [133] or [146] to [268] or [292] to [293] or the method according to any one of items [134] to [141] or [146] to [268] or [292] to [293] or the use according to any one of items [142] to [268] or [292] to [293], wherein said antibody-drug conjugate comprises up to 2 solubility tags.
[301] The molecule according to any one of items [130] to [131] or [146] to [268] or [292] to [295] or the antibody-drug conjugate according to any one of items [132] to [133] or [146] to [268] or [292] to [295] or the method according to any one of items [134] to [141] or [146] to [268] or [292] to [295] or the use according to any one of items [142] to [268] or [292] to [295], wherein said antibody-drug conjugate comprises at least 1 and up to 4 solubility tags.
[302] The molecule according to any one of items [130] to [131] or [146] to [268] or [292] to [299] or [301] or the antibody-drug conjugate according to any one of items [132] to [133] or [146] to [268] or [292] to [299] or [301] or the method according to any one of items [134] to [141] or [146] to [268] or [292] to [299] or [301] or the use according to any one of items [142] to [268] or [292] to [299] or [301], wherein said antibody-drug conjugate comprises at least 3 and up to 10 solubility tags.
[303] The molecule according to any one of items [130] to [131] or [146] to [268] or [292] or the antibody-drug conjugate according to any one of items [132] to [133] or [146] to [268] or [292] or the method according to any one of items [134] to [141] or [146] to [268] or [292] or the use according to any one of items [142] to [268] or [292], wherein said antibody-drug conjugate comprises only one solubility tag.
[304] The molecule according to any one of items [130] to [131] or [146] to [268] or [292] to [303] or the antibody-drug conjugate according to any one of items [132] to [133] or [146] to [268] or [292] to [303] or the method according to any one of items [134] to [141] or [146] to [268] or [292] to [303] or the use according to any one of items [142] to [268] or [292] to [303], wherein at least one solubility tag is covalently linked to said antibody-drug conjugate.
[305] The molecule according to any one of items [130] to [131] or [146] to [268] or [292] to [302] or [304] or the antibody-drug conjugate according to any one of items [132] to [133] or [146] to [268] or [292] to [302] or [304] or the method according to any one of items [134] to [141] or [146] to [268] or [292] to [302] or [304] or the use according to any one of items [142] to [268] or [292] to [302] or [304], wherein at least 2 solubility tags are covalently linked to said antibody-drug conjugate.
[306] The molecule according to any one of items [130] to [131] or [146] to [268] or [292] to [299] or [301] to [302] or [304] or the antibody-drug conjugate according to any one of items [132] to [133] or [146] to [268] or [292] to [299] or [301] to [302] or [304] or the method according to any one of items [134] to [141] or [146] to [268] or [292] to [299] or [301] to [302] or [304] or the use according to any one of items [142] to [268] or [292] to [299] or [301] to [302] or [304], wherein at least 3 solubility tags are covalently linked to said antibody-drug conjugate.
[307] The molecule according to any one of items [130] to [131] or [146] to [268] or [292] to [299] or [301] to [302] or [304] or the antibody-drug conjugate according to any one of items [132] to [133] or [146] to [268] or [292] to [299] or [301] to [302] or [304] or the method according to any one of items [134] to [141] or [146] to [268] or [292] to [299] or [301] to [302] or [304] or the use according to any one of items [142] to [268] or [292] to [299] or [301] to [302] or [304], wherein at least 4 solubility tags are covalently linked to said antibody-drug conjugate.
[308] The molecule according to any one of items [130] to [131] or [146] to [268] or [292] to [304] or the antibody-drug conjugate according to any one of items [132] to [133] or [146] to [268] or [292] to [304] or the method according to any one of items [134] to [141] or [146] to [268] or [292] to [304] or the use according to any one of items [142] to [268] or [292] to [304], wherein only one solubility tag is covalently linked to said antibody-drug conjugate.
[309] The molecule according to any one of items [130] to [131] or [146] to [268] or [292] to [305] or the antibody-drug conjugate according to any one of items [132] to [133] or [146] to [268] or [292] to [305] or the method according to any one of items [134] to [141] or [146] to [268] or [292] to [305] or the use according to any one of items [142] to [268] or [292] to [305], wherein up to 2 solubility tags are covalently linked to said antibody-drug conjugate.
[310] The molecule according to any one of items [130] to [131] or [146] to [268] or [292] to [309] or the antibody-drug conjugate according to any one of items [132] to [133] or [146] to [268] or [292] to [309] or the method according to any one of items [134] to [141] or [146] to [268] or [292] to [309] or the use according to any one of items [142] to [268] or [292] to [309], wherein up to 4 solubility tags are covalently linked to said antibody-drug conjugate.
[311] The molecule according to any one of items [130] to [131] or [146] to [268] or [292] to [310] or the antibody-drug conjugate according to any one of items [132] to [133] or [146] to [268] or [292] to [310] or the method according to any one of items [134] to [141] or [146] to [268] or [292] to [310] or the use according to any one of items [142] to [268] or [292] to [310], wherein up to 6 solubility tags are covalently linked to said antibody-drug conjugate.
[312] The molecule according to any one of items [130] to [131] or [146] to [268] or [292] to [311] or the antibody-drug conjugate according to any one of items [132] to [133] or [146] to [268] or [292] to [311] or the method according to any one of items [134] to [141] or [146] to [268] or [292] to [311] or the use according to any one of items [142] to [268] or [292] to [311], wherein up to 8 solubility tags are covalently linked to said antibody-drug conjugate.
[313] The molecule according to any one of items [130] to [131] or [146] to [268] or [292] to [312] or the antibody-drug conjugate according to any one of items [132] to [133] or [146] to [268] or [292] to [312] or the method according to any one of items [134] to [141] or [146] to [268] or [292] to [312] or the use according to any one of items [142] to [268] or [292] to [312], wherein up to 10 solubility tags are covalently linked to said antibody-drug conjugate.
[314] The molecule according to any one of items [130] to [131] or [146] to [268] or [292] to [313] or the antibody-drug conjugate according to any one of items [132] to [133] or [146] to [268] or [292] to [313] or the method according to any one of items [134] to [141] or [146] to [268] or [292] to [313] or the use according to any one of items [142] to [268] or [292] to [313], wherein at least 1 and up to 4 solubility tags are covalently linked to said antibody-drug conjugate.
[315] The molecule according to any one of items [130] to [131] or [146] to [268] or [292] to [299] or [301] to [302] or [304] to [307] or [310] to [314] or the antibody-drug conjugate according to any one of items [132] to [133] or [146] to [268] or [292] to [299] or [301] to [302] or [304] to [307] or [310] to [314] or the method according to any one of items [134] to [141] or [146] to [268] or [292] to [299] or [301] to [302] or [304] to [307] or [310] to [314] or the use according to any one of items [142] to [268] or [292] to [299] or [301] to [302] or [304] to [307] or [310] to [314], wherein at least 3 and up to 10 solubility tags are covalently linked to said antibody-drug conjugate.
[316] The molecule according to any one of items [3] or [6] or [27] to [131] or [146] to [315] or the antibody-drug conjugate according to any one of items [132] to [133] or [146] to [315] or the method according to any one of items [9] or [12] or [15] or [18] to [19] or [27] to [131] or [134] to [141] or [146] to [315] or the use according to any one of items [22] or [25] to [131] or [144] to [315], wherein not more than three solubility tags are covalently linked per linker.
[317] The molecule according to any one of items [3] or [6] or [27] to [131] or [146] to [316] or the antibody-drug conjugate according to any one of items [132] to [133] or [146] to [316] or the method according to any one of items [9] or [12] or [15] or [18] to [19] or [27] to [131] or [134] to [141] or [146] to [316] or the use according to any one of items [22] or [25] to [131] or [144] to [316], wherein not more than two solubility tags are covalently linked per linker.
[318] The molecule according to any one of items [3] or [6] or [27] to [131] or [146] to [317] or the antibody-drug conjugate according to any one of items [132] to [133] or [146] to [317] or the method according to any one of items [9] or [12] or [15] or [18] to [19] or [27] to [131] or [134] to [141] or [146] to [317] or the use according to any one of items [22] or [25] to [131] or [144] to [317], wherein not more than one solubility tag is covalently linked per linker.
[319] The molecule according to any one of items [75] to [131] or [146] to [318] or the antibody-drug conjugate according to any one of items [132] to [133] or [146] to [318] or the method according to any one of items [75] to [131] or [134] to [141] or [146] to [318] or the use according to any one of items [75] to [131] or [144] to [318], wherein not more than three solubility tags are covalently linked per payload.
[320] The molecule according to any one of items [75] to [131] or [146] to [319] or the antibody-drug conjugate according to any one of items [132] to [133] or [146] to [319] or the method according to any one of items [75] to [131] or [134] to [141] or [146] to [319] or the use according to any one of items [75] to [131] or [144] to [319], wherein not more than two solubility tags are covalently linked per payload.
[321] The molecule according to any one of items [75] to [131] or [146] to [320] or the antibody-drug conjugate according to any one of items [132] to [133] or [146] to [320] or the method according to any one of items [75] to [131] or [134] to [141] or [146] to [320] or the use according to any one of items [75] to [131] or [144] to [320], wherein not more than one solubility tag is covalently linked per payload.
[322] The molecule according to any one of items [39] to [42] or [44] or [46] to [47] or [49] or [51] to [52] or [54] to [131] or [146] to [321] or the antibody-drug conjugate according to any one of items [132] to [133] or [146] to [321] or the method according to any one of items [39] to [42] or [44] or [46] to [47] or [49] or [51] to [52] or [54] to [131] or [134] to [141] or [146] to [321] or the use according to any one of items [39] to [42] or [44] or [46] to [47] or [49] or [51] to [52] or [54] to [131] or [144] to [321], wherein not more than three solubility tags are covalently linked per antibody component.
[323] The molecule according to any one of items [39] to [42] or [44] or [46] to [47] or [49] or [51] to [52] or [54] to [131] or [146] to [322] or the antibody-drug conjugate according to any one of items [132] to [133] or [146] to [322] or the method according to any one of items [39] to [42] or [44] or [46] to [47] or [49] or [51] to [52] or [54] to [131] or [134] to [141] or [146] to [322] or the use according to any one of items [39] to [42] or [44] or [46] to [47] or [49] or [51] to [52] or [54] to [131] or [144] to [322], wherein not more than two solubility tags are covalently linked per antibody component.
[324] The molecule according to any one of items [39] to [42] or [44] or [46] to [47] or [49] or [51] to [52] or [54] to [131] or [146] to [323] or the antibody-drug conjugate according to any one of items [132] to [133] or [146] to [323] or the method according to any one of items [39] to [42] or [44] or [46] to [47] or [49] or [51] to [52] or [54] to [131] or [134] to [141] or [146] to [323] or the use according to any one of items [39] to [42] or [44] or [46] to [47] or [49] or [51] to [52] or [54] to [131] or [144] to [323], wherein not more than one solubility tag is covalently linked per antibody component.
[325] The molecule according to any one of items [130] to [131] or [146] to [324] or the antibody-drug conjugate according to any one of items [132] to [133] or [146] to [324] or the method according to any one of items [134] to [141] or [146] to [324] or the use according to any one of items [142] to [324], wherein only one kind of solubility tag is covalently attached to said antibody-drug conjugate.
[326] The molecule according to any one of items [130] to [131] or [146] to [324] or the antibody-drug conjugate according to any one of items [132] to [133] or [146] to [324] or the method according to any one of items [134] to [141] or [146] to [324] or the use according to any one of items [142] to [324], wherein more than one kind of solubility tag is covalently attached to said antibody-drug conjugate.
[327] The molecule according to any one of items [130] to [131] or [146] to [324] or [326] or the antibody-drug conjugate according to any one of items [132] to [133] or [146] to [324] or [326] or the method according to any one of items [134] to [141] or [146] to [324] or [326] or the use according to any one of items [142] to [324] or [326], wherein up to two kinds of solubility tags are covalently attached to said antibody-drug conjugate.
[328] The molecule according to any one of items [130] to [131] or [146] to [327] or the antibody-drug conjugate according to any one of items [132] to [133] or [146] to [327] or the method according to any one of items [134] to [141] or [146] to [327] or the use according to any one of items [142] to [327], wherein said antibody-drug conjugate comprises only one kind of solubility tag.
[329] The molecule according to any one of items [130] to [131] or [146] to [327] or the antibody-drug conjugate according to any one of items [132] to [133] or [146] to [327] or the method according to any one of items [134] to [141] or [146] to [327] or the use according to any one of items [142] to [327], wherein said antibody-drug conjugate comprises more than one kind of solubility tag.
[330] The molecule according to any one of items [130] to [131] or [146] to [327] or [329] or the antibody-drug conjugate according to any one of items [132] to [133] or [146] to [327] or [329] or the method according to any one of items [134] to [141] or [146] to [327] or [329] or the use according to any one of items [142] to [327] or [329], wherein said antibody-drug conjugate comprises up to two different kinds of solubility tags.
[331] The molecule according to any one of items [1] to [6] or [27] to [131] or [146] to [330] or the antibody-drug conjugate according to any one of items [132] to [133] or [146] to [330] or the method according to any one of items [7] to [19] or [27] to [131] or [134] to [141] or [146] to [330] or the use according to any one of items [20] to [131] or [142] to [330], wherein said solubility tag comprises/said solubility tags comprise monosaccharide units.
[332] The molecule according to any one of items [1] to [6] or [27] to [131] or [146] to [331] or the antibody-drug conjugate according to any one of items [132] to [133] or [146] to [331] or the method according to any one of items [7] to [19] or [27] to [131] or [134] to [141] or [146] to [331] or the use according to any one of items [20] to [131] or [142] to [331], wherein said solubility tag consists of/said solubility tags consist of monosaccharide units.
[333] The molecule according to any one of items [1] to [6] or [27] to [131] or [146] to [332] or the antibody-drug conjugate according to any one of items [132] to [133] or [146] to [332] or the method according to any one of items [7] to [19] or [27] to [131] or [134] to [141] or [146] to [332] or the use according to any one of items [20] to [131] or [142] to [332], wherein said solubility tag comprises/said solubility tags comprise an oligosaccharide consisting of monosaccharide units.
[334] The molecule according to any one of items [1] to [6] or [27] to [131] or [146] to [333] or the antibody-drug conjugate according to any one of items [132] to [133] or [146] to [333] or the method according to any one of items [7] to [19] or [27] to [131] or [134] to [141] or [146] to [333] or the use according to any one of items [20] to [131] or [142] to [333], wherein said solubility tag consists of/said solubility tags consist of an oligosaccharide consisting of monosaccharide units.
[335] The molecule according to any one of items [1] to [6] or [27] to [131] or [146] to [334] or the antibody-drug conjugate according to any one of items [132] to [133] or [146] to [334] or the method according to any one of items [7] to [19] or [27] to [131] or [134] to [141] or [146] to [334] or the use according to any one of items [20] to [131] or [142] to [334], wherein the solubility tag comprises/each solubility tag comprises up to 25 monosaccharide units.
[336] The molecule according to any one of items [1] to [6] or [27] to [131] or [146] to [335] or the antibody-drug conjugate according to any one of items [132] to [133] or [146] to [335] or the method according to any one of items [7] to [19] or [27] to [131] or [134] to [141] or [146] to [335] or the use according to any one of items [20] to [131] or [142] to [335], wherein the solubility tag comprises/each solubility tag comprises up to 20 monosaccharide units.
[337] The molecule according to any one of items [1] to [6] or [27] to [131] or [146] to [336] or the antibody-drug conjugate according to any one of items [132] to [133] or [146] to [336] or the method according to any one of items [7] to [19] or [27] to [131] or [134] to [141] or [146] to [336] or the use according to any one of items [20] to [131] or [142] to [336], wherein the solubility tag comprises/each solubility tag comprises up to 15 monosaccharide units.
[338] The molecule according to any one of items [1] to [6] or [27] to [131] or [146] to [337] or the antibody-drug conjugate according to any one of items [132] to [133] or [146] to [337] or the method according to any one of items [7] to [19] or [27] to [131] or [134] to [141] or [146] to [337] or the use according to any one of items [20] to [131] or [142] to [337], wherein the solubility tag comprises/each solubility tag comprises up to 12 monosaccharide units.
[339] The molecule according to any one of items [1] to [6] or [27] to [131] or [146] to [338] or the antibody-drug conjugate according to any one of items [132] to [133] or [146] to [338] or the method according to any one of items [7] to [19] or [27] to [131] or [134] to [141] or [146] to [338] or the use according to any one of items [20] to [131] or [142] to [338], wherein the solubility tag comprises/each solubility tag comprises up to 10 monosaccharide units.
[340] The molecule according to any one of items [1] to [6] or [27] to [131] or [146] to [339] or the antibody-drug conjugate according to any one of items [132] to [133] or [146] to [339] or the method according to any one of items [7] to [19] or [27] to [131] or [134] to [141] or [146] to [339] or the use according to any one of items [20] to [131] or [142] to [339], wherein the solubility tag comprises/each solubility tag comprises up to 9 monosaccharide units.
[341] The molecule according to any one of items [1] to [6] or [27] to [131] or [146] to [340] or the antibody-drug conjugate according to any one of items [132] to [133] or [146] to [340] or the method according to any one of items [7] to [19] or [27] to [131] or [134] to [141] or [146] to [340] or the use according to any one of items [20] to [131] or [142] to [340], wherein the solubility tag comprises/each solubility tag comprises up to 8 monosaccharide units.
[342] The molecule according to any one of items [1] to [6] or [27] to [131] or [146] to [341] or the antibody-drug conjugate according to any one of items [132] to [133] or [146] to [341] or the method according to any one of items [7] to [19] or [27] to [131] or [134] to [141] or [146] to [341] or the use according to any one of items [20] to [131] or [142] to [341], wherein the solubility tag comprises/each solubility tag comprises up to 7 monosaccharide units.
[343] The molecule according to any one of items [1] to [6] or [27] to [131] or [146] to [342] or the antibody-drug conjugate according to any one of items [132] to [133] or [146] to [342] or the method according to any one of items [7] to [19] or [27] to [131] or [134] to [141] or [146] to [342] or the use according to any one of items [20] to [131] or [142] to [342], wherein the solubility tag comprises/each solubility tag comprises up to 6 monosaccharide units.
[344] The molecule according to any one of items [1] to [6] or [27] to [131] or [146] to [343] or the antibody-drug conjugate according to any one of items [132] to [133] or [146] to [343] or the method according to any one of items [7] to [19] or [27] to [131] or [134] to [141] or [146] to [343] or the use according to any one of items [20] to [131] or [142] to [343], wherein the solubility tag comprises/each solubility tag comprises up to 5 monosaccharide units.
[345] The molecule according to any one of items [1] to [6] or [27] to [131] or [146] to [344] or the antibody-drug conjugate according to any one of items [132] to [133] or [146] to [344] or the method according to any one of items [7] to [19] or [27] to [131] or [134] to [141] or [146] to [344] or the use according to any one of items [20] to [131] or [142] to [344], wherein the solubility tag comprises/each solubility tag comprises at least 2 monosaccharide units.
[346] The molecule according to any one of items [1] to [6] or [27] to [131] or [146] to [345] or the antibody-drug conjugate according to any one of items [132] to [133] or [146] to [345] or the method according to any one of items [7] to [19] or [27] to [131] or [134] to [141] or [146] to [345] or the use according to any one of items [20] to [131] or [142] to [345], wherein the solubility tag comprises/each solubility tag comprises at least 3 monosaccharide units.
[347] The molecule according to any one of items [1] to [6] or [27] to [131] or [146] to [346] or the antibody-drug conjugate according to any one of items [132] to [133] or [146] to [346] or the method according to any one of items [7] to [19] or [27] to [131] or [134] to [141] or [146] to [346] or the use according to any one of items [20] to [131] or [142] to [346], wherein the solubility tag comprises/each solubility tag comprises at least 4 monosaccharide units.
[348] The molecule according to any one of items [1] to [6] or [27] to [131] or [146] to [347] or the antibody-drug conjugate according to any one of items [132] to [133] or [146] to [347] or the method according to any one of items [7] to [19] or [27] to [131] or [134] to [141] or [146] to [347] or the use according to any one of items [20] to [131] or [142] to [347], wherein the solubility tag comprises/each solubility tag comprises at least 5 monosaccharide units.
[349] The molecule according to any one of items [1] to [6] or [27] to [131] or [146] to [348] or the antibody-drug conjugate according to any one of items [132] to [133] or [146] to [348] or the method according to any one of items [7] to [19] or [27] to [131] or [134] to [141] or [146] to [348] or the use according to any one of items [20] to [131] or [142] to [348], wherein the solubility tag comprises/each solubility tag comprises 5 monosaccharide units.
[350] The molecule according to any one of items [1] to [6] or [27] to [131] or [146] to [349] or the antibody-drug conjugate according to any one of items [132] to [133] or [146] to [349] or the method according to any one of items [7] to [19] or [27] to [131] or [134] to [141] or [146] to [349] or the use according to any one of items [20] to [131] or [142] to [349], wherein the monosaccharide units comprised in said solubility tag/each solubility tag are linked by covalent bonds, forming an oligosaccharide.
[351] The molecule according to any one of items [1] to [6] or [27] to [131] or [146] to [350] or the antibody-drug conjugate according to any one of items [132] to [133] or [146] to [350] or the method according to any one of items [7] to [19] or [27] to [131] or [134] to [141] or [146] to [350] or the use according to any one of items [20] to [131] or [142] to [350], wherein the solubility tag/each solubility tag consists of 3 to 8 monosaccharide units.
[352] The molecule according to any one of items [1] to [6] or [27] to [131] or [146] to [351] or the antibody-drug conjugate according to any one of items [132] to [133] or [146] to [351] or the method according to any one of items [7] to [19] or [27] to [131] or [134] to [141] or [146] to [351] or the use according to any one of items [20] to [131] or [142] to [351], wherein the solubility tag/each solubility tag consists of 4 to 8 monosaccharide units.
[353] The molecule according to any one of items [1] to [6] or [27] to [131] or [146] to [352] or the antibody-drug conjugate according to any one of items [132] to [133] or [146] to [352] or the method according to any one of items [7] to [19] or [27] to [131] or [134] to [141] or [146] to [352] or the use according to any one of items [20] to [131] or [142] to [352], wherein the solubility tag/each solubility tag consists of 4 to 7 monosaccharide units.
[354] The molecule according to any one of items [1] to [6] or [27] to [131] or [146] to [353] or the antibody-drug conjugate according to any one of items [132] to [133] or [146] to [353] or the method according to any one of items [7] to [19] or [27] to [131] or [134] to [141] or [146] to [353] or the use according to any one of items [20] to [131] or [142] to [353], wherein the solubility tag/each solubility tag consists of 4 to 6 monosaccharide units.
[355] The molecule according to any one of items [1] to [6] or [27] to [131] or [146] to [354] or the antibody-drug conjugate according to any one of items [132] to [133] or [146] to [354] or the method according to any one of items [7] to [19] or [27] to [131] or [134] to [141] or [146] to [354] or the use according to any one of items [20] to [131] or [142] to [354], wherein the solubility tag/each solubility tag consists of 4 or 5 monosaccharide units.
[356] The molecule according to any one of items [1] to [6] or [27] to [131] or [146] to [355] or the antibody-drug conjugate according to any one of items [132] to [133] or [146] to [355] or the method according to any one of items [7] to [19] or [27] to [131] or [134] to [141] or [146] to [355] or the use according to any one of items [20] to [131] or [142] to [355], wherein the solubility tag/each solubility tag consists of 5 or 6 monosaccharide units.
[357] The molecule according to any one of items [1] to [6] or [27] to [131] or [146] to [356] or the antibody-drug conjugate according to any one of items [132] to [133] or [146] to [356] or the method according to any one of items [7] to [19] or [27] to [131] or [134] to [141] or [146] to [356] or the use according to any one of items [20] to [131] or [142] to [356], wherein the solubility tag/each solubility tag consists of 5 monosaccharide units.
[358] The molecule according to any one of items [1] to [6] or [27] to [131] or [146] to [357] or the antibody-drug conjugate according to any one of items [132] to [133] or [146] to [357] or the method according to any one of items [7] to [19] or [27] to [131] or [134] to [141] or [146] to [357] or the use according to any one of items [20] to [131] or [142] to [357], wherein the monosaccharide units of which said solubility tag/each solubility tag consists are linked by covalent bonds, forming an oligosaccharide.
[359] The molecule according to any one of items [1] to [6] or [27] to [131] or [146] to [358] or the antibody-drug conjugate according to any one of items [132] to [133] or [146] to [358] or the method according to any one of items [7] to [19] or [27] to [131] or [134] to [141] or [146] to [358] or the use according to any one of items [20] to [131] or [142] to [358], wherein the solubility tag/each solubility tag comprises a chito-oligosaccharide.
[360] The molecule according to any one of items [1] to [6] or [27] to [131] or [146] to [359] or the antibody-drug conjugate according to any one of items [132] to [133] or [146] to [359] or the method according to any one of items [7] to [19] or [27] to [131] or [134] to [141] or [146] to [359] or the use according to any one of items [20] to [131] or [142] to [359], wherein the solubility tag/each solubility tag is a chito-oligosaccharide.
[361] The molecule or the antibody-drug conjugate or the method or the use according to any one of items [359] to [360], wherein said chito-oligosaccharide is selected from the chito-oligosaccharides shown in Table T1:

**Table T1:**

| **Number of monosaccharide units** | **Chito-oligosaccharide** |
|---|---|
| 2 | (GlcN)₂ / Chitobiose |
| 2 | GlcN-GlcNAc |
| 2 | GlcNAc-GlcN |
| 2 | (GlcNAc)₂ / Di-*N*-acetylchitobiose |
| 3 | (GlcN)₃ / Chitotriose |
| 3 | (GlcN)₂-GlcNAc |
| 3 | (GlcNAc)₂-GlcN |
| 3 | GlcN-(GlcNAc)₂ |
| 3 | (GlcNAc)₃ / Tri-*N*-acetylchitotriose |
| 4 | (GlcN)₄ / Chitotetraose |
| 4 | (GlcN)₃-GlcNAc |
| 4 | (GlcNAc)₂-(GlcN)₂ |
| 4 | GlcNAc-GlcN-GlcNAc-GlcN |
| 4 | GlcNAc-(GlcN)₂-GlcNAc |
| 4 | GlcN-(GlcNAc)₂-GlcN |
| 4 | GlcN-GlcNAc-GlcN-GlcNAc |
| 4 | (GlcN)₂-(GlcNAc)₂ |
| 4 | (GlcNAc)₃-GlcN |
| 4 | (GlcNAc)₂-GlcN-GlcNAc |
| 4 | GlcNAc-GlcN-(GlcNAc)₂ |
| 4 | GlcN-(GlcNAc)₃ |
| 4 | (GlcNAc)₄ / Tetra-*N*-acetylchitotetraose |
| 5 | (GlcN)₅ / Chitopentaose |
| 5 | (GlcN)₃-(GlcNAc)₂ |
| 5 | (GlcNAc)₂-(GlcN)₂-GlcNAc |
| 5 | GlcNAc-GlcN-GlcNAc-GlcN-GlcNAc |
| 5 | GlcNAc-(GlcN)₂-(GlcNAc)₂ |
| 5 | GlcN-(GlcNAc)₂-GlcN-GlcNAc |
| 5 | GlcN-GlcNAc-GlcN-(GlcNAc)₂ |
| 5 | GlcN-(GlcNAc)₄ |
| 5 | (GlcNAc)₅ / Penta-*N*-acetylchitopentaose |
| 6 | (GlcN)₆ / Chitohexaose |
| 6 | (GlcN)₂-GlcNAc-(GlcN)₃ |
| 6 | GlcNAc-(GlcN)₄-GlcNAc |
| 6 | GlcN-GlcNAc-GlcN-GlcNAc-(GlcN)₂ |
| 6 | GlcNAc-(GlcN)₂-GlcNAc-GlcN-GlcNAc |
| 6 | GlcNAc-(GlcN)₃-(GlcNAc)₂ |
| 6 | GlcN-GlcNAc-GlcN-(GlcNAc)₃ |
| 6 | GlcNAc-(GlcN)₂-(GlcNAc)₃ |
| 6 | (GlcNAc)₆ / Hexa-*N*-acetylchitohexaose |
| 7 | (GlcN)₇ / Chitoheptaose |
| 7 | (GlcNAc)₇ / Hepta-*N*-acetylchitoheptaose |

[362] The molecule or the antibody-drug conjugate or the method or the use according to any one of items [359] to [361], wherein said chito-oligosaccharide is a chito-oligosaccharide with 3 to 7 monosaccharide units shown in Table T1.
[363] The molecule or the antibody-drug conjugate or the method or the use according to any one of items [359] to [362], wherein said chito-oligosaccharide is a chito-oligosaccharide with 4 to 6 monosaccharide units shown in Table T1.
[364] The molecule or the antibody-drug conjugate or the method or the use according to any one of items [359] to [363], wherein said chito-oligosaccharide is a chito-oligosaccharide with 4 or 5 monosaccharide units shown in Table T1.
[365] The molecule or the antibody-drug conjugate or the method or the use according to any one of items [331] to [364], wherein said monosaccharide units are independently selected from the group consisting of aldoses, ketoses and chemically modified forms of said aldoses or ketoses.
[366] The molecule or the antibody-drug conjugate or the method or the use according to any one of items [331] to [365], wherein said monosaccharide units are independently selected from the group consisting of aldoses and chemically modified forms of said aldoses.
[367] The molecule or the antibody-drug conjugate or the method or the use according to any one of items [331] to [366], wherein said monosaccharide units are individually selected from the group consisting of tetroses, pentoses, hexoses, heptoses, octoses and chemically modified forms of tetroses, pentoses, hexoses, heptoses and octoses.
[368] The molecule or the antibody-drug conjugate or the method or the use according to any one of items [331] to [367], wherein said monosaccharide units are individually selected from the group consisting of tetroses, pentoses, hexoses, and chemically modified forms of tetroses, pentoses and hexoses.
[369] The molecule or the antibody-drug conjugate or the method or the use according to any one of items [331] to [368], wherein said monosaccharide units are individually selected from the group consisting of pentoses, hexoses and chemically modified forms of pentoses and hexoses.
[370] The molecule or the antibody-drug conjugate or the method or the use according to any one of items [331] to [369], wherein said monosaccharide units are selected from the group consisting of hexoses and chemically modified forms of hexoses.
[371] The molecule or the antibody-drug conjugate or the method or the use according to any one of items [331] to [370], wherein said monosaccharide units are individually selected from the group consisting of tetroses, pentoses, hexoses, heptoses and octoses.
[372] The molecule or the antibody-drug conjugate or the method or the use according to any one of items [331] to [371], wherein said monosaccharide units are individually selected from the group consisting of tetroses, pentoses and hexoses.
[373] The molecule or the antibody-drug conjugate or the method or the use according to any one of items [331] to [372], wherein said monosaccharide units are individually selected from the group consisting of pentoses and hexoses.
[374] The molecule or the antibody-drug conjugate or the method or the use according to any one of items [331] to [373], wherein said monosaccharide units are hexoses.
[375] The molecule or the antibody-drug conjugate or the method or the use according to any one of items [331] to [374], wherein said monosaccharide units of said solubility tag are not modified by chemical modification.
[376] The molecule or the antibody-drug conjugate or the method or the use according to any one of items [367] to [376], wherein said tetroses, pentoses, hexoses, heptoses and octoses have the chemical formula Cₙ(H₂O)ₙ.
[377] The molecule or the antibody-drug conjugate or the method or the use according to any one of items [365] to [376], wherein said aldoses have the chemical formula Cₙ(H₂O)ₙ.
[378] The molecule or the antibody-drug conjugate or the method or the use according to any one of items [365] to [377], wherein said ketoses have the chemical formula Cₙ(H₂O)ₙ.
[379] The molecule or the antibody-drug conjugate or the method or the use according to any one of items [376] to [378], wherein n is an integer number and 4 ≤ n ≤ 12.
[380] The molecule or the antibody-drug conjugate or the method or the use according to any one of items [376] to [379], wherein n is an integer number and 4 ≤ n ≤ 8.
[381] The molecule or the antibody-drug conjugate or the method or the use according to any one of items [376] to [380], wherein n is an integer number and 5 ≤ n ≤ 7.
[382] The molecule or the antibody-drug conjugate or the method or the use according to any one of items [376] to [381], wherein n is 5 or 6.
[383] The molecule or the antibody-drug conjugate or the method or the use according to any one of items [376] to [382], wherein n is 6.
[384] The molecule or the antibody-drug conjugate or the method or the use according to any one of items [367] to [383], wherein said tetroses are individually selected from the group consisting of erythrose and threose.
[385] The molecule or the antibody-drug conjugate or the method or the use according to any one of items [367] to [384], wherein said pentoses are individually selected from the group consisting of ribose, arabinose, xylose and lyxose.
[386] The molecule or the antibody-drug conjugate or the method or the use according to any one of items [367] to [385], wherein said hexoses are individually selected from the group consisting of allose, altrose, glucose, mannose, gulose, idose, galactose and talose.
[387] The molecule or the antibody-drug conjugate or the method or the use according to any one of items [331] to [387], wherein said monosaccharide units are D-sugars.
[388] The molecule or the antibody-drug conjugate or the method or the use according to any one of items [331] to [387], wherein at least one monosaccharide unit of said solubility tag is modified by at least one chemical modification.
[389] The molecule or the antibody-drug conjugate or the method or the use according to any one of items [331] to [388], wherein all monosaccharide units of said solubility tag are modified by at least one chemical modification.
[390] The molecule or the antibody-drug conjugate or the method or the use according to any one of items [331] to [389], wherein no monosaccharide unit of said solubility tag is modified by more than three chemical modifications.
[391] The molecule or the antibody-drug conjugate or the method or the use according to any one of items [331] to [390], wherein no monosaccharide unit of said solubility tag is modified by more than two chemical modifications.
[392] The molecule or the antibody-drug conjugate or the method or the use according to any one of items [331] to [391], wherein no monosaccharide unit of said solubility tag is modified by more than one chemical modification.
[393] The molecule or the antibody-drug conjugate or the method or the use according to any one of items [331] to [392], wherein the average number of chemical modifications per monosaccharide unit of said solubility tag is ≤ 3.
[394] The molecule or the antibody-drug conjugate or the method or the use according to any one of items [331] to [393], wherein the average number of chemical modifications per monosaccharide unit of said solubility tag is ≤ 2.
[395] The molecule or the antibody-drug conjugate or the method or the use according to any one of items [331] to [394], wherein the average number of chemical modifications per monosaccharide unit of said solubility tag is ≤ 1.5.
[396] The molecule or the antibody-drug conjugate or the method or the use according to any one of items [331] to [395], wherein the average number of chemical modifications per monosaccharide unit of said solubility tag is ≤ 1.
[397] The molecule or the antibody-drug conjugate or the method or the use according to any one of items [393] to [396], wherein the average number of chemical modifications per monosaccharide unit of said solubility tag is calculated by dividing the overall number of chemical modifications on all monosaccharide units of said solubility tag by the number of monosaccharide units in said solubility tag.
[398] The molecule or the antibody-drug conjugate or the method or the use according to any one of items [365] to [397], wherein said chemically modified forms of said monosaccharide units are forms of said monosaccharide units with at least one chemical modification.
[399] The molecule or the antibody-drug conjugate or the method or the use according to any one of items [365] to [398], wherein said chemically modified forms of said monosaccharide units are forms of said monosaccharide units with up to three chemical modifications.
[400] The molecule or the antibody-drug conjugate or the method or the use according to any one of items [365] to [399], wherein said chemically modified forms of said monosaccharide units are forms of said monosaccharide units with up to two chemical modifications.
[401] The molecule or the antibody-drug conjugate or the method or the use according to any one of items [365] to [400], wherein said chemically modified forms of said monosaccharide units are forms of said monosaccharide units with one chemical modification.
[402] The molecule or the antibody-drug conjugate or the method or the use according to any one of items [365] to [401], wherein said chemical modification(s) are individually selected from the following:
- replacement of a hydroxyl group by a substituent selected from the group consisting of hydrogen, alkyl, acyl, acyloxy, alkenyl, alkynyl, O-alkyl, S-alkyl, carboxyalkyl, halogen, amino, N-acylamino, azido, sulfate, selenyl and azido;
- replacement of a hydroxyl group by a sulfur-containing moiety selected from the group consisting of a sulfoxide, a sulfone, a sulfuric acid, a sulfuric ester, a thiosulfate, a thioester, a thioether and a sulfoximine;
- replacement of a hydroxyl group by a phosphor-containing moiety selected from the group consisting of a phosphate, a phosphonate, a phosphines, a phosphoric acid and a phosphoester;
- replacement of a hydroxyl group by a silyl group or covalent linkage of a silyl group to said hydroxyl group by formation of a silyl ether;
- replacement of a hydroxyl group by a branched polyalcohol;
- replacement of a hydroxyl group by an amino acid or a peptide of up to 3 amino acids, or covalent linkage of an amino acid or a peptide of up to 3 amino acids to said monosaccharide unit;
- acetal formation with a hydroxyl group of said monosaccharide unit;
- covalent linkage of a PEG (polyethylene glycol) group to said monosaccharide unit;
- covalent linkage to an aromatic or heteroaromatic substituent;
- endocyclic double-bond formation within the sugar ring of said monosaccharide unit.
[403] The molecule or the antibody-drug conjugate or the method or the use according to any one of items [365] to [402], wherein said chemical modification(s) are individually selected from the following: replacement of a hydroxyl group by a substituent selected from the group consisting of hydrogen, alkyl, acyl, acyloxy, alkenyl, alkynyl, O-alkyl, S-alkyl, carboxyalkyl, halogen, amino, N-acylamino, azido, sulfate, selenyl and azido.
[404] The molecule or the antibody-drug conjugate or the method or the use according to any one of items [365] to [403], wherein said alkyl is substituted or unsubstituted C₁-C₅ alkyl, said acyl is substituted or unsubstituted C₁-C₅ acyl, said acyloxy is substituted or unsubstituted C₁-C₅ acyloxy, said alkenyl is substituted or unsubstituted C₁-C₅ alkenyl, said alkynyl is substituted or unsubstituted C₁-C₅ alkynyl, said O-alkyl is substituted or unsubstituted C₁-C₅ O-alkyl, said S-alkyl is substituted or unsubstituted C₁-C₅ S-alkyl, said carboxyalkyl is substituted or unsubstituted C₁-C₅ carboxyalkyl, and said N-acylamino is substituted or unsubstituted C₁-C₅ N-acylamino.
[405] The molecule or the antibody-drug conjugate or the method or the use according to any one of items [402] to [404], wherein said alkyl is unsubstituted C₁-C₅ alkyl, said acyl is unsubstituted C₁-C₅ acyl, said acyloxy is unsubstituted C₁-C₅ acyloxy, said alkenyl is unsubstituted C₁-C₅ alkenyl, said alkynyl is unsubstituted C₁-C₅ alkynyl, said O-alkyl is unsubstituted C₁-C₅ O-alkyl, said S-alkyl is unsubstituted C₁-C₅ S-alkyl, said carboxyalkyl is unsubstituted C₁-C₅ carboxyalkyl, and said N-acylamino is unsubstituted C₁-C₅ N-acylamino.
[406] The molecule or the antibody-drug conjugate or the method or the use according to any one of items [402] to [405], wherein said alkyl is substituted or unsubstituted C₁-C₂ alkyl, said acyl is substituted or unsubstituted C₁-C₂ acyl, said acyloxy is substituted or unsubstituted C₁-C₂ acyloxy, said alkenyl is substituted or unsubstituted C₁-C₂ alkenyl, said alkynyl is substituted or unsubstituted C₁-C₂ alkynyl, said O-alkyl is substituted or unsubstituted C₁-C₂ O-alkyl, said S-alkyl is substituted or unsubstituted C₁-C₂ S-alkyl, said carboxyalkyl is substituted or unsubstituted C₁-C₂ carboxyalkyl, and said N-acylamino is substituted or unsubstituted C₁-C₂ N-acylamino.
[407] The molecule or the antibody-drug conjugate or the method or the use according to any one of items [402] to [406], wherein said alkyl is unsubstituted C₁-C₂ alkyl, said acyl is unsubstituted C₁-C₂ acyl, said acyloxy is unsubstituted C₁-C₂ acyloxy, said alkenyl is unsubstituted C₁-C₂ alkenyl, said alkynyl is unsubstituted C₁-C₂ alkynyl, said O-alkyl is unsubstituted C₁-C₂ O-alkyl, said S-alkyl is unsubstituted C₁-C₂ S-alkyl, said carboxyalkyl is unsubstituted C₁-C₂ carboxyalkyl, and said N-acylamino is unsubstituted C₁-C₂ N-acylamino.
[408] The molecule or the antibody-drug conjugate or the method or the use according to any one of items [402] to [407], wherein said alkyl, acyl, acyloxy, alkenyl, alkynyl, O-alkyl, S-alkyl, carboxyalkyl, or N-acylamino is linear or branched.
[409] The molecule or the antibody-drug conjugate or the method or the use according to any one of items [402] to [408], wherein said alkyl, acyl, acyloxy, alkenyl, alkynyl, O-alkyl, S-alkyl, carboxyalkyl, or N-acylamino is linear.
[410] The molecule or the antibody-drug conjugate or the method or the use according to any one of items [402] to [409], wherein said substituted alkyl, acyl, acyloxy, alkenyl, alkynyl, O-alkyl, S-alkyl, carboxyalkyl, or N-acylamino is substituted with a group selected from halogen, CN, OH, amino, methyl, ethyl, methoxy, ethoxy.
[411] The molecule or the antibody-drug conjugate or the method or the use according to any one of items [402] to [410], wherein said substituted alkyl, acyl, acyloxy, alkenyl, alkynyl, O-alkyl, S-alkyl, carboxyalkyl, or N-acylamino is substituted with an atom or group having a molecular weight of ≤ 100 Dalton.
[412] The molecule or the antibody-drug conjugate or the method or the use according to any one of items [402] to [411], wherein said substituted alkyl, acyl, acyloxy, alkenyl, alkynyl, O-alkyl, S-alkyl, carboxyalkyl, or N-acylamino is substituted with an atom or group having a molecular weight of ≤ 80 Dalton.
[413] The molecule or the antibody-drug conjugate or the method or the use according to any one of items [402] to [412], wherein said substituted alkyl, acyl, acyloxy, alkenyl, alkynyl, O-alkyl, S-alkyl, carboxyalkyl, or N-acylamino is substituted with an atom or group having a molecular weight of ≤ 50 Dalton.
[414] The molecule or the antibody-drug conjugate or the method or the use according to any one of items [365] to [413], said chemical modification(s) are individually selected from the following: replacement of a hydroxyl group by a sulfur-containing moiety selected from the group consisting of a sulfoxide, a sulfone, a sulfuric acid, a sulfuric ester, a thiosulfate, a thioester, a thioether and a sulfoximine.
[415] The molecule or the antibody-drug conjugate or the method or the use according to any one of items [402] to [414], wherein said sulfur-containing moiety has a molecular weight of up to 100 Da, preferably up to 50 Da.
[416] The molecule or the antibody-drug conjugate or the method or the use according to any one of items [365] to [415], wherein said chemical modification(s) are individually selected from the following: replacement of a hydroxyl group by a phosphor-containing moiety selected from the group consisting of a phosphate, a phosphonate, a phosphines, a phosphoric acid and a phosphoester.
[417] The molecule or the antibody-drug conjugate or the method or the use according to any one of items [402] to [416], wherein said phosphor-containing moiety has a molecular weight of up to 100 Da, preferably up to 50 Da.
[418] The molecule or the antibody-drug conjugate or the method or the use according to any one of items [365] to [417], wherein said chemical modification(s) are individually selected from the following: replacement of a hydroxyl group by a silyl group or covalent linkage of a silyl group to said hydroxyl group by formation of a silyl ether.
[419] The molecule or the antibody-drug conjugate or the method or the use according to any one of items [402] to [418], wherein said silyl group has a molecular weight of up to 150 Da, preferably up to 100 Da.
[420] The molecule or the antibody-drug conjugate or the method or the use according to any one of items [365] to [419], wherein said chemical modification(s) are individually selected from the following: replacement of a hydroxyl group by an amino acid or a peptide of up to 3 amino acids or covalent linkage of an amino acid or a peptide of up to 3 amino acids to said monosaccharide unit;
[421] The molecule or the antibody-drug conjugate or the method or the use according to any one of items [365] to [420], wherein said chemical modification(s) are individually selected from the following: acetal formation with a hydroxyl group of said monosaccharide unit.
[422] The molecule or the antibody-drug conjugate or the method or the use according to any one of items [402] to [411], wherein each substituent of the acetal that is not a covalent linkage to the monosaccharide unit has a molecular weight of up to 100 Da, preferably up to 50 Da.
[423] The molecule or the antibody-drug conjugate or the method or the use according to any one of items [365] to [422], wherein said chemical modification(s) are individually selected from the following: replacement of a hydroxyl group by a branched polyalcohol.
[424] The molecule or the antibody-drug conjugate or the method or the use according to any one of items [402] to [423], wherein said branched polyalcohol is a branched polyalcohol with up to 8 hydroxyl groups.
[425] The molecule or the antibody-drug conjugate or the method or the use according to any one of items [402] to [424], wherein said branched polyalcohol is a branched polyalcohol with up to 5 hydroxyl groups.
[426] The molecule or the antibody-drug conjugate or the method or the use according to any one of items [365] to [425], wherein said chemical modification(s) are individually selected from the following: covalent linkage of a PEG (polyethylene glycol) group to said monosaccharide unit.
[427] The molecule or the antibody-drug conjugate or the method or the use according to any one of items [402] to [426], wherein said PEG group has a molecular weight of up to 1000 Dalton.
[428] The molecule or the antibody-drug conjugate or the method or the use according to any one of items [402] to [427], wherein said PEG group has a molecular weight of up to 500 Dalton.
[429] The molecule or the antibody-drug conjugate or the method or the use according to any one of items [402] to [428], wherein said PEG group has a molecular weight of up to 120 Dalton.
[430] The molecule or the antibody-drug conjugate or the method or the use according to any one of items [365] to [429], wherein said chemical modification(s) are individually selected from the following: covalent linkage to an aromatic or heteroaromatic substituent.
[431] The molecule or the antibody-drug conjugate or the method or the use according to any one of items [402] to [428], wherein said aromatic or heteroaromatic substituent has a molecular weight of up to 200 Da, preferably up to 100 Da.
[432] The molecule or the antibody-drug conjugate or the method or the use according to any one of items [365] to [431], wherein said chemical modification(s) are individually selected from the following: endocyclic double-bond formation within the sugar ring of said monosaccharide unit.
[433] The molecule or the antibody-drug conjugate or the method or the use according to any one of items [365] to [432], wherein said chemical modification of said monosaccharides is the replacement of a hydroxyl group of said monosaccharide by a substituent selected from the group consisting of hydrogen, amino, N-acetylamino, methyl, methoxy and sulfate.
[434] The molecule or the antibody-drug conjugate or the method or the use according to any one of items [365] to [433], wherein said chemical modification of said monosaccharides is the replacement of a hydroxyl group of said monosaccharide by a substituent selected from the group consisting of hydrogen, amino, N-acetylamino, methyl and methoxy.
[435] The molecule or the antibody-drug conjugate or the method or the use according to any one of items [365] to [434], wherein said chemical modification of said monosaccharides is the replacement of a hydroxyl group of said monosaccharide by a substituent selected from the group consisting of hydrogen, amino and N-acetylamino.
[436] The molecule or the antibody-drug conjugate or the method or the use according to any one of items [365] to [435], wherein said chemically modified forms of said monosaccharide units are forms of said monosaccharide units with one, two or three chemical modifications.
[437] The molecule or the antibody-drug conjugate or the method or the use according to any one of items [365] to [436], wherein said chemically modified forms of said monosaccharide units are forms of said monosaccharide units with one or two chemical modifications.
[438] The molecule or the antibody-drug conjugate or the method or the use according to any one of items [365] to [437], wherein said chemically modified forms of said monosaccharide units are forms of said monosaccharide units with one chemical modification.
[439] The molecule or the antibody-drug conjugate or the method or the use according to any one of items [365] to [438], wherein said chemically modified forms of said monosaccharide units are forms of said monosaccharide units in which one, two or three hydroxyl groups have independently been replaced.
[440] The molecule or the antibody-drug conjugate or the method or the use according to any one of items [365] to [439], wherein said chemically modified forms of said monosaccharide units are forms of said monosaccharide units in which one or two hydroxyl groups have independently been replaced.
[441] The molecule or the antibody-drug conjugate or the method or the use according to any one of items [365] to [440], wherein said chemically modified forms of said monosaccharide units are forms of said monosaccharide units in which one hydroxyl group has independently been replaced.
[442] The molecule or the antibody-drug conjugate or the method or the use according to any one of items [365] to [441], wherein said chemically modified forms of said monosaccharide units are forms of said monosaccharide units in which one or two hydroxyl groups have independently been replaced by a hydrogen, amino group, N-acetylamino group, methyl group, methoxy group or sulfate group.
[443] The molecule or the antibody-drug conjugate or the method or the use according to any one of items [365] to [442], wherein said chemically modified forms of said monosaccharide units are forms of said monosaccharide units in which one or two hydroxyl groups have independently been replaced by a hydrogen, amino group, N-acetylamino group, methyl group or methoxy group.
[444] The molecule or the antibody-drug conjugate or the method or the use according to any one of items [365] to [443], wherein said chemically modified forms of said monosaccharide units are forms of said monosaccharide units in which one or two hydroxyl groups have independently been replaced by a hydrogen, amino group or N-acetylamino group.
[445] The molecule or the antibody-drug conjugate or the method or the use according to any one of items [365] to [444], wherein the substituent to the carbon backbone of the monosaccharide unit that results from the chemical modification has a molecular weight of not more than 800 Dalton.
[446] The molecule or the antibody-drug conjugate or the method or the use according to any one of items [365] to [445], wherein the substituent to the carbon backbone of the monosaccharide unit that results from the chemical modification has a molecular weight of not more than 400 Dalton.
[447] The molecule or the antibody-drug conjugate or the method or the use according to any one of items [365] to [446], wherein the substituent to the carbon backbone of the monosaccharide unit that results from the chemical modification has a molecular weight of not more than 200 Dalton.
[448] The molecule or the antibody-drug conjugate or the method or the use according to any one of items [365] to [447], wherein the substituent to the carbon backbone of the monosaccharide unit that results from the chemical modification has a molecular weight of not more than 100 Dalton.
[449] The molecule according to any one of items [1] to [6] or [27] to [131] or [146] to [448] or the antibody-drug conjugate according to any one of items [132] to [133] or [146] to [448] or the method according to any one of items [7] to [19] or [27] to [131] or [134] to [141] or [146] to [448] or the use according to any one of items [20] to [131] or [142] to [448], wherein said solubility tag comprises/said solubility tags comprise no monosaccharide units other than monosaccharide units selected from the group consisting of glucose, chemically modified forms of glucose, galactose and chemically modified forms of galactose.
[450] The molecule according to any one of items [1] to [6] or [27] to [131] or [146] to [449] or the antibody-drug conjugate according to any one of items [132] to [133] or [146] to [449] or the method according to any one of items [7] to [19] or [27] to [131] or [134] to [141] or [146] to [449] or the use according to any one of items [20] to [131] or [142] to [449], wherein said solubility tag comprises/said solubility tags comprise no monosaccharides other than monosaccharides selected from glucosamine (GlcN), N-acetyl-glucosamine (GlcNAc), fucose (Fuc) and 6-methyl-fucose.
[451] The molecule according to any one of items [1] to [6] or [27] to [131] or [146] to [450] or the antibody-drug conjugate according to any one of items [132] to [133] or [146] to [450] or the method according to any one of items [7] to [19] or [27] to [131] or [134] to [141] or [146] to [450] or the use according to any one of items [20] to [131] or [142] to [450], wherein said solubility tag comprises/said solubility tags comprise only monosaccharides selected from glucosamine (GlcN) and N-acetyl-glucosamine (GlcNAc) as monosaccharide units.
[452] The molecule according to any one of items [1] to [6] or [27] to [131] or [146] to [451] or the antibody-drug conjugate according to any one of items [132] to [133] or [146] to [451] or the method according to any one of items [7] to [19] or [27] to [131] or [134] to [141] or [146] to [451] or the use according to any one of items [20] to [131] or [142] to [451], wherein said solubility tag comprises/said solubility tags comprise only N-acetyl-glucosamine (GlcNAc) as monosaccharide units.
[453] The molecule according to any one of items [1] to [6] or [27] to [131] or [146] to [452] or the antibody-drug conjugate according to any one of items [132] to [133] or [146] to [452] or the method according to any one of items [7] to [19] or [27] to [131] or [134] to [141] or [146] to [452] or the use according to any one of items [20] to [131] or [142] to [452], wherein said solubility tag consists/said solubility tags consist only of covalently linked monosaccharide units selected from the group consisting of glucosamine (GlcN), N-acetyl-glucosamine (GlcNAc) and 6-methyl-fucose.
[454] The molecule according to any one of items [1] to [6] or [27] to [131] or [146] to [453] or the antibody-drug conjugate according to any one of items [132] to [133] or [146] to [453] or the method according to any one of items [7] to [19] or [27] to [131] or [134] to [141] or [146] to [453] or the use according to any one of items [20] to [131] or [142] to [453], wherein said solubility tag consists/said solubility tags consist only of covalently linked monosaccharide units selected from the group consisting of glucosamine (GlcN) and N-acetyl-glucosamine (GlcNAc).
[455] The molecule according to any one of items [1] to [6] or [27] to [131] or [146] to [454] or the antibody-drug conjugate according to any one of items [132] to [133] or [146] to [454] or the method according to any one of items [7] to [19] or [27] to [131] or [134] to [141] or [146] to [454] or the use according to any one of items [20] to [131] or [142] to [454], wherein said solubility tag consists/said solubility tags consist only of covalently linked N-acetyl-glucosamine (GlcNAc) units.
[456] The molecule according to any one of items [1] to [6] or [27] to [131] or [146] to [455] or the antibody-drug conjugate according to any one of items [132] to [133] or [146] to [455] or the method according to any one of items [7] to [19] or [27] to [131] or [134] to [141] or [146] to [455] or the use according to any one of items [20] to [131] or [142] to [455], wherein said solubility tag comprises/said solubility tags comprise at least three N-acetyl-glucosamine (GlcNAc) units.
[457] The molecule according to any one of items [1] to [6] or [27] to [131] or [146] to [456] or the antibody-drug conjugate according to any one of items [132] to [133] or [146] to [456] or the method according to any one of items [7] to [19] or [27] to [131] or [134] to [141] or [146] to [456] or the use according to any one of items [20] to [131] or [142] to [456], wherein said solubility tag comprises/said solubility tags comprise at least four N-acetyl-glucosamine (GlcNAc) units.
[458] The molecule according to any one of items [1] to [6] or [27] to [131] or [146] to [457] or the antibody-drug conjugate according to any one of items [132] to [133] or [146] to [457] or the method according to any one of items [7] to [19] or [27] to [131] or [134] to [141] or [146] to [457] or the use according to any one of items [20] to [131] or [142] to [457], wherein said solubility tag comprises/said solubility tags comprise one glucosamine (GlcN) and four N-acetyl-glucosamine (GlcNAc) units.
[459] The molecule according to any one of items [1] to [6] or [27] to [131] or [146] to [458] or the antibody-drug conjugate according to any one of items [132] to [133] or [146] to [458] or the method according to any one of items [7] to [19] or [27] to [131] or [134] to [141] or [146] to [458] or the use according to any one of items [20] to [131] or [142] to [458], wherein said solubility tag consists/said solubility tags consist of one glucosamine (GlcN) and four N-acetyl-glucosamine (GlcNAc) units.
[460] The molecule or the antibody-drug conjugate or the method or the use according to any one of items [331] to [459], wherein the monosaccharide units of said solubility tag are linked in a linear fashion without branches.
[461] The molecule or the antibody-drug conjugate or the method or the use according to any one of items [331] to [459], wherein the monosaccharide units of said solubility tag are linked in a branched fashion.
[462] The molecule or the antibody-drug conjugate or the method or the use according to any one of items [331] to [461], wherein said monosaccharide units are linked covalently by glycosidic linkages.
[463] The molecule or the antibody-drug conjugate or the method or the use according to any one of items [331] to [462], wherein the monosaccharide units of the solubility tag are in ring form.
[464] The molecule according to any one of items [3] or [6] or [27] to [131] or [146] to [463] or the antibody-drug conjugate according to any one of items [132] to [133] or [146] to [463] or the method according to any one of items [9] or [12] or [15] or [18] to [19] or [27] to [131] or [134] to [141] or [146] to [463] or the use according to any one of items [22] or [25] to [131] or [144] to [463], wherein said solubility tag is/said solubility tags are linked to said antibody-drug conjugate (resp. said molecule) via a covalent bond between a GlcN monosaccharide unit of said solubility tag and said linker.
[465] The molecule according to any one of items [3] or [6] or [27] to [131] or [146] to [464] or the antibody-drug conjugate according to any one of items [132] to [133] or [146] to [464] or the method according to any one of items [9] or [12] or [15] or [18] to [19] or [27] to [131] or [134] to [141] or [146] to [464] or the use according to any one of items [22] or [25] to [131] or [144] to [464], wherein said solubility tag is/said solubility tags are linked to said antibody-drug conjugate (resp. said molecule) via a beta-alanine group that covalently links a GlcN monosaccharide unit of said solubility tag to said linker.
[466] The molecule according to any one of items [3] or [6] or [27] to [131] or [146] to [465] or the antibody-drug conjugate according to any one of items [132] to [133] or [146] to [465] or the method according to any one of items [9] or [12] or [15] or [18] to [19] or [27] to [131] or [134] to [141] or [146] to [465] or the use according to any one of items [22] or [25] to [131] or [144] to [465], wherein said solubility tag is/said solubility tags are linked to said antibody-drug conjugate (resp. said molecule) by formation of a covalent bond between a GlcN monosaccharide unit of said solubility tag and said linker.
[467] The molecule according to any one of items [3] or [6] or [27] to [131] or [146] to [466] or the antibody-drug conjugate according to any one of items [132] to [133] or [146] to [466] or the method according to any one of items [9] or [12] or [15] or [18] to [19] or [27] to [131] or [134] to [141] or [146] to [466] or the use according to any one of items [22] or [25] to [131] or [144] to [466], wherein said solubility tag is/said solubility tags are linked to said antibody-drug conjugate (resp. said molecule) via a covalent bond between said linker and an amino group linked to carbon 2 of a monosaccharide unit of said solubility tag.
[468] The molecule or the antibody-drug conjugate or the method or the use according to item [467], wherein said amino group is linked to carbon 2 of the terminal monosaccharide unit at the non-reducing end of said oligosaccharide (i.e. of the oligosaccharide comprised in said solubility tag resp. of which said solubility tag consists).
[469] The molecule according to any one of items [1] to [6] or [27] to [131] or [146] to [468] or the antibody-drug conjugate according to any one of items [132] to [133] or [146] to [468] or the method according to any one of items [7] to [19] or [27] to [131] or [134] to [141] or [146] to [468] or the use according to any one of items [20] to [131] or [142] to [468], wherein the solubility tag comprises/each solubility tag comprises the oligosaccharide
GlcN-GlcNAc-GlcNAc-GlcNAc-GlcNAc.
[470] The molecule according to any one of items [1] to [6] or [27] to [131] or [146] to [469] or the antibody-drug conjugate according to any one of items [132] to [133] or [146] to [469] or the method according to any one of items [7] to [19] or [27] to [131] or [134] to [141] or [146] to [469] or the use according to any one of items [20] to [131] or [142] to [469], wherein the solubility tag consists of/each solubility tag consists of the oligosaccharide
GlcN-GlcNAc-GlcNAc-GlcNAc-GlcNAc.
[471] The molecule or the antibody-drug conjugate or the method or the use according to any one of items [469] to [470], wherein said oligosaccharide GlcN-GlcNAc-GlcNAc-GlcNAc-GlcNAc is linked to another component of the antibody-drug conjugate by a covalent bond to the GlcN monosaccharide unit of said oligosaccharide.
[472] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) *O*-(2-desoxy-2-amino-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D*-glucopyranosyl)-(1→4)-*O*-(2-acetamido-2-desoxy-*D*-glucopyranose.
[473] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) *O*-(2-Desoxy-2-amino-*β*-*D*-glucopyranosyl)-(→4)-*O*-(2-acetamido-2-desoxy-*β*-*D*-glucopyranosyl)-(1→4)-*O*-(2-acetamido-2-desoxy-*β*-*D-*glucopyranosyl)-(1→4)-*O*-(2-acetamido-2-desoxy-*β*-*D*-glucopyranosyl)-(1→4)-*O-*{(6-desoxy-2-*O*-methyl-*α*-*L*-galactopyranosyl)-(1→)-*O*}-(2-acetamido-2-desoxy-D-glucopyranose).
[474] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) *O*-(2-Desoxy-2-amino-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D-*glucopyranosyl)-(1→4)-*O*-(2-acetamido-2-desoxy-*D*-glucopyranose).
[475] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) the sodium salt of *O*-(2-Desoxy-2-amino-*β*-*D*-glucopyranosyl)-(1→4)-*O*-(2-acetamido-2-desoxy-*β*-*D*-glucopyranosyl)-(1→4)-*O*-(2-acetamido-2-desoxy-*β-D*-glucopyranosyl)-(1→4)-*O*-(2-acetamido-2-desoxy-6-*O*-sulfo-*D*-glucopyranose).
[476] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) *O*-(2-Desoxy-2-amino-*β*-*D*-glucopyranosyl)-(1→4)-*O*-(2-acetamido-2-desoxy-*D*-glucopyranose).
[477] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) N-[(3R,4R,6R)-5-[(2S,3R,4R,6R)-3-acetamido-5-[(2S,3R,4R,6R)-3-acetamido-5-[(2S,3R,4R,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide.
[478] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) N-[(3R,4R,5S,6R)-5-[(2S,3R,4R,5S,6R)-3-acetamido-5-[(2S,3R,4R,5S,6R)-3-acetamido-5-[(2S,3R,4R,5S,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-6-[[(2R,3S,4R,5S,6S)-4,5-dihydroxy-3-methoxy-6-methyl-tetrahydropyran-2-yl]oxymethyl]-2,4-dihydroxy-tetrahydropyran-3-yl]acetamide.
[479] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) N-[(3R,4R,5S,6R)-5-[(2S,3R,4R,5S,6R)-3-acetamido-5-[(2S,3R,4R,5S,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide.
[480] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) ((2R,3S,4R,5R)-5-acetamido-3-(((2S,3R,4R,5S,6R)-3-acetamido-5-(((2S,3R,4R,5S,6R)-3-acetamido-5-(((2S,3R,4R,5S,6R)-3-amino-4,5-dihydroxy-6-(hydroxymethyl)tetrahydro-2H-pyran-2-yl)oxy)-4-hydroxy-6-(hydroxymethyl)tetrahydro-2H-pyran-2-yl)oxy)-4-hydroxy-6-(hydroxymethyl)tetrahydro-2H-pyran-2-yl)oxy)-4,6-dihydroxytetrahydro-2H-pyran-2-yl)methyl sulfate Natrium (I).
[481] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is or comprises (preferably is) N-[(3R,4R,5S,6R)-5-[(2S,3R,4R,5S,6R)-3-amino-4,5-dihydroxy-6-(hydroxymethyl)tetrahydropyran-2-yl]oxy-2,4-dihydroxy-6-(hydroxymethyl)tetrahydropyran-3-yl]acetamide.
[482] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag comprises a chemical group with the structural formula (I): 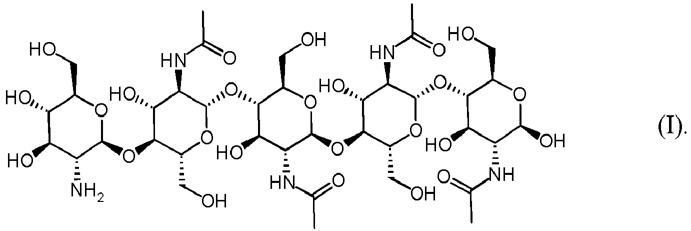 [483] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag comprises a chemical group with the structural formula (II): 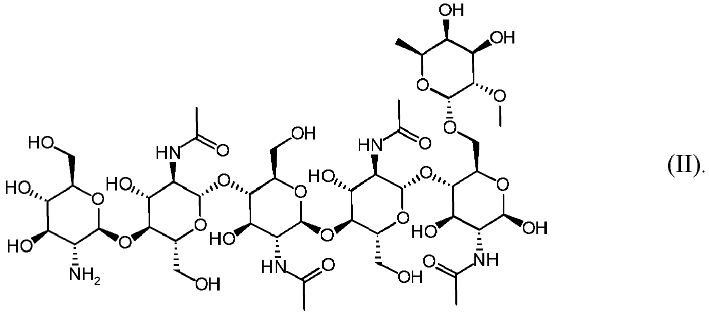 [484] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag comprises a chemical group with the structural formula (III): 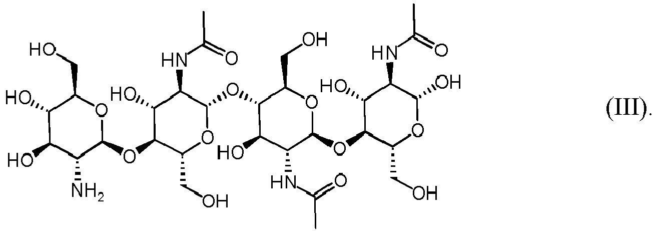 [485] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag comprises a chemical group with the structural formula (IV): 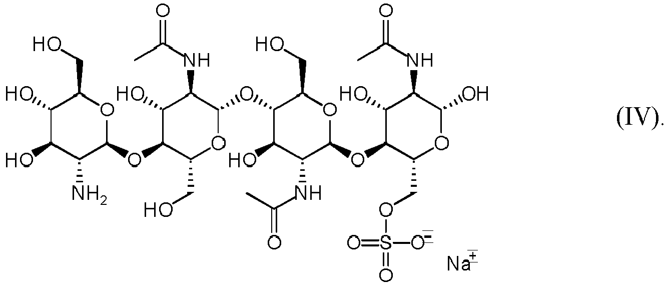 [486] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is a chemical group with the structural formula (I): 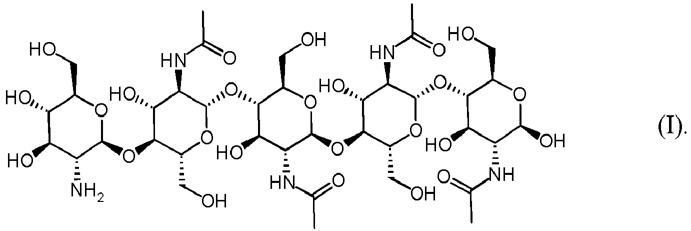 [487] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is a chemical group with the structural formula (II): 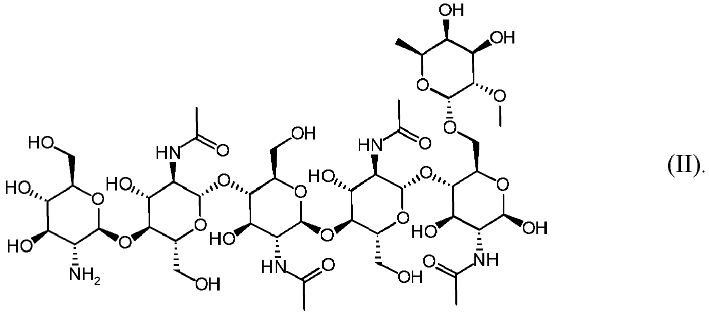 [488] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is a chemical group with the structural formula (III): 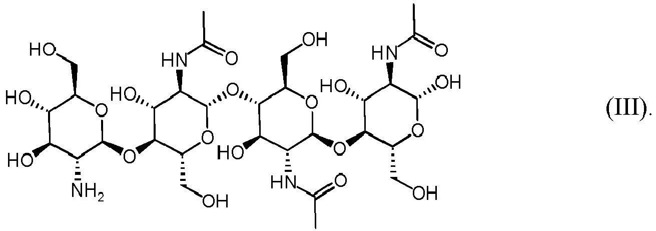 [489] The molecule according to any one of items [1] to [6] or [27] to [131] or [146] to [471] or the antibody-drug conjugate according to any one of items [132] to [133] or [146] to [471] or the method according to any one of items [7] to [19] or [27] to [131] or [134] to [141] or [146] to [471] or the use according to any one of items [20] to [131] or [142] to [471], wherein said solubility tag/each solubility tag is a chemical group with the structural formula (IV): 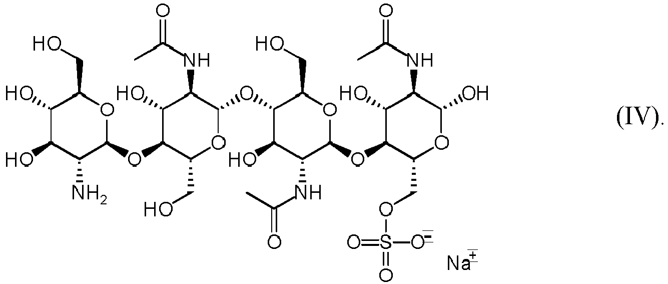 [490] The molecule according to any one of items [1] to [6] or [27] to [131] or [146] to [489] or the antibody-drug conjugate according to any one of items [132] to [133] or [146] to [489] or the method according to any one of items [7] to [19] or [27] to [131] or [134] to [141] or [146] to [489] or the use according to any one of items [20] to [131] or [142] to [489], wherein said solubility tag/each solubility tag is not an N-linked glycan.
[491] The molecule according to any one of items [1] to [6] or [27] to [131] or [146] to [490] or the antibody-drug conjugate according to any one of items [132] to [133] or [146] to [490] or the method according to any one of items [7] to [19] or [27] to [131] or [134] to [141] or [146] to [490] or the use according to any one of items [20] to [131] or [142] to [490], wherein said solubility tag/each solubility tag is not an N-linked glycan, does not comprise an N-linked glycan and is not a molecular group within an N-linked glycan.
[492] The molecule according to any one of items [1] to [6] or [27] to [131] or [146] to [491] or the antibody-drug conjugate according to any one of items [132] to [133] or [146] to [491] or the method according to any one of items [7] to [19] or [27] to [131] or [134] to [141] or [146] to [491] or the use according to any one of items [20] to [131] or [142] to [491], wherein said solubility tag/each solubility tag is not an N-linked glycan or O-linked glycan.
[493] The molecule according to any one of items [1] to [6] or [27] to [131] or [146] to [492] or the antibody-drug conjugate according to any one of items [132] to [133] or [146] to [492] or the method according to any one of items [7] to [19] or [27] to [131] or [134] to [141] or [146] to [492] or the use according to any one of items [20] to [131] or [142] to [492], wherein said solubility tag/each solubility tag is not an N-linked glycan or O-linked glycan, does not comprise an N-linked glycan or O-linked glycan, and is not a molecular group within an N-linked glycan or O-linked glycan.
[494] The molecule according to any one of items [38] to [42] or [44] or [46] to [47] or [49] or [51] to [52] or [54] to [131] or [146] to [493] or the antibody-drug conjugate according to any one of items [132] to [133] or [146] to [493] or the method according to any one of items [38] to [42] or [44] or [46] to [47] or [49] or [51] to [52] or [54] to [131] or [134] to [141] or [146] to [493] or the use according to any one of items [38] to [42] or [44] or [46] to [47] or [49] or [51] to [52] or [54] to [131] or [144] to [493], wherein said antibody component (resp. antibody or antigen-binding fragment of item [38]) does not comprise any chito-oligosaccharide.
[495] The molecule according to any one of items [38] to [42] or [44] or [46] to [47] or [49] or [51] to [52] or [54] to [131] or [146] to [494] or the antibody-drug conjugate according to any one of items [132] to [133] or [146] to [494] or the method according to any one of items [38] to [42] or [44] or [46] to [47] or [49] or [51] to [52] or [54] to [131] or [134] to [141] or [146] to [494] or the use according to any one of items [38] to [42] or [44] or [46] to [47] or [49] or [51] to [52] or [54] to [131] or [144] to [494], wherein said antibody component (resp. antibody or antigen-binding fragment of item [38]) is either not glycosylated or only carries a glycosylation at position Asn 297 (EU numbering).
[496] The molecule or the antibody-drug conjugate or the method or the use according to item [495], wherein said glycosylation at Asn 297 is the natural antibody glycosylation.
[497] The molecule according to any one of items [38] to [42] or [44] or [46] to [47] or [49] or [51] to [52] or [54] to [131] or [146] to [496] or the antibody-drug conjugate according to any one of items [132] to [133] or [146] to [496] or the method according to any one of items [38] to [42] or [44] or [46] to [47] or [49] or [51] to [52] or [54] to [131] or [134] to [141] or [146] to [496] or the use according to any one of items [38] to [42] or [44] or [46] to [47] or [49] or [51] to [52] or [54] to [131] or [144] to [496], wherein said antibody component (resp. said antibody or antigen-binding fragment of item [38]) does not carry any monosaccharides or carries no monosaccharides besides the monosaccharides in the antibody glycosylation.
[498] The molecule according to any one of items [38] to [42] or [44] or [46] to [47] or [49] or [51] to [52] or [54] to [131] or [146] to [497] or the antibody-drug conjugate according to any one of items [132] to [133] or [146] to [497] or the method according to any one of items [38] to [42] or [44] or [46] to [47] or [49] or [51] to [52] or [54] to [131] or [134] to [141] or [146] to [497] or the use according to any one of items [38] to [42] or [44] or [46] to [47] or [49] or [51] to [52] or [54] to [131] or [144] to [497], wherein said antibody component (resp. said antibody or antigen-binding fragment of item [38]) does not carry any antibody glycosylation.
[499] The molecule according to any one of items [38] to [42] or [44] or [46] to [47] or [49] or [51] to [52] or [54] to [131] or [146] to [498] or the antibody-drug conjugate according to any one of items [132] to [133] or [146] to [498] or the method according to any one of items [38] to [42] or [44] or [46] to [47] or [49] or [51] to [52] or [54] to [131] or [134] to [141] or [146] to [498] or the use according to any one of items [38] to [42] or [44] or [46] to [47] or [49] or [51] to [52] or [54] to [131] or [144] to [498], wherein said antibody component (resp. said antibody or antigen-binding fragment of item [38]) does not comprise any monosaccharide units.
[500] The molecule according to any one of items [1] to [6] or [27] to [131] or [146] to [499] or the antibody-drug conjugate according to any one of items [132] to [133] or [146] to [499] or the method according to any one of items [7] to [19] or [27] to [131] or [134] to [141] or [146] to [499] or the use according to any one of items [20] to [131] or [142] to [499], wherein said antibody-drug conjugate (resp. said molecule) comprises no chito-oligosaccharide beyond the chito-oligosaccharide(s) of the solubility tag(s).
[501] The molecule according to any one of items [38] to [42] or [44] or [46] to [47] or [49] or [51] to [52] or [54] to [131] or [146] to [500] or the antibody-drug conjugate according to any one of items [132] to [133] or [146] to [500] or the method according to any one of items [38] to [42] or [44] or [46] to [47] or [49] or [51] to [52] or [54] to [131] or [134] to [141] or [146] to [500] or the use according to any one of items [38] to [42] or [44] or [46] to [47] or [49] or [51] to [52] or [54] to [131] or [144] to [500], wherein said antibody-drug conjugate (resp. said molecule) comprises no other monosaccharide units beyond the monosaccharide units of the solubility tag(s) and optionally monosaccharide units that are part of the glycosylation of the antibody component (resp. of the antibody or antigen-binding fragment of item [38]).
[502] The molecule according to any one of items [1] to [6] or [27] to [131] or [146] to [501] or the antibody-drug conjugate according to any one of items [132] to [133] or [146] to [501] or the method according to any one of items [7] to [19] or [27] to [131] or [134] to [141] or [146] to [501] or the use according to any one of items [20] to [131] or [142] to [501], wherein said antibody-drug conjugate (resp. said molecule) comprises no other monosaccharide units beyond the monosaccharide units of the solubility tag(s).
[503] The molecule according to any one of items [1] to [6] or [27] to [131] or [146] to [502] or the antibody-drug conjugate according to any one of items [132] to [133] or [146] to [502] or the method according to any one of items [7] to [19] or [27] to [131] or [134] to [141] or [146] to [502] or the use according to any one of items [20] to [131] or [142] to [502], wherein said molecule with solubility tag(s) has a higher solubility than a molecule of the same structure, but without said solubility tag(s).
[504] The molecule according to any one of items [130] to [131] or [146] to [502] or the antibody-drug conjugate according to any one of items [132] to [133] or [146] to [502] or the method according to any one of items [134] to [141] or [146] to [502] or the use according to any one of items [142] to [502], wherein said ADC with solubility tag(s) has a higher solubility than an ADC of the same structure, but without said solubility tag(s).
[505] The molecule according to any one of items [130] to [131] or [146] to [504] or the antibody-drug conjugate according to any one of items [132] to [133] or [146] to [504] or the method according to any one of items [134] to [141] or [146] to [504] or the use according to any one of items [142] to [504], wherein a corresponding molecule without said payload (i.e. a molecule composed of only the antibody component, the linker and the solubility tag of said antibody-drug conjugate) is non-toxic to mice at a dose of 6 mg per kg of body weight of said mice administered by intravenous administration.
[506] The molecule according to any one of items [130] to [131] or [146] to [505] or the antibody-drug conjugate according to any one of items [132] to [133] or [146] to [505] or the method according to any one of items [134] to [141] or [146] to [505] or the use according to any one of items [142] to [505], wherein said ADC is such that a corresponding molecule without said payload (i.e. a molecule composed of only the antibody component, the linker and the solubility tag of said antibody-drug conjugate) does not lead to any signs of liver toxicity in an animal study with mice.
[507] The molecule or the antibody-drug conjugate or the method or the use according to item [506], wherein said animal study with mice involves the administration of said molecule without said payload to adult female BALB/c Nude mice in a single intravenous administration at a dose of 6 mg of ADC per kg of body weight of said mice, preparation of formalin-fixed liver tissue stained with hematoxylin & eosin and histopathological analysis under the light microscope, wherein the absence of visible lesions under the light microscope indicates the absence of liver toxicity.
[508] A method for preparing an antibody-drug conjugate as defined in any one of items [130] to [507], said method comprising the step of
- carrying out a reaction resulting in the formation of a covalent bond between (a) a molecule comprising an antibody component (resp. antibody or antigen-binding fragment) as defined in any one of items [38] to [42] or [44] or [46] to [47] or [49] or [51] to [52] or [54] to [141] or [144] to [507], a payload as defined in any one of items [75] to [141] or [144] to [507] and a linker as defined in any one of items [3] or [6] or [9] or [12] or [15] or [18] to [19] or [22] or [25] to [141] or [144] to [507] and (b) a solubility tag as defined in any one of items [1] to [19] or [27] to [141] or [146] to [507], or
- carrying out a reaction resulting in the formation of a covalent bond between (a) an antibody component (resp. antibody or antigen-binding fragment) as defined in any one of items [38] to [42] or [44] or [46] to [47] or [49] or [51] to [52] or [54] to [141] or [144] to [507] and (b) a molecule comprising a payload as defined in any one of items [75] to [141] or [144] to [507], a linker as defined in any one of items [3] or [6] or [9] or [12] or [15] or [18] to [19] or [22] or [25] to [141] or [144] to [507] and a solubility tag as defined in any one of items [1] to [19] or [27] to [141] or [146] to [507], or
- carrying out a reaction resulting in the formation of a covalent bond between (a) a molecule comprising an antibody component (resp. antibody or antigen-binding fragment) as defined in any one of items [38] to [42] or [44] or [46] to [47] or [49] or [51] to [52] or [54] to [141] or [144] to [507], a linker as defined in any one of items [3] or [6] or [9] or [12] or [15] or [18] to [19] or [22] or [25] to [141] or [144] to [507] and a solubility tag as defined in any one of items [1] to [19] or [27] to [141] or [146] to [507] and (b) a payload as defined in any one of items [75] to [141] or [144] to [507], or
- carrying out a reaction resulting in the formation of a covalent bond between (a) a molecule comprising an antibody component (resp. antibody or antigen-binding fragment) as defined in any one of items [38] to [42] or [44] or [46] to [47] or [49] or [51] to [52] or [54] to [141] or [144] to [507] and a linker as defined in any one of items [3] or [6] or [9] or [12] or [15] or [18] to [19] or [22] or [25] to [141] or [144] to [507] and (b) a molecule comprising a solubility tag as defined in any one of items [1] to [19] or [27] to [141] or [146] to [507] and a payload as defined in any one of items [75] to [141] or [144] to [507],
to yield an antibody-drug conjugate with a covalently linked solubility tag.
[509] A method for preparing an antibody-drug conjugate according to item [508], wherein said payload is a therapeutic agent or a detectable label.
[510] A compound for use in the preparation of an antibody-drug compound according to any one of items [132] to [133] or [146] to [507], wherein said compound comprises a solubility tag as defined in any one of items [1] to [19] or [27] to [141] or [146] to [507] linked to an activator group.
[511] The compound according to item [510], wherein said compound consists of a solubility tag as defined in any one of items [1] to [19] or [27] to [141] or [146] to [507] linked to an activator group.
[512] The compound according to any one of items [510] or [511], wherein said activator group is selected from the group consisting of a maleimide, a halogen-acetamide, an alkyl halogen, a Michael acceptor (wherein said Michael acceptor is preferably a vinylpyridine) and a group suitable for cycloaddition (wherein said group suitable for cycloaddition is preferably a ketone, hydrazone, semicarbazone, carboxylic acid, alkene or alkyne suitable for cycloaddition).
[513] An antibody-drug compound that has been prepared by a method according to any one of items [508] to [509].
[514] A pharmaceutical composition comprising the antibody-drug compound according to any one of items [130] to [131] or [146] to [507] or [513].
[515] The pharmaceutical composition according to item [514], wherein said pharmaceutical composition comprises a pharmaceutically acceptable carrier, diluent and/or excipient.
[516] The pharmaceutical composition according to any one of items [514] to [515], wherein said pharmaceutical composition further includes at least one additional adjuvant, antioxidant, buffering agent, bulking agent, colorant, emulsifier, filler, flavoring agent, preservative, stabilizer, suspending agent and/or other customary pharmaceutical auxiliary.
[517] An antibody-drug conjugate according to any one of items [130] to [131] or [146] to [507] or [513] or a pharmaceutical composition according to any one of items [514] to [516] for use as a medicament.
[518] An antibody-drug conjugate according to any one of items [130] to [131] or [146] to [507] or [513] or a pharmaceutical composition according to any one of items [514] to [516] for use in the treatment of cancer.
[519] An antibody-drug conjugate according to any one of items [130] to [131] or [146] to [507] or [513] or a pharmaceutical composition according to any one of items [514] to [516] for use in the treatment of a malignant tumor.
[520] An antibody-drug conjugate of any one of items [130] to [131] or [146] to [507] or [513] or a pharmaceutical composition of any one of items [514] to [516] for use in the treatment of an inflammatory disease.
[521] The antibody-drug conjugate or the pharmaceutical composition for use according to any one of items [517] to [520], wherein said antibody-drug conjugate and said pharmaceutical composition are for use in the treatment of a human.
[522] A method for treating a disease in a patient in need thereof, comprising the step of administering to said patient a therapeutically effective amount of the antibody-drug conjugate according to any one of items [130] to [131] or [146] to [507] or [513] or the pharmaceutical composition according to any one of items [514] to [516].
[523] The method according to item [522], wherein said disease is cancer.
[524] The method according to item [522], wherein said disease is a malignant tumor.
[525] The method according to item [522], wherein said disease is an inflammatory disease.
[526] The method according to any one of items [522] to [525], wherein said patient is a human.
[527] Use of the antibody-drug conjugate according to any one of items [130] to [131] or [146] to [507] or [513] or of the pharmaceutical composition according to any one of items [514] to [516] for the manufacture of a medicament.
[528] Use of the antibody-drug conjugate according to any one of items [130] to [131] or [146] to [507] or [513] or of the pharmaceutical composition according to any one of items [514] to [516] for the manufacture of a medicament for the treatment of cancer.
[529] Use of the antibody-drug conjugate according to any one of items [130] to [131] or [146] to [507] or [513] or of the pharmaceutical composition according to any one of items [514] to [516] for the manufacture of a medicament for the treatment of a malignant tumor.
[530] Use of the antibody-drug conjugate according to any one of items [130] to [131] or [146] to [507] or [513] or of the pharmaceutical composition according to any one of items [514] to [516] for the manufacture of a medicament for the treatment of an inflammatory disease.
[531] The use according to any one of items [527] to [530], wherein said medicament is prepared for administration to a human.
[532] The antibody-drug conjugate or the pharmaceutical composition for use according to any one of items [520] to [521] or the method according to any one of items [525] to [526] or the use according to any one of items [530] to [531], wherein said inflammatory disease is an autoimmune disease.
[533] The antibody-drug conjugate or the pharmaceutical composition for use according to any one of items [520] to [521] or [532] or the method according to any one of items [525] to [526] or [532] or the use according to any one of items [530] to [532], wherein said inflammatory disease is selected from the group consisting of inflammatory bowel disease (IBD), systemic lupus erythematosus (SLE), multiple sclerosis, rheumatoid arthritis, Sjogren's syndrome and Hidradenitis suppurativa (HS).
[534] The antibody-drug conjugate or the pharmaceutical composition for use according to any one of items [518] to [521] or the method according to any one of items [523] to [526] or the use according to any one of items [528] to [521], wherein said cancer, malignant tumor or inflammatory disease is a human disease.

### EXAMPLES

The following examples describe the preparation and characterization of antibody-drug conjugates with the oligosaccharide tags as described in the present disclosure, as well as related compounds and methods, along with comparative disclosure. It is understood that various embodiments of the disclosure reflected in the examples may be practiced, given the general description provided above. Although the foregoing invention has been described in some detail by way of illustration and example for purposes of clarity of understanding, the description and examples should not be construed as limiting the scope of the invention.

If in the structural formula of a molecule below an oxygen atom is depicted as if it formed only a single covalent bond (-O), but no hydrogen atom linked to that oxygen atom is depicted (i.e. not -OH or -O-H), it is understood that this oxygen atom is saturated by in addition forming a covalent bond to a hydrogen atom (even if this hydrogen atom linked to that oxygen atom by a covalent bond is not depicted in the structural formula).

Similarly, if in the structural formula of a molecule below a nitrogen atom is depicted that is depicted as if it formed only one or two covalent bonds, it is understood that this nitrogen atom is saturated by in addition being covalently bound to hydrogen atom(s) such that the nitrogen forms three covalent bonds in total (even if not all hydrogen atoms linked to that nitrogen atom by a covalent bond are depicted in the structural formula).

### Example 1: Preparation of Linker-Payload Constructs

### Overview of Linker-Payload Constructs Prepared

**Table 2: Overview of linker-payload constructs and related compounds prepared**

| **Linker- Payload** | **Linker cleavage** | **Payload** | **Oligosaccharide tag** |
|---|---|---|---|
| Compound 1 | Cathepsin B | Auri statin¹ | --- |
| Compound 2 | Cathepsin B | Auristatin | CO-V² |
| Compound 3 | Cathepsin B | Duocarmycin | CO-V |
| Compound 4 | Cathepsin B | Auristatin | CO-V |
| Compound 5 | Cathepsin B | Duocarmycin | --- |
| Compound 6 | Cathepsin B | Duocarmycin | CO-V |
| Compound 7 | Cathepsin B | Duocarmycin | CO-V |
| Compound 8 | --- | --- | CO-V |
| Compound 9 | Disulfide | Maytansinoid³ | CO-V |
| Compound 10 | Disulfide | Maytansinoid | CO-V |
| Compound 11 | Disulfide | Maytansinoid | CO-V |
| Compound 12 | Cathepsin B | CBI⁴ | --- |
| Compound 13 | Cathepsin B | CBI | CO-V |
| Compound 14 | Cathepsin B | Duocarmycin | CO-V |
| Compound 15 | Cathepsin B | Auristatin | CO-V |
| Compound 16 | Glucuronide | Duocarmycin | --- |
| Compound 17 | Glucuronide | Duocarmycin | PEG |
| Compound 18 | Glucuronide | Duocarmycin | CO-V |
| Compound 19 | Glucuronide | Duocarmycin | CO-V |
| Compound 20 | Glucuronide | Auristatin | CO-V |
| Compound 21 | Cathepsin B | Auristatin | CO-V |
| Compound 22 | Cathepsin B | Auristatin | CO-V |

| | | | |
|---|---|---|---|
| ¹ The auristatin in the listed compounds was MMAE (monomethyl auristatin E). ² For details about CO-V, see below, section "Oligosaccharide Synthesis". ³ The maytansinoid in the listed compounds was DM4 (= ravtansine). ⁴ Cyclopropanebenz[e]indoline dimer | | | |

### Chemical Structure of Linker-Payload Constructs

Linker payload constructs were synthesized by standard methods of chemical synthesis as described below. The reactions were monitored and all reaction products were characterized by HPLC-MS (High performance liquid chromatography-mass spectrometry) using an Agilent LC MS-1200-6120B. In all cases, characterization confirmed that the described product was obtained.

The following linker-payload constructs linked to an oligosaccharide solubility tag were prepared/obtained:
MC-VC-MMAE (Compound 1): 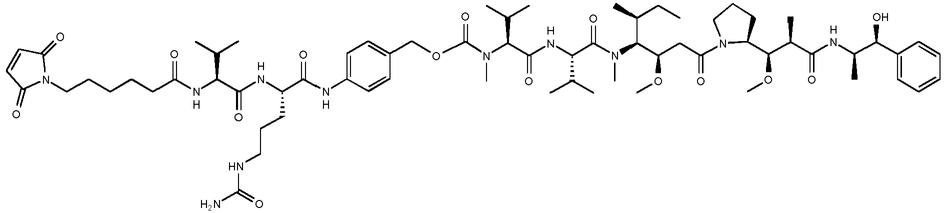
CRD012/619 (Compound 2): 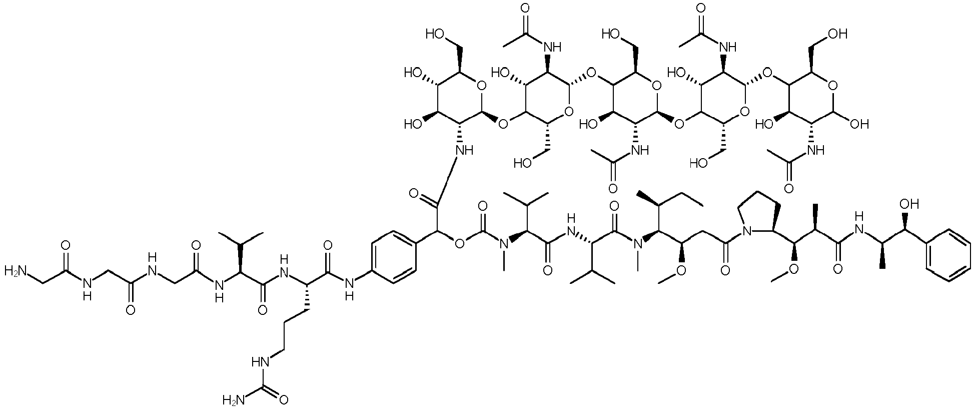
CRD013/413 (Compound 3): 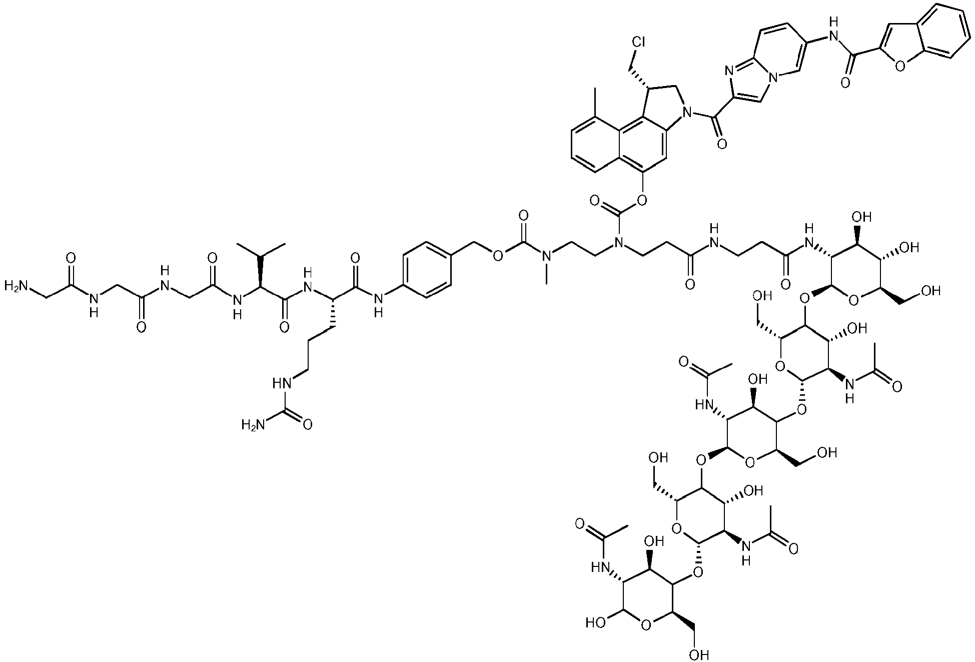
CRD012/636 (Compound 4): 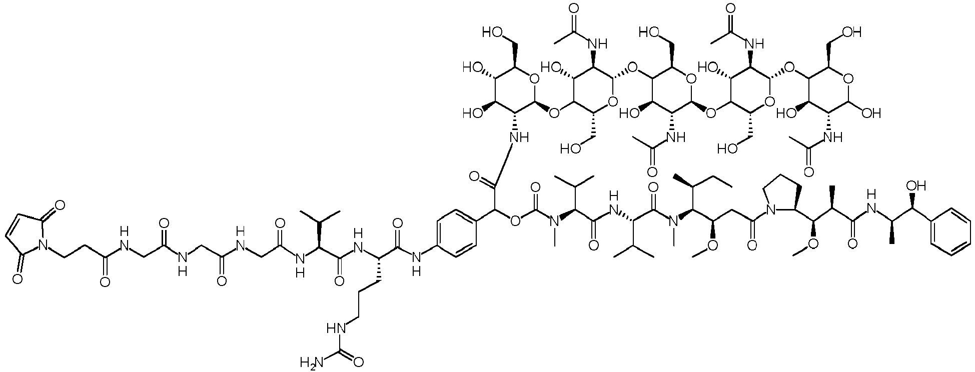
MC901_335-02 (Compound 5): 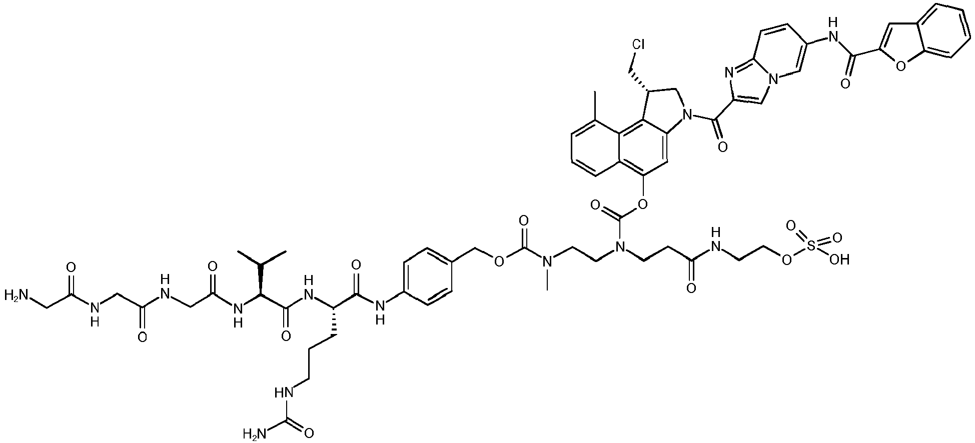
MC901_333-10 (Compound 6): 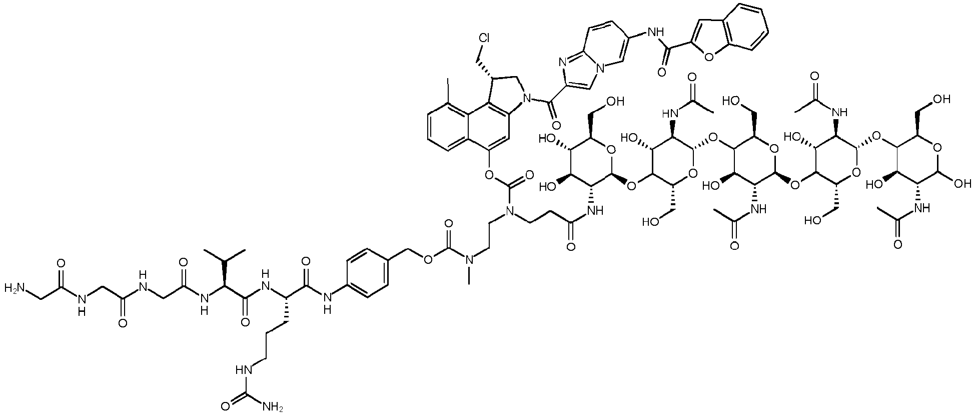
MC901_337-6 (Compound 7): 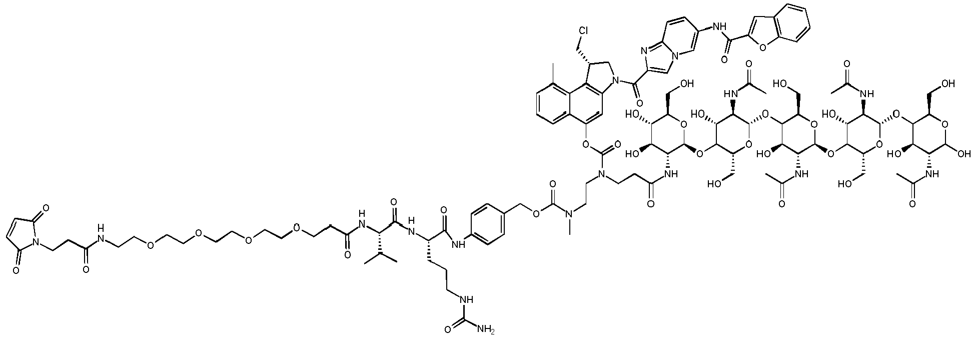
CRD018/206 (Compound 8):

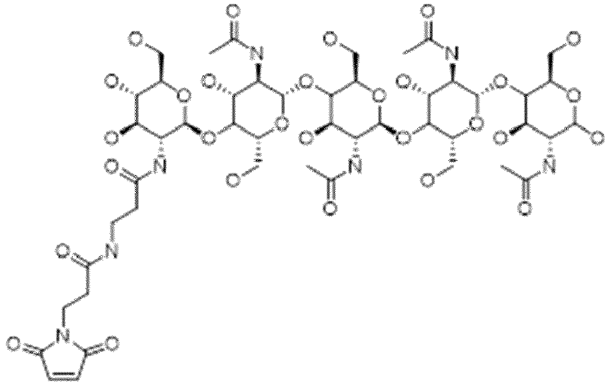

This compound is an oligosaccharide linked to maleimide as activator group. The compound can be used to attach a solubility tag/solubility tags according to the present disclosure to an ADC in order to directly study the effect of the tag on the solubility of a compound.
MC901_346-11 (Compound 9): 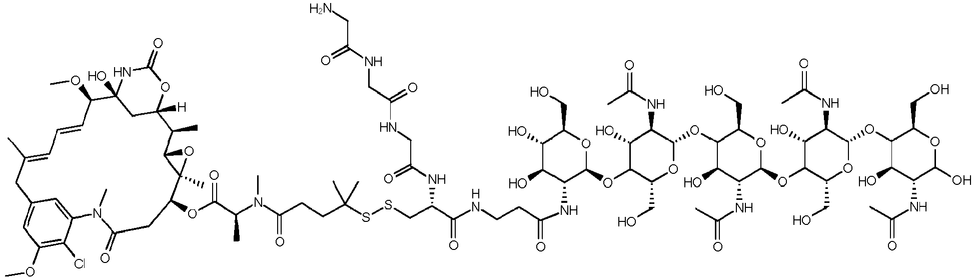
MC901_347-9 (Compound 10): 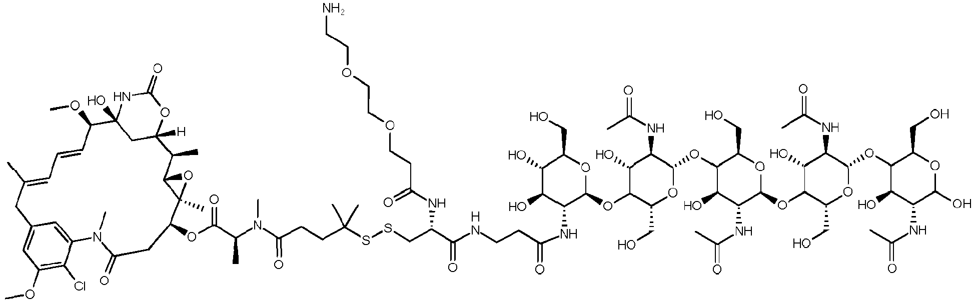
MC901_347-10 (Compound 11): 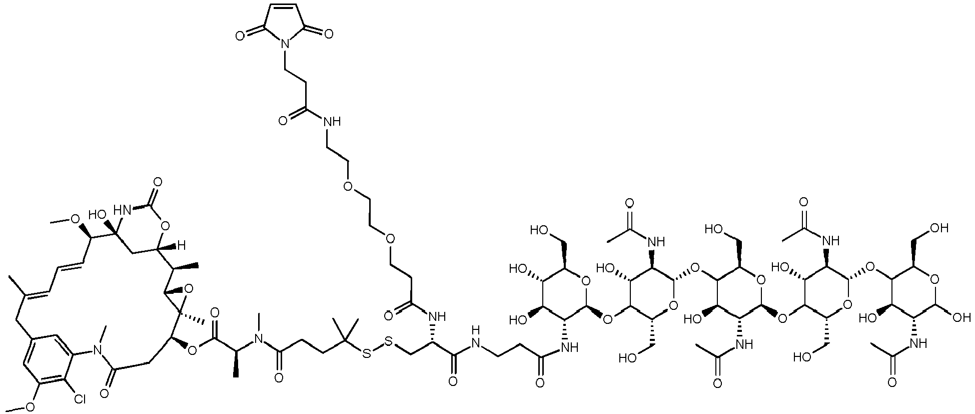
MC901_453 (Compound 12): 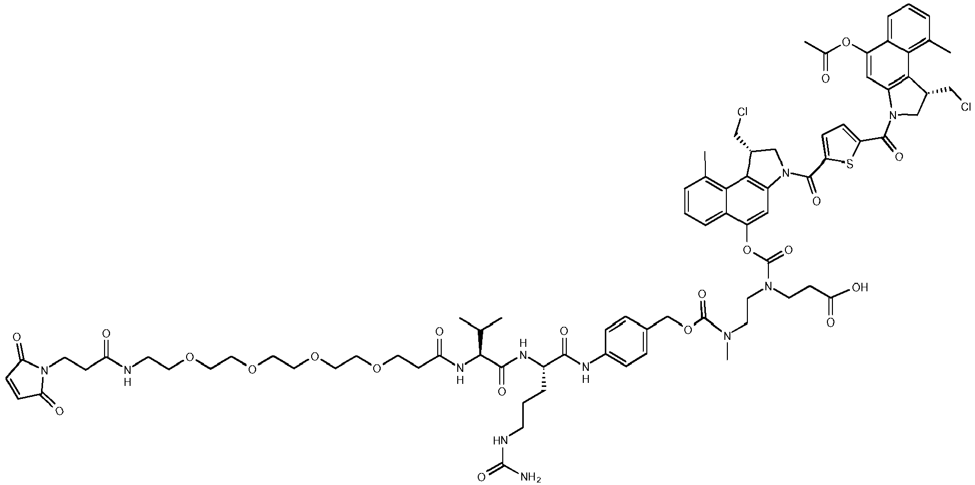
MC901_433-93 (Compound 13): 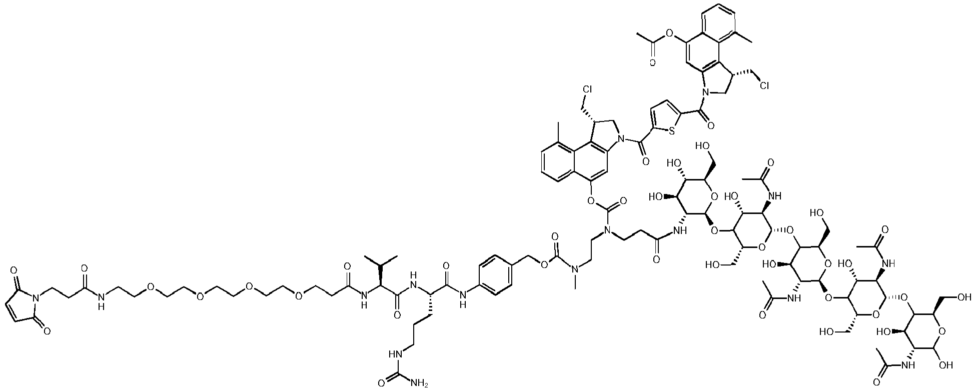
MC901_458 (Compound 14): 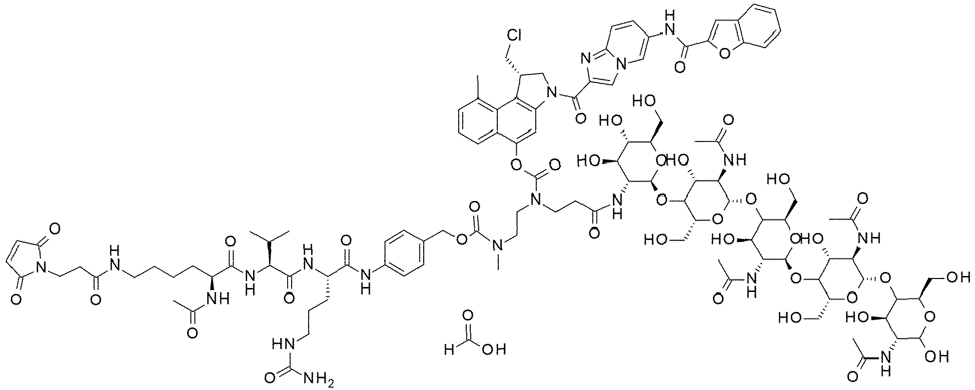
MC901_362-42 (Compound 15):
CRD010/072 (Compound 16): 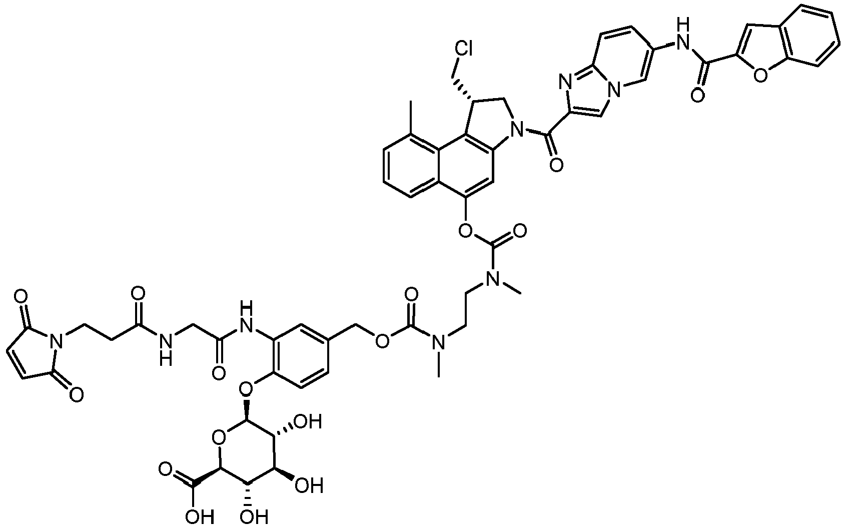
MC901_482 (Compound 17): 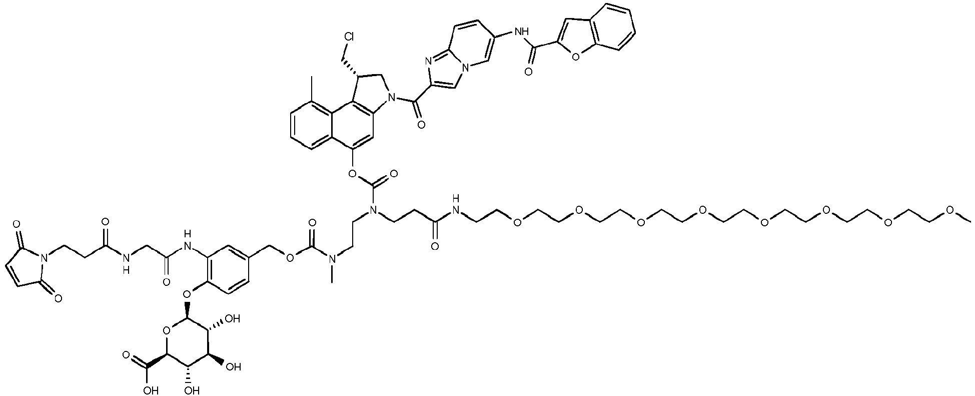
CRD012/789 (Compound 18): 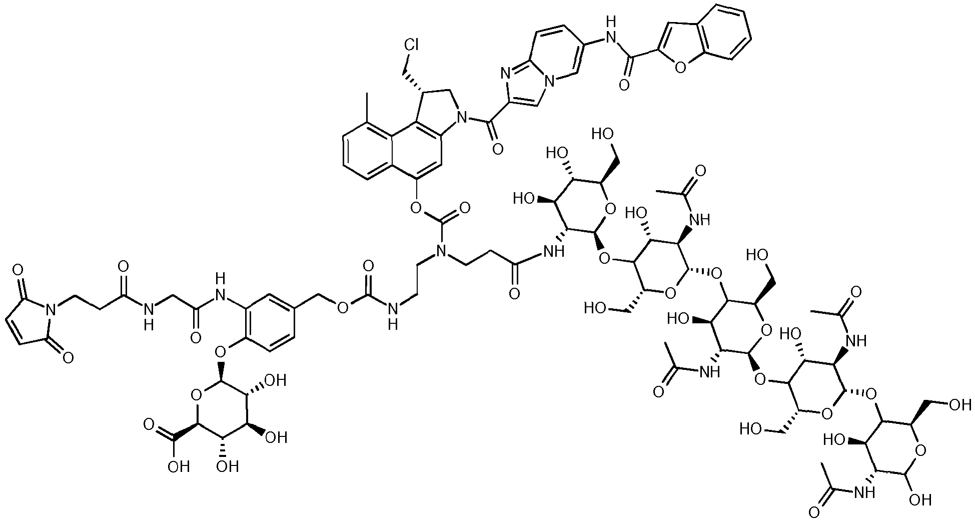
MC901_488-7 (Compound 19): 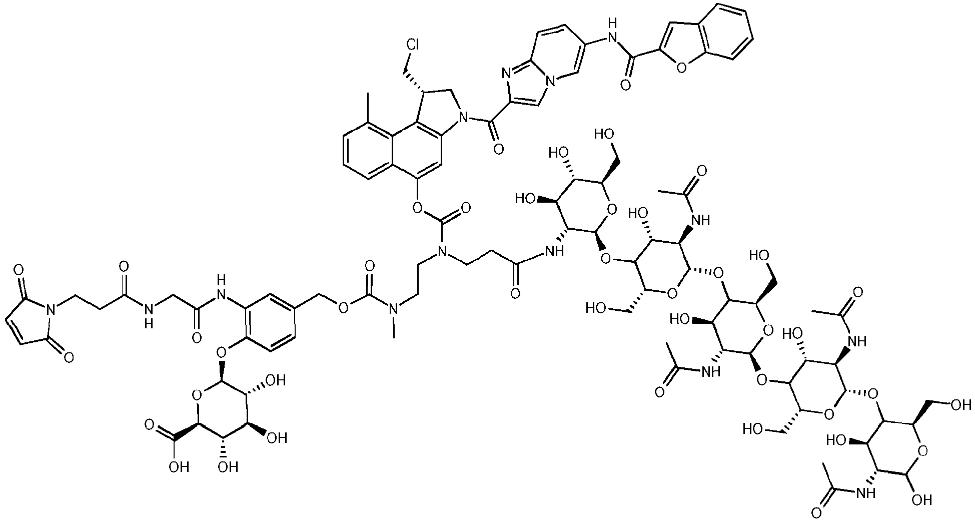
MC901_455-45 (Compound 20): 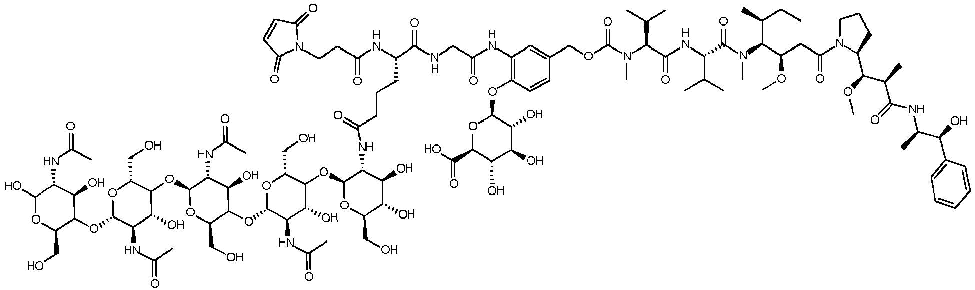
MC901_614 (Compound 21): 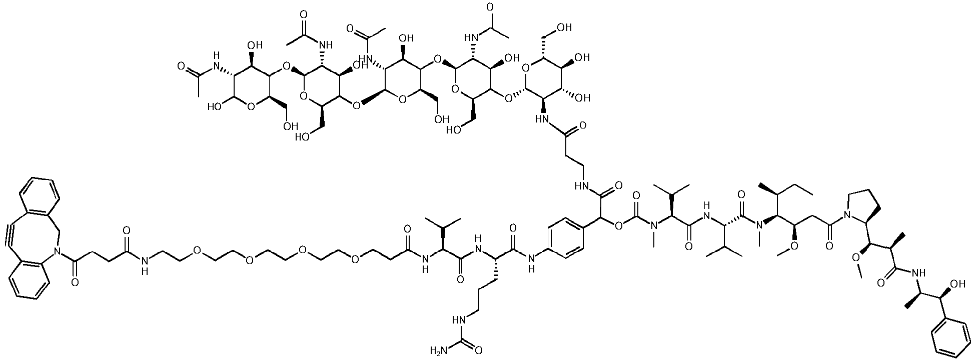
CRD012/604 (Compound 22):

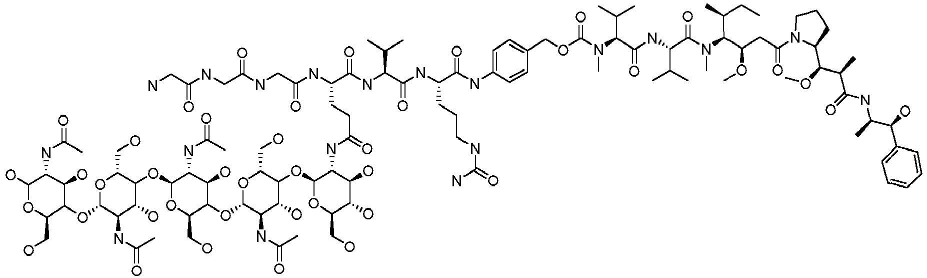

### Oligosaccharide Synthesis

Oligosaccharides were synthesized via a biotechnological approach. Specifically, the chito-oligosaccharide
CO-V: 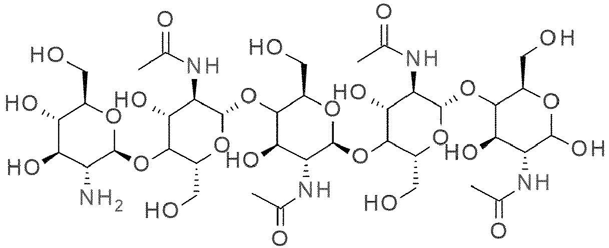 which can be designated as:
*O*-(2-desoxy-2-amino-*β*-*D*-glucopyranosyl)-(1→4)-*O*-(2-acetamido-2-desoxy-*β*-*D-*glucopyranosyl)-(1→4)-*O*-(2-acetamido-2-desoxy-*β*-*D*-glucopyranosyl)-(1→4)-*O-*(2-acetamido-2-desoxy-*β*-*D*-glucopyranosyl)-(1→4)-*O*-(2-acetamido-2-desoxy-*D-*glucopyranose
or in IUPAC nomenclature as:
N-[(3R,4R,6R)-5-[(2S,3R,4R,6R)-3-acetamido-5-[(2S,3R,4R,6R)-3-acetamido-5-[(2S,3R,4R,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide
was produced by use of a recombinant *E. coli* strain according to Samain et al., Carbohydrate Research (1997), vol. 302, p. 35-42 and Samain et al., Biotechnol. (1999), vol. 72, p. 33-47. The oligosaccharide was subsequently purified by charcoal adsorption followed by ion exchange chromatography and, if required for obtaining a fully purified product (typically > 90 %, at least > 70 %), HPLC purification. The identity of the oligosaccharide was confirmed by HPLC - MS.

By the same approach, the following chito-oligosaccharides are prepared, and their identity is confirmed:
CO-V(MeFuc): 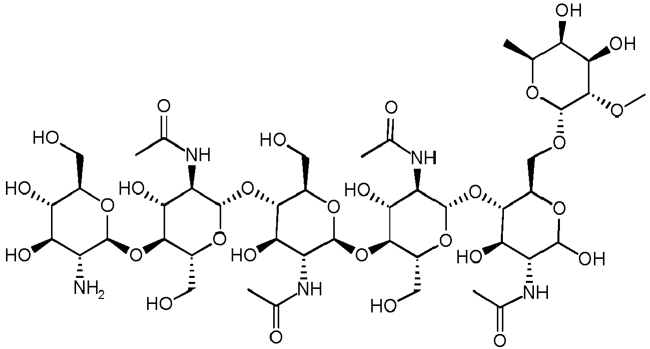 which can be designated as:
*O*-(2-Desoxy-2-amino-*β*-*D*-glucopyranosyl)-(1→4)-*O*-(2-acetamido-2-desoxy-*β*-*D-*glucopyranosyl)-(1→4)-*O*-(2-acetamido-2-desoxy-*β*-*D*-glucopyranosyl)-(1→4)-*O-*(2-acetamido-2-desoxy-*β*-*D*-glucopyranosyl)-(1→4)-*O*-{(6-desoxy-2-*O*-methyl-*α-L-*galactopyranosyl)-(1→6)-*O*}-(2-acetamido-2-desoxy-D-glucopyranose)
or in IUPAC nomenclature as:
N-[(3R,4R,5S,6R)-5-[(2S,3R,4R,5S,6R)-3-acetamido-5-[(2S,3R,4R,5S,6R)-3-acetamido-5-[(2S,3R,4R,5S,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-6-[[(2R,3S,4R,5S,6S)-4,5-dihydroxy-3-methoxy-6-methyl-tetrahydropyran-2-yl]oxymethyl]-2,4-dihydroxy-tetrahydropyran-3-yl]acetamide
CO-IV: 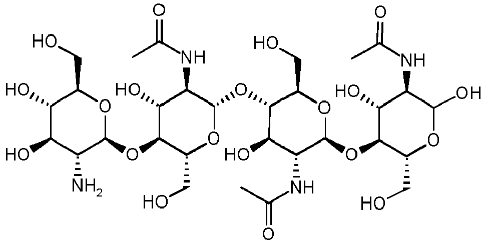 which can be designated as:
*O*-(2-Desoxy-2-amino-*β*-*D*-glucopyranosyl)-(1→4)-*O*-(2-acetamido-2-desoxy-*β*-*D-*glucopyranosyl)-(1→4)-*O*-(2-acetamido-2-desoxy-*β*-*D*-glucopyranosyl)-(1→4)-*O-*(2-acetamido-2-desoxy-*D*-glucopyranose)
or in IUPAC nomenclature as:
N-[(3R,4R,5S,6R)-5-[(2S,3R,4R,5S,6R)-3-acetamido-5-[(2S,3R,4R,5S,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide
CO-IV(S): 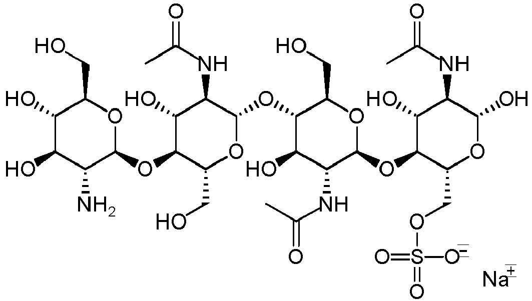 which can be designated as:
the sodium salt of *O*-(2-Desoxy-2-amino-*β*-*D*-glucopyranosyl)-(1→4)-*O*-(2-acetamido-2-desoxy-*β*-*D*-glucopyranosyl)-(1→4)-*O*-(2-acetamido-2-desoxy-*β*-*D-*glucopyranosyl)-(1→4)-*O*-(2-acetamido-2-desoxy-6-*O*-sulfo-*D*-glucopyranose)
or in IUPAC nomenclature as:
((2R,3S,4R,5R)-5-acetamido-3-(((2S,3R,4R,5S,6R)-3-acetamido-5-(((2S,3R,4R,5S,6R)-3-acetamido-5-(((2S,3R,4R,5S,6R)-3-amino-4,5-dihydroxy-6-(hydroxymethyl)tetrahydro-2H-pyran-2-yl)oxy)-4-hydroxy-6-(hydroxymethyl)tetrahydro-2H-pyran-2-yl)oxy)-4-hydroxy-6-(hydroxymethyl)tetrahydro-2H-pyran-2-yl)oxy)-4,6-dihydroxytetrahydro-2H-pyran-2-yl)methyl sulfate Natrium (I)
CO-II: 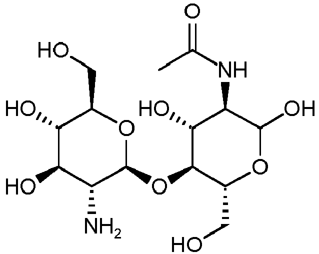 which can be designated as:
*O*-(2-Desoxy-2-amino-*β*-D-glucopyranosyl)-(1→4)-*O*-(2-acetamido-2-desoxy-D-glucopyranose)
or in IUPAC nomenclature as:
N-[(3R,4R,5S,6R)-5-[(2S,3R,4R,5S,6R)-3-amino-4,5-dihydroxy-6-(hydroxymethyl)tetrahydropyran-2-yl]oxy-2,4-dihydroxy-6-(hydroxymethyl)tetrahydropyran-3-yl]acetamide

### MC-VC-MMAE (Compound 1)

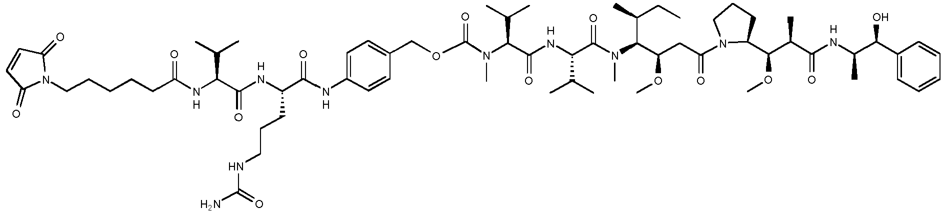

Compound 1 was purchased from Levena Biopharma (San Diego, USA).

### Synthesis of CRD012/619 (Compound 2)

### Synthesis of Intermediate CRD012/450:

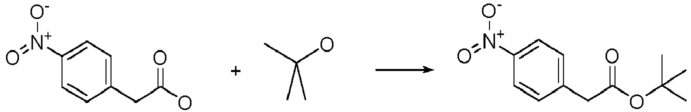

To a solution of 4-N-nitrophenylacetic acid (5.000 g; 1.00 eq.) in chloroform (55.000 ml), dried pyridine (11.140 ml; 5.00 eq.) and tert-butanol (26.230 ml; 10.00 eq.) were added, followed by the addition of phosphoryl chloride (3.295 ml; 1.30 eq.) dropwise over 2 min. The reaction mixture was stirred at room temperature overnight.

The reaction mixture was poured into an ice-cold solution containing dried dichloromethane (12.000 ml) and hydrochloric acid (10%) (5.032 ml; 0.50 eq.). The organic layer was separated, washed with brine and dried over sodium sulfate to provide the crude product as a yellow oil. The residue was purified by flash chromatography on silica gel eluted with cyclohexane/ethyl acetate.

The fractions containing the product were combined and the solvent was removed *in vacuo* to afford (4-nitro-phenyl)-acetic acid tert-butyl ester (5.340 g; 22.305 mmol) as a pale yellow oil. (5.34 g, 80.8%); M-H = 236 (negative)

### Synthesis of CRD012/646:

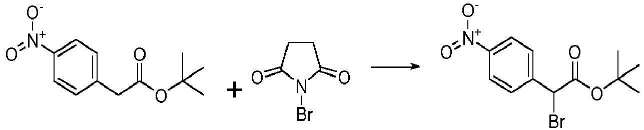

(4-Nitro-phenyl)-acetic acid tert-butyl ester (5.050 g; 1.00 eq.) was dissolved in carbon tetrachloride (50.000 ml). N-bromosuccinimide (1.949 ml; 1.10 eq.) and benzoyl peroxide (with 25% H₂O) (0.338 g; 0.05 eq.) were added. The reaction mixture was heated at reflux overnight.

The reaction mixture was allowed to cool down and filtered through a pad of celite. The filtrate was concentrated under reduced pressure and the residue was purified by flash chromatography on silica gel eluted with cyclohexane/ethyl acetate.

The fractions containing the product were combined and the solvent was removed *in vacuo* to afford bromo-(4-nitro-phenyl)-acetic acid tert-butyl ester (6.018 g; 16.808 mmol) as a yellow oil.
(6 g, 80.4%); M-2H = 314 (negative).

### Synthesis of CRD012/456:

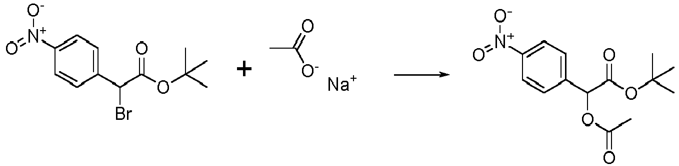

Bromo-(4-nitro-phenyl)-acetic acid tert-butyl ester (7.810 g; 1.00 eq.) was dissolved in a mixture of N,N-dimethylformamide (60.000 ml) and water (30.000 ml) under nitrogen with constant stirring. Following by the addition of sodium acetate (2.199 g; 1.20 eq.), the mixture was stirred at 100 °C for 3 h.

The reaction solution was concentrated under reduced pressure and the residue was partitioned between ethyl acetate and brine. The organic layer was washed with 10% sol. HCl, then with brine and after drying over sodium sulfate, the filtrate was concentrated *in vacuo* to give the crude product as a yellow oil. The residue was purified by flash chromatography on silica gel eluted with cyclohexane / ethyl acetate.

The fractions containing the product were combined and the solvent was removed to afford acetoxy-(4-nitro-phenyl)-acetic acid tert-butyl ester as a yellow oil.
(4.3 g, 60.1%) M-H = 294 (negative).

### Synthesis of CRD012/657:

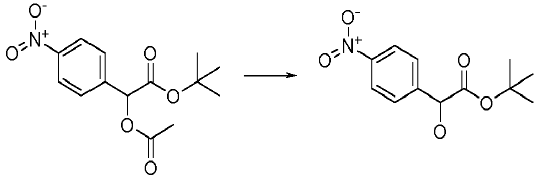

A solution of acetoxy-(4-nitro-phenyl)-acetic acid tert-butyl ester (4.880 g; 1.00 eq.) in dried methanol (10.000 ml) was treated with cesium carbonate extra pure (0.087 ml; 0.10 eq.) in dried methanol (40.000 ml) and stirred at room temperature overnight.

The solvent was removed, and the crude product was purified via flash chromatography on silica gel eluted with cyclohexane/ethyl acetate.

The fractions containing the product were combined and the solvent was removed to afford hydroxy-(4-nitro-phenyl)-acetic acid tert-butyl ester as a yellow solid.
(957 mg, 25.5%); M-H = 252 (negative)

### Synthesis of CRD012/471:

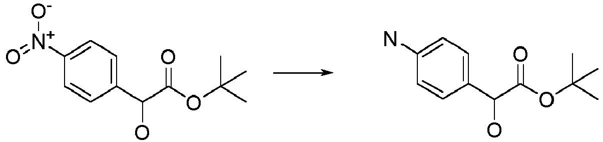

Hydroxy-(4-nitro-phenyl)-acetic acid tert-butyl ester (495.000 mg; 1.00 eq.) was dissolved in dried methanol (20.000 ml) and the solution was filtered over a Whatman AutoCup 0.45 µm (Nylon). The colorless solution was subjected to hydrogenation in the H-cube (Thales Nanotechnology).

The solvent was removed and the residue was lyophilized overnight to afford (4-amino-phenyl)-hydroxy-acetic acid tert-butyl ester as a pale yellow solid.
(437 mg, 100%); M = 224

### Synthesis of CRD012/477:

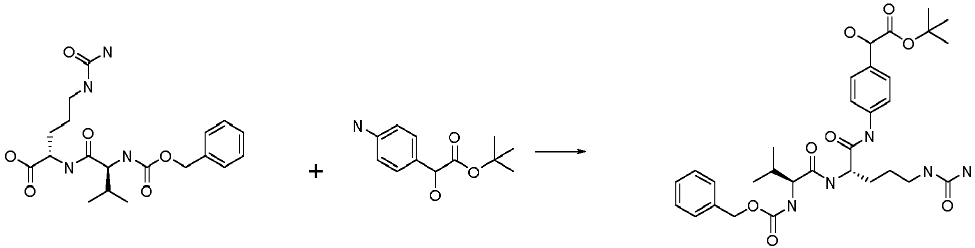

(2S)-2-[[(2S)-2-(benzyloxycarbonylamino)-3-methyl-butanoyl]amino]-5-ureido-pentanoic acid was dissolved in N,N-dimethylformamide (4.000 ml) and the reaction mixture was cooled down to 0°C. 4-Methylmorpholine for synthesis (275 µl; 1.50 eq.) was added dropwise, then HATU ([Dimethylamino-([1,2,3]triazolo[4,5-b]pyridin-3-yloxy)-methylene]-dimethylammonium; hexafluoro phosphate; 700.000 mg; 1.10 eq.) was added, followed by the addition of a solution of (4-amino-phenyl)-hydroxy-acetic acid tert-butyl ester (437.000 mg; 1.00 eq.) in N,N-dimethylformamide (6.000 ml). The ice bath was removed, and the reaction mixture was stirred at room temperature for 5 h.

The reaction solution was concentrated *in vacuo* and 20 mL water were added. The pale-yellow precipitation was collected with suction, washed with water and dried under vacuo overnight to afford a crude product as a yellow solid. The residue was purified by flash chromatography on silica gel eluted with dichloromethane/methanol.

The fractions containing the product were combined and the solvent was removed to afford {4-[(S)-2-((S)-2-Benzyloxycarbonylamino-3-methyl-butyrylamino)-5-ureido-pentanoylamino]-phenyl}-hydroxy-acetic acid tert-butyl ester as a pale yellow solid.
(1.12 g, 92.8%); M+H = 614

### Synthesis of CRD012/480:

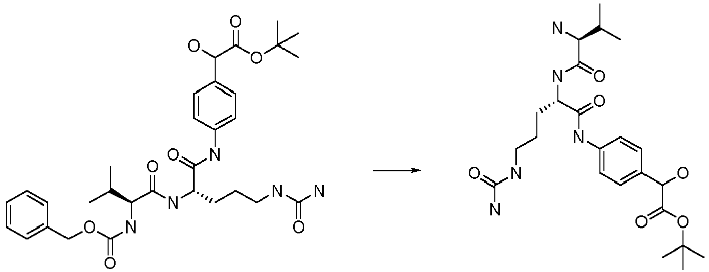

{4-[(S)-2-((S)-2-Benzyloxycarbonylamino-3-methyl-butyrylamino)-5-ureido-pentanoylamino]-phenyl}-hydroxy-acetic acid tert-butyl ester (851.000 mg; 1.00 eq.) was dissolved in dried methanol (100.000 ml) and the solution was filtered over a Whatman AutoCup 0.45 µm (Nylon). The light-yellow solution was subjected to hydrogenation in the H-cube (Thales Nanotechnology).

The solvent was removed under reduced pressure to afford {4-[(S)-2-((S)-2-amino-3-methyl-butyrylamino)-5-ureido-pentanoylamino]-phenyl}-hydroxy-acetic acid tert-butyl ester as a light-yellow viscose oil.
(675 mg, 98.7%); M+H = 480

### Synthesis of CRD012/485:

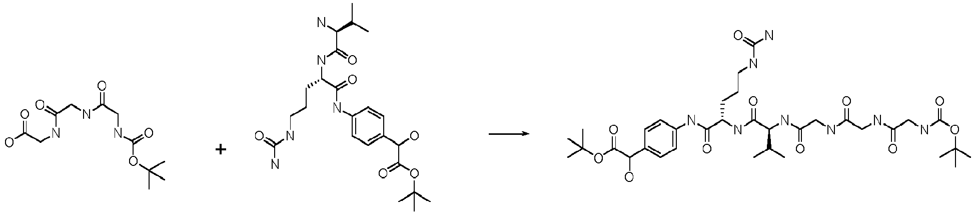

2-[[2-[[2-(tert-butoxycarbonylamino)acetyl]amino]acetyl]amino]acetic acid (885.491 mg; 2.00 eq.) was dissolved in N,N-dimethylformamide (10.000 ml) and the reaction mixture was cooled down to 0°C. 4-methylmorpholine (234 µl; 1.50 eq.) was added dropwise, then HATU (995.109 mg; 1.80 eq.) was added and the pale-yellow solution was stirred for 30 min. Then, a solution of {4-[(S)-2-((S)-2-Amino-3-methyl-butyrylamino)-5-ureido-pentanoylamino]-phenyl}-hydroxy-acetic acid tert-butyl ester (847.000 mg; 1 eq.) in N,N-dimethylformamide (5.000 ml) was added dropwise. The ice bath was removed, and the reaction mixture was stirred at room temperature overnight.

The pale-yellow solution was concentrated to dryness and the crude product was purified by RP (reversed-phase) chromatography eluted with water/acetonitrile.

The fractions containing the product were combined, concentrated *in vacuo* and lyophilized overnight to afford {4-[(S)-2-((S)-2-{2-[2-(2-tert-butoxycarbonylamino-acetylamino)-acetylamino]-acetylamino}-3-methyl-butyrylamino)-5-ureido-pentanoylamino]-phenyl}-hydroxy-acetic acid tert-butyl ester as a white foam.
(897 mg, 72.3%); M+H = 751

### Synthesis of CRD012/528:

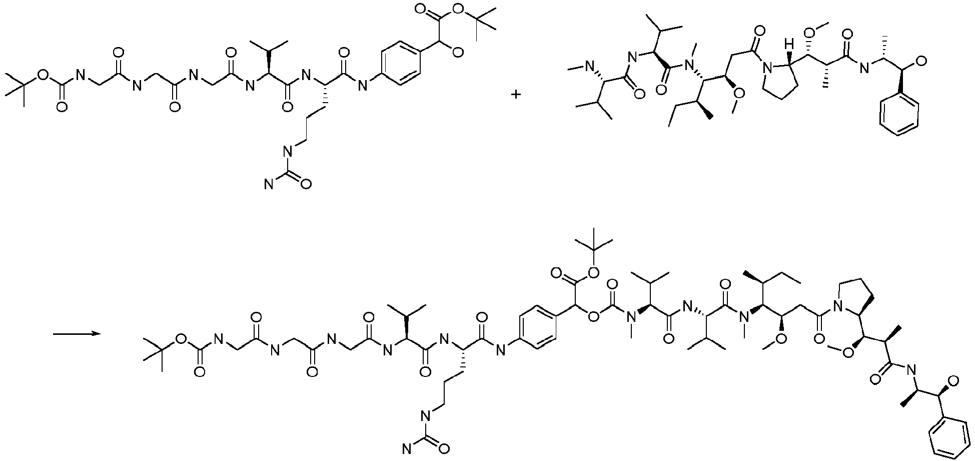

To a solution of {(S)-2-((S)-2-{2-[2-(2-tert-butoxycarbonylamino-acetylamino)-acetylamino]-acetylamino}-3-methyl-butyrylamino)-5-ureido-pentanoylamino]-phenyl}-hydroxy-acetic acid tert-butyl ester (200.000 mg; 1.00 eq.) in N,N-dimethylformamide (5.000 ml) was added bis(4-nitrophenyl) carbonate (106.890 mg; 1.50 eq.) and N-E-ethyldiisopropylamine (120 µl; 3.00 eq.) at room temperature and the reaction mixture was stirred overnight. Monomethyl auristatin E (274.584 mg; 1.60 eq.) and HOBT (1-Hydroxybenzotriazole; 47.478 mg; 1.50 eq.) were added and the yellow solution was stirred at room temperature overnight. The reaction solution was concentrated *in vacuo* as a yellow honey. The crude product was purified by RP flash chromatography.

The fractions containing the product were combined and the solvent was removed to afford tert-butyl 2-[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]-2-[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxopropyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxy-acetate as an off-white solid.
(145 mg, 37.1%); M = 1494

### Synthesis of CRD012/543:

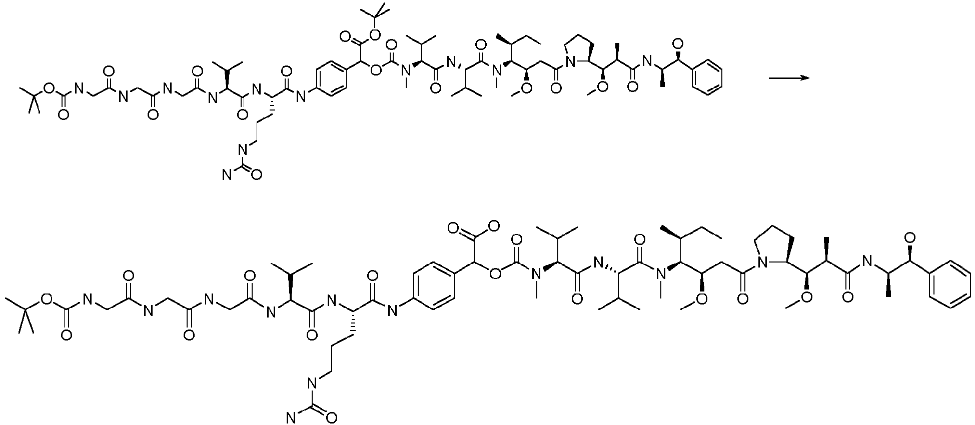

To a cooled solution (ice-bath) of tert-butyl 2-[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]-2-[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxopropyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxy-acetate (125.000 mg; 1.00 eq.) in dried methanol (0.750 ml), a solution of lithium hydroxide monohydrate (10 mg; 3.2 eq.) in dest. water (0.750 ml) was added dropwise. After 15 min the ice-bath was removed, and the yellow solution was stirred at room temperature over 5 days.

The reaction solution was directly purified by preparative HPLC.

The fractions containing the product were combined and lyophilized to afford 2-[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]-2-[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxy-acetic acid as a white solid.
(46 mg, 41.3%); M = 1438

### Synthesis of CRD012/614:

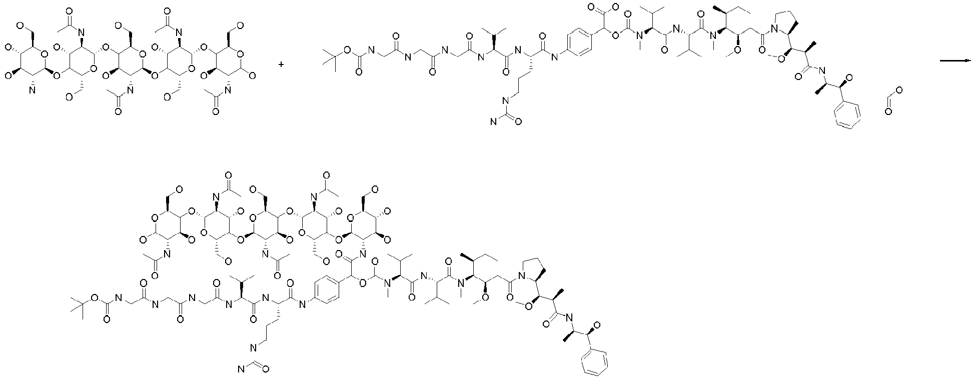

Activation with EEDQ (N-Ethoxycarbonyl-2-ethoxy-1,2-dihydroquinoline: A solution of 2-[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]-2-[[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxopropyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-methyl-carbamoyl]oxy-acetic acid (25.000 mg; 0.56 eq.) and EEDQ (12.087 mg; 1.60 eq.) in N,N-dimethylformamide (1.000 ml) was stirred at room temperature for 3 h.

In a second reaction vessel, N-[(3R,4R,6R)-5-[(2S,3R,4R,6R)-3-acetamido-5-[(2S,3R,4R,6R)-3-acetamido-5-[(2S,3R,4R,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide (30.000 mg; 1.00 eq.) was suspended in N,N-dimethylformamide (1.000 ml) and lithium chloride (7.981 mg; 6.10 eq.) was added. The suspension was sonicated for 5 min. Then, the solution containing the activated acid was added to the amine suspension. The reaction mixture (yellow solution) was stirred at room temperature over 5 days. The solvent was removed, the crude product was purified by preparative HPLC.
The fractions containing the product were combined and lyophilized overnight to afford [2-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-1-[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]-2-oxo-ethyl]N-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methylcarbamoyl]-2-methyl-propyl]carbamoyl]-2-methyl-propyl]-N-methyl-carbamate as a white solid.
(21 mg, 28.6%); M/2 = 1206

### Synthesis of CRD012/619 (Compound 2):

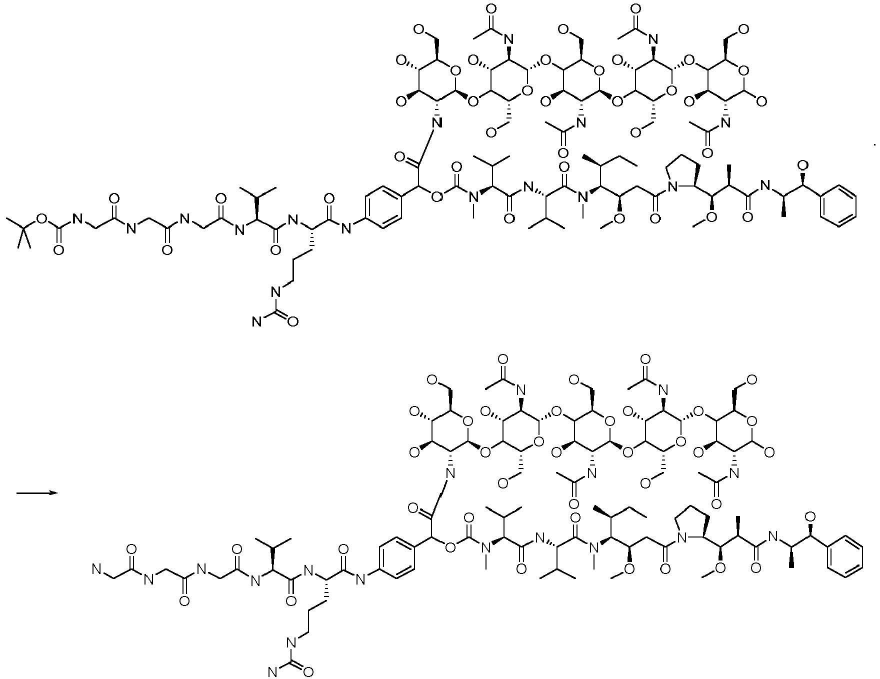

[2-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-1-[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]-2-oxo-ethyl] N-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxo-propyl]pyrrolidin-1-yl]-2-methoxy-1-[(1S)-1-methylpropyl]-4-oxo-butyl]-methylcarbamoyl]-2-methyl-propyl]carbamoyl]-2-methyl-propyl]-N-methyl-carbamate (20.000 mg; 1.00 eq.) was dissolved in acetonitrile extra pure (1.600 ml) and dest. water (0.400 ml), then trifluoroacetic acid (20.000 µl; 31.50 eq.) was added and the solution was heated up to 50 °C. The reaction mixture was stirred for 20 h.

The solution was concentrated *in vacuo* and the crude product was purified by preparative HPLC.

The fractions containing the product were combined and lyophilized overnight to afford: [2-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-1-[4-[[(2S)-2-[[(2S)-2-[[2-[[2-[(2-aminoacetyl)amino]acetyl]amino]acetyl]amino]-3-methyl-butanoyl]amino]-5-ureido-pentanoyl]amino]phenyl]-2-oxo-ethyl] N-[(1S)-1-[[(1S)-1-[[(1S,2R)-4-[(2S)-2-[(1R,2R)-3-[[(1R,2S)-2-hydroxy-1-methyl-2-phenyl-ethyl]amino]-1-methoxy-2-methyl-3-oxopropyl]pyrrolidin-1-yl]-2-methoxy-1-[(1S)-1-methylpropyl]-4-oxo-butyl]-methyl-carbamoyl]-2-methyl-propyl]carbamoyl]-2-methyl-propyl]-N-methyl-carbamate as a white solid.
(12 mg, 63%); M/2 = 1156

### Synthesis of CRD013/413 (Compound 3)

### Synthesis of CRD018/185 (Compound 8):

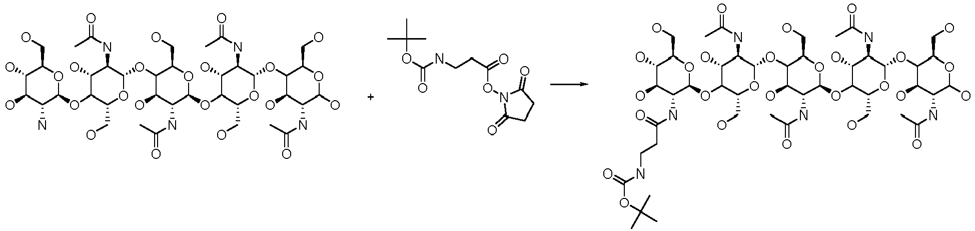

To a stirred solution of N-[(3R,4R,6R)-5-[(2S,3R,4R,6R)-3-acetamido-5-[(2S,3R,4R,6R)-3-acetamido-5-[(2S,3R,4R,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide (200.00 mg; 0.16 mmol; 1.00 eq.) and triethylamine (0.03 ml; 0.24 mmol; 1.50 eq.) in dried dimethyl sulfoxide (2.00 ml) was added a solution of (2,5-dioxopyrrolidin-1-yl) 3-(tert-butoxycarbonylamino)propanoate (69.97 mg; 0.24 mmol; 1.50 eq.) in dried dimethyl sulfoxide (2.00 ml) at room temperature and the colorless solution was stirred at room temperature overnight.

The reaction solution was injected directly onto the column of the preparative HPLC. The fractions containing the desired product were combined and lyophilized overnight to afford tert-butyl N-[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]carbamate.
(80 mg, 42.6%); (M+Na) = 1185

### Synthesis of CRD018/205:

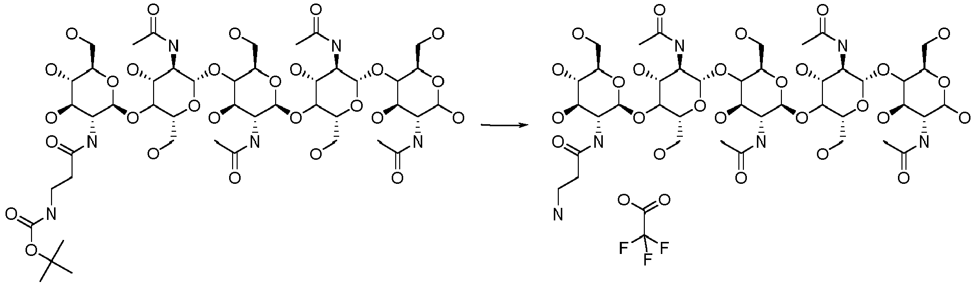

Tert-butyl N-[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]carbamate (80.00 mg; 0.07 mmol; 1.00 eq.) was dissolved in trifluoroacetic acid (0.01 ml; 0.14 mmol; 2.00 eq.) and water (1.00 ml). The solution was heated to 50 °C for 2 h.

The reaction mixture was lyophilized overnight to afford N-[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]-3-amino-propanamide trifluoroacetate
(80 mg, 98.8%); M = 1063

### Synthesis of CRD013/408:

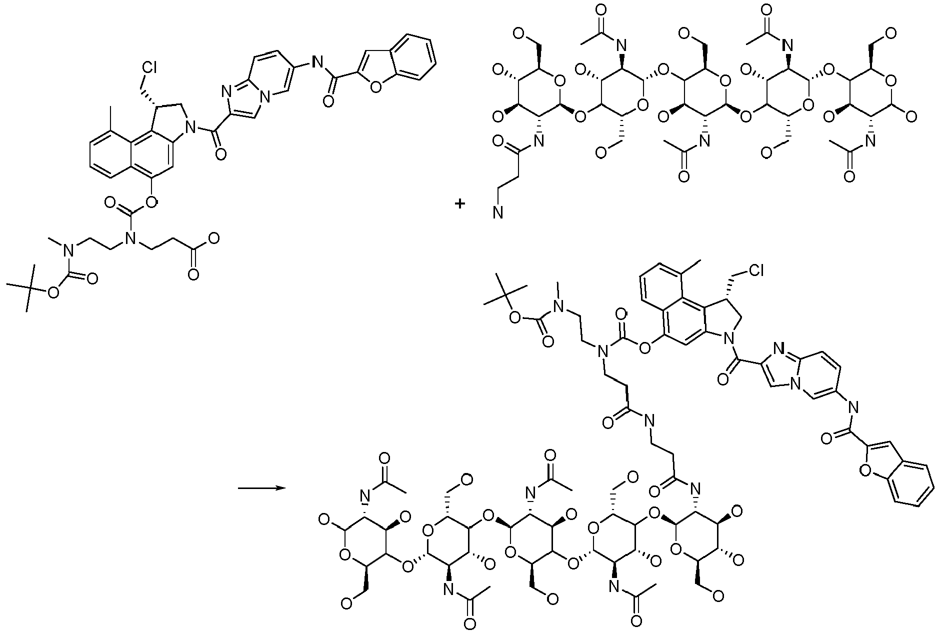

3-[({[(1S)-3-[6-(1-benzofuran-2-amido)imidazo[1,2-a]pyridine-2-carbonyl]-1-(chloromethyl)-9-methyl-1H,2H,3H-benzo[e]indol-5-yl]oxy}carbonyl)(2-{[(tert-butoxy)carbonyl](methyl)amino}ethyl)amino]propanoic acid (30.00 mg; 0.04 mmol; 1.00 eq.) was dissolved in N,N-dimethylformamide (2.00 ml). HATU (10.29 mg; 0.04 mmol; 1.20 eq.) was added and the solution was stirred at room temperature for 30 min. Then N-[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]-3-amino-propanamide trifluoroacetate and 4-methylmorpholine (0.01 ml; 0.07 mmol; 2.00 eq.) were added and the solution was stirred at room temperature for 1 h.

The reaction solution was injected directly onto the column of the preparative HPLC. The fractions containing the desire product were combined and lyophilized overnight to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]amino]-3-oxo-propyl]-N-[2-[tert-butoxycarbonyl(methyl)amino]ethyl]carbamate.
(28 mg, 41%); M+H = 1868

### Synthesis of CRD013/410:

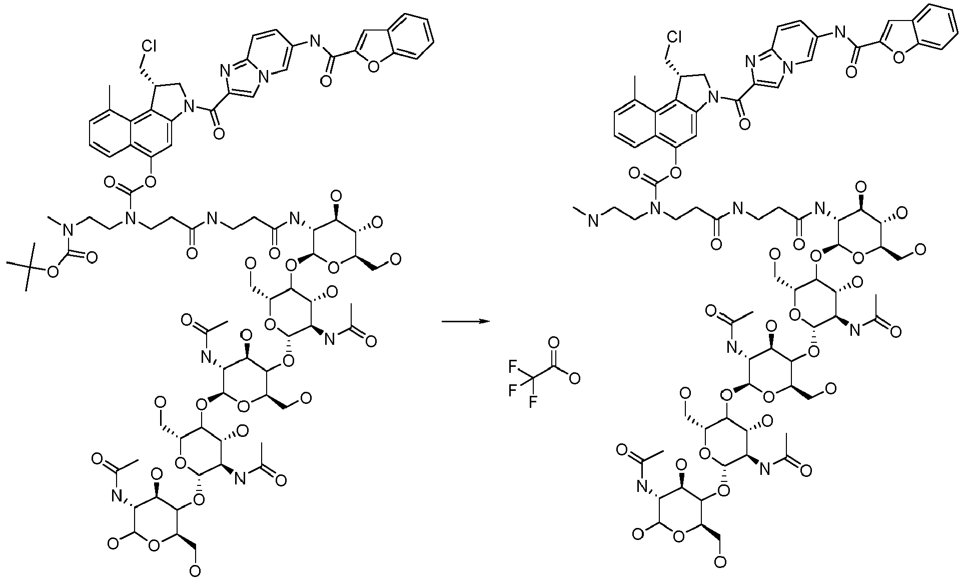

[(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[3-[[(2S,3R,4R,SS,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]amino]-3-oxo-propyl]-N-[2-[tert-butoxycarbonyl(methyl)amino]ethyl]carbamate (28.00 mg; 0.01 mmol; 1.00 eq.) was dissolved in water/acetonitrile 1:1 and heated at 50 °C for 2 days.

The solution was concentrated and lyophilized overnight to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]amino]-3-oxo-propyl]-N-[2-(methylamino)ethyl]carbamate;2,2,2-trifluoroacetic acid.
(27 mg, 95%); M+H = 1769

The product was used in the next step without further purification.

### Synthesis of CRD013/412:

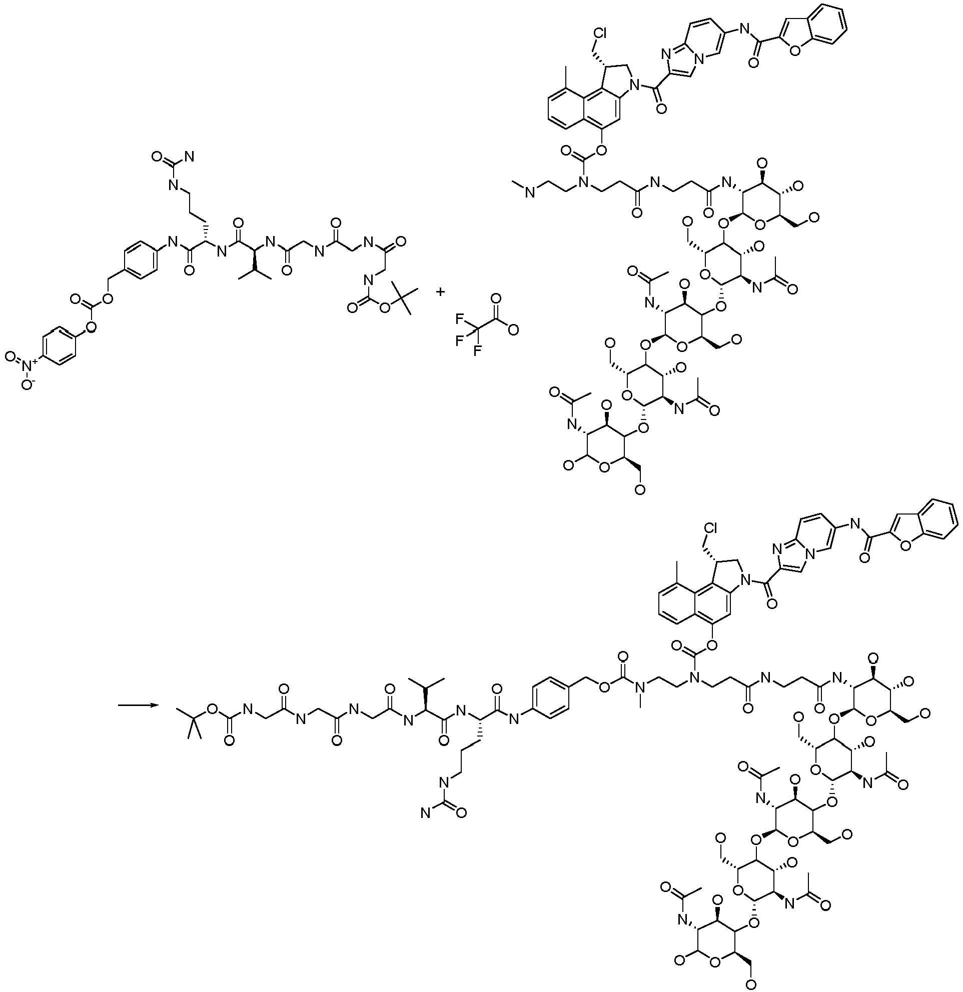

To a solution of carbonic acid 4-[(S)-2-((S)-2-{2-[2-(2-tert-butoxycarbonylamino-acetylamino)-acetylamino]-acetylamino}-3-methyl-butyrylamino)-5-ureido-pentanoylamino]-benzyl ester 4-nitro-phenyl ester (11.70 mg; 0.01 mmol; 1.00 eq.) in N,N-dimethylformamide (2.00 ml), N-ethyldiisopropylamine (4.88 µl; 0.03 mmol; 2.00 eq.), [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[3-[[(2S,3R,4R,SS,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]amino]-3-oxo-propyl]-N-[2-(methylamino)ethyl]carbamate;2,2,2-trifluoroacetic acid (27.00 mg; 0.01 mmol; 1.00 eq.) and HOBT (0.97 mg; 0.01 mmol; 0.50 eq.) were added and the yellow solution was stirred at room temperature for 2 h.

The reaction solution was injected directly onto the column of the preparative HPLC. The fractions containing the desired product were combined and lyophilized overnight to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]amino]-3-oxo-propyl]-N-[2-[[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]methoxycarbonyl-methyl-amino]ethyl]carbamate (4.8 mg, 13.7%) M/2 = 1223

### Synthesis of CRD013/413 (Compound 3):

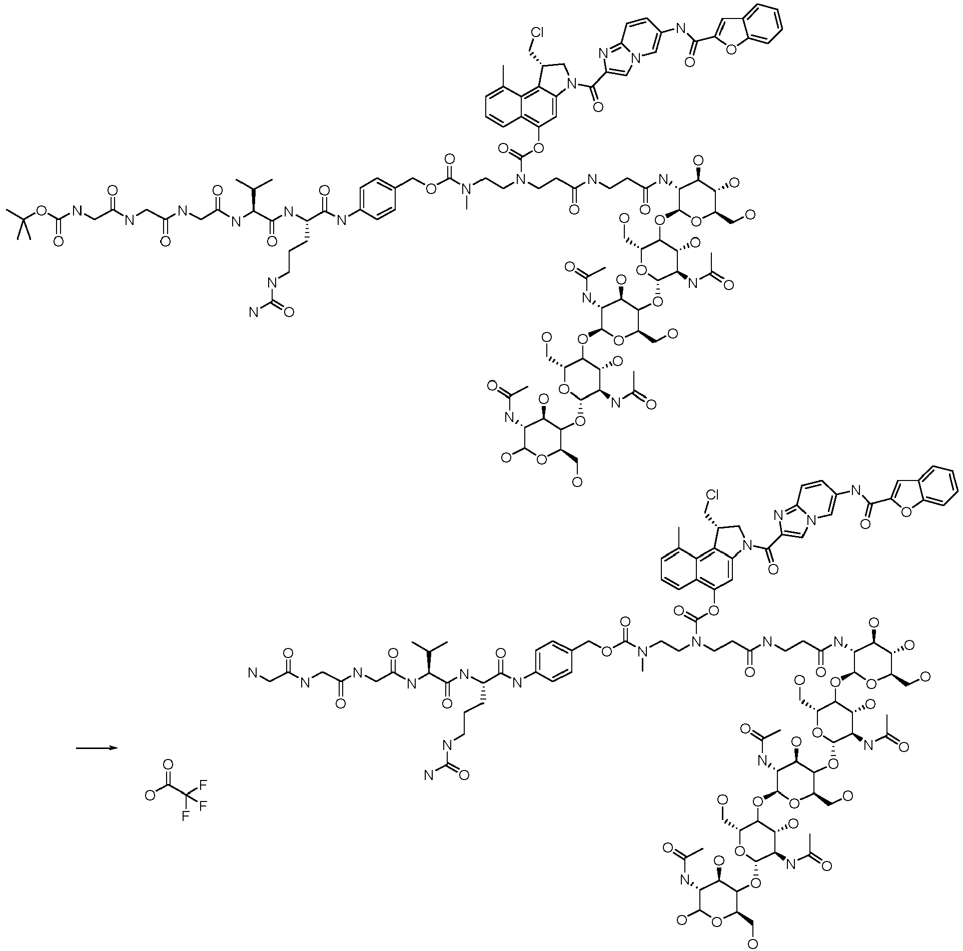

[(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[3-[[(2S,3R,4R,SS,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]amino]-3-oxo-propyl]-N-[2-[[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]methoxycarbonyl-methyl-amino]ethyl]carbamate (4.80 mg; 0.002 mmol; 1.00 eq.) was dissolved in trifluoroacetic acid (0.151 µl; 0.002 mmol; 1.00 eq.) and water (1.00 ml). The solution was stirred at room temperature overnight and heated to 50 °C for 2 h.

The reaction mixture was lyophilized overnight to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]amino]-3-oxo-propyl]-N-[2-[[4-[[(2S)-2-[[(2S)-2-[[2-[[2-[(2-aminoacetyl)amino]acetyl]amino]acetyl]amino]-3-methyl-butanoyl]amino]-5-ureido-pentanoyl]amino]phenyl]methoxycarbonyl-methyl-amino]ethyl]carbamate;2,2,2-trifluoroacetic acid.
(4.9 mg, 96.4%) M/2+H = 1173

### Synthesis of CRD012/636 (Compound 4)

### Synthesis of CRD012/636 (Compound 4):

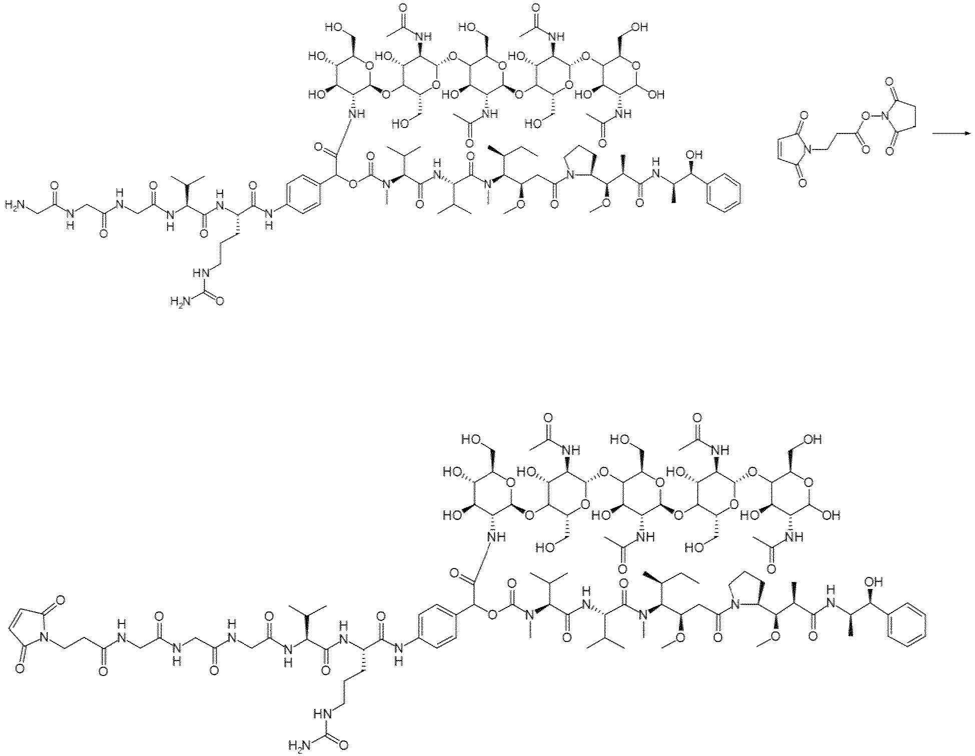

To a stirred solution of {4-[(2S)-2-[(2S)-2-{2-[2-(2-aminoacetamido)acetamido]acetamido}-3-methylbutanamido]-5-(carbamoylamino)pentanamido]phenyl}({[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl})methyl N-[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamate + 2,5-dioxopyrrolidin-1-yl 3-(2,5-dioxo-2,5-dihydro-1H-pyrrol-1-yl)propanoate → {4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}({[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl})methyl N-[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamate (9.600 mg; 1.00 eq.) in N,N-dimethylformamide (0.096 ml; 10.00 V) were added 4-methylmorpholine (1.0 µl; 3.00 eq.) and N-succinimidyl-3-maleimidopropionate (0.900 mg; 1.10 eq.) at 0 °C. The reaction mixture was stirred for 5 min, then the ice bath was removed, and it was stirred at room temperature overnight.

The reaction mixture was purified by preparative HPLC.

The fractions containing product were combined, the solvent was removed and the residue was lyophilized overnight to afford {4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}({[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl})methyl N-[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamatemaleimidopropionate as a light yellow solid.
(3.100 mg; 98%); (M+H)/2

### Synthesis of MC901 335-02 (Compound 5)

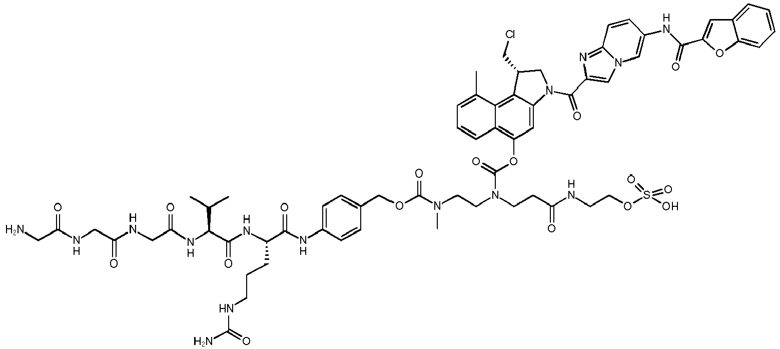

The linker payload construct was synthesized like Compound 3, but with use of a sulfone instead of the oligosaccharide.

### Synthesis of MC901 333-10 (Compound 6)

### Synthesis of MC901_333-5:

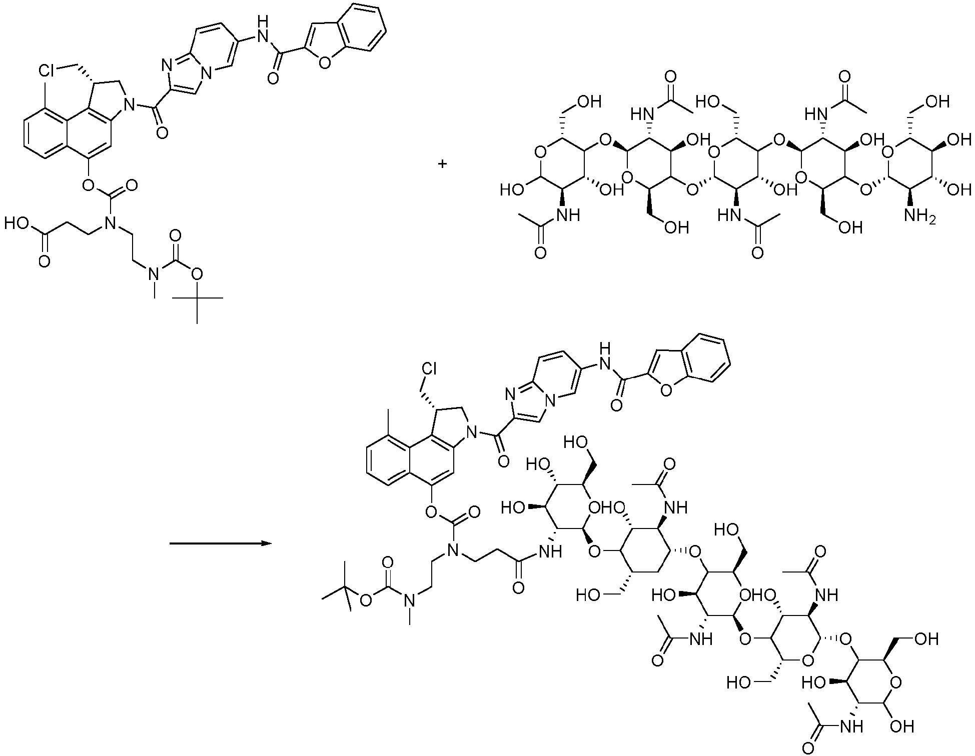

To a stirred solution of 3-[({[(1S)-3-[6-(1-benzofuran-2-amido)imidazo[1,2-a]pyridine-2-carbonyl]-1-(chloromethyl)-9-methyl-1H,2H,3H-benzo[e]indol-5-yl]oxy}carbonyl)(2-{[(tert-butoxy)carbonyl](methyl)amino}ethyl)amino]propanoic acid (150.00 mg; 0.15 mmol; 1.00 eq.) in DMF (20.00 ml; 133.33 V), were added benzotriazol-1-ol (25.21 mg; 0.15 mmol; 1.00 eq.), followed by (3-dimethylamino-propyl)-ethyl-carbodiimide hydrochloride (44.98 mg; 0.23 mmol; 1.50 eq.) at 0 °C under nitrogen atmosphere. The reaction mixture was stirred at room temperature for 30 min. To this N-[(2S,3R,4R,6R)-5-{[(2S,3R,4R,5S,6R)-3-amino-4,5-dihydroxy-6-(hydroxymethyl)oxan-2-yl]oxy}-2-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-6-(hydroxymethyl)oxan-3-yl]acetamide (153.62 mg; 0.15 mmol; 1.00 eq.) was added and the reaction mixture was stirred at room temperature for 2 days. The reaction mixture was concentrated at 35 °C.

The residue was washed with methanol and dried to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[(2R,3R,4R,5S,6R)-2-[(2R,3R,4R,6R)-3-acetamido-4-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-2-hydroxy-6-(hydroxymethyl)cyclohexoxy]-4,5-dihydroxy-6-(hydroxymethyl)tetrahydropyran-3-yl]amino]-3-oxo-propyl]-N-[2-[tert-butoxycarbonyl(methyl)amino]ethyl]carbamate.
(120 mg, 30.5%), M+H = 1796

The crude product was used for next step without purification.

### Synthesis of MC901_333-6:

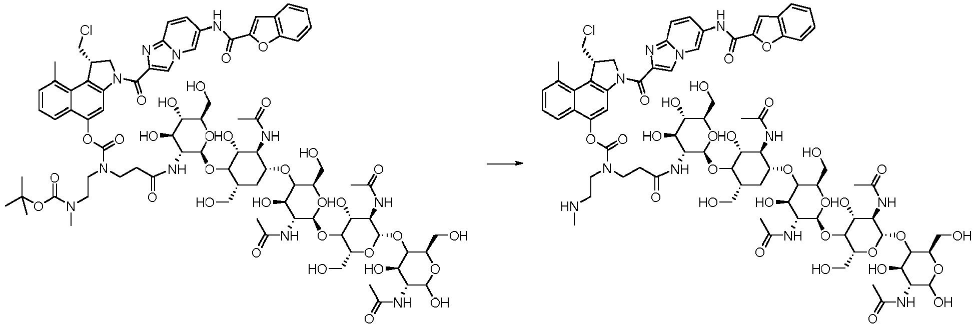

To a stirred solution of [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[(2R,3R,4R,5S,6R)-2-[(2R,3R,4R,6R)-3-acetamido-4-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-2-hydroxy-6-(hydroxymethyl)cyclohexoxy]-4,5-dihydroxy-6-(hydroxymethyl)tetrahydropyran-3-yl]amino]-3-oxo-propyl]-N-[2-[tert-butoxycarbonyl(methyl)amino]ethyl]carbamate in acetonitrile (40.00 ml; 333 V) and water (60.00 ml; 500.00 V), was added TFA (trifluoroacetic acid; 0.10 ml; 1.25 mmol; 24.28 eq.). The reaction mixture was stirred at 50 °C for 18 h.

The reaction mixture was cooled to room temperature and the solvents were removed by lyophilization to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[(2R,3R,4R,5S,6R)-2-[(2R,3R,4R,6R)-3-acetamido-4-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-2-hydroxy-6-(hydroxymethyl)cyclohexoxy]-4,5-dihydroxy-6-(hydroxymethyl)tetrahydropyran-3-yl]amino]-3-oxo-propyl]-N-[2-(methylamino)ethyl]carbamate.
(100 mg, 68.5%), M+H = 1696

The crude product was used for next step without purification.

### Synthesis of MC901_333-8:

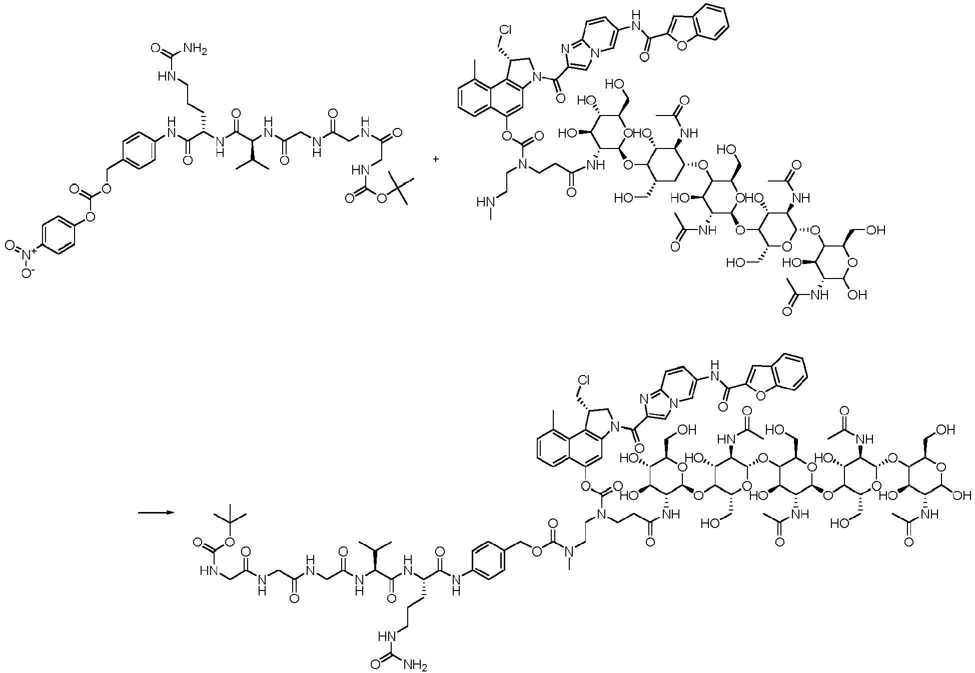

To a stirred solution of {4-[(2S)-2-[(2S)-2-{2-[2-(2-{[(tert-butoxy)carbonyl]amino}acetamido)acetamido]acetamido}-3-methylbutanamido]-5-(carbamoylamino)pentanamido]phenyl}methyl 4-nitrophenyl carbonate (80.00 mg; 0.06 mmol; 1.00 eq.) in THF (0.10 ml; 1.25 V) were added triethyl amine (0.03 ml; 0.23 mmol; 4.00 eq.) at 0 °C under nitrogen atmosphere followed by the addition of [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[(2R,3R,4R,5S,6R)-2-[(2R,3R,4R,6R)-3-acetamido-4-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-2-hydroxy-6-(hydroxymethyl)cyclohexoxy]-4,5-dihydroxy-6-(hydroxymethyl)tetrahydropyran-3-yl]amino]-3-oxo-propyl]-N-[2-(methylamino)ethyl]carbamate. The reaction mixture was stirred at room temperature for 6 h. The reaction mixture was concentrated under reduced pressure. The residue was washed with methanol to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[(2S,3R,4R,5 S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]-N-[2-[[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]methoxycarbonyl-methyl-amino]ethyl]carbamate.
(72 mg, 49%) M/2 = 1186

The crude product was used for the next step without purification.

### Synthesis of MC901_333-10 (Compound 6):

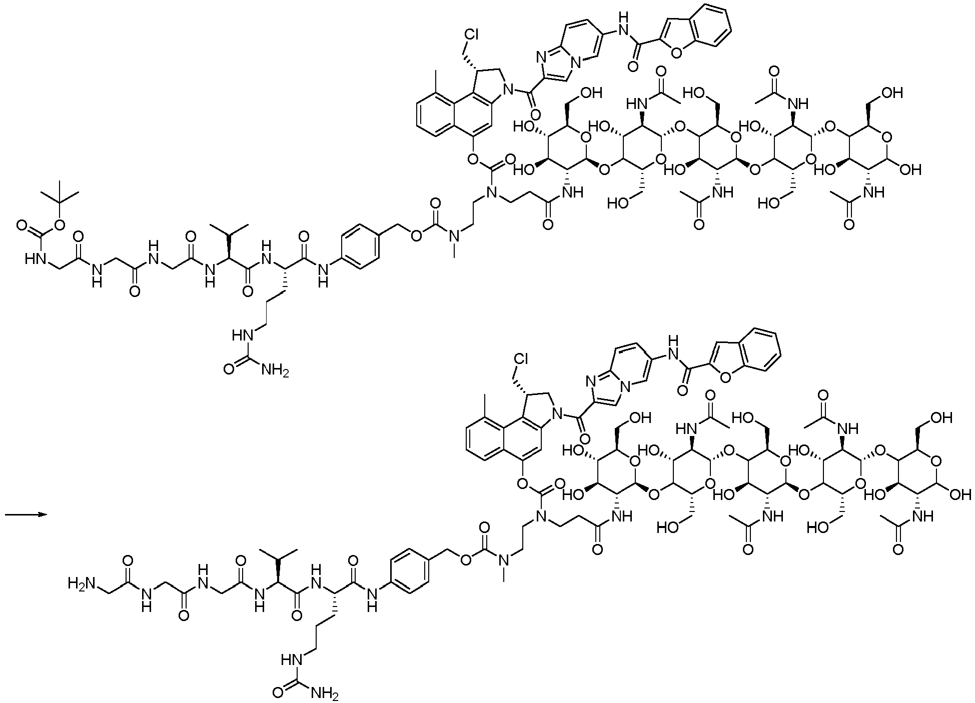

To a stirred solution of [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]-N-[2-[[4-[[(2S)-2-[[(2S)-2-[[2-[[2-[[2-(tert-butoxycarbonylamino)acetyl]amino]acetyl]amino]acetyl]amino]-3-methyl-butanoyl]amino]-5-ureido-pentanoyl]amino]phenyl]methoxycarbonyl-methyl-amino]ethyl]carbamate in acetonitrile (40.00 ml) and water (60.00 ml), was added TFA (0.20 ml; 2.50 mmol; 132.92 eq.). The reaction mixture was stirred at 50 °C for 18 h.

The reaction mixture was cooled to room temperature and solvents were removed by lyophilization. The crude was purified by preparative HPLC and solvents were removed by lyophilization to afford [(1S)-3-[6-(benzofuran-2-carbonylamino)imidazo[1,2-a]pyridine-2-carbonyl]-1-(chloromethyl)-9-methyl-1,2-dihydrobenzo[e]indol-5-yl] N-[3-[[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]amino]-3-oxo-propyl]-N-[2-[[4-[[(2S)-2-[[(2S)-2-[[2-[[2-[(2-aminoacetyl)amino]acetyl]amino]acetyl]amino]-3-methyl-butanoyl]amino]-5-ureido-pentanoyl]amino]phenyl]methoxycarbonyl-methyl-amino]ethyl]carbamate.
(11 mg, 25.7%); M/2 = 1136

### Synthesis of MC901 337-6 (Compound 7)

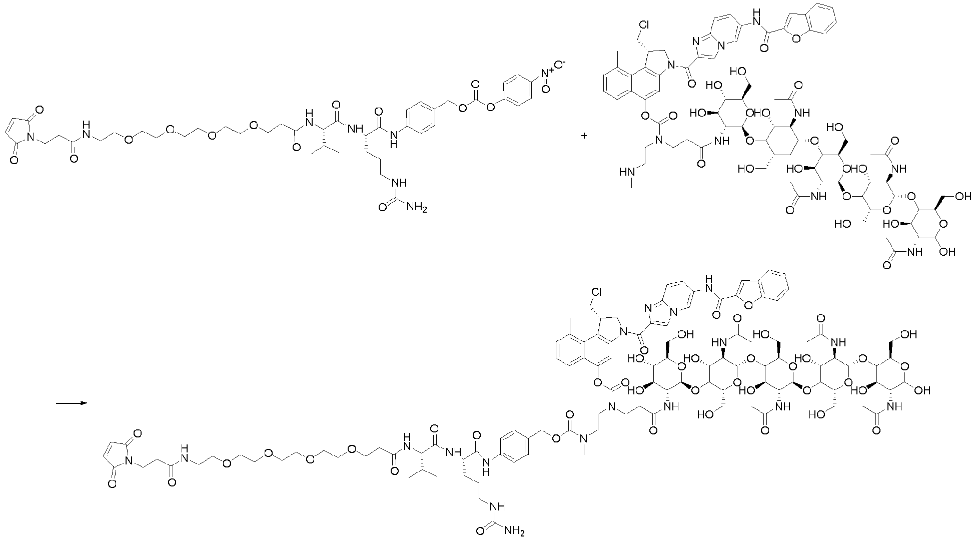

MC901_337-6 was synthesized using the intermediate CRD013/410 yielding 5 mg of the desired product as off-white solid.

### Synthesis of CRD018/206 (Compound 8)

Compound 8 is a ready-to-conjugate form of the chito-oligosaccharide CO-V.

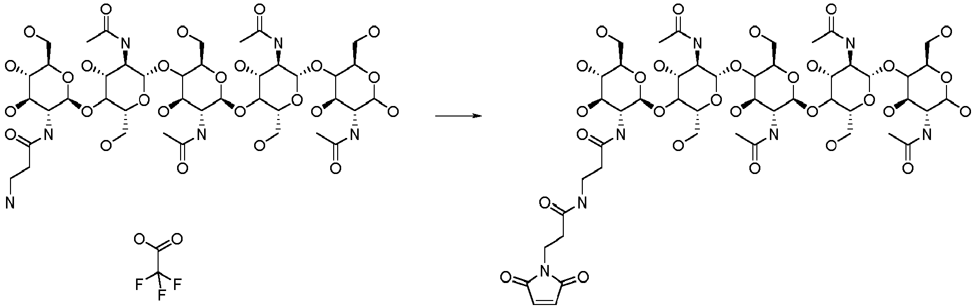

To a stirred solution of N-[(2S,3R,4R,5S,6R)-2-[(2R,4R,5R,6S)-5-acetamido-6-[(2R,4R,5R,6S)-5-acetamido-6-[(2R,4R,5R,6S)-5-acetamido-6-[(2R,4R,5R)-5-acetamido-4,6-dihydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4-hydroxy-2-(hydroxymethyl)tetrahydropyran-3-yl]oxy-4,5-dihydroxy-6-(hydroxymethyl)tetrahydropyran-3-yl]-3-amino-propanamide trifluoroacetate (31,300 mg; 0.027 mmol; 1.0 eq.) in N,N-dimethylformamide (0.313 ml; 10.0 V) were added 4-methylmorpholine (9 µl; 0.080 mmol; 3.0 eq.) and N-succinimidyl 3 maleimidopropionate (7.787 mg; 0.029 mmol; 1.1 eq.) at 0 °C. The reaction mixture was stirred for 5 min, then the ice bath was removed. The reaction mixture was stirred at room temperature overnight.

The reaction mixture was purified by preparative HPLC, and the fractions containing the desired product were combined and lyophilized overnight to afford 3-(2,5-dioxo-2,5-dihydro-1H-pyrrol-1-yl)-N-(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)propenamide as a white powder
(3.2 mg; 10%), M = 1214

### Synthesis of Compound 9

### Synthesis of MC901_346-10:

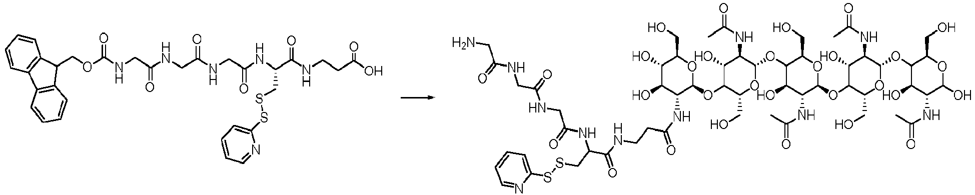

To a solution of 3-[(2R)-2-(2-{2-[2-({[(9H-fluoren-9-yl)methoxy]carbonyl}amino)acetamido]acetamido}acetamido)-3-(pyridin-2-yldisulfanyl)propanamido]propanoic acid (50.00 mg; 0.06 mmol; 1.00 eq.) and N-[(2S,3R,4R,6R)-5-{[(2S,3R,4R,5S,6R)-3-amino-4,5-dihydroxy-6-(hydroxymethyl)oxan-2-yl]oxy}-2-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-6-(hydroxymethyl)oxan-3-yl]acetamide (60.49 mg; 0.06 mmol; 1.00 eq.) and HATU (33.37 mg; 0.09 mmol; 1.50 eq.) in dry DMSO (1.00 ml; 20.00 V) was added ethyl-diisopropyl-amine (0.02 ml; 0.09 mmol; 1.50 eq.).The reaction mixture was stirred at room temperature for 2 days. The reaction was monitored by LCMS. The reaction mixture was diluted with diethyl ether and the precipitate was collected by filtration and dried to get 2-{2-[2-(2-aminoacetamido)acetamido]acetamido}-N-(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3- yl]carbamoyl}ethyl)-3-(pyridin-2-yldisulfanyl)propenamide as an off-white solid.
(40.00 mg; 33.1%); (M+2)/2 = 724

### Synthesis of MC901_346-11 (Compound 9):

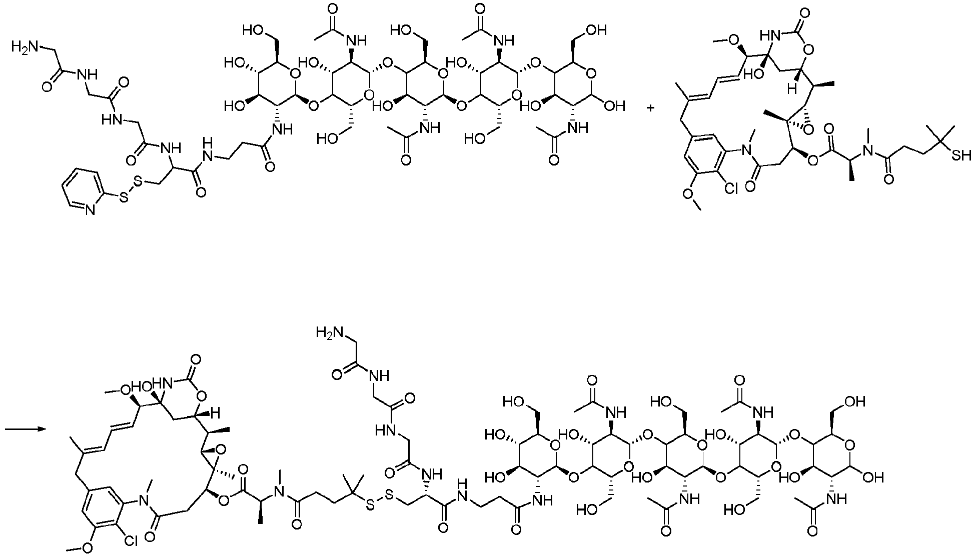

To a solution of DM4 (ravtansine) (14.96 mg; 0.02 mmol; 1.00 eq.) in N,N-Dimethylacetamide (0.50 ml; 12.50 V) was added saturated sodium bicarbonate solution (0.04 ml; 1.00 V), water (0.04 ml; 1.00 V) followed by 2)-{2-[2-(2-aminoacetamido)acetamido]acetamido}-N-(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3- yl]carbamoyl}ethyl)-3-(pyridin-2-yldisulfanyl)propanamide (40.00 mg; 0.02 mmol; 1.00 eq.) in N,N-Dimethylacetamide (0.50 ml; 12.50 V). The reaction mixture turned to a yellow solution. The reaction mixture was stirred at room temperature for 24 h. UPLC showed product formation. The crude reaction mixture was purified by preparative HPLC purification, to get (1S,2R,3S,5S,6S,16E,18E,20R,21S)-11-chloro-21-hydroxy-12,20-dimethoxy-2,5,9,16-tetramethyl-8,23-dioxo-4,24-dioxa-9,22-diazatetracyclo[19.3.1.1¹⁰,¹⁴.0³,⁵]hexacosa-10(26),11,13,16,18-pentaen-6-yl (2S)-2-(4-{[(2R)-2-[3-(2-{2-[3-(2,5-dioxo-2,5-dihydro-1H-pyrrol-1-yl)propanamido]ethoxy}ethoxy)propanamido]-2-[(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)carbamoyl]ethyl]disulfanyl}-N,4-dimethylpentanamido)propanoate; (1S,2R,3S,5S,6S,16E,18E,20R,21S)-11-chloro-21-hydroxy-12,20-dimethoxy-2,5,9,16-tetramethyl-8,23-dioxo-4,24-dioxa-9,22-diazatetracyclo[19.3.1.1¹⁰,¹⁴.0³,⁵]hexacosa-10(26),11,13,16,18-pentaen-6-yl (2S)-2-(4-{[(2R)-2-{2-[2-(2-aminoacetamido)acetamido]acetamido}-2-[(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3- yl]carbamoyl}ethyl)carbamoyl]ethyl]disulfanyl}-N,4-dimethylpentanamido)propanoate; formic acid as an off-white solid.
(6.00 mg; 14.2%); (M+H) = 2116

### Synthesis of MC901 347-9 (Compound 10)

### Synthesis of MC901_347-8:

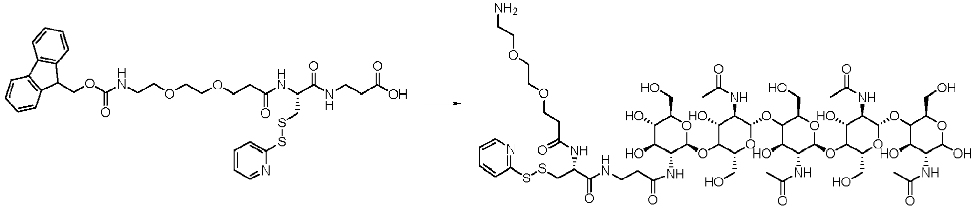

To a solution of 3-[(2R)-2-(3-{2-[2-({[(9H-fluoren-9-yl)methoxy]carbonyl}amino)ethoxy]ethoxy}propanamido)-3-(pyridin-2-yldisulfanyl)propanamido]propanoic acid (70.00 mg; 0.09 mmol; 1.00 eq.) and N-(2S,3R,4R,6R)-5-{[(2S,3R,4R,5S,6R)-3-amino-4,5-dihydroxy-6-(hydroxymethyl)oxan-2-yl]oxy}-2-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-6-(hydroxymethyl)oxan-3-yl]acetamide (95.62 mg; 0.09 mmol; 1.00 eq.) and HATU (52.76 mg; 0.14 mmol; 1.50 eq.) in dry DMSO (1.40 ml; 20.00 V) was added ethyl-diisopropyl-amine (0.02 ml; 0.14 mmol; 1.50 eq.).The reaction mixture was stirred at room temperature for 2 days. The reaction was monitored by UPLC (Ultra High-Performance Liquid Chromatography). The reaction mixture was diluted with diethyl ether and the precipitate was collected by filtration and dried to get (2R)-2-{3-[2-(2-aminoethoxy)ethoxy]propanamido}-N-(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)-3-(pyridin-2-yldisulfanyl)propanamide. (100.00 mg; 0.04 mmol; 39.5%; off-white solid; crude product)

### Synthesis of MC901_347-9 (Compound 10):

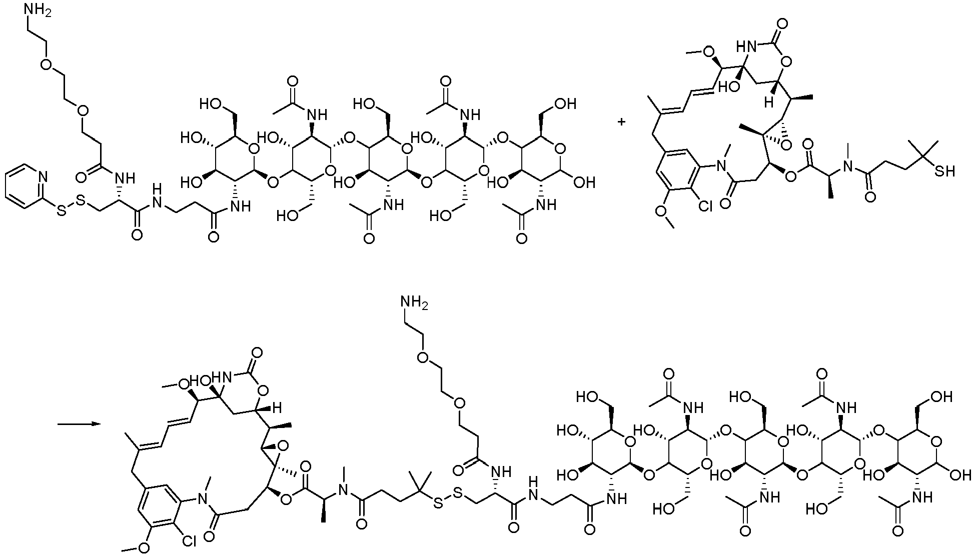

To a solution of DM4 (ravtansine, a maytansinoid; 47.10 mg; 0.06 mmol; 1.00 eq.) in N,N-Dimethylacetamide (0.50 ml; 5.00 V) was added saturated sodium bicarbonate solution (0.04 ml; 0.40 V) followed by (2R)-2-{3-[2-(2-aminoethoxy)ethoxy]propanamido}-N-(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)-3-(pyridin-2-yldisulfanyl)propanamide (100.00 mg; 0.06 mmol; 1.00 eq.) in N,N-Dimethylacetamide (0.50 ml; 5.00 V). The reaction mixture was stirred at room temperature for 24 h. UPLC showed product formation.

The reaction mixture was purified by preparative HPLC, to get (14S,16S,32S,33S,2R,4S,10E,12E,14R)-86-chloro-14-hydroxy-85,14-dimethoxy-33,2,7,10-tetramethyl-12,6-dioxo-7-aza-1(6,4)-oxazinana-3(2,3)-oxirana-8(1,3)-benzenacyclotetradecaphane-10,12-dien-4-yl (11R,20S)-11-((3-(((2S,3R,4R,5S,6R)-2-(((2R,4R,5R,6S)-5-acetamido-6-(((2R,4R,5R,6S)-5-acetamido-6-(((2R,4R,5R,6S)-5-acetamido-6-(((2R,4R,5R)-5-acetamido-4,6-dihydroxy-2-(hydroxymethyl)tetrahydro-2H-pyran-3-yl)oxy)-4-hydroxy-2-(hydroxymethyl)tetrahydro-2H-pyran-3-yl)oxy)-4-hydroxy-2-(hydroxymethyl)tetrahyd as light blue solid.
(9.40 mg; 6.8 %); (M+H) = 2104

### Synthesis ofMC901 347-10 (Compound 11)

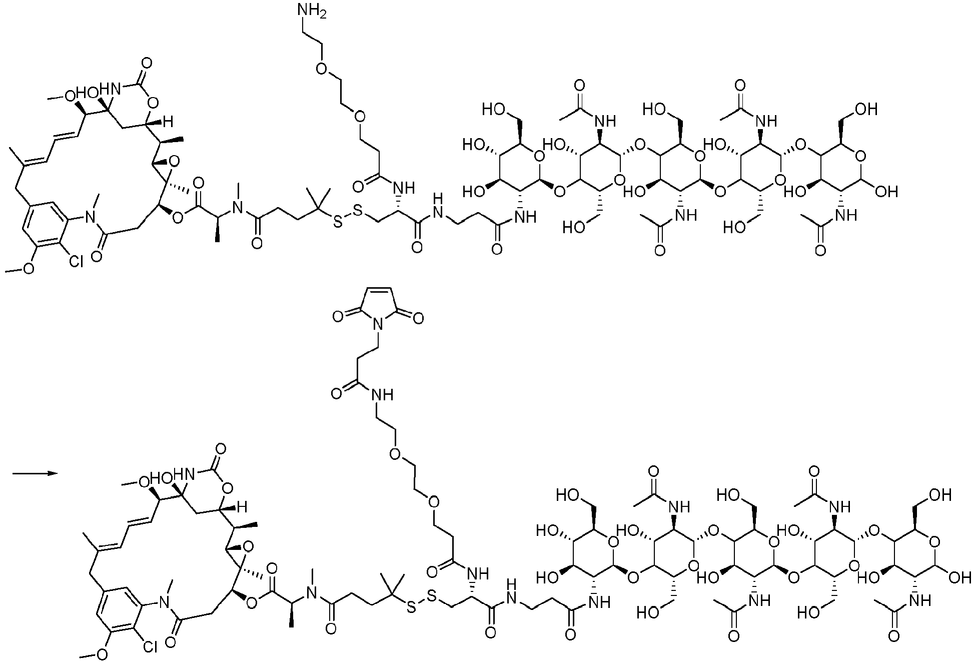

To a solution of (14S,16S,32S,33S,2R,4S,10E,12E,14R)-86-chloro-14-hydroxy-85,14-dimethoxy-33,2,7,10-tetramethyl-12,6-dioxo-7-aza-1(6,4)-oxazinana-3(2,3)-oxirana-8(1,3)-benzenacyclotetradecaphane-10,12-dien-4-yl (11R,20S)-11-((3-(((2S,3R,4R,5S,6R)-2-(((2R,4R,5R,6S)-5-acetamido-6-(((2R,4R,5R,6S)-5-acetamido-6-(((2R,4R,5R,6S)-5-acetamido-6-(((2R,4R,5R)-5-acetamido-4,6-dihydroxy-2-(hydroxymethyl)tetrahydro-2H-pyran-3-yl)oxy)-4-hydroxy-2-(hydroxymethyl)tetrahydro-2H-pyran-3-yl)oxy)-4-hydroxy-2-(hydroxymethyl)tetrahyd (8.00 mg; 0.004 mmol; 1.00 eq.) and 2,5-dioxopyrrolidin-1-yl3-(2,5-dioxo-2,5-dihydro-1H-pyrrol-1-yl)propanoate (1.90 mg; 0.01 mmol; 2.00 eq.) in dry DMF (0.08 ml; 10.00 V),was added 4-methylmorpholine; 1 µl; 0.01 mmol; 3.00 eq.) The reaction mixture was stirred at room temperature for 16 h. The reaction was monitored by LCMS. The reaction mixture was purified by preparative purification, to get (1S,2R,3S,5S,6S,16E,18E,20R,21S)-11-chloro-21-hydroxy-12,20-dimethoxy-2,5,9,16-tetramethyl-8,23-dioxo-4,24-dioxa-9,22-diazatetracyclo[19.3.1.1¹⁰,¹⁴.0³,⁵]hexacosa-10(26),11,13,16,18-pentaen-6-yl (2S)-2-(4-{[(2R)-2-[3-(2-{2-[3-(2,5-dioxo-2,5-dihydro-1H-pyrrol-1-yl)propanamido]ethoxy}ethoxy)propanamido]-2-[(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)carbamoyl]ethyl]disulfanyl}-N,4-dimethylpentanamido)propanoate as an off-white solid.
(1.50 mg; 15.3%); (M+H)/2 = 1127

### Synthesis of MC901 453 (Compound 12)

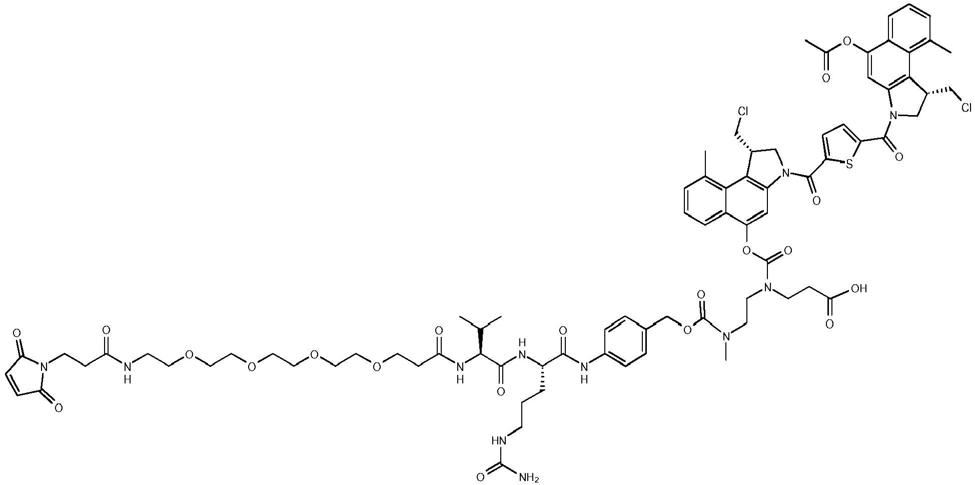

Compound 12 was synthesized by a similar procedure as Compound 13, but without inclusion of the oligosaccharide.
30 mg, yellow solid, M-H = 1648

### Synthesis of MC901 433-93 (Compound 13)

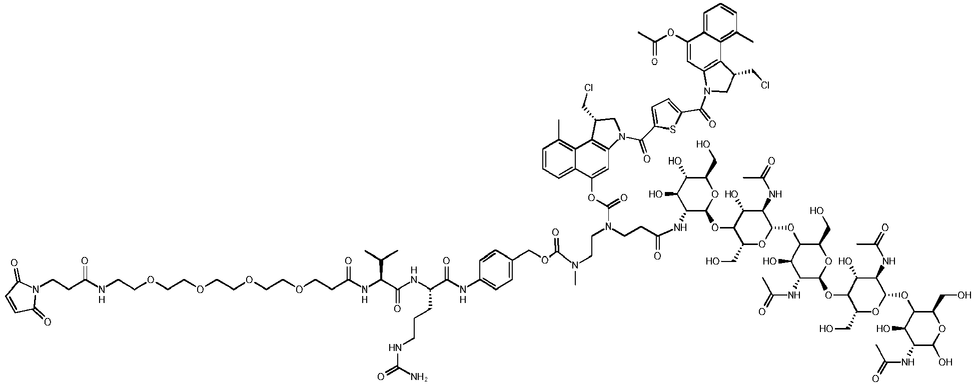

Compound 13 was synthesized based on the procedure described in Tietze et al., Angew. Chem. Int. Ed. (2010), vol. 49, p. 7336 -7339 with adaptions from the intermediate MC901_333-6. 5 mg of the desired product were obtained with a yield of 3.8%.
M/2 = 1311

### Synthesis of MC901 458 (Compound 14)

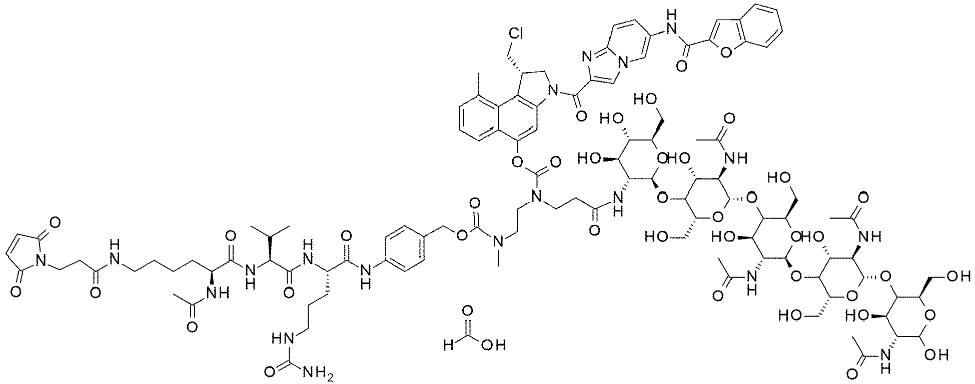

MC901_458 was synthesized similar to MC901_333-10 (Compound 6) with an acetyl-lysine as spacer and maleimide attachment.
(M+2)/2 = 1212/M+Na = 2445

### Synthesis of MC901 362-42 (Compound 15)

The starting material was synthesized according to CRD012/619 (Compound 2).

### Synthesis of MC901_362-16:

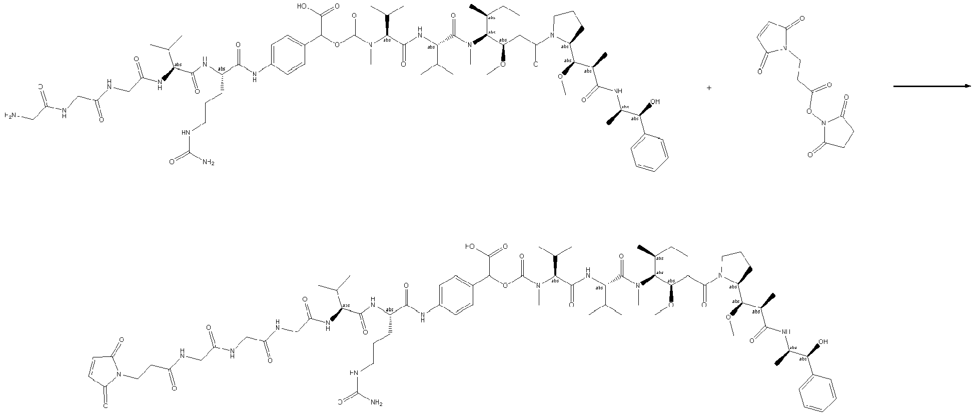

A solution of 2-{4-[(2S)-2-[(2S)-2-{2-[2-(2-aminoacetamido)acetamido]acetamido}-3-methylbutanamido]-5-(carbamoylamino)pentanamido]phenyl}-2-({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)acetic acid (183.00 mg; 1.00 eq.) and 3-(2,5-Dioxo-2,5-dihydro-pyrrol-1-yl)-propionic acid 2,5-dioxo-pyrrolidin-1-yl ester (37.18 mg; 0.14 mmol; 1.10 eq.) in N,N-dimethylformamide; (7.00 ml; 38.25 V) was cooled with ice. To this, 4-methyl-morpholine (0.1 M in DMF) (0.04 ml; 3.00 eq.) was added and the reaction mixture was stirred at room temperature for 5 h. The solvent was removed under reduced pressure at 35 °C. The crude was purified by reversed-phase chromatography. The combined fractions were lyophilized to get 2-{4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}-2-({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)acetic acid as a white solid.
(190.00 mg; 92.6%); (M+H) = 1490

### Synthesis of MC901_362-39:

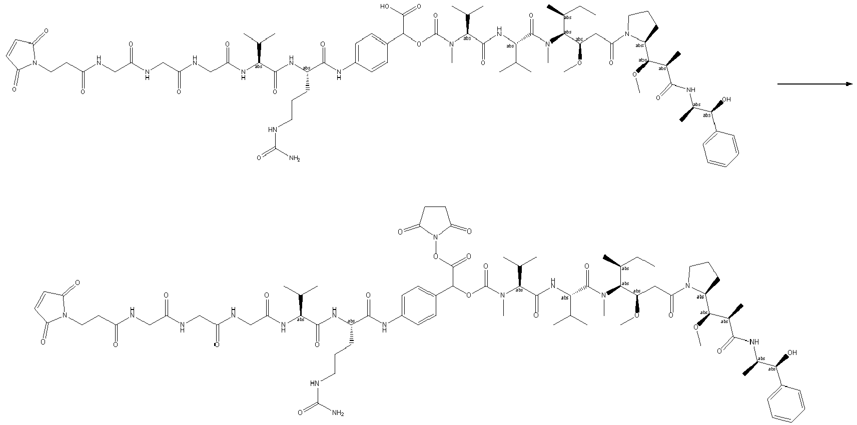

2-{4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}-2-({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)acetic acid (102.00 mg; 1.00 eq.) and 1-hydroxy-pyrrolidine-2,5-dione (1 M in DMF) (684.70 µl; 1.00 eq.) and dicyclohexyl-carbodiimide (0.1 M in DMF) (684.70 µl; 1.00 eq.) were added. The reaction mixture was stirred at room temperature for 16 h. The reaction mixture containing the product 2,5-dioxopyrrolidin-1-yl2-{4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}-2-({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)acetate (108.00 mg; 71.7 %; colorless solution) was filtered, rinsed with 0.5 mL of DMF and used in the next reaction without purification.
M = 1586

### Synthesis of MC901_362-42 (Compound 15):

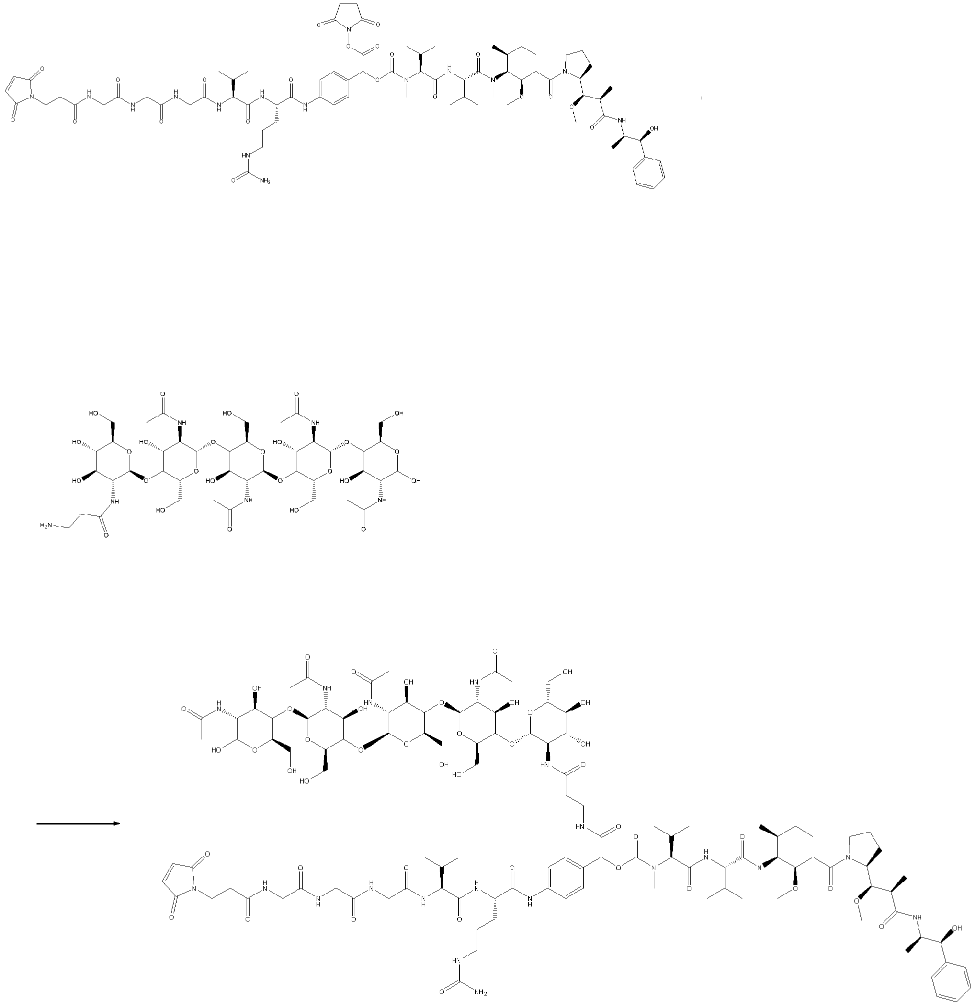

To a solution of 2,5-dioxopyrrolidin-1-yl2-{4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}-2-({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)acetate (98.00 mg; 1.00 eq.) in DMF (2.36 ml) was added 3-amino-N-[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]propanamide (236.35 mg; 3.60 eq.) in water (1.50 ml). 4-Methyl-morpholine (0.1 M in DMF) (0.31 ml; 5.00 eq.) was added immediately and the reaction mixture was stirred at room temperature for 10 min.

The reaction mixture was quenched with an aqueous solution of 20 % citric acid (0.5 mL).

The crude reaction mixture was purified by preparative HPLC. The fractions containing the product were lyophilized to get {4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}[(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)carbamoyl]methyl N-[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamate as a white solid.
(17.50 mg; 11.0 %); (M+2)/2 = 1268

### Synthesis of CRDO10/072 (Compound 16):

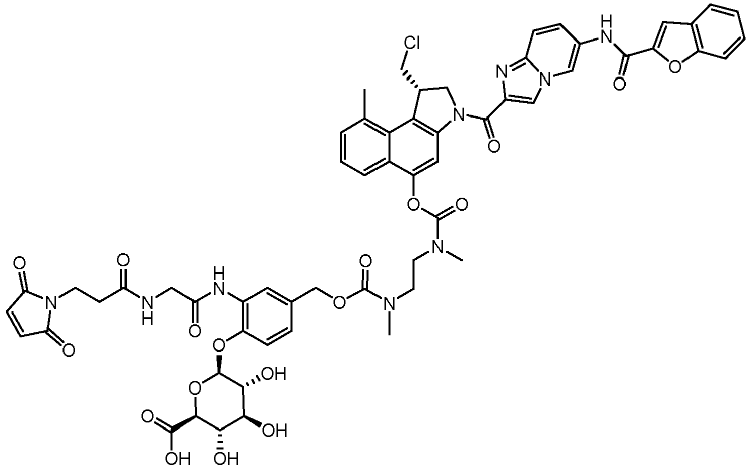

Compound 16 was obtained by standard procedures of organic chemical synthesis including synthesis via a MC901_333-6 derivative without oligosaccharide tag as intermediate followed by addition of the beta-glucuronide linker. The reaction mixture was purified by HPLC delivering the final product (10.3 mg) as white solid.
(M+H) = 1214.5

### Synthesis of MC901 482 (Compound 17)

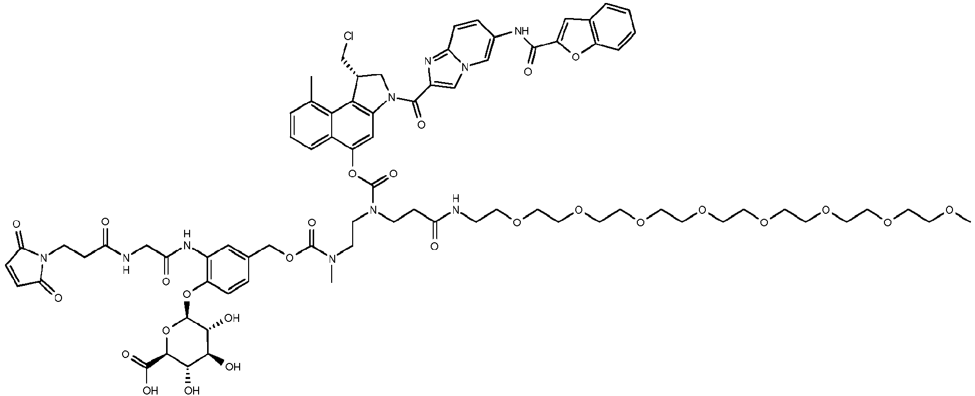

MC901_482 (Compound 17) was synthesized similar to MC901_488-7 (Compound 19, see below) using 2,5,8,11,14,17,20,23-octaoxapentacosan-25-amine as coupling partner within the sequence.
35 mg, white solid, [M+H] = 1639

### Synthesis of CRD012/789 (Compound 18):

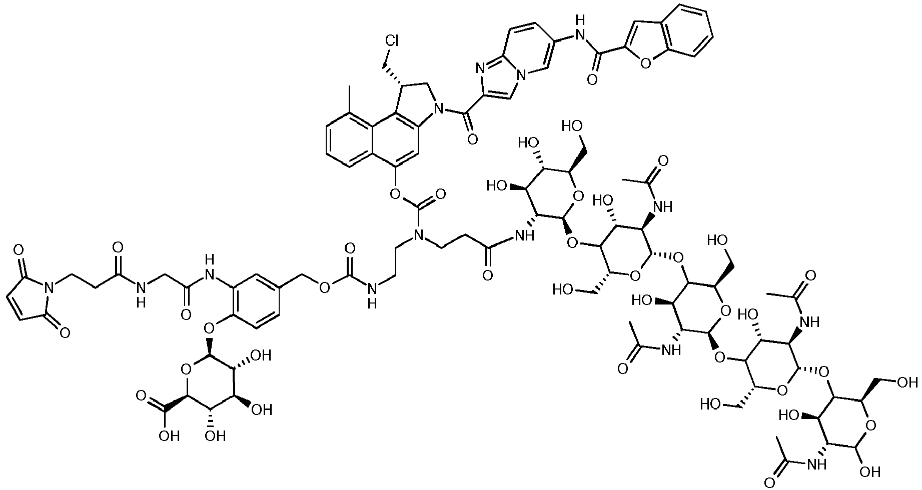

Compound 18 was obtained by standard procedures of organic chemical synthesis including synthesis via MC901_333-6 as intermediate followed by addition of the beta-glucuronide linker. The reaction mixture was purified by HPLC delivering the final product (5.6 mg) as white solid.
(M+H)/2 = 1116.5

### Synthesis of MC901 488-7 (Compound 19)

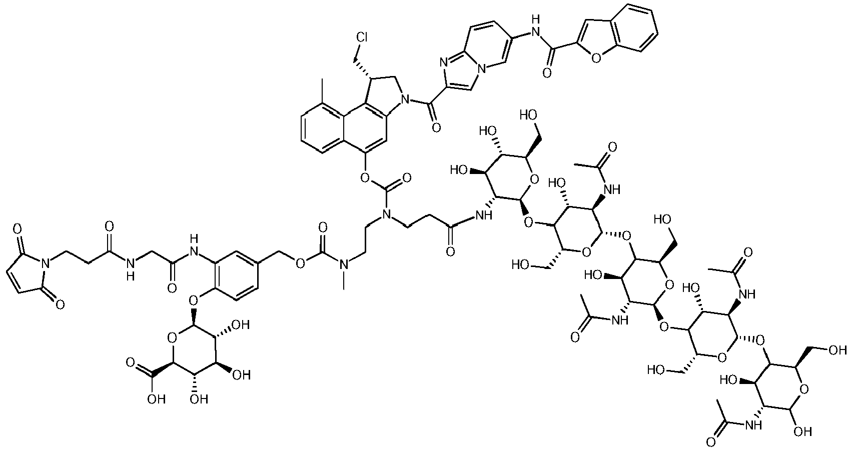

Compound 19 was synthesized via MC901_333-6 as intermediate by standard procedures of chemical synthesis. 14 mg of the desired product were obtained with a yield of 45%.
(M+H)/2 = 1123

### Synthesis of MC901 455 45 (Compound 20)

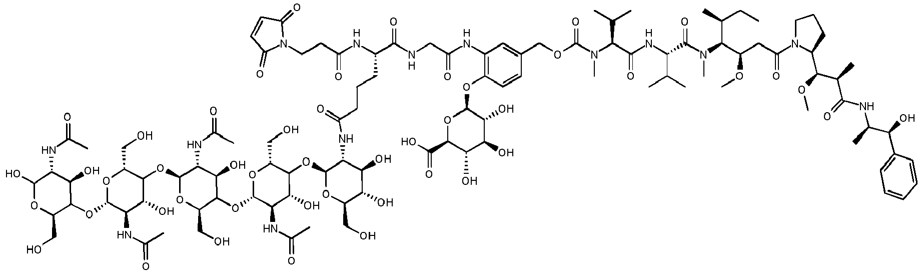

Compound 20 was synthesized by a similar synthesis route as CRD012/604, using standard procedures of chemical synthesis.

The product was purified by preparative HPLC. The product containing fractions were combined and lyophilized overnight to afford (2S,3S,4S,5R,6S)-6-(2-{2-[(2S)-2-[3-(2,5-dioxo-2,5-dihydro-1H-pyrrol-1-yl)propanamido]-5-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}pentanamido]acetamido}-4-[({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)methyl]phenoxy)-3,4,5-trihydroxyoxane-2-carboxylic acid; {4-[(2S)-2-[(2S)-2-[1-(4-{2-azatricyclo[10.4.0.0⁴,⁹]hexadeca-1(12),4(9),5,7,13,15-hexaen-10-yn-2-yl}-4-oxobutanamido)-3,6,9,12-tetraoxapentadecan-15-amido]-3-methylbutanamido]-5-(carbamoylamino)pentanamido]phenyl}[(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3- yl]carbamoyl}ethyl)carbamoyl]methyl N-[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamate as a white solid.
(4 mg, 33%); (M+2H)/2 = 1193

### Synthesis of MC901 614 (Compound 21)

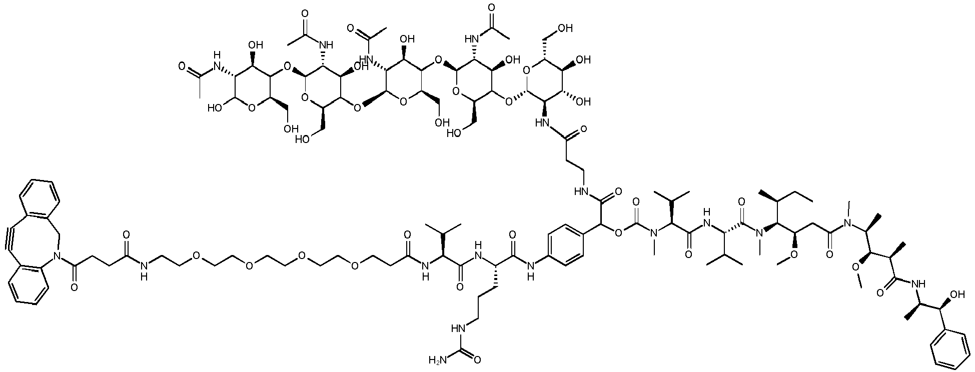

The molecule was synthesized by a similar synthesis route as MC901_362 (MC901_362_16, MC901_262_39 and MC901_362-42) using standard procedures of chemical synthesis.

The product was purified by preparative HPLC. The product containing fractions were combined and lyophilized overnight to afford {4-[(2S)-2-[(2S)-2-[1-(4-{2-azatricyclo[10.4.0.0⁴,⁹]hexadeca-1(12),4(9),5,7,13,15-hexaen-10-yn-2-yl}-4-oxobutanamido)-3,6,9,12-tetraoxapentadecan-15-amido]-3-methylbutanamido]-5-(carbamoylamino)pentanamido]phenyl}[(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)carbamoyl]methyl N-[(1S)-1-{[(1 S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamate as white solid.
(17.20 mg, 6.9%); (M+H)/2 = 1374

### Synthesis of CRD012/604 (Compound 22)

### Synthesis of CRD018/177:

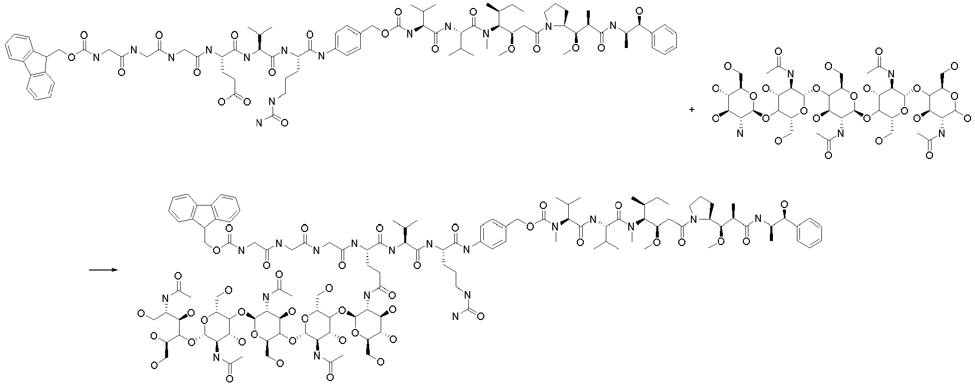

(4S)-4-{[(1S)-1-{[(1S)-4-(carbamoylamino)-1-({4-[({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)methyl]phenyl}carbamoyl)butyl]carbamoyl}-2-methylpropyl]carbamoyl}-4-(2-{2-[2-({[(9H-fluoren-9-yl)methoxy]carbonyl}amino)acetamido]acetamido}acetamido)butanoic acid; trifluoroacetic acid; {4-[(2S)-5-(carbamoylamino)-2-[(2S)-2-[2-(2-{2-[3-(2,5-dioxo-2,5-dihydro-1H-pyrrol-1-yl)propanamido]acetamido}acetamido)acetamido]-3-methylbutanamido]pentanamido]phenyl}[(2-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}ethyl)carbamoyl]methyl N-[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamate (30.000 mg; 0.012 mmol; 1.0 eq.) was dissolved in N,N-dimethylformamide (2.000 ml) and it was cooled down to 0 °C. N-Ethyldiisopropylamine (0.006 ml; 0.036 mmol; 3.0 eq.) was added then HATU, [Dimethylamino-([1,2,3]triazolo[4,5-b]pyridin-3-yloxy)-methylene]-dimethyl-ammonium; hexafluoro phosphate (9.074 mg; 0.024 mmol; 2.0 eq.) and finally N-[(3R,4R,6R)-5-[(2S,3R,4R,6R)-3-acetamido-5-[(2S,3R,4R,6R)-3-acetamido-5-[(2S,3R,4R,6R)-3-acetamido-5-[(2S,3R,4R,5S,6R)-3-amino-4,5-dihydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-4-hydroxy-6-(hydroxymethyl)tetrahydropyran-2-yl]oxy-2,4-dihydroxy-6-(hydroxymethyl)tetrahydropyran-3-yl]acetamide (23.672 mg; 0.024 mmol; 2.0 eq.) were added. The ice bath was removed, and the reaction mixture was stirred at room temperature overnight. The reaction solution was concentrated *in vacuo.* The residue was purified by preparative HPLC. The fractions containing the product were lyophilized to get (9H-fluoren-9-yl)methyl N-[({[({[(1S)-1-{[(1S)-1-{[(1S)-4-(carbamoylamino)-1-({4-[({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)methyl]phenyl}carbamoyl)butyl]carbamoyl}-2-methylpropyl]carbamoyl}-3-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}propyl]carbamoyl}methyl)carbamoyl]methyl}carbamoyl)methyl]carbamate as off-white solid.
(4 mg); (M+H)/2 = 1310

### Synthesis of CRD012/604 (Compound 22):

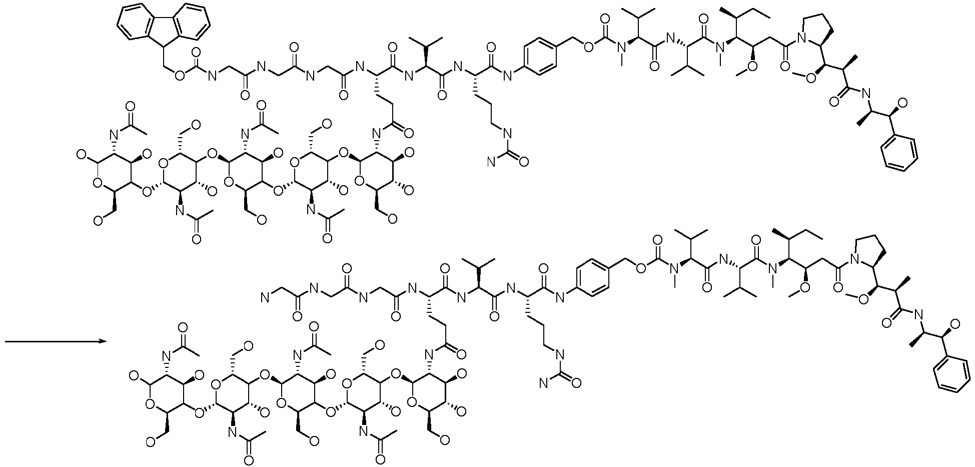

Polymer-bound piperazine (122 mg; 80.00 eq.) was left for swelling for 30 min in N,N-dimethylformamide (1.600 ml; 400.00 V), (9H-fluoren-9-yl)methyl N-[({[({[(1S)-1-{[(1S)-1-{[(1S)-4-(carbamoylamino)-1-({4-[({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)methyl]phenyl}carbamoyl)butyl]carbamoyl}-2-methylpropyl]carbamoyl}-3-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}propyl]carbamoyl}methyl)carbamoyl]methyl}carbamoyl)methyl]carbamate (4.000 mg; 1.00 eq.) was added and the reaction mixture was shaken over 6 days.
The reaction mixture was filtered, the filtrate was concentrated *in vacuo* and the residue was purified by preparative HPLC.

The product containing fractions were combined and lyophilized overnight to afford (9H-fluoren-9-yl)methyl N-[({[({[(1S)-1-{[(1S)-1-{[(1S)-4-(carbamoylamino)-1-({4-[({[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl](methyl)carbamoyl}oxy)methyl]phenyl}carbamoyl)butyl]carbamoyl}-2-methylpropyl]carbamoyl}-3-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3 - yl]carbamoyl}propyl]carbamoyl}methyl)carbamoyl]methyl}carbamoyl)methyl]carbamate; {4-[(2S)-2-[(2S)-2-[(2S)-2-{2-[2-(2-aminoacetamido)acetamido]acetamido}-4-{[(2S,3R,4R,5S,6R)-2-{[(2R,4R,5R,6S)-5-acetamido-6-{[(2R,4R,5R,6S)-5-acetamido-6-{[(2R,4R,5R,6S)-5-acetamido-6-{[(2R,4R,5R)-5-acetamido-4,6-dihydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4-hydroxy-2-(hydroxymethyl)oxan-3-yl]oxy}-4,5-dihydroxy-6-(hydroxymethyl)oxan-3-yl]carbamoyl}butanamido]-3-methylbutanamido]-5-(carbamoylamino)pentanamido]phenyl}methyl N-[(1S)-1-{[(1S)-1-{[(3R,4S,5S)-1-[(2S)-2-[(1R,2R)-2-{[(1S,2R)-1-hydroxy-1-phenylpropan-2-yl]carbamoyl}-1-methoxy-2-methylethyl]pyrrolidin-1-yl]-3-methoxy-5-methyl-1-oxoheptan-4-yl](methyl)carbamoyl}-2-methylpropyl]carbamoyl}-2-methylpropyl]-N-methylcarbamate as a white solid.
(2.1 mg, 33%); (M+H)/2 = 1199

### Example 2: Preparation of Antibody Component

The monoclonal anti-HER2 antibody trastuzumab (for amino acid sequence see e.g. DrugBank at https://www.drugbank.ca/drugs/DB00072) was obtained from Roche Pharma AG resp. Canoma Pharma GmbH.

A trastuzumab-derived antibody with a transglutaminase recognition tag in the antibody light chain was produced by genetic engineering and recombinant expression as described in Dickgiesser et al., Site-Specific Antibody-Drug Conjugation Using Microbial Transglutaminase, in: Methods in Molecular Biology: Enzyme-Mediated Ligation Methods (2019), editors Nuijens and Schmidt, vol. 2012, p. 135-149. This antibody ("Trastuzumab-T") has the following amino acid sequence (differences to trastuzumab are marked):

Sequence of light chain of Trastuzumab-T (SEQ ID NO: 1):
DIQMTQSPSS LSASVGDRVT ITCRASQDVN TAVAWYQQKP GKAPKLLIYS
ASFLYSGVPS RFSGSRSGTD FTLTISSLQP EDFATYYCQQ HYTTPPTFGQ
GTKVEIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGECGGLLQG
(MW = 24,162.93 Da)

Sequence of heavy chain of Trastuzumab-T (SEQ ID NO: 2):
EVQLVESGGG LVQPGGSLRL SCAASGFNIK DTYIHWVRQA PGKGLEWVAR
IYPTNGYTRY ADSVKGRFTI SADTSKNTAY LQMNSLRAED TAVYYCSRWG
GDGFYAMDYW GQGTLVTVSS ASTKGPSVFP LAPSSKSTSG GTAALGCLVK
DYFPEPVTVS WNSGALTSGV HTFPAVLQSS GLYSLSSVVT VPSSSLGTQT
YICNVNHKPS NTKVDKKVEP PKSCDKTHTC PPCPAPELLG GPSVFLFPPK
PKDTLMISRT PEVTCVVVDV SHEDPEVKFN WYVDGVEVHN AKTKPREEAY
NSTYRVVSVL TVLHQDWLNG KEYKCKVSNK ALPAPIEKTI SKAKGQPREP
QVYTLPPSRD ELTKNQVSLT CLVKGFYPSD IAVEWESNGQ PENNYKTTPP
VLDSDGSFFL YSKLTVDKSR WQQGNVFSCS VMHEALHNHY TQKSLSLSPG
K
(MW = 49,292.65 Da)

The antibody was subsequently purified by standard procedures of antibody purification.

Anti-TumorAntigenA is a monoclonal full-length IgG antibody directed against antigen A, a tumor antigen on the cell surface of breast cancer cells (the extracellular domain of a transmembrane protein). The antibody was expressed in CHO cells and subsequently purified by standard procedures of antibody purification. The antibody was designed to be suitable for transglutaminase conjugation at position Q295 (EU numbering) of the antibody heavy chain.

Anti-TumorAntigenB&C is a bispecific antibody in the SEED format (an approach for generation of bispecific antibodies in which structurally related sequences within the conserved CH3 domains of human IgA and IgG are exchanged to form two asymmetric but complementary domains, see WO 2016/087650). The antibody is directed against two tumor antigens, wherein both antigen B and antigen C are tumor antigens expressed on the cell surface of different tumor cells (such as lung cancer cells, head and neck cancer cells and colorectal cancer cells). Since Anti-TumorAntigenB&C binds to both antigen B and antigen C, this results in enhanced selectivity for tumor cells expressing both antigens (antigen b and antigen c) over tumor cells expressing only one of the antigens (either antigen b or antigen c) due to a strong avidity effect mediated by concurrent binding to both antigens B and C on the surface of the same cell. Anti-TumorAntigenB&C was prepared as described in Muda et al., Protein Engineering, Design & Selection (2011), vol. 24(5), p. 447-454 and Davis et al., Protein Engineering, Design & Selection (2010), vol. 23(4), p. 195-202, including purification by standard procedures of antibody purification.

The identity and purity of each antibody was confirmed by SDS-PAGE, MS analytics, as well as verification of specific binding to positive cell lines (i.e. cell lines expressing the respective antigen on their cell surface) by FACS binding and Octet binding experiments.

### Example 3: Conjugation

The different antibody components were covalently linked to linker-payload constructs as shown in Table 3 below using standard procedures.

Linkage by transglutaminase was carried out with wild type microbial transglutaminase (WT Transglutaminase purchased from Zedira, Germany) to the light chain of trastuzumab-T by standard methods, following a procedure that is also described in Dickgiesser et al., Bioconjugate Chem. (2020), vol. 31(4), p. 1070-1076. To this end, 1 eq antibody in a buffer with 150 mM NaCl, 25 mM Tris (pH 8.0) was mixed with a solution of the respective payload (10x or 20x surplus to the concentration of the antibody, depending on the number of linkage sites) and 6 U/ml of transglutaminase. The mixture was incubated for 16 h in a thermomixer at 37 °C and 450 rpm.

For linkage by maleimide chemistry, a 5 mg/mL solution of the respective antibody component in PBS (pH 7.4) was prepared (∼33 µM antibody). The antibody was reduced with TCEP ((tris(2-carboxyethyl)phosphine); ratio of antibody component to TCEP 1:2 to 1:6, depending on the desired DAR; TCEP was used as 2 mM stock, pH 7.0) or in some cases with DTT (dithiothreitol; 20 mM). After incubation for 0.5-2 hours (depending on the number of cysteines to be activated) in a 37 °C water bath, the reaction was allowed to cool down to room temperature.

The solution was desalted to conjugation buffer (10 mM sodium phosphate, pH 6.0, 2 mM EDTA, N₂ degassed) on a Sephadex G25 column and adjusted to an antibody concentration of 0.2 mg/mL in conjugation buffer.

Conjugation was initiated by adding the reduced antibody to a solution of the appropriate maleimide-activated linker-payload construct at a suitable ratio (e.g. 1:4 to 1:8 ratio antibody to linker-payload construct for DAR 4; e.g. 1:20 ratio antibody to linker-payload construct for DAR 8). The reaction was incubated for 1 h at 22 °C with slow rocking, then the conjugation was checked by LCMS. If necessary, the reaction was continued until the desired DAR was reached.

The conjugated sample was purified by hydrophobic interaction chromatography (HIC) on a 15 PHE (Phenyl) column (GE Healthcare) or HiTrap HP or FF Butyl Sepharose column (GE Healthcare). Elution fractions were concentrated, and buffer exchanged to PBS pH 7.4 or 10 mM potassium phosphate, 200 mM NaCl, 10 mM histidine, 50 mM trehalose, pH 7.0.

The identity and purity of each prepared ADC was confirmed by LC-MS and SDS-PAGE.

The DAR (drug-antibody ratio) of an ADC is the (average) number of payloads per ADC molecule divided by the number of antibody components per ADC molecule. The DAR for each prepared ADC was calculated and confirmed from HIC (hydrophobic interaction chromatography) data and mass spectrometry data. Results are summarized in Table 3 below.

The stability of each prepared ADC was tested by a freeze-thawing experiment. Specifically, the ADC in histidine buffer was shock-frozen in liquid nitrogen to -80 °C and stored at this temperature for several weeks up to a few months. After putting the sample to room temperature until the sample had been completely thawed, the sample was subjected to SE-HPLC (size exclusion-high performance liquid chromatography) analysis to test for compound degradation, and the activity of the thawed ADC was examined. Specifically, target antigen binding was examined by Octet binding assay and payload-mediated cytotoxicity was tested by cell titer glow assay on positive and negative cell lines. The results for the freeze-thawed ADC was compared to the corresponding ADC without freeze-thawing. In each case, the ADC with solubility tag was found to be stable in this freeze-thawing procedure.

Endotoxin was determined by the PTS (Portable Test System) cartridge method (Nexgen) according to the manufacturer's instructions under standard conditions. For each prepared ADC, it was found that the endotoxin level was < 5.0 endotoxin units (EU)/mg.

**Table 3: Overview of ADCs prepared**

| **ADC** | **Antibody Component** | **Linker- Payload Construct** | **Payload** | **Oligosaccharide** | **DAR** | **Coupling mechanism** |
|---|---|---|---|---|---|---|
| ADC1 | Trastuzumab- T | Compound 2 | Auristatin¹ | CO-V² | 1.71 | TGAse LC³ |
| ADC2⁴ | Trastuzumab- T | Compound 3 | Duocarmycin | CO-V | 1.56 | TGAse LC |
| ADC3 | Trastuzumab- T | Compound 5 | Duocarmycin | --- | 0.4 | TGAse LC |
| ADC4⁵ | Anti-TumorAntigenB&C | Compound 1 | Auristatin | --- | 2.3⁶ | Inter-chain⁷ |
| ADC5 | Anti-TumorAntigenB&C | Compound 4 | Auristatin | CO-V | 5.8 | Inter-chain |
| ADC6 | Trastuzumab | Compound 4 | Auristatin | CO-V | 3.8 | Inter-chain |
| ADC7 | Trastuzumab | Compound 7 | Duocarmycin | CO-V | 1.5 | Inter-chain |
| ADC8 | Trastuzumab | Compound 8 | --- | CO-V | 2.9 | Inter-chain |
| ADC9 | Trastuzumab- T | Compound 9 | Maytansinoid⁸ | CO-V | 1.3 | TGAse LC |
| ADC10 | Trastuzumab | Compound 11 | Maytansinoid | CO-V | 3.1 | Inter-chain |
| ADC11 | Trastuzumab | Compound 12 | CBI⁹ | --- | N.A. | ADC could not be prepared due to aggregation |
| ADC12 | Trastuzumab | Compound 15 | Auristatin | CO-V | 7.76 | Inter-chain |
| ADC13 | Trastuzumab | Compound 15 | Auristatin | CO-V | 8 | Inter-chain |
| ADC14 | Trastuzumab | Compound 15 | Auristatin | CO-V | 4.5 | Inter-chain |
| ADC15 | Trastuzumab | Compound 1 | Auristatin | --- | 4 | Inter-chain |
| ADC16 | Anti- TumorAntigenA | Compound 14 | Duocarmycin | CO-V | 1.9 | Q295 via thiol spacer¹⁰ |
| ADC17 | Trastuzumab | Compound 15 | Auristatin | CO-V | 4.6 | Inter-chain |
| ADC18 | Trastuzumab | Compound 20 | Auristatin | CO-V | 3.2 | Inter-chain |
| ADC19 | Trastuzumab | Compound 1 | Auristatin | --- | 2.5 | Inter-chain |
| ADC20 | Anti- TumorAntigenA | Compound 16 | Duocarmycin | --- | 2.3 | Q295 via thiol spacer |
| ADC21 | Anti- TumorAntigenA | Compound 19 | Duocarmycin | CO-V | 2.5 | Q295 via thiol spacer |
| ADC22 | Anti- TumorAntigenA | Compound 17 | Duocarmycin | PEG | 2.1 | Q295 via thiol spacer |
| ADC23 | Anti- TumorAntigenA | Compound 18 | Duocarmycin | CO-V | 2.0 | Q295 via thiol spacer |
| ADC24 | Anti- TumorAntigenA | Compound 13 | CBI | CO-V | 2.2 | Q295 via thiol spacer |

| | | | | | | |
|---|---|---|---|---|---|---|
| ¹ The auristatin in the listed compounds was MMAE (monomethyl auristatin E) ² For details about CO-V, see Example 1, section "Oligosaccharide Synthesis". ³ Transglutaminase-catalyzed linkage to TGAse sequence in light chain of antibody component ⁴ The antibody component and linker-payload construct of ADC4 is identical to ADC5, with the distinction that the linker-payload construct of ADC4 lacks an oligosaccharide tag. ⁵ The antibody component and linker-payload construct of ADC2 is identical to ADC3, with the distinction that the linker-payload construct of ADC3 lacks an oligosaccharide tag. ⁶ no higher DAR possible ⁷ Conjugation to cysteines in the antibody hinge region. Inter-chain cysteine bonds were broken and therefore available for conjugation. ⁸ The maytansinoid in the listed compounds was DM4 (= ravtansine). ⁹ Cyclopropanebenz[e]indoline dimer ¹⁰ 2-Aminoethanethiol was conjugated via transglutaminase-catalyzed coupling to position Q295 (EU numbering) of the antibody component, subsequently a maleimide-activated linker-payload-solubility tag construct was added by chemical reaction to obtain an ADC with site-specific linkage to the antibody component. | | | | | | |

It is known that ADCs including highly hydrophobic payloads like duocarmycin often cannot be formed, because conjugation fails due to solubility issues (C. O'Donnell, Pfizer, presentation at World ADC San Diego 2016). In line with this understanding, it was not possible to form ADC3 based on a linker-payload construct including the combination of a cathepsin B-cleavable linker and duocarmycin as payload, but lacking an oligosaccharide tag (Compound 5). Various attempts to conjugate this construct to an antibody component by transglutaminase coupling failed since the solubility of the linker-payload construct under coupling conditions was insufficient. However, the corresponding ADC2, which includes a corresponding linker-payload construct with an oligosaccharide tag (Compound 3), was formed successfully. Also in all other cases where formation of duocarmycin-based ADCs with an oligosaccharide tag according to the present disclosure was attempted, conjugation was successful (ADC7, ADC16 and ADC21).

Similarly, ADCs with CBI dimer as payload are notoriously challenging. In fact, it has been reported that such ADCs frequently cannot be formed at all because the conjugation process fails due to solubility issues (presentation by O'Donnell of Pfizer at the World ADC San Diego 2016). In line with this report, also in the experiments underlying the present examples ADCs based on CBI dimers could not be obtained in the absence of an oligosaccharide tag. For example, conjugation of a Compound 13-like linker-payload construct *without* an oligosaccharide tag was not possible. All attempts to obtain a clean conjugate from such a construct failed either due to limited solubility of the linker-payload construct in conjugation media or due to the formation of aggregates in the conjugation process. However, the corresponding CBI dimer-based ADC *with* oligosaccharide solubility tag was successfully obtained (ADC24 based on the linker-payload construct Compound 13 with oligosaccharide tag).

Solubility often also limits the DAR of ADCs with hydrophobic payloads like duocarmycin. In line with this understanding, attempts to obtain ADC3 (a duocarmycin-based ADC) with a DAR > 1 failed at the step of transglutaminase conjugation. Surprisingly, however, for the corresponding ADC including an oligosaccharide solubility tag (ADC2), a DAR of 1.56 could be achieved.

Similarly, for an ADC based on a bispecific SEED antibody with auristatin as payload, in the absence of an oligosaccharide tag (ADC4) a DAR of only ∼2 could be obtained (since the components aggregated and conjugation failed), while for the corresponding construct with oligosaccharide tag (ADC5) a DAR of ∼6 could be achieved.

Thus, an oligosaccharide tag as described in the present disclosure allows access to ADCs that otherwise cannot be prepared.

The prepared ADCs include a broad spectrum of antibody components. For example, ADC6, ADC7 and ADC13 include trastuzumab as antibody component, i.e. a humanized IgG1 monoclonal antibody that targets the Her2 receptor. ADC2 and ADC9 are based on trastuzumab-T, a form of trastuzumab with a mutated sequence optimized for transglutaminase conjugation. ADC23 is based on an antibody directed against a different cell surface antigen. This antibody is a humanized IgG1 with modifications in the Fc part to modulate pharmacokinetic properties. ADC5 is a bispecific antibody against two different tumor antigens, wherein this antibody was prepared in the SEED format that utilizes sequence stretches from both IgA and IgG (see Muda et al., Protein Engineering, Design & Selection (2011), vol. 24(5), p. 447-454 and Davis et al., Protein Engineering, Design & Selection (2010), vol. 23(4), p. 195-202). Thus, a variety of different antibodies was used in the examples, confirming that the solubility tag of the present disclosure can be used with ADCs based on different antibody types and formats.

Similarly, the ADCs prepared in this example include payloads of very different classes. ADC9 and ADC10, for example, include a payload of the widely used payload class of maytansinoids (DM4). ADC5, ADC6, ADC12, ADC14 and ADC18 include the rather hydrophilic payload monomethyl auristatin E. ADC2, ADC7, ADC16 and ADC21, on the other hand, include the strongly hydrophobic payload duocarmycin, and ADC24 includes CBI (cyclopropanebenz[e]indoline-dimer). From the ADCs in this example, it can be seen that the solubility tags according to the present disclosure are compatible with any of these payload classes and can enhance the solubility of an ADC irrespective of the payload class.

The ADCs prepared also reflect a broad range of linker types. For example, ADC10 includes a disulfide linker for thiol-mediated cleavage, ADC16 a protease-cleavable linker (cathepsin B recognition site), and ADC18 and ADC21 a beta-glucuronide linker (cleavable by the lysosomal enzyme β-glucuronidase). ADC2 with a cathepsin B cleavage site includes a short spacer, whereas ADC7 includes a much longer spacer.

The ADCs in the table above were prepared by very different conjugation methods and principles. For example, ADC5, ADC6 and ADC7 were conjugated by cysteine coupling with a maleimide (a common conjugation approach for ADCs). In contrast, ADC1, ADC9 and ADC2 were conjugated by enzymatic coupling with transglutaminase. Thus, the oligosaccharide tag of the present disclosure is broadly compatible with different conjugation methods, ranging from chemical coupling to enzymatic coupling methods.

Also the attachment site of the oligosaccharide tags varies widely among the different ADCs prepared. For example, in ADC18 the solubility tag was attached to the linker right next to the beta-glucuronide cleavage motif, while in ADC13, a cathepsin B cleavable linker, the tag is attached to a PABC (p-aminobenzyloxycarbonyl) group, which is often found in ADC linkers to allow for increased flexibility. Similarly, ADC1 includes an oligosaccharide tag as part of PABC; the specific linker of ADC1 can be used for transglutaminase coupling delivering a DAR of 1.7 without impacting the coupling enzyme efficacy. In ADC7, ADC24 and ADC16 (cleavage by cathepsin B) the oligosaccharide tag was attached to the self-immolative part. In contrast, the oligosaccharide group of ADC10 was attached in close range to the disulfide cleavage bridge without hampering either efficacy or conjugatability. In ADC12 and ADC14, a spacer was included between the oligosaccharide tag and the PABC, whereas ADC6 did not include such a spacer. It is noteworthy that the spacer, allowing a higher flexibility, has no negative impact. In all constructs mentioned above, both the solubility tag and linker cleavage were functional, indicating that the solubility tag of the present disclosure is broadly compatible with all kinds of cleavage mechanisms and attachment sites. For example, with respect to duocarmycins, which must be tracelessly released to be active, the oligosaccharide tag did not hamper the activation, as can be seen by the ADC activity on positive and negative cell lines (see below).

Moreover, while for many linker-payload constructs in the ADCs of this example the oligosaccharide tag was attached at an intermediate step of the synthesis procedure, the linker-payload construct of ADC1 was prepared by a divergent approach in which the oligosaccharide unit was attached in the second to last step, showing that variation is possible also in the preparation procedure. Within ADC12, ADC13 and ADC14 even further modifications (cleavage of protecting groups followed by Amide couplings) were possible after attachment of the solubility tag and well tolerated by the tag.

In this example, ADCs with quite different DAR were prepared. This includes ADCs with a low DAR of about 1-2 (e.g. ADC2, ADC9, ADC16), ADCs with a DAR around 3-4 (e.g. ADC6, ADC10, ADC14, ADC15, ADC18) up to ADCs with a much higher DAR of close to 8 (e.g. ADC12). In no situation was aggregation observed for ADCs including an oligosaccharide tag according to the present disclosure.

To further study the effect of an oligosaccharide tag compared to either the absence of a tag or the presence of a different tag (PEG tag), the ADCs ADC20 (no tag), ADC22 (PEG tag) and ADC21 (oligosaccharide tag CO-V) were prepared. Although in all three cases conjugated product could be obtained, clear differences were observed during the conjugation reaction. ADC20 (no tag) was the most challenging material as it showed significant aggregates. While ADC22 (PEG tag) showed, compared the untagged ADC, a somewhat reduced level of aggregates, the lowest level of aggregates was observed with ADC21 (oligosaccharide-tag). The aggregate formation during the manufacturing process was also reflected in the conjugation yield of 42% for ADC20 (no tag) vs. 53% for ADC22 (PEG tag) and 53% for ADC21 (oligosaccharide-tag). (In this context, it is noteworthy that the conjugation yield of ADC23, which is identical to ADC21 except for a methyl group at the self-immolative part of the linker, was 75%, thus providing further confirmation for the favorable effect of the oligosaccharide-tag.) HIC retention times indicated a higher solubility for the oligosaccharide-tagged ADC compared to the other systems (see below, Example 4).

In additional conjugation reactions, ADCs based on the linker-payload constructs Compound 6, Compound 10 and Compound 21 are prepared. Compound 6 and Compound 10 are conjugated by enzymatic coupling with transglutaminase. Compound 21 has an alkyne motif as attachment site and is coupled by an (copper free) click chemistry approach as described in Peplow, Nature Biotechnology (2019), vol. 37, p. 829-841. The characterization of the obtained ADCs by the methods described in the present examples shows similar effects as for the other ADCs according to the present disclosure. Thus, this provides further evidence that variation is possible with regard to the design and preparation of the ADC according to the present disclosure while still achieving the desired effects.

In further experiments, the chito-oligosaccharide CO-V(MeFuc), CO-IV, CO-IV(S) and CO-II are used to build ADCs with alternative oligosaccharide-based solubility tags. Characterization of the obtained ADCs by the methods described in the present examples shows that ADCs tagged with CO-V(MeFuc), CO-IV and CO-IV(S) have similar effects as the ADCs described above. In contrast, the effects observed for CO-II lag behind.

### Example 4: HIC Retention Time

In this example, the retention time of different ADCs in hydrophobic interaction chromatography (HIC) was determined.

The retention time was measured was determined against an internal standard, anti-HEL (hen egg white lysozyme) antibodies modified to generate antibodies of increasing hydrophobicity. The unmodified anti-HEL antibody has a retention time of 10.72 min under these conditions, whereas the anti-HEL antibody with the highest degree of modification shows a peak at 19.66 min. These retention times were reproducible in all experiments. Thus, these internal standards were always used as reference to ensure consistency of the measurements.

A shift in the retention time to earlier elution shows an increased hydrophilicity. This, in turn, indicates an increased solubility in aqueous environment and thus a positive impact on the behavior of the ADC.

The HIC retention times obtained from these experiments are summarized in Table 4 below.

**Table 4: HIC retention time of different ADCs**

| **ADC** | **Payload** | **Oligosaccharide** | **DAR** | **HIC Retention time [min]** |
|---|---|---|---|---|
| Trastuzumab | --- | --- | --- | 11.5 |
| ADC1 | Auristatin | CO-V | 1.71 | 13.9 |
| ADC2 | Duocarmycin | CO-V | 1.56 | 12.39 |
| ADC3 | Duocarmycin | --- | 0.4 | 14.80¹ |
| ADC6 | Auristatin | CO-V | 3.8 | 13.25 |
| ADC7 | Duocarmycin | CO-V | 1.5 | 13.23 |
| ADC8 | --- | CO-V | 2.9 | 10.47 |
| ADC10 | Maytansinoid | CO-V | 3.1 | 12.40 |
| ADC16 | Duocarmycin | CO-V | 1.9 | 10.9 |
| ADC19 | Auristatin | --- | 2.5 | 14.30 |
| ADC20 | Duocarmycin | --- | 2.3 | 13.90 |
| ADC21 | Duocarmycin | CO-V | 2.5 | 12.90 |
| ADC22 | Duocarmycin | PEG | 2.1 | 13.4 |
| ADC23 | Duocarmycin | CO-V | 2.0 | 13.2 |
| ADC24 | CBI | CO-V | 2.2 | 14.65 |

| | | | | |
|---|---|---|---|---|
| ¹ The auristatin in the listed compounds was MMAE (monomethyl auristatin E). ² HIC retention time refers to species with DAR 1. | | | | |

As can be seen from Table 4, all tagged ADCs have moderate retention time shifts if compared to the naked antibody trastuzumab. However, some effects are striking and surprising.

ADC8 is an interchain construct of trastuzumab that does not include a payload, but is modified by covalent attachment of a CO-V oligosaccharide tag to cysteine residues of the antibody that are involved in linking the two antibody heavy chains. Compared to the retention time of the naked trastuzumab without oligosaccharide tag, the retention time of ADC8 is shifted from 11.5 min to 10.47 min, indicating that the oligosaccharide-tagged antibody is more hydrophilic than the native antibody.

Improved solubility in the presence of an oligosaccharide tag is also seen in ADCs with payloads:
ADC2 is a duocarmycin-based ADC with trastuzumab-T as antibody component and carrying an oligosaccharide solubility tag, whereas ADC3 is a corresponding duocarmycin-based trastuzumab-T-ADC without oligosaccharide tag. The minor ADC3 species with DAR 1 had a retention time of 14.80 min, whereas ADC2 with oligosaccharide tag, despite containing a larger number of highly hydrophobic duocarmycin payloads per ADC, still showed a lower retention time of only 12.39 min and thus had a better solubility.

ADC6 and ADC1 are auristatin-based ADCs with trastuzumab/trastuzumab-T as antibody component and carrying an oligosaccharide solubility tag, whereas ADC19 is a corresponding auristatin-based trastuzumab-ADC without oligosaccharide tag. The DAR values of ADC6 and ADC1 are slightly higher resp. lower than the DAR of ADC19 (3.8 and 1.71 vs. 2.5). However, both auristatin ADCs with oligosaccharide tag have significantly lower HIC retention values than the ADC without oligosaccharide tag (13.25 min and 13.9 min vs. 14.30 min).

Similarly, PB-8704 and ADC23 are duocarmycin-based ADCs against Anti-TumorAntigenA with oligosaccharide solubility tag, whereas ADC20 is a corresponding duocarmycin-based ADC without such a tag. The DAR values of ADC21 and ADC23 spread around the DAR of ADC20 (2.5 and 2.0 vs. 2.3). Nevertheless, both ADCs with oligosaccharide tag have lower HIC retention times than ADC20 (12.90 min and 13.2 min vs. 13.9 min).

A comparison of ADC3 (without oligosaccharide tag) against the oligosaccharide-tagged variant ADC7 reveals on the one hand that the conjugation of the untagged variant was not successful at higher DARs due to solubility issues, while for the oligosaccharide-tagged ADC a DAR of 1.5 could be achieved. On the other hand, the minor ADC3 species with DAR 1 had a retention time of 14.80 min, whereas ADC7 with oligosaccharide tag, despite containing a larger number of highly hydrophobic duocarmycin payloads per ADC, still showed a lower retention time of only 13.23 min and thus had a better solubility.

Similar observations were also made for the CO-V-tagged auristatin-based ADC6 against the untagged auristatin-based ADC19 (DAR 3.8 vs. 2.5; retention time 13.25 min vs. 14.30 min), providing further evidence that this effect is translatable between different payload classes.

Thus, the oligosaccharide tag increases the solubility of ADCs, irrespective of the specific antibody component, payload and DAR.

### Example 5: Measurement of Melting Point

In this example, the melting point of different ADCs including the linker-payload construct Compound 15 (which includes the oligosaccharide CO-V) or a corresponding linker-payload construct without the oligosaccharide tag was measured.

Specifically, the unfolding/denaturation temperatures were measured using the Prometheus NT.Plex from NanoTemper and the intrinsic fluorescence of the proteins. Results of these measurements are shown in Table 5.

**Table 5: Melting points of ADCs**

| **ADC** | **Linker payload** | **Payload** | **Oligosaccharide tag** | **DAR** | **Melting point [°C]** |
|---|---|---|---|---|---|
| ADC12 | Compound 15 | Auristatin | CO-V | 7.76 | 63.5 |
| ADC13¹ | Compound 15 | Auristatin | CO-V | 8 | 63.6 |
| ADC14 | Compound 15 | Auristatin | CO-V | 4.5 | 65.6 |
| ADC15 | Compound 1² | Auristatin | --- | 4 | 51.6 |
| ADC17³ | Compound 15 | Auristatin | CO-V | 4.6 | 65.5 |

| | | | | | |
|---|---|---|---|---|---|
| ¹ ADC12 and ADC13 are ADCs prepared from the same linker-payload construct and antibody and with similar DAR value. ² corresponds to Compound 15 without oligosaccharide tag ³ ADC14 and ADC17 are ADCs prepared from the same linker-payload construct and antibody and with similar DAR value. | | | | | |

These results show that, irrespective of the DAR value, the presence of the oligosaccharide tag results in a significantly higher melting point. This higher melting point indicates that the oligosaccharide tags as described in the present disclosure have a surprising and not envisioned stabilizing effect on ADCs and provides the oligosaccharide-tagged ADCs according to the present disclosure with advantages regarding storage, stability and manufacturability, compared to corresponding ADCs without such a tag. Moreover, the comparison of ADC12 with ADC13 and of ADC17 with ADC14 proves the consistency between batches and the reproducibility of the measured effect.

### Example 6: Stability in Serum

In this example, the stability of the prepared ADCs in human serum was determined.

The respective ADC was added into either mouse serum or human serum (stabilized at pH 7.4) to a concentration of 50 µg ADC per ml of serum and incubated at 37 °C/5% CO₂. After 72 h and 96 h, samples were taken (3 samples per time point) and analyzed by LC-MS for the amount of remaining ADC with still intact linker sequence and degradation products.

Results for the ADCs ADC10 and ADC14 are summarized in Table 6.

**Table 6: Serum stability of different ADCs**

| | **ADC10** | | **ADC14** | |
|---|---|---|---|---|
| | Remaining at 72 h | Remaining at 96 h | Remaining at 72 h | Remaining at 96 h |
| **Mouse serum** | 91.1±13.1 % | 91.6±9.0 % | 100 % | 100 % |
| **Human serum** | 71.4±1.2 % | 65.7±5.6 % | 100 % | 100 % |

ADC10 is an ADC with a disulfide cleavage linker. It has been reported that disulfide linkers in ADCs are unstable in both human and mouse serum and degraded with a half life of ∼80 h (Kellogg et al., Bioconjugate Chem. (2011), vol. 22(4), p. 717-727). However, the serum stability data obtained with ADC10 shows that in the presence of the oligosaccharide tag according to the present disclosure, the linker is stabilized in both mouse and human serum.

ADC14 is an ADC with a linker including a cleavage site for cleavage by the intracellular protease Cathepsin B. It has been reported that ADCs with this type of linker are highly unstable in mouse serum due to an unspecific clearance by the mouse enzyme carboxyesterase 1C which is known to unspecifically cleave these linkers (see Dorywalska et al., Mol Cancer Ther (2016), vol. 15(5), p. 958-970 and Ubink et al., Mol Cancer Ther, vol. 17(11), p. 2389-2398). This effect makes it challenging to use the mouse model system to develop or characterize novel payloads with this linker system. The serum stability data obtained with ADC14 shows that in the presence of the oligosaccharide tag according to the present disclosure, the linker is stabilized in both mouse and human serum.

ADC14 was also compared in animal studies to ADC15 which has a very similar DAR and is based on a linker-payload construct that is identical to that of ADC14 with the only difference that it lacks the oligosaccharide tag. Here ADC14 showed tumor reduction, whereas ADC15 the same linker without the tag only delivered a delayed tumor growth. This effect might be a result of an improved stability against protease cleavage in serum and not only driven by a solubility effect.

This experiment suggests that oligosaccharide tags as described in the present disclosure confer higher serum stability. This, in turn, indicates improved pharmacokinetics and thus superior *in vivo* efficacy of ADCs carrying the oligosaccharide tag according to the present invention, compared to corresponding ADCs without such a tag.

### Example 7: Toxicity Assessment in Cell Culture

In this example, the toxicity of the antibody-drug conjugates prepared in Example 3 was assessed in cultured cells.

The experiment was carried out with the antibody trastuzumab/trastuzumab-t against Her2 or antibodies based on the described antigen A which is directed against a tumor antigen that is observed *i.a.* in CRC (colorectal cancer) and gastric cancer.

As positive cells, cells of tumor cell lines (CRC, breast cancer or lung cancer) expressing about 100,000-1,000,000 copies of the antigen at the cell surface of each cell were used. As negative cells, different tumor cell lines were used that did not express the respective antigen at their cell surface.

On day 0, 1000-5000 cells (depending on the cell line), were seeded into the wells of 384 well culture plates (= assay plates) in 40 µL/well RPMI 1640 or DMEM (Dulbecco's Modified Eagle's Medium) as seeding medium. Cells were incubated for 20-24 h at 37 °C at the appropriate CO₂ level (5% or 10%) and 95% rH (relative humidity). On day 1 the ADCs to be tested resp. controls were added to the cells (at a dose suitable for a proper dose response curve covering at least 4 log units in concentration), followed by incubation for 3 days for non-DNA targeting payloads or for 6 days for DNA damaging payloads at 37 °C, the appropriate CO₂ level and 95% rH. On day 4, the number of viable cells was determined using the CellTiter-Glo^{®} Luminescent Cell Viability Assay (Promega) according to the manufacturer's instructions. Specifically, plates were equilibrated at room temperature for 30 min, then 30 µl CellTiterGlo^{®} Reagent was added to each well. Plates were incubated for 2 minutes on a shaker and then kept for 10 min in the dark, followed by measurement on an Envision plate reader (PerkinElmer) at 560 to 590 nm.

The CellTiter-Glo^{®} Luminescent Cell Viability Assay results in cell lysis and generation of a luminescent signal proportional to the amount of ATP present which, in turn, measures the presence of metabolically active cells. Thus, the assay indicates proliferation and viability of the cultured cells and hence allows to determine the toxicity of the tested antibody-drug conjugates on the cells.

**Table 7: Results of Cytotoxicity Measurements in Cell Culture**

| **ADC** | **Antibody component** | **Linker Payload** | **Payload class** | **Positive cell line 1** | **Positive cell line 2** | **Negative cell line** |
|---|---|---|---|---|---|---|
| ADC1 | Trastuzumab-T | Compound 2 | Auri statin¹ | +++ | ++ | -- |
| ADC2 | Trastuzumab-T | Compound 3 | Duocarmycin | ++ | + | - |
| ADC5 | Anti-Tumor-AntigenB&C | Compound 4 | Auristatin | ++ | ND | - |
| ADC6 | Trastuzumab | Compound 4 | Auristatin | +++ | ++ | -- |
| ADC7 | Trastuzumab | Compound 7 | Duocarmycin | ++ | ++ | - |
| ADC9 | Trastuzumab-T | Compound 9 | Maytansinoid² | ++ | + | - |
| ADC10 | Trastuzumab | Compound 11 | Maytansinoid | ++ | ++ | - |
| ADC12 | Trastuzumab | Compound 15 | Auristatin | +++ | +++ | - |
| ADC14 | Trastuzumab | Compound 15 | Auristatin | +++ | +++ | - |
| ADC16 | Anti-TumorAntigenA | Compound 14 | Duocarmycin | +++ | ND | - |
| ADC18 | Trastuzumab | Compound 20 | Auristatin | +++ | ND | - |
| ADC21 | Anti-TumorAntigenA | Compound 19 | Duocarmycin | +++ | ND | - |
| ADC23 | Anti-TumorAntigenA | Compound 18 | Duocarmycin | +++ | ND | - |

| | | | | | | |
|---|---|---|---|---|---|---|
| IC₅₀ concentration of ADC: +++: < 0.1 nM/++: < 1 nM/+: single-digit nM/-: >10 nM/ --: no effect/ND: not determined ¹ The auristatin in the listed compounds was MMAE (monomethyl auristatin E). ³ The maytansinoid in the listed compounds was DM4 (= ravtansine). | | | | | | |

From the data in Table 7, it can be seen that the ADCs are highly toxic to cells expressing at their surface the target antigen of the ADC, but not toxic to cells that do not express this target antigen. This shows that all linker payload constructs, regardless of payload, conjugation method or position of the oligosaccharide tag, maintain full functionality in the presence of the oligosaccharide tag.

### Example 8: Anti-Tumoral Activity and Toxicity In Vivo

### Experimental Design and Treatment

This study was carried out with 210 healthy, 7-8 weeks old female BALB/c Nude (CByJ.Cg-F*oxn1^{nu}*/J) mice (Charles River, France).

On D0 (day 0), tumors were induced by subcutaneous (SC) injection of SK-OV-3 cells (a human ovarian adenocarcinoma cell line, purchased from Oncodesign) into the right flank of 198 female animals. SK-OV-3 tumor cell implantation was performed 48 hours after a whole-body irradiation with a gamma-source (2 Gy, ⁶⁰Co, BioMep, France).

On D31, animals were randomized by individual tumor volume when tumors reached a mean volume of 150 - 200 mm³. 120 animals out of 198 were randomized into 10 groups of 12 animals each.

Administration of the different ADCs or vehicle control was performed on the day of randomization (D31) as summarized in Table 8 below.

**Table 8: Treatment schedule**

| **Group** | **Number of animals** | **Test item** | **Dose (mg/kg/adm)** |
|---|---|---|---|
| 1 | 9+3* | Vehicle (PBS) | - |
| 2 | 9+3 | ADC12 | 0.5 |
| 3 | 9+3 | ADC12 | 2 |
| 4 | 9+3 | ADC12 | 6 |
| 5 | 9+3 | ADC14 | 0.5 |
| 6 | 9+3 | ADC14 | 2 |
| 7 | 9+3 | ADC14 | 6 |
| 8 | 9+3 | ADC15 | 0.5 |
| 9 | 9+3 | ADC15 | 2 |
| 10 | 9+3 | ADC15 | 6 |

| | | | |
|---|---|---|---|
| * 9 animals for analysis of anti-tumoral activity + 3 animals for toxicological analysis | | | |

Vehicle was PBS, pH 7.3-7.4. The ADCs or vehicle were administered by one intravenous (IV) injection into the caudal vein. The administration volume was 5 mL/kg adjusted to the most recent individual body weight of each mouse.

### Results

### Randomization

On the day of randomization (D31), tumors had a mean volume of 185-186 mm³. There were no statistically significant differences between all groups.

### Health parameters

In order to evaluate the impact of the treatment on the health of mice, body weight of animals was monitored throughout the experiment. The mean body weight change (MBWC) per group was calculated by comparing the mean body weight on D34 (the day with the first drop in body weight after treatment) to the mean body weight on the day of randomization (D31). For the period D31 to D34, all groups lost body weight, but these body weight losses were minor and there were no statistically significant differences between all groups.

### Toxicological analysis

Three satellite mice per group were sacrificed on D38. Tumors were collected for fixation in formalin (30 samples). A transverse section was made at the largest extension of the tumor for paraffin embedding. Tissues (lung, heart, liver, spleen, stomach, intestine and mesenteric lymph nodes) were collected and fixed in formalin. The carcasses with the skin were fixed after section of the thoracic and spinal cord and between the eyes.

H&E (Hematoxylin & Eosin) staining of the following tissues was carried out: heart, lung, liver, kidneys, spleen, stomach, intestines, mesenteric lymph nodes, brain, eyes, bone marrow, bone (femur and sternum). Moreover, skin from Groups 1, 4, 7 and 10 (control group and high dose groups) was examined by light microscopy.

Results are summarized in Table 9 below. No treatment-related findings were detected in the organs examined from mice of group 4,7 and 10. As no target organs were identified in the high dose group animals, the examination of the intermediate group mice was omitted. All remaining findings listed in Table 9 are incidental/spontaneous in nature.

**Table 9: Histopathological findings**

| **Tissue** | **Diagnosis** | **Group 1** | **Group 4** | **Group 7** | **Group 10** |
|---|---|---|---|---|---|
| | | PBS | 6 mg/kg ADC12 | 6 mg/kg ADC14 | 6 mg/kg ADC15 |
| | | N=3 | N=3 | N=3 | N=3 |
| | | Animal IDs | Animal IDs | Animal IDs | Animal IDs |
| Heart | NVL | all | all | all | all |
| | Lesion | | | | |
| | | PBS | 6 mg/kg ADC12 | 6 mg/kg ADC14 | 6 mg/kg ADC15 |
| | | N=3 | N=3 | N=3 | N=3 |
| | | Animal IDs | Animal IDs | Animal IDs | Animal IDs |
| Lung | NVL | all | all | all | all |
| | Lesion | | | | |
| Liver | NVL | all | 10, 12 | all | all |
| | Extramedullary hematopoiesis, increased, minimal | | 11 | | |
| Spleen | NVL | all | all | 19, 21 | all |
| | Congestion blood, acute | | | 20 | |
| Kidney | NVL | all | all | all | all |
| | Lesion | | | | |
| Ln mesenter. | NVL | all | all | 20, 21 | all |
| | Tissue missing | | | 19 | |
| Peyers Patches | NVL | all | all | all | all |
| | Lesion | | | | |
| Bone | NVL | all | all | all | all |
| | Sternum missing | 2, 3 | 11, 12 | 19, 20 | 30 |
| Bone marrow | NVL | all | all | all | all |
| | Lesion | | | | |
| Brain | NVL | all | all | all | all |
| | Lesion | | | | |
| Stomach | NVL | all | all | all | 29, 30 |
| | Tissue missing | | | | 28 |
| Duodenum | NVL | all | all | all | all |
| | Lesion | | | | |
| Jejunum | NVL | all | all | all | all |
| | Lesion | | | | |
| | | PBS | 6 mg/kg ADC12 | 6 mg/kg ADC14 | 6 mg/kg ADC15 |
| | | N=3 | N=3 | N=3 | N=3 |
| | | Animal IDs | Animal IDs | Animal IDs | Animal IDs |
| Ileum | NVL | 1,2 | all | all | all |
| | Autolysis | 3 | | | |
| Cecum | NVL | all | all | all | all |
| | Lesion | | | | |
| Colon | NVL | all | all | all | all |
| | Lesion | | | | |
| Rectum | NVL | all | all | all | all |
| | Lesion | | | | |
| Eye | NVL | all | all | all | all |
| | Mineralization, muscle fiber eye, unilateral, minimal | | 12 | | 29 |
| Nerve, optic | NVL | 1,2 | all | 19, 21 | all |
| | Tissue missing Tissue missing, unilateral | 3 | | 20 | - |
| | | 1 | | - | 29, 30 |
| Skin | NVL | all | all | all | all |
| | Lesion | | | | |

| | | | | | |
|---|---|---|---|---|---|
| NVL: no visible lesion | | | | | |

Direct comparison of toxicology and health parameters of the different ADCs tested in this example and vehicle control shows that the oligosaccharide tag according to the present disclosure is well-tolerated *in vivo* in the mouse model system, even in case of a high DAR of almost 8 (ADC12). This was unexpected, since there is precedence that upon metabolic processing in the body even molecular groups that as such are non-toxic may still have toxic effects on target organs like kidney or liver, as known for example for PEGs (Fruijtier-Pölloth, Toxicology (2005), vol. 214, p. 1-38).

### Anti-tumoral activity

The growth of subcutaneous SK-OV-3 tumors in BALB/c Nude mice was monitored throughout the experiment. The parameters of tumor doubling time, tumor growth delay and tumor growth inhibition were used in order to evaluate the anti-tumoral activity of the three test substances ADC12, ADC14 and ADC15 IV administered at 0.5, 2 and 6 mg/kg to mice bearing subcutaneous SK-OV-3 tumors.

### Tumor growth

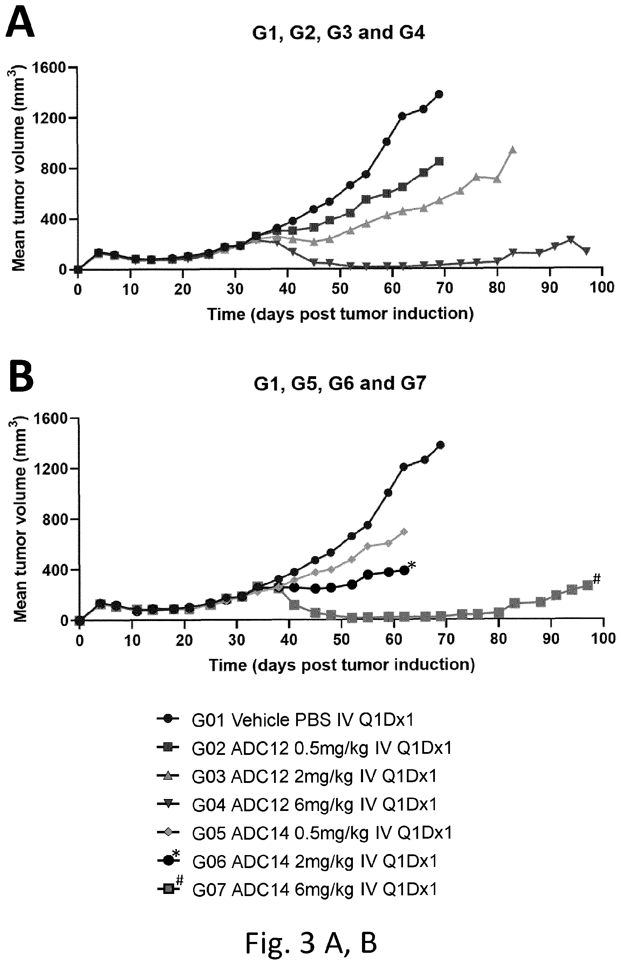

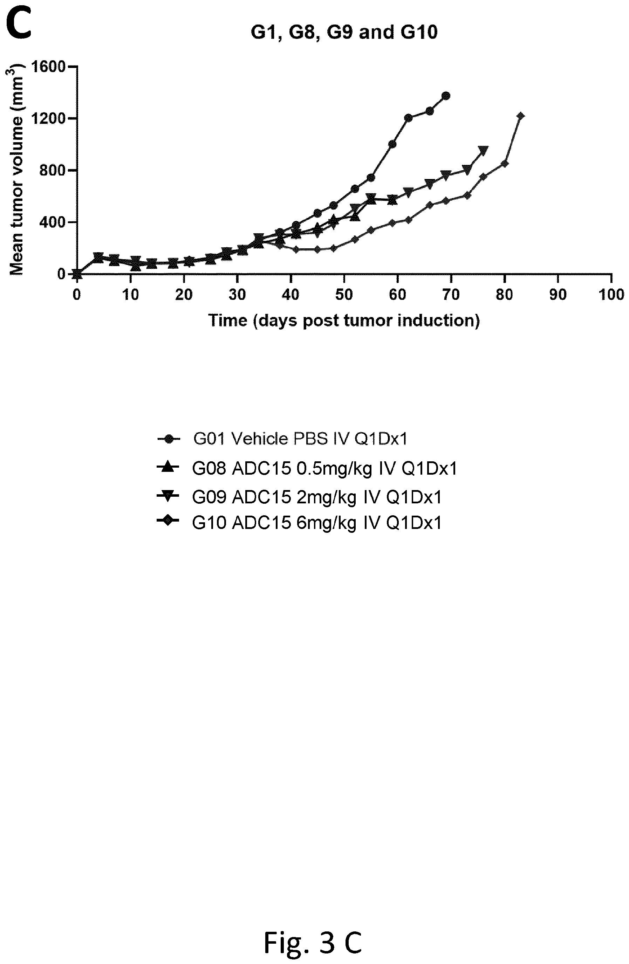

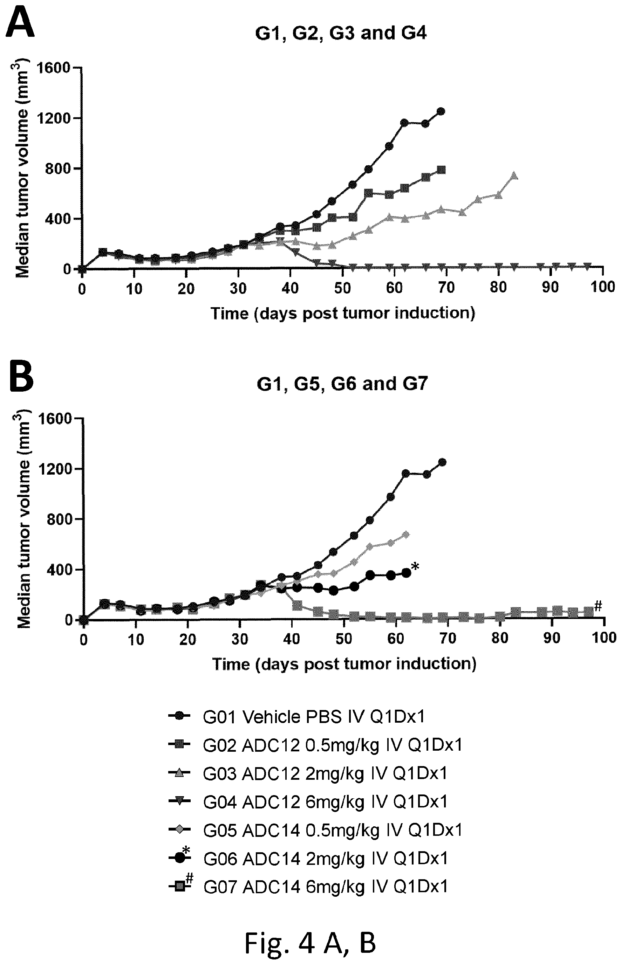

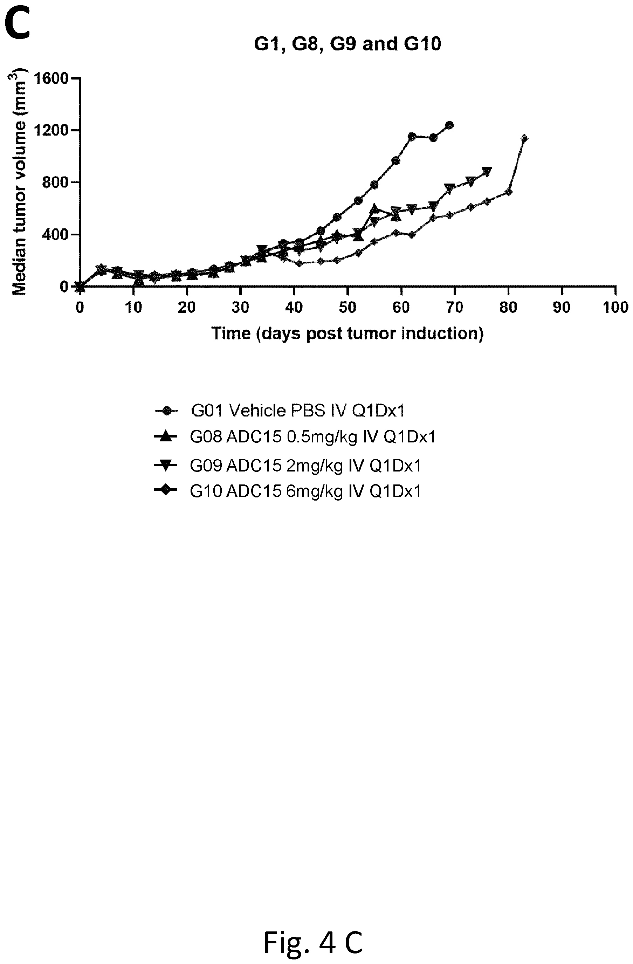

The growth of SK-OV-3 tumors on BALB/c Nude mice is presented in Figure 3 and Figure 4. Individual tumor volume measurement showed that vehicle-treated mice in Group 1 displayed exponentially growing tumors reaching 2000 mm³ in approximately 70 days.

For mice treated with ADC12 at 0.5 mg/kg (Group 2), 2 mg/kg (Group 3) and 6 mg/kg (Group 4), a dose-dependent anti-tumoral response was observed. In Group 4, six out of nine animals had tumors that completely regressed (TV (tumor volume) = 0 mm³) from D52-D59 to the end of the experiment. One animal displayed tumor that completely regressed (TV = 0 mm³) from D59 to D76 and then re-grew from D80 to the end of the experiment (TV = 308.71 mm³). Two animals in Group 4 had tumors that did not grow from the day of randomization (D31) to D80 and D69, respectively, and then re-grew exponentially until the end of the experiment.

For mice treated with ADC14 at 0.5 mg/kg (Group 5), 2 mg/kg (Group 6) and 6 mg/kg (Group 7), a dose-dependent anti-tumoral response was observed. In Group 7, four out of nine animals displayed tumors that completely regressed (TV = 0 mm³). In addition, two animals had tumors that regressed until the end of the experiment (respectively, TV = 134.52 and 48.57 mm³). These tumors were considered as regressed since their TVs at the end of the study (D97) were lower than those recorded (TV = 255.24 and 196.88 mm³, respectively) on the day of randomization (D31). Tumors of three out of nine animals in Group 7 regressed from D41 to D76 or D80 and then re-grew exponentially until the end of the study.

For mice treated with ADC15 at 0.5 mg/kg (Group 8), 2 mg/kg (Group 9) and 6 mg/kg (Group 10), a slight dose dependent anti-tumoral response was observed. In Groups 8 and 9, tumors grew in the same manner. However, the tumor volumes in Group 10 were lower than those in Groups 8 and 9 throughout the experiment.

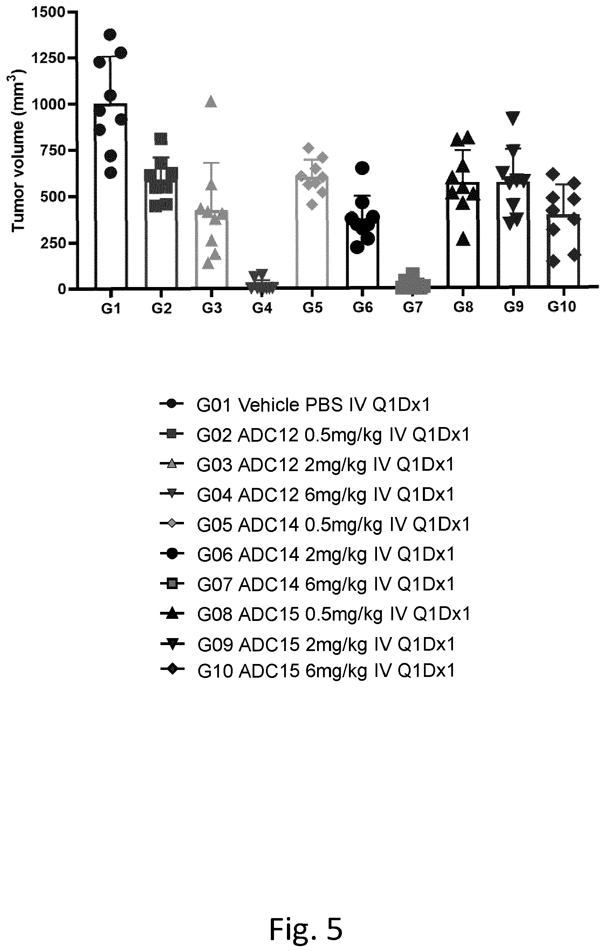

The analysis of tumor volumes was performed on D59, corresponding to the last day with at least 80% of animals alive in all groups (Figure 5). In Group 1, the mean TV was 1002 mm³, and was significantly higher compared with the mean TV in all the groups (p < 0.0001). Animals in Groups 2 and 3 displayed tumor volumes at 422.5 and 591.3 mm³, respectively. Despite a slight dose-dependent response decrease, no statistical difference was observed between Groups 2 and 3. However, tumors in Group 4 (TV = 14.98 mm³) were significantly smaller (p < 0.0001) than Groups 2 and 3. Animals in Groups 5, 6 and 7 displayed TV at 601, 374 mm³ and 18.91 mm³, respectively. A statistical difference was observed between Groups 5 and 7 and Groups 6 and 7 (p ≤ 0.0005) but not between Groups 5 and 6 (p = 0.16). Finally, tumor volumes in Groups 8, 9 and 10 (TV = 572.2, 571.1 and 393.7 mm³, respectively) were similar and there were no statistically significant differences between Groups 8, 9 and 10.

### Tumor doubling time

The efficacy of treatments was assessed by evaluating the tumor doubling time (DT) calculated from the day of randomization (D31) to the end of the experiment (D97). Tumor doubling times ranged from 15.17 days (Group 1) to 24.39 days (Group 6). Tumors in Groups 1, 2, 5, 8, 9 and 10 displayed tumor doubling times between 15.17 ≤ DT ≤ 21.90 days; there were no statistically significant differences between these groups. Tumors doubling times in Groups 3 (ADC12, 0.5 mg/kg) and 6 (ADC14, 0.5 mg/kg) (DT = 23.46 and 24.39 days, respectively) were significantly longer than those in the aforementioned groups. For Groups 4 and 7, tumor doubling time parameter could not be precisely calculated since no tumor grew exponentially throughout the experiment. In this case, tumor doubling times for Groups 4 and 7 were higher than 97 days.

### Tumor growth delay

The tumor growth delay was calculated by estimating the time for the SK-OV-3 tumors to reach a mean volume of 800 mm³. Tumor in the control Group 1 (n = 9) took 56 days to reach the target volume. Tumor growth delays in Groups 2 (n = 6), 3 (n = 8) and 4 (n = 1) were 69, 80 and 91 days, respectively. Tumors in Group 3 took 1.16 times longer than those in Group 2 to reach the target volume; this difference was statistically significant (p = 0.0006). Tumor growth delay in Group 4 should be interpreted with caution since it was calculated with one animal. Tumor growth delays in Groups 5 (n = 7), 6 (n = 6) and 7 (n = 1) were 72, 85 and 91 days, respectively. Similar to what was observed in Group 4, the tumor growth delay in Group 7 which was calculated with one animal, should be cautiously interpreted. In Groups 8 (n = 5), 9 (n = 7) and 10 (n = 8), tumors took 64, 71 and 79 days, respectively, and there were no significant differences between these groups.

### Tumor growth inhibition

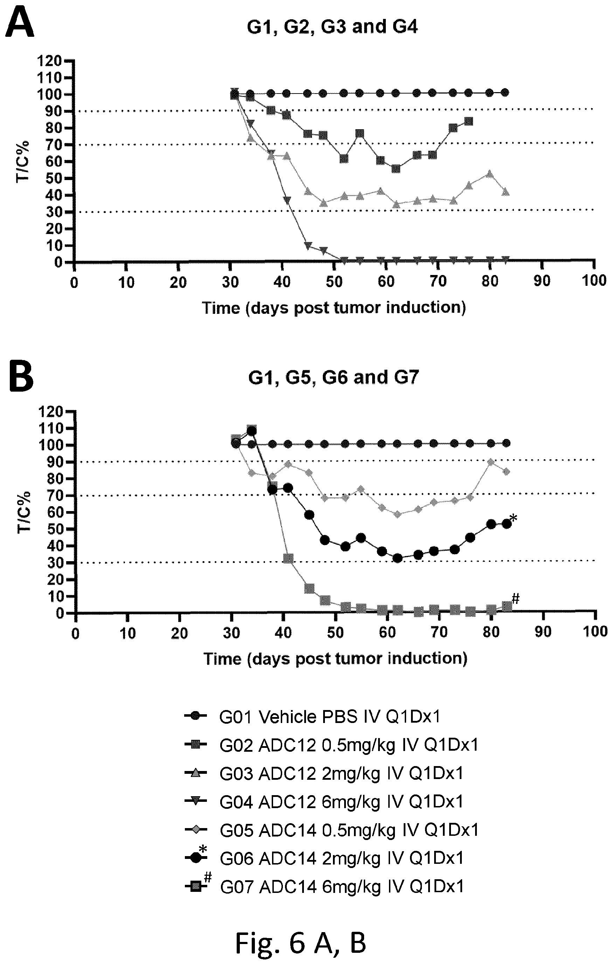

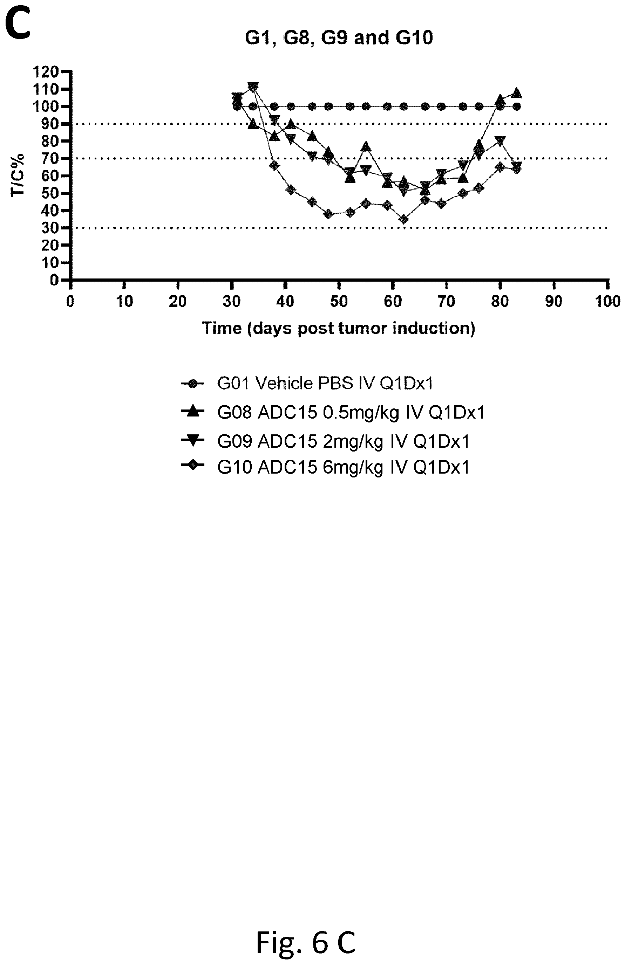

Tumor growth inhibition (T/C%) was calculated to evaluate the anti-tumoral activity of the treatments by comparing the median tumor volume of each treated group to the control Group 1 (Figure 6). For most of the study, low-dose treatments (Group 2, 5 and 8) resulted in marginal to moderate anti-tumoral activities with T/C% optimal values of 55, 58 and 52%, respectively. Furthermore, intermediate-dose treatment with ADC12 and ADC14 (Group 3 and 6) resulted in moderate anti-tumoral activity throughout the experiment, with T/C% optimal values of 34 and 32%, respectively. However, intermediate and high dose treatment with ADC15 (Groups 9 and 10) resulted in a moderate anti-tumoral activity (T/C% = 51% and 35% respectively) which was less efficient and later than those of Groups 3, 4, 6 and 7. Finally, the anti-tumoral activities of Group 4 and 7 treatments were marked for most of the experiment with T/C% optimal values of 0%.

### Conclusions

In conclusion, all treatments were well tolerated. All body weight losses were minor and there were no significant differences to the control Group 1.

A dose-dependent antitumoral effect on SK-OV3 cell-derived ovarian tumors was observed with treatment with ADCs ADC12 and ADC14, ranging from marginal, to moderate and marked, respectively, when increasing doses from 0.5 to 2 and 6 mg/kg. No statistical difference was observed when comparing treatments with ADC12 and ADC14 administered at the same dose of 0.5 mg/kg (Groups 2 and 5), 2 mg/kg (Groups 3 and 6) or 6 mg/kg (Groups 4 and 7).

In contrast, treatment with ADC15 (corresponding compound without an oligosaccharide tag according to the present disclosure) did not result in a dose dependent effect with increasing doses as observed for ADC12 and ADC14; efficacy parameters where not significantly different when ADC15 was administered at 0.5, 2 or 6 mg/kg (Groups 8, 9 and 10), ranging from marginal to moderate.

Efficacy parameters showed that treatments with ADC12 and ADC14 at lower doses (0.5 and 2 mg/kg) (Groups 2, 3, 5 and 6) were increased compared with ADC15 administered at the high dose of 6 mg/kg (Group 10).

Parameters of tumor doubling time, tumor growth delay and tumor growth inhibition showed that treatments with ADC12 and ADC14 IV administered at 6 mg/kg (Groups 4 and 7) resulted in a significantly marked anti-tumoral activity compared with the control vehicle (G1) and with the other groups.

### REFERENCES

Al-Lazikani et al., J. Molec. Biol. (1997), vol. 273, p. 927-948
Anderl et al., Methods in Molecular Biology (2013), vol. 1045, p. 51-70
Antibodies: A Laboratory Manual, 2nd edition (2014), editor Greenfield, Cold Spring Harbor Laboratory Press (USA)
Antibody-Drug Conjugates: Fundamentals, Drug Development, and Clinical Outcomes to Target Cancer, 1st edition (2016), editors Olivier and Hurvitz, publisher John Wiley & Sons, Inc. (U.S.)
Antibody Engineering - Methods and Protocols, 2nd edition (2010), editors Nevoltris and Chames, publisher Springer (Germany)
Bander, Methods Mol Biol (2013), vol. 1045, p. 29-40
Barfield and Rabuka, Methods in Molecular Biology (2018), vol. 1728, p. 3-16
Behrens et al., Molecular Pharmaceutics (2015), vol. 12(11), p. 3986-3998
Boerner et al., J. Immunol. (1991), vol. 147(1), p. 86-95
Bohe and Crich, in: Comprehensive Organic Synthesis, 2nd edition (2014), vol. 6, editors Knochel and Molander, Elsevier Ltd.
Bornstein, AAPS J. (2015), vol. 17(3), p. 525-534
Brennan et al., Science (1985), vol. 229, p. 81
Carbohydrate Chemistry (1988), editor El Khadem, Academic Press (San Diego)
Carbohydrate Chemistry: Proven Synthetic Methods (2015), editors Roy and Vidal, CRC Press
Carbohydrate Chemistry: State of the Art and Challenges for Drug Development (2016), editor Cipolla, Imperial College Press (London)
Chau, Lancet (2019), vol. 394 (10200), p. 793-804
Chen et al., ACS Chem Biol. (2017), vol. 12(4), p. 1038-1046
Coats et al., Clinical Cancer Research (2019), vol. 25(18), p. 5441-5448
Cole et al., in: Monoclonal Antibodies and Cancer Therapy (1985), editors Reisfeld and Sell, publisher Alan R. Liss Inc. (New York), p. 77-96
Corsaro et al., in: Microwaves in Organic Synthesis, 2nd edition (2006), publisher Wiley-VCH Verlag, p. 579-614
CRC Handbook of Oligosaccharides (1990), Vol. I-III, (published 2019), editors: Liptak et al., CHR Press, Inc.
Cytotoxic Payloads for Antibody-Drug Conjugates (Drug Discovery, Band 71), 1st edition (2019), editors Thurston and Jackson, Royal Society of Chemistry (U.K.)
Davis et al., Protein Engineering, Design & Selection (2010), vol. 23(4), p. 195-202
de Souza et al., Cancer Immun. (2012), vol. 12, p. 15
Dickgiesser et al., Bioconjugate Chem. (2020), vol. 31(4), p. 1070-1076
(https://dx.doi.org/10.1021/acs.bioconjchem.0c00061, published online March 5, 2020)
Dickgiesser et al., in: Methods in Molecular Biology: Enzyme-Mediated Ligation Methods (2019), editors Nuijens and Schmidt, vol. 2012, p. 135-149
Dickgiesser et al., Site-Specific Antibody-Drug Conjugation Using Microbial Transglutaminase, in: Enzyme-Mediated Ligation Methods. Methods in Molecular Biology (2019), p. 135-149
Dorywalska et al., Mol Cancer Ther (2016), vol. 15(5), p. 958-970
Dubowchik and Walker, Pharm. Therapeutics (1999), vol. 83, p. 67-123
Duerr and Friess, European Journal of Pharmaceutics and Biopharmaceutics (2019), vol. 139, p. 168-176
Edelman et al., Proc Natl Acad Sci USA (1969), vol. 63, p. 78-85
Fei et al., Journal of Biomedical Nanotechnology (2018), vol. 14(3), p. 405-429
Feuillatre et al., ACS Omega (2020), vol. 5(3), p. 1557-1565
Fruijtier-Pölloth, Toxicology (2005), vol. 214, p. 1-38
Garcia-Echeverria, Journal of Medicinal Chemistry (2014), vol. 57(19), p. 7888-7889
Gibbs and Wayt, Nanobodies, Scientific American Magazine (2005)
Gorka et al., Accounts of Chemical Research (2018), vol. 51(12), p. 3226-3235
Gromek et al., Current Topics in Medicinal Chemistry (2014), vol. 14(24), p. 2822-2834
Handbook of Therapeutic Antibodies (2014), editors Dübel and Reichert, publisher Wiley-VCH Verlag GmbH & Co. KGaA (Germany)
Harper, Methods in Molecular Biology (2013), vol. 1045, p. 41-49
Hermanson, Bioconjugate Techniques (1996), Academic Press (New York), p 234-242
Harris, Biochem. Soc. Transactions (1995), vol. 23, p. 1035-1038
Hollinger et al., Proc. Natl. Acad. Sci. USA (1993), vol. 90, p. 6444-6448
Hong et al., BMC Syst Biol. (2018), vol. 12 (Suppl 2), p. 17
Hong, Journal of Pharmacological and Toxicological Methods (2020), vol. 102, p. 106678
Hoogenboom and Winter, J. Mol. Biol. (1991), vol. 227, p. 381
Hurle and Gross, Curr. Op. Biotech. (1994), vol. 5, p. 428-433
Immunobiology, 5th Ed. (2001), editors Janeway et al., Garland Publishing (USA)
Johnson et al., Anticancer Res. (1995), vol. 15, p. 1387-1393
Jones et al., Nature (1986), vol. 321, p. 522-525
Kalonia et al., J. Phys. Chem. B (2016), vol. 120, p. 7062-7075
Karpov et al., ACS Medicinal Chemistry Letters (2019), vol. 10(12), p. 1674-1679
Kellogg et al., Bioconjugate Chem. (2011), vol. 22(4), p. 717-727
Köhler and Milstein, Eur. J. Immunol. (1976), vol. 5, p. 511-519
Koviach-Cote and Pirinelli, Chemical Reviews (2018), vol. 118(17), p. 7986-8004
Lau et al., Bioorg-Med-Chem. (1995), vol. 3(10), p. 1299-1304
Lau et al., Bioorg-Med-Chem. (1995), vol. 3(10), p. 1305-1312
Liaqat and Eltem, Carbohydrate Polymers (2018), vol. 184, p. 243-259
Lichtenthaler, in: Methods and Reagents for Green Chemistry (2007), editors Tundo et al., publisher John Wiley & Sons, p. 23-63
Li et al., Proc. Natl. Acad. Sci. USA (2006), vol. 103, p. 3557-3562
Macroni et al., Molecules (2019), vol. 24(21), p. 3948
Maderna et al., Molecular Pharmaceutics (2015), vol. 12(6), p. 1798-1812
Marks et al., J. Mol. Biol. (1991), vol. 222, p. 581
Matsuda et al., Organic Process Research & Development (2019), vol. 23(12), p. 2647-2654
Meyer et al., Biotechnological Production of Oligosaccharides - Applications in the Food Industry, Food Production and Industry (2015), Ayman Hafiz Amer Eissa, IntechOpen, DOI: 10.5772/60934, available from: https://www.intechopen.com/books/food-production-and-industry/biotechnological-production-of-oligosaccharides-applications-in-the-food-industry
Milstein et al., Nature (1983), vol. 305, p. 537-539
Morrison et al., Proc. Natl. Acad. Sci USA (1984), vol. 81, p. 6851-6855
Muda et al., Protein Engineering, Design & Selection (2011), vol. 24(5), p. 447-454
Neville et al., Biol. Chem. (1989), vol. 264, p. 14653-14661
Nicolaou et al., Accounts of Chemical Research (2019), vol. 52(1), p. 127-139
Nimoy, Pharmaceuticals (2018), vol. 11 (2), p. 32/1-32/22
Peplow, Nature Biotechnology (2019), vol. 37, p. 829-841
Pinelo et al., Separation and Purification Technology (2009), vol. 70(1), p. 1-11
Pluckthun, in: The Pharmacology of Monoclonal Antibodies, vol. 113 (1994), editors Rosenburg and Moore, Springer-Verlag (New York), p. 269-315
Preparative Carbohydrate Chemistry (1997), editor Hanessian, publisher Marcel Dekker, Inc. (New York)
Presta, Curr. Op. Struct. Biol. (1992), vol. 2, p. 593-596
Quiles et al., Journal of Medicinal Chemistry (2010), vol. 53(2), p. 586-594
Remington: The Science and Practice of Pharmacy, 22nd ed. (2012), Pharmaceutical Press
Riechmann et al., Nature (1988), vol. 332, p. 323-329
Rohrer, in: Process Scale Purification of Antibodies, 2nd edition (2017), editor: Gottschalk, John Wiley & Sons, Inc., p. 595-614
Rondon and Degoul, Bioconjugate Chemistry (2020), vol. 31(2), p. 159-173
Rosenberg, Ann. Rev. Med. (1996), vol. 47, p. 481-491
Rossin et al., Bioconjugate Chemistry (2016), vol. 27(7), p. 1697-1706
Rudra, Bioconjugate Chemistry (2020), vol. 31(3), p. 462-473
Saloranta and Leino, Synlett (2015), vol. 26(4), p. 421-425
Samain et al., Biotechnol. (1999), vol. 72, p. 33-47
Samain et al., Carbohydrate Research (1997), vol. 302, p. 35-42
Schmitz et al., Marine Drugs (2019), vol. 17(8), p. 452
Seeberger et al., Nature (2015), vol. 526(7572), p. 241-244
Sequences of Proteins of Immunological Interest, 5th ed. (1991), editors Kabat et al., National Institutes of Health (Bethesda, USA)
Simmons et al., Toxicology and Applied Pharmacology (2020), vol. 392, p. 114932
Singh et al., Therapeutic Antibodies: Methods and Protocols (2009), vol. 525, p 445-457
Solid Support Oligosaccharide Synthesis and Combinatorial Carbohydrate Libraries (2001), editor Seeberger, John Wiley & Sons, Inc.
Sonzini, Bioconjugate Chemistry (2020), vol. 31(1), p. 123-129
Stefano, Methods in Molecular Biology (2013), vol. 1045, p. 145-171
Tamburrini et al., Medicinal Research Reviews (2020), vol. 40(2), p. 495-531
The Organic Chemistry of Sugars, editors Levy and Fügedi (2005), CRC Press/Taylor & Francis (USA)
Thi et al., Polymers (2020), vol. 12(2), p. 298
Thorpe et al., Cancer Res. (1987), vol. 47, p. 5924-5931
Tiberghien et al., Journal of Organic Chemistry (2019), vol. 84(8), p. 4830-4836
Tiberghien, Organic Process Research & Development (2018), vol. 22(9), p. 1241-1256
Tietze et al., Angew. Chem. Int. Ed. (2010), vol. 49, p. 7336 -7339
Toader, Topics in Medicinal Chemistry (2018), vol. 28 (Cancer II), p. 289-332
Ubink et al., Mol Cancer Ther, vol. 17(11), p. 2389-2398
Vaklavas and Forero, Methods in Molecular Biology (2012), vol. 899, p. 489-497
van Dijk and van de Winkel, Curr. Opin. Pharmacol. (2001), vol. 5, p. 368-374
Vaswani and Hamilton, Ann. Allergy, Asthma & Immunol. (1998), vol. 1, p. 105-115
Vigneron et al., Cancer Immun. (2013), vol. 13, p. 15
Walker et al., Bioconjugate Chemistry (2019), vol. 30(9), p. 2452-2457
Watkinson, BioProcess International (2017), vol. 15(10), p. 22-33
Wawrzynczak et al., in: Immunoconjugates: Antibody Conjugates in Radioimagery and Therapy of Cancer (1987), editor Vogel, Oxford U. Press
EP 0404097
US 2009/0304721
U.S. Patent 4,816,567
U.S. Patent 4,880,935
U.S. Patent 5,122,368
U.S. Patent 5,622,929
U.S. Patent 5,824,805
U.S. Patent 6,075,181
U.S. Patent 6,150,584
U.S. Patent 6,214,345
U.S. Patent 6,982,321
U.S. Patent 7,087,409
WO 93/11161
WO 01/038318
WO 03/097625
WO 2004/010957
WO 2016/087650

## Claims

1. A molecule comprising
(i) a targeting moiety
(ii) at least one payload wherein said at least one payload is a therapeutic agent or a detectable label,
(iii) a linker/linkers covalently linking said payload/payloads and said targeting moiety, and
(iv) at least one solubility tag,
wherein said solubility tag comprises at least 3 and up to 12 monosaccharide units.

2. The molecule according to claim 1, wherein said solubility tag is/said solubility tags are linked by a covalent bond to said at least one payload and/or to said linker(s).

3. The molecule according to any of claims 1 or 2, wherein said monosaccharide units are independently selected from the group consisting of aldoses, ketoses and chemically modified forms of said aldoses or ketoses.

4. The molecule according to any of claims 1 to 3, wherein said monosaccharide units are individually selected from the group consisting of tetroses, pentoses, hexoses, and chemically modified forms of tetroses, pentoses and hexoses,
wherein said tetroses are individually selected from the group consisting of erythrose and threose,
said pentoses are individually selected from the group consisting of ribose, arabinose, xylose and lyxose, and
said hexoses are individually selected from the group consisting of allose, altrose, glucose, mannose, gulose, idose, galactose and talose.

5. The molecule according to any of claims 1 to 4, wherein the solubility tag comprises or consists of a chito-oligosaccharide.

6. The molecule according to claim 5, wherein said chito-oligosaccharide is a chito-oligosaccharide selected from the following table:
| **Number of monosaccharide units** | **Chito-oligosaccharide** |
|---|---|
| 3 | (GlcN)₃ / Chitotriose |
| 3 | (GlcN)₂-GlcNAc |
| 3 | (GlcNAc)₂-GlcN |
| 3 | GlcN-(GlcNAc)₂ |
| 3 | (GlcNAc)₃ / Tri-*N*-acetylchitotriose |
| 4 | (GlcN)₄ / Chitotetraose |
| 4 | (GlcN)₃-GlcNAc |
| 4 | (GlcNAc)₂-(GlcN)₂ |
| 4 | GlcNAc-GlcN-GlcNAc-GlcN |
| 4 | GlcNAc-(GlcN)₂-GlcNAc |
| 4 | GlcN-(GlcNAc)₂-GlcN |
| 4 | GlcN-GlcNAc-GlcN-GlcNAc |
| 4 | (GlcN)₂-(GlcNAc)₂ |
| 4 | (GlcNAc)₃-GlcN |
| 4 | (GlcNAc)₂-GlcN-GlcNAc |
| 4 | GlcNAc-GlcN-(GlcNAc)₂ |
| 4 | GlcN-(GlcNAc)₃ |
| 4 | (GlcNAc)₄ / Tetra-*N*-acetylchitotetraose |
| 5 | (GlcN)₅ / Chitopentaose |
| 5 | (GlcN)₃-(GlcNAc)₂ |
| 5 | (GlcNAc)₂-(GlcN)₂-GlcNAc |
| 5 | GlcNAc-GlcN-GlcNAc-GlcN-GlcNAc |
| 5 | GlcNAc-(GlcN)₂-(GlcNAc)₂ |
| 5 | GlcN-(GlcNAc)₂-GlcN-GlcNAc |
| 5 | GlcN-GlcNAc-GlcN-(GlcNAc)₂ |
| 5 | GlcN-(GlcNAc)₄ |
| 5 | (GlcNAc)₅ / Penta-*N*-acetylchitopentaose |
| 6 | (GlcN)₆ / Chitohexaose |
| 6 | (GlcN)₂-GlcNAc-(GlcN)₃ |
| 6 | GlcNAc-(GlcN)₄-GlcNAc |
| 6 | GlcN-GlcNAc-GlcN-GlcNAc-(GlcN)₂ |
| 6 | GlcNAc-(GlcN)₂-GlcNAc-GlcN-GlcNAc |
| 6 | GlcNAc-(GlcN)₃-(GlcNAc)₂ |
| 6 | GlcN-GlcNAc-GlcN-(GlcNAc)₃ |
| 6 | GlcNAc-(GlcN)₂-(GlcNAc)₃ |
| 6 | (GlcNAc)₆ / Hexa-*N*-acetylchitohexaose |
| 7 | (GlcN)₇ / Chitoheptaose |
| 7 | (GlcNAc)₇ / Hepta-*N*-acetylchitoheptaose |

7. The molecule according to any of claims 1 to 6, wherein said solubility tag comprises or is a chemical group with a structural formula selected from the following (I) to (IV):

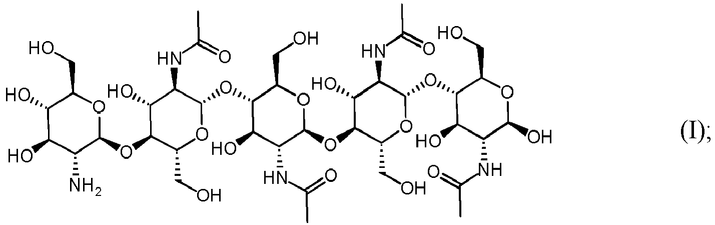

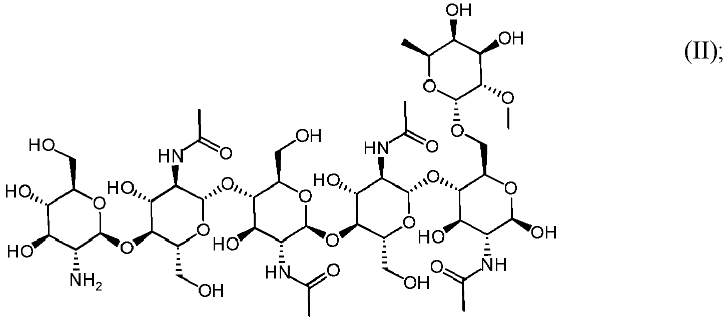

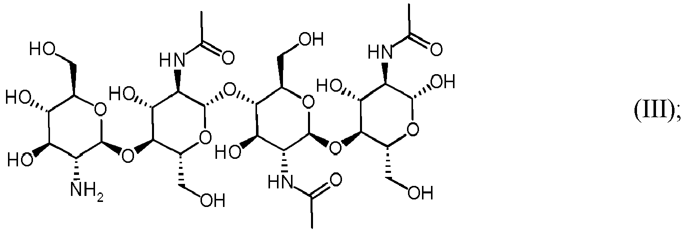

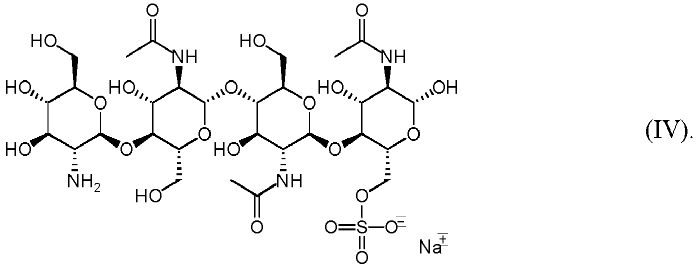

8. The molecule according to any of claims 1 to 7, wherein said targeting moiety is selected from the group consisting of a protein, a peptide, a peptide mimetic, a nucleic acid, an oligonucleotide and a small molecule.

9. The molecule according to any of claims 1 to 8, wherein said targeting moiety is an antibody against an antigen present on the surface of a target cell or an antigen-binding fragment of such an antibody.

10. The molecule according to any of claims 1 to 9, wherein said targeting moiety specifically is capable of binding to a tumor antigen.

11. The molecule according to any of claims 1 to 10, wherein said therapeutic agent is a cytotoxic agent, anti-inflammatory agent, immunostimulatory agent or immunosuppressive agent.

12. The molecule according to any of claims 1 to 11, wherein said linker/each of said linkers has a molecular weight of up to 1,500 Da.

13. A method for increasing the solubility of a chemical compound, said chemical compound comprising
(i) a targeting moiety,
(ii) at least one payload wherein said at least one payload is a therapeutic agent or a detectable label, and
(iii) a linker/linkers covalently linking said payload/payloads and said targeting moiety,
wherein said method comprises the preparation of a molecule in which said chemical compound is covalently linked to at least one solubility tag, wherein said solubility tag comprises at least 3 and up to 12 monosaccharide units.

14. A pharmaceutical composition comprising the molecule of any of claims 1 to 12, wherein said pharmaceutical composition further comprises a pharmaceutically acceptable carrier, diluent and/or excipient.

15. A molecule according to any of claims 1 to 12 or a pharmaceutical composition according to claim 14 for use as a medicament.

16. A compound for use in the preparation of a molecule according to any of claims 1 to 12, wherein said compound comprises a solubility tag as defined in any of claims 1 to 7 linked to an activator group.

17. The molecule according to any of claims 1 to 12, wherein said molecule comprises an antibody-drug conjugate.

18. The molecule according to any of claims 1 to 12, wherein said molecule comprises a peptide-drug conjugate.
